(19) Europäisches Patentamt
European Patent Office
Office européen des brevets

(11) EP 0 706 083 A1

(12) EUROPÄISCHE PATENTANMELDUNG

(43) Veröffentlichungstag:
**10.04.1996 Patentblatt 1996/15**

(51) Int Cl.[6]: **G03C 1/815**

(21) Anmeldenummer: **95810602.3**

(22) Anmeldetag: **27.09.1995**

(84) Benannte Vertragsstaaten:
**DE FR GB IT NL**

(30) Priorität: **04.10.1994 CH 2988/94**
**10.10.1994 CH 3039/94**
**08.02.1995 CH 364/95**
**08.02.1995 CH 365/95**

(71) Anmelder: **CIBA-GEIGY AG**
**CH-4002 Basel (CH)**

(72) Erfinder:
- **Toan, Vien Van, Dr.**
  **CH-1745 Lentigny (CH)**
- **Leppard, David George, Dr.**
  **CH-1723 Marly (CH)**
- **Rytz, Gerhard, Dr.**
  **CH-3007 Bern (CH)**
- **Würms, Norbert**
  **CH-1717 St. Ursen (CH)**
- **Hayoz, Pascal, Dr.**
  **CH-1752 Villars-sur-Glâne (CH)**

(54) **Fotografisches Aufzeichnungsmaterial enthaltend einen UV-Absorber**

(57) Es wird ein fotografisches Aufzeichnungsmaterial beschrieben, welches auf einem Träger mindestens eine Silberhalogenidemulsionsschicht sowie gegebenenfalls mindestens eine Zwischenschicht und/oder eine Protektionsschicht enthält und worin mindestens eine der genannten Schichten einen UV-Absorber vom Typ der 2-Hydroxyphenyltriazine enthält, wie in Anspruch 1 näher erläutert; dieser UV-Absorber kann auch als Homopolymer oder Copolymer vorliegen.

EP 0 706 083 A1

## Beschreibung

Die vorliegende Erfindung betrifft ein neues fotografisches Aufzeichnungsmaterial, das einen UV-Absorber vom 2-Hydroxyphenyltriazinyltyp enthält.

Typischerweise basiert fotografisches Aufzeichnungsmaterial auf Siberhalogenidemulsionen, wobei Silberhalogenid und bei farbfotografischem Material auch die Farbstoffe oder Farbstoffvorläufer für UV-Strahlung empfindlich sind. Besonders durch UV-Strahlung der Wellenlängen zwischen 300 und 400 nm verändert sich das Material beziehungsweise verfärbt sich oder bleicht aus. Diese Empfindlichkeit gegenüber UV-Strahlung ist unerwünscht. Die genannten Effekte können ganz oder teilweise unterdrückt werden, indem man den Farbstoffen Cyan, Magenta und Gelb sowie den Kupplern Stabilisatoren zusetzt, typischerweise UV-Absorber, deren Absorptionsmaximum zwischen 300 und 400 nm liegt; bekannte Beispiele dafür sind Verbindungen aus der Klasse der 2-Hydroxyphenyl-benztriazole.

Die Verwendung der bisher bekannten UV-Absorber (UVA) führt jedoch häufig zu unerwünschten Effekten wie beispielsweise Verfärbung und/oder Fleckenbildung als Folge ungenügender Eigenstabilität gegenüber Licht, Wärme oder Feuchtigkeit. Weiterhin kann als Folge des hochsiedenden organischen Lösungsmittels, welches zur Herstellung der UVA-Emulsion verwendet wird, eine Erweichung der Schicht und verschlechterte Adhäsion zwischen den diversen Schichten eintreten. Eine Kompensation dieses Effektes durch Erhöhung des Gelatineanteils führt in der Regel nur zu einer Destabilisierung der Schicht, während eine zusätzliche Gelatine-Schutzschicht über den UVA-enthaltenden Schichten eine unerwünschte Zunahme der Gesamtschichtdicke bewirkt. Andere Nachteile konventioneller UVA-Systeme können sein: Migration, Oberflächenkristallisation oder Blooming, Zusammenklumpen und Lichtstreuung an übermäßig großen Öltröpfchen, die den UVA enthalten und gemäß bekannten Emulgiermethoden hergestellt werden.

Es ist bekannt, daß Polymer-Latices, hergestellt durch Polymerisation von bestimmten UVA-Monomeren, die oben erwähnten Probleme teilweise lösen können, wie das z.B. in EP-A-577122 für polymere 2-Hydroxyphenyl-benztriazole diskutiert wird.

Auch die Verwendung einiger UV-Absorber vom Typ 2-Hydroxyphenyltriazin in Fotomaterial wurde bereits vorgeschlagen (EP-A-530 135, US-A-5 364 749, US-A-5 300 414). Weitere Verbindungen dieses Typs sind beispielsweise in EP-A-434 608 und US-A-5 189 084 beschrieben.

Es wurde nun eine spezielle Gruppe von 2-Hydroxyphenyltriazin-UV-Absorbern gefunden, deren Verwendung zur Stabilisierung von fotografischem Aufzeichnungsmaterial sich überraschenderweise weitgehend frei von den genannten Nachteilen erweist, die über eine verbesserte Eigenlichtstabilität verfügen, und die darüberhinaus geeignet sind, die Stabilität der Cyan-, Magenta- und Gelbschicht fotografischer Materialien zu erhöhen. Die erfindungsgemäßen fotografischen Materialien bieten gegenüber bekannten, unter Verwendung von Hydroxyphenyltriazin UV-Absorbern stabilisierten Materialien (z.B. US-A-5 364 749) beispielsweise den Vorteil geringerer Gelbfärbung ohne Beeinträchtigung der Lichtschutzwirkung.

Die erfindungsgemäßen UV-Absorber können für alle Arten silberhalogenidhaltigen Aufzeichnungsmaterials verwendet werden. Beispielsweise können sie für Farbpapier, Farbumkehrpapier, Direkt-Positiv-Farbmaterial, Farbnegativfilm, Farbpositivfilm, Farbumkehrfilm und weitere eingesetzt werden. Unter anderem werden sie bevorzugt für fotosensitives Farbmaterial, welches ein Umkehrsubstrat enthält oder welches Positive bildet, verwendet.

Ferner können diese Triazine mit Vorteil mit UV-Absorbern vom Hydroxyphenylbenzotriazoltyp, insbesondere bei Raumtemperatur flüssigen Vertretern hiervon (vgl. beispielsweise US-A-4,853,471, US-A-4,973,702, US-A-4,921,966 und US-A-4,973,701) kombiniert werden.

Auch Kombinationen der Hydroxyphenyltriazine mit anderen Typen von UV-Absorbern wie Benzophenonen, Oxaniliden, Cyanoacrylaten, Salicylsäureestern, Acrylnitrilen oder Thiazolinen eignen sich zur Verwendung in fotografischen Aufzeichnungsmaterialien.

Gegenstand vorliegender Anmeldung ist somit fotografisches Aufzeichnungsmaterial enthaltend auf einem Träger mindestens eine Silberhalogenidemulsionsschicht sowie gegebenenfalls mindestens eine Zwischenschicht und/oder eine Protektionsschicht, wobei mindestens eine der genannten Schichten einen UV-Absorber enthält, dadurch gekennzeichnet, daß der genannte UV-Absorber ein durch Additionspolymerisation eines Monomeren der Formel I erhaltenes Homopolymer, ein Copolymer mindestens zweier verschiedener Verbindungen der Formel I, **oder** ein Copolymer mindestens einer Verbindung der Formel I und mindestens einer weiteren ethylenisch ungesättigten Verbindung ist

(I) 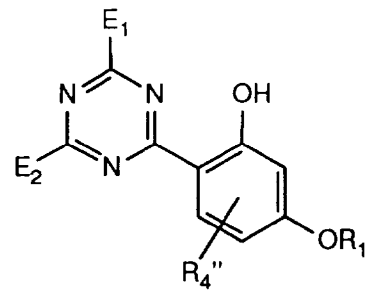 ,

worin die Reste

$E_1$ und $E_2$, unabhängig voneinander, eine Gruppe der Formel Ia oder Ib darstellen

(Ia) 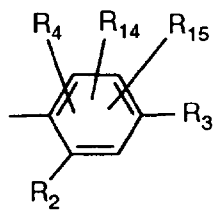

(Ib) 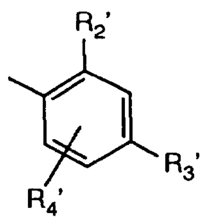 ;

$R_1$, unabhängig voneinander, -A, $-CH_2-CH(XA)-CH_2-O-R_7$, $-CR_8R'_8-(CH_2)_1-XA$, $-CH_2-CH(OA)-R_9$, $-CH_2-CH(OH)-CH_2-XA$,

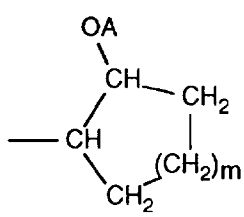 ,

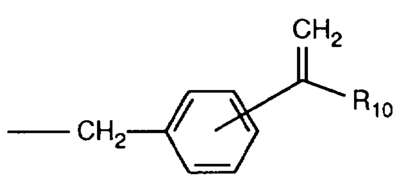 ,

$-CH_2-C(=CH_2)-R_{10}$, $-(CH_2)_p-SiR_{11}R_{11}'-CH=CH_2$, $-C(=O)-(CH_2)_q-CH=CH_2$, $-CHR_8-(CH_2)_r-C(=O)-O-CH_2-CH(OH)-CH_2-OA$, $-CR_8R'_8-(CH_2)_l-C(=O)-XA$ oder $-C(=O)-O-CH_2-C(=CH_2)-R_{10}$; wobei A $-C(=O)-CR_5=CH-R_6$ ist;

$R_2$, unabhängig voneinander, H, $C_1$-$C_{12}$-Alkyl, $C_5$-$C_{12}$-Cycloalkyl, $C_3$-$C_6$-Alkenyl, Halogen, Phenyl oder Trifluormethyl;

$R_2'$, unabhängig voneinander, $C_1$-$C_{18}$-Alkoxyl $C_3$-$C_{18}$-Alkenoxy, $-O-CO-R_{12}$, -OH oder -OA;

$R_3$ und $R_3'$, unabhängig voneinander, H, -OH, $-OR_1$, $-OR_{131}$, $C_1$-$C_{18}$-Alkyl, $C_3$-$C_{18}$-Alkenyl, $C_6$-$C_{12}$-Cycloalkyl, Halogen, Trifluormethyl, Phenyl, Phenyl-$C_1$-$C_4$-Alkyl, -CN, $C_1$-$C_{18}$-Alkyl-$S(=O)_t$- oder Phenyl-$S(=O)_t$-;

$R_4$, $R_4'$ und $R_4''$, unabhängig voneinander, H, $C_1$-$C_{18}$-Alkyl, $C_3$-$C_6$-Alkenyl, $-OR_{131}$, Halogen, Trifluormethyl, Phenyl, Phenyl-$C_1$-$C_4$-alkyl mit ein bis drei Resten $C_1$-$C_4$-Alkyl substituiertes Phenyl-$C_1$-$C_4$-alkyl -CN, $C_1$-$C_{18}$-Alkyl-$S(=O)_t$- oder Phenyl-$S(=O)_t$-;

$R_5$ H, $-CH_2-COOR_{13}$, $C_1$-$C_4$-Alkyl oder -CN;

$R_6$ H, $-COOR_{13}$, $C_1$-$C_{17}$-Alkyl oder Phenyl;

$R_7$ $C_1$-$C_{18}$-Alkyl; $C_5$-$C_{12}$-Cycloalkyl; $C_3$-$C_{18}$-Alkenyl; Phenyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; durch ein oder mehrere -O- unterbrochenes $C_3$-$C_{50}$-Alkyl; 1-Adamantyl; 2-Adamantyl; Norbornyl; Norbornan-2-methyl; $-C(=O)-R_{12}$; -A;

$R_8$ und $R_8'$, unabhängig voneinander, H, $C_1$-$C_{18}$-Alkyl; Phenyl; Phenyl-$C_1$-$C_4$-alkyl; oder mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl;

$R_9$ $C_1$-$C_{18}$-Alkyl, Phenyl oder Phenyl-$C_1$-$C_4$-alkyl;

$R_{10}$ H oder $-CH_3$;

$R_{11}$ und $R_{11}'$, unabhängig voneinander, $C_1$-$C_4$-Alkyl oder Phenyl oder mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl;

$R_{12}$ H, $C_1$-$C_{18}$-Alkyl, Phenyl, Phenyl-$C_1$-$C_4$-alkyl, $C_5$-$C_{12}$-Cycloalkyl, $C_1$-$C_{12}$-Alkoxy, Phenoxy, Norbornan-2-yl, 5-Norbornen-2-yl, 1-Adamantyl;

$R_{13}$ $C_1$-$C_{18}$-Alkyl, $C_3$-$C_{18}$-Alkenyl, Phenyl, $C_5$-$C_{12}$-Cycloalkyl, durch ein oder mehrere -O- unterbrochenes $C_3$-$C_{50}$-Alkyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; 2-Adamantyl; Norbornyl, Norbornan-2-methyl;

$R_{14}$ und $R_{15}$, unabhängig voneinander, H, $C_1$-$C_{18}$-ASkyl, $C_3$-$C_{18}$-Alkenyl, $C_6$-$C_{12}$-Cycloalkyl, Halogen, Trifluormethyl, Phenyl, Phenyl-$C_1$-$C_4$-alkyl, -CN, $C_1$-$C_{18}$-Alkyl-$(S{=}O)_t$-, Phenyl-$(S{=}O)_t$- oder $-OR_{131}$;

$R_{131}$ $C_1$-$C_{18}$-Alkyl; $C_1$-$C_{18}$-Alkyl, welches substituiert ist durch -OH, $C_1$-$C_{18}$-Alkoxy, $C_5$-$C_{12}$-Cycloalkoxy, $C_3$-$C_6$-Alkenyloxy, Halogen, $-COOR_{13}$, $-CONH_2$, $-COHNR_{132}$, $-CON(R_{132})(R_{133})$, $-NHCOR_{12}$, CN, $-OCOR_{12}$, Phenoxy, und/oder durch $C_1$-$C_{18}$-Alkyl, $C_1$-$C_{18}$-Alkoxy oder Halogen substituiertes Phenoxy; $C_3$-$C_{18}$-Alkenyl; $C_6$-$C_{12}$-Cycloalkyl; durch $C_1$-$C_4$-Alkyl und/oder $-OCOR_{12}$ substituiertes $C_6$-$C_{12}$-Cycloalkyl; durch ein oder mehrere -O- unterbrochenes $C_3$-$C_{50}$-Alkyl; durch ein oder mehrere -O- unterbrochenes $C_3$-$C_{50}$-Alkyl, welches durch -OA oder $-O-CO-R_{12}$ substituiert ist; Phenyl; Phenyl-$C_1$-$C_4$-alkyl; $-COR_{12}$; $-SO_2R_{12}$;

$R_{132}$ und $R_{133}$, unabhängig voneinander, $C_1$-$C_{12}$-Alkyl, $C_3$-$C_{12}$-Alkoxyalkyl, $C_4$-$C_{16}$-Dialkylaminoalkyl, $C_5$-$C_{12}$-Cycloalkyl; oder

$R_{132}$ und $R_{133}$ gemeinsam $C_3$-$C_9$-Alkylen, $C_3$-$C_9$-Oxaalkylen oder -Azaalkylen; X $-NR_8-$, -O-, $-NH-(C_nH_{2n})-NH-$ oder $-O-(C_kH_{2k})-NH-$;

k eine Zahl 2-4;

l eine Zahl 0-19;

m eine Zahl 2-8;

n eine Zahl 0-4;

p eine Zahl 0-10;

q eine Zahl 1-8;

r eine Zahl 0-18; und

t eine Zahl 0, 1 oder 2 bedeuten.

Enthalten Monomereneinheiten der Formel I mehrere gleich benannte Reste, so können diese im Rahmen der angegebenen Bedeutungen gleich oder verschieden sein.

Ein weiterer Gegenstand der Anmeldung ist die Verwendung der obigen Verbindungen zum Stabilisieren von fotografischem Aufzeichnungsmaterial und das Verfahren zum Stabilisieren von fotografischem Aufzeichnungsmaterial, indem man obige Verbindungen in das Material einarbeitet.

Bedeuten Substituenten in Verbindungen der Formel(I) Alkyl mit bis zu 18 Kohlenstoffatomen, so kommen hierfür Reste wie Methyl, Ethyl, Propyl, Butyl, Pentyl, Hexyl, Heptyl, Octyl, Nonyl, Decyl, Undecyl, Dodecyl, Tetradecyl, Hexadecyl oder Octadecyl sowie entsprechende verzweigte Isomere in Frage.

Bedeuten Substituenten in Verbindungen der Formel(I) Alkoxy mit bis zu 18 Kohlenstoffatomen, so kommen hierfür Reste wie Methoxy oder Ethoxy sowie den obigen Alkylresten analoge Reste in Frage.

Bedeuten Substituenten in Verbindungen der Formel(I) Alkenyl mit bis zu 18 Kohlenstoffatomen, so kommen hierfür Reste wie Vinyl, Prop-1-enyl ($-CH=CH-CH_3$) oder Prop-2-enyl ($-CH_2-CH=CH_2$) sowie den obigen Alkylresten analoge Reste in Frage.

Bedeuten Substituenten in Verbindungen der Formel(I) Alkenoxy mit bis zu 18 Kohlenstoffatomen, so kommen hierfür Reste wie Ethenoxy, Prop-1-enoxy ($-O-CH=CH-CH_3$) oder Prop-2-enoxy ($-O-CH_2-CH=CH_2$) sowie den obigen Alkylresten analoge Reste in Frage.

Bedeuten Substituenten in Verbindungen der Formel(I) Phenyl-$C_1$-$C_4$-Alkyl bzw. mit ein bis drei Resten $C_1$-$C_4$-Alkyl substituiertes Phenyl-$C_1$-$C_4$-alkyl, so kommen hierfür Reste wie

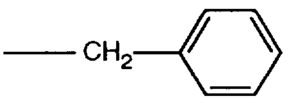

,

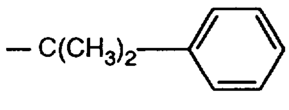

,

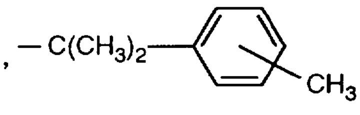

oder

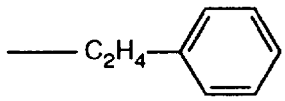

in Frage.

Bedeuten Substituenten in Verbindungen der Formel(I) Phenyl-$C_1$-$C_4$-Alkoxy, so kommen hierfür Reste wie

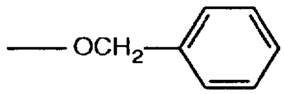 oder 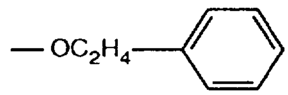

in Frage.

Bedeuten Substituenten in Verbindungen der Formel(I) Halogen, so kommt hierfür Fluor, Chlor, Brom oder Jod in Frage.

Bevorzugt bedeutet $R_1$ -A, -$CH_2$-CH(XA)-$CH_2$-O-$R_7$, -$CR_8R'_8$-$(CH_2)_l$-XA, -$CH_2$-CH(OA)-$R_9$, -$CH_2$-CH(OH)-$CH_2$-XA,

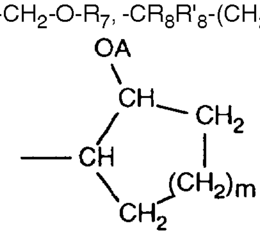 ,

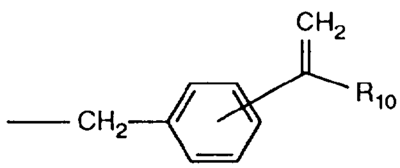 ,

-$CHR_8$-$(CH_2)_r$-C(=O)-O-$CH_2$-CH(OH)-$CH_2$-OA; besonders bevorzugt -A, -$CH_2$-CH(OA)-$CH_2$-O-$R_7$, -$CHR_8$-$(CH_2)_l$-OA, -$CH_2$-CH(OA)-$R_9$, -$CH_2$-CH(OH)-$CH_2$-OA,

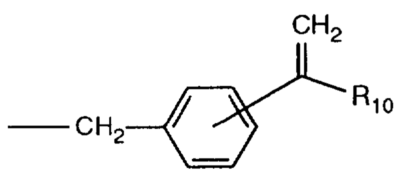

oder -$CHR_8$-$(CH_2)_r$-C(=O)-O-$CH_2$-CH(OH)-$CH_2$-OA; und ganz besonders bevorzugt -A, -$CH_2$-CH(OA)-$CH_2$-O-$R_7$, -$CHR_8$-$(CH_2)_l$-OA, -$CH_2$-CH(OA)-$R_9$ oder

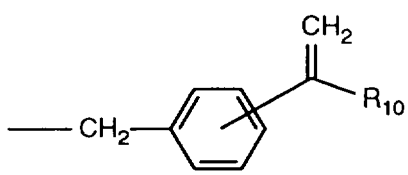 .

Bevorzugt bedeutet $R_2$ H, $C_1$-$C_4$-Alkyl, $C_3$-Alkenyl, F, Cl oder Phenyl; besonders bevorzugt H, -$CH_3$ oder Cl; und ganz besonders bevorzugt H oder -$CH_3$.

Bevorzugt bedeutet $R_2$' -OH, $C_1$-$C_4$-Alkoxy, $C_3$-Alkenoxy; besonders $C_1$-$C_2$-Alkoxy, oder -OH.

Bevorzugt bedeutet $R_3$ oder $R_3$' H, -OH, -$OR_1$, -$OR_{131}$, $C_1$-$C_4$-Alkyl, Cyclohexyl, $C_3$-Alkenyl, F, Cl, Trifluormethyl, Phenyl, Benzyl oder -CN; besonders bevorzugt H, -OH, -$OR_1$, -$CH_3$, $C_1$-$C_{12}$-Alkoxyl, durch $C_2$-$C_6$-Alkanoyloxy substituiertes $C_2$-$C_{18}$-Alkoxy, $C_3$-Alkenoxy, F, Cl, Phenyl, Benzyloxy oder -CN; und ganz besonders bevorzugt H, -$OR_1$, -$CH_3$, $C_1$-$C_{12}$-Alkoxy, Cl, Phenyl oder -CN.

Häufig bedeutet $R_3$' -OH, -$OR_1$ oder -$OR_{131}$, und $R_3$ umfaßt nicht die Bedeutung -$OR_1$.

Bevorzugt bedeutet $R_4$, $R_4$' oder $R_4$'' H, $C_1$-$C_4$-Alkyl, $C_3$-Alkenyl, $C_1$-$C_4$-Alkoxy, $C_3$-Alkenoxy, F, Cl, Trifluormethyl, Phenyl, Phenyl-$C_1$-$C_3$-alkyl oder -CN; besonders bevorzugt H, -$CH_3$, $C_3$-Alkenyl, -$OCH_3$, $C_3$-Alkenoxy, F, Cl, Phenyl-$C_3$-alkyl oder -CN; und ganz besonders bevorzugt H, -$OCH_3$ oder -$CH_3$.

Bevorzugt bedeutet $R_5$ H oder -$CH_3$.

Bevorzugt bedeutet $R_6$ H, -$COOR_{13}$, -$CH_3$ oder Phenyl; und besonders bevorzugt H oder -$CH_3$.

Bevorzugt bedeutet $R_7$ $C_1$-$C_8$-Alkyl, $C_5$-$C_8$-Cycloalkyl, $C_3$-$C_8$-Alkenyl, Phenyl, mit ein bis drei Resten $C_1$-$C_4$-Alkyl oder $C_1$-$C_4$-Alkoxy substituiertes Phenyl oder Benzyl;und besonders bevorzugt $C_1$-$C_8$-Alkyl, Cyclopentyl, Cyclohexyl, $C_3$-Alkenyl, Phenyl oder Benzyl.

Bevorzugt bedeuten $R_8$ und $R_8$', unabhängig voneinander, H oder $C_1$-$C_{18}$-Alkyl.

$R_{10}$ ist bevorzugt Wasserstoff.

$R_{11}$ und $R_{11}$' sind, unabhängig voneinander, vorzugsweise $C_1$-$C_4$-Alkyl oder Phenyl, vor allem Methyl.

X steht vorzugsweise für -O- oder -$NR_8$-, besonders für ein Sauerstoffatom.

Der Wert des Index 1 beträgt vorzugsweise 1-15.

Der **erfindungsgemäße UV Absorber kann auch ein Monomer sein. Gegenstand der Erfindung ist daher auch ein f**otografisches Aufzeichnungsmaterial enthaltend auf einem Träger mindestens eine Silberhalogenidemulsionsschicht sowie gegebenenfalls mindestens eine Zwischenschicht und/oder eine Protektionsschicht, wobei mindestens eine der genannten Schichten einen UV-Absorber enthält, dadurch gekennzeichnet, daß der genannte UV-Absorber eine Verbindung der Formel I ist

(I) 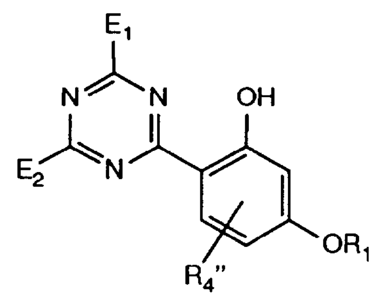 ,

worin die Reste

$E_1$ und $E_2$, unabhängig voneinander, jeweils eine Gruppe der Formel Ia oder Ib darstellen

(Ia) 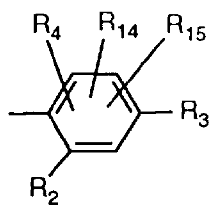

(Ib) 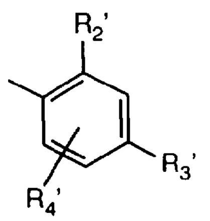 ;

$R_1$, unabhängig voneinander, -A,

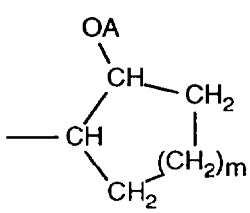 , 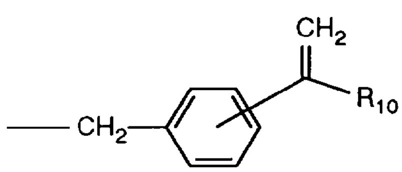 ,

-$CH_2$-C(=$CH_2$)-$R_{10}$, -$(CH_2)_p$-$SiR_{11}R_{11'}$-CH=$CH_2$, C(=O)-$(CH_2)_q$-CH=$CH_2$, -$CHR_8$-$(CH_2)_r$-C(=O)-O-$CH_2$-CH(OH)-$CH_2$-OA, -$CR_8R'_8$-$(CH_2)_l$-C(=O)-XA oder -C(=O)-O-$CH_2$-C(=$CH_2$)-$R_{10}$, **und, im Fall daß $E_1$ oder $E_1$ und $E_2$ eine Gruppe der Formel Ib sind, $R_1$ zusätzlich die Bedeutungen -$CH_2$-CH(XA)-$CH_2$-O-$R_7$, -$CR_8R'_8$-$(CH_2)_l$-XA, -$CH_2$-CH(OA)-$R_9$, -$CH_2$-CH(OH)-$CH_2$-XA umfaßt;** wobei A -C(=O)-$CR_5$=CH-$R_6$ ist;

$R_2$, unabhängig voneinander, H, $C_1$-$C_{12}$-Alkyl, $C_5$-$C_{12}$-Cycloalkyl, $C_3$-$C_6$-Alkenyl, Halogen, Phenyl oder Trifluormethyl;

$R_2$', unabhängig voneinander, $C_1$-$C_{18}$-Alkoxy, $C_3$-$C_{18}$-Alkenoxy, -O-CO-$R_{12}$, -OH oder -OA;

$R_3$ und $R_3$', unabhängig voneinander, H, -OH, -$OR_1$, -$OR_{131}$, $C_1$-$C_{18}$-Alkyl, $C_3$-$C_{18}$-Alkenyl, $C_6$-$C_{12}$-Cycloalkyl, Halo-

gen, Trifluormethyl, Phenyl, Phenyl-$C_1$-$C_4$-Alkyl, -CN, $C_1$-$C_{18}$-Alkyl-S(=O)$_t$- oder Phenyl-S(=O)$_t$-;

$R_4$, $R_4$' und $R_4$", unabhängig voneinander, H, $C_1$-$C_{18}$-Alkyl, $C_3$-$C_6$-Alkenyl, -$OR_{131}$, Halogen, Trifluormethyl, Phenyl, Phenyl-$C_1$-$C_4$-alkyl, mit ein bis drei Resten $C_1$-$C_4$-Alkyl substituiertes Phenyl-$C_1$-$C_4$-alkyl, -CN, $C_1$-$C_{18}$-Alkyl-S(=O)$_t$- oder Phenyl-S(=O)$_t$-;

$R_5$ H, -$CH_2$-$COOR_{13}$, $C_1$-$C_4$-Alkyl oder -CN;

$R_6$ H, -$COOR_{13}$, $C_1$-$C_{17}$-Alkyl oder Phenyl;

$R_7$ $C_1$-$C_{18}$-Alkyl; $C_5$-$C_{12}$-Cycloalkyl; $C_3$-$C_{18}$-Alkenyl; Phenyl; mit ein bis drei Resten $C_1$-$C_5$-Alkyl, $C_1$-$C_8$-Alkoxy, $C_3$-$C_5$-Alkenoxy, Halogen oder Trifluormethyl substituiertes Phenyl; Phenyl-$C_1$-$C_4$-alkyl; durch ein oder mehrere -O- unterbrochenes $C_3$-$C_{50}$-Alkyl; 1-Adamantyl; 2-Adamantyl; Norbornyl; Norbornan-2-methyl; -C(=O)-$R_{12}$; -A;

$R_8$ und $R_8$', unabhängig voneinander, H, $C_1$-$C_{18}$-Alkyl; Phenyl; Phenyl-$C_1$-$C_4$-alkyl; oder mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl;

$R_9$ $C_1$-$C_{18}$-Alkyl, Phenyl oder Phenyl-$C_1$-$C_4$-alkyl;

$R_{10}$ H oder -$CH_3$;

$R_{11}$ und $R_{11}$', unabhängig voneinander, $C_1$-$C_4$-Alkyl oder Phenyl oder mit ein bis drei Resten $C_1$-$C_8$-Alkyl- $C_1$-$C_8$-Alkoxyl, $C_3$-$C_8$-Alkenoxy, Halogen oder Trifluormethyl substituiertes Phenyl;

$R_{12}$ H, $C_1$-$C_{18}$-Alkyl, Phenyl, Phenyl-$C_1$-$C_4$-alkyl, $C_5$-$C_{12}$-Cycloalkyl- $C_1$-$C_{12}$-Alkoxy, Phenoxy, Norbornan-2-yl, 5-Norbornen-2-yl, 1-Adamantyl;

$R_{13}$ $C_1$-$C_{18}$-Alkyl, $C_3$-$C_{18}$-Alkenyl, Phenyl, $C_5$-$C_{12}$-Cycloalkyl, durch ein oder mehrere -O- unterbrochenes $C_3$-$C_{50}$-Alkyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; 2-Adamantyl; Norbornyl, Norbornan-2-methyl;

$R_{14}$ und $R_{15}$, unabhängig voneinander, H, $C_1$-$C_{18}$-Alkyl, $C_3$-$C_{18}$-Alkenyl, $C_6$-$C_{12}$-Cycloalkyl, Halogen, Trifluormethyl, Phenyl, Phenyl-$C_1$-$C_4$-alkyl, -CN, $C_1$-$C_{18}$-Alkyl-(S=O)$_t$-, Phenyl-(S=O)$_t$- oder -$OR_{131}$;

$R_{131}$ $C_1$-$C_{18}$-Alkyl; $C_1$-$C_{18}$-Alkyl, welches substituiert ist durch -OH, $C_1$-$C_{18}$-Alkoxy, $C_5$-$C_{12}$-Cycloalkoxy, $C_3$-$C_6$-Alkenyloxy, Halogen, -$COOR_{13}$, -$CONH_2$, -$COHNR_{132}$, -CON($R_{132}$)($R_{133}$))R -$NHCOR_{12}$, CN, -$OCOR_{12}$, Phenoxy, und/oder durch $C_1$-$C_{18}$-Alkyl, $C_1$-$C_{18}$-Alkoxy oder Halogen substituiertes Phenoxy; $C_3$-$C_{18}$-Alkenyl; $C_6$-$C_{12}$-Cycloakyl; durch $C_1$-$C_4$-Alkyl und/oder -$OCOR_{12}$ substituiertes $C_6$-$C_{12}$-Cycloalkyl; durch ein oder mehrere -O- unterbrochenes $C_3$-$C_{50}$-Alkyl; durch ein oder mehrere -O- unterbrochenes $C_3$-$C_{50}$-Alkyl, welches durch -OA oder -O-CO-$R_{12}$ substituiert ist; Phenyl; Phenyl-$C_1$-$C_4$-alkyl; -$COR_{12}$; -$SO_2R_{12}$;

$R_{132}$ und $R_{133}$, unabhängig voneinander, $C_1$-$C_{12}$-Alkyl, $C_3$-$C_{12}$-Alkoxyalkyl, $C_4$-$C_{16}$-Dialkylaminoalkyl, $C_5$-$C_{12}$-Cycloalkyl; oder

$R_{132}$ und $R_{133}$ gemeinsam $C_3$-$C_9$-Alkylen, $C_3$-$C_9$-Oxaalkylen oder -Azaalkylen; X -$NR_8$-, -O-, -NH-($C_nH_{2n}$)-NH- oder -O-($C_kH_{2k}$)-NH-;

k eine Zahl 2-4;

l eine Zahl 0-19;

m eine Zahl 2-8;

n eine Zahl 0-4;

p eine Zahl 0-10;

q eine Zahl 1-8;

r eine Zahl 0-18; und

t eine Zahl 0, oder 2 bedeuten.

Anwendungsweise und Bevorzugungen der polymeren UV Absorber gelten für die genannten monomeren Verbindungen entsprechend.

Unter den polymeren UV Absorbern sind diejenigen Verbindungen von gesondertem Interesse, die sich durch Polymerisation der genannten monomeren UV Absorber erhalten lassen, d.h. aus solchen Verbindungen der Formel I, worin $R_1$ -A,

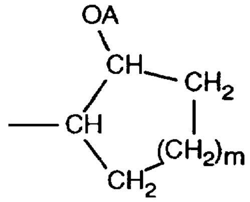
,

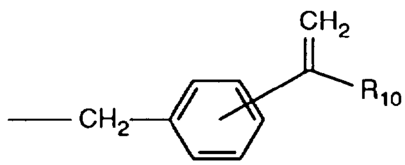 ,

$-CH_2-C(=CH_2)-R_{10}$, $-(CH_2)_p-SiR_{11}R_{11}'-CH=CH_2$, $-C(=O)-(CH_2)_q-CH=CH_2$, $-CHR_8-(CH_2)_r-C(=O)-O-CH_2-CH(OH)-CH_2-OA$, $-CR_8R'_8-(CH_2)_l-C(=O)-XA$ oder $-C(=O)-O-CH_2-C(=CH_2)-R_{10}$, und, im Fall daß $E_1$ oder $E_1$ und $E_2$ eine Gruppe der Formel Ib sind (von Bis(2-hydroxyphenyl)triazin bzw. Tris(2-hydroxyphenyl)triazin abgeleitete Verbindungen), $R_1$ zusätzlich die Bedeutungen $-CH_2-CH(XA)-CH_2-O-R_7$, $-CR_8R'_8-(CH_2)_l-XA$, $-CH_2-CH(OA)-R_9$, $-CH_2-CH(OH)-CH_2-XA$ umfaßt; insbesondere solche, worin $R_1$

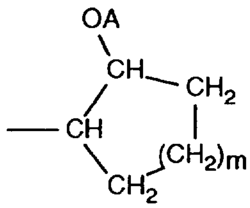 ,

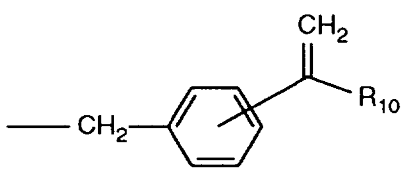 ,

$-(CH_2)_p-SiR_{11}R_{11}'-CH=CH_2$, $-CHR_8-(CH_2)_r-C(=O)-O-CH_2-CH(OH)-CH_2-OA$, $-CR_8R'_8-(CH_2)_l-C(=O)-XA$ oder $-C(=O)-O-CH_2-C(=CH_2)-R_{10}$, und, im Fall daß $E_1$ oder $E_1$ und $E_2$ eine Gruppe der Formel Ib sind, $R_1$ zusätzlich die Bedeutungen -A, $-CH_2-C(=CH_2)-R_{10}$, $-C(=O)-(CH_2)_q-CH=CH_2$, $-CH_2-CH(XA)-CH_2-O-R_7$, $-CR_8R'_8-(CH_2)_l-XA$, $-CH_2-CH(OA)-R_9$, $-CH_2-CH(OH)-CH_2-XA$ umfaßt; eine besonders bevorzugte Gruppe sind darunter diejenigen, worin $E_1$ der Gruppe Ib und $E_2$ der Gruppe Ia entspricht (von Bis(2-hydroxyphenyl)triazin abgeleitete Verbindungen).

Erfindungsgemäß einsetzbare **polymere** Verbindungen der Formel I **leiten sich** daher beispielsweise **von** der Formel Ic **ab**

(Ic) 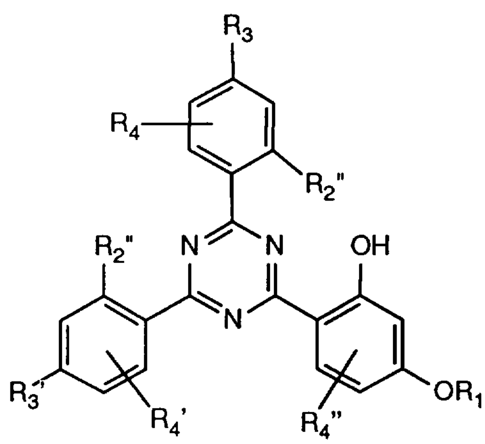 ,

worin die Reste

A $-C(=O)-CR_5=CH-R_6$;

$R_1$, unabhängig voneinander, -A, $-CH_2-CH(OA)-CH_2-O-R_7$, $-CHR_8-(CH_2)_l-OA$, $-CH_2-CH(OA)-R_9$, $-CH_2-CH(OH)-CH_2-OA$,

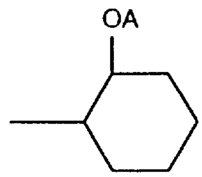
,

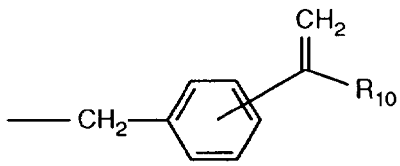
,

$-CH_2-C(=CH_2)-R_{10}$, $-(CH_2)_p-SiR_{11}R_{11}'-CH=CH_2$, $-C(=O)-(CH_2)_q-CH=CH_2$, $-CHR_8-(CH_2)_r-C(=O)-O-CH_2-CH(OH)-CH_2-OA$ oder $-C(=O)-O-CH_2-C(=CH_2)-R_{10}$;

$R_2''$, unabhängig voneinander, H, -OH, -OA, $C_1$-$C_{12}$-Alkyl, Cyclohexyl, $C_3$-$C_6$-Alkenyl, $C_1$-$C_{18}$-Alkoxy, $C_3$-$C_{18}$-Alkenoxy, Halogen, Phenyl oder Trifluormethyl;

$R_3$ und $R_3'$, unabhängig voneinander, H, -OH, $-OR_1$, $C_1$-$C_{12}$-Alkyl, Cyclohexyl, $C_3$-$C_6$-Alkenyl, $C_1$-$C_{18}$-Alkoxy, $C_3$-$C_{18}$-Alkenoxy, Halogen, Trifluormethyl, Phenyl, Phenyloxy, Phenyl-$C_1$-$C_4$-alkyl, Phenyl-$C_1$-$C_4$-alkoxy, -CN, $C_1$-$C_{18}$-Alkyl-$S(=O)_t$- oder Phenyl-$S(=O)_t$-;

$R_4$, $R_4'$ und $R_4''$, unabhängig voneinander, H, $C_1$-$C_{12}$-Alkyl, $C_3$-$C_6$-Alkenyl, $C_1$-$C_{18}$-Alkoxyl, $C_3$-$C_{18}$-Alkenoxy, Halogen, Trifluormethyl, Phenyl, Phenyloxy, Phenyl-$C_1$-$C_4$-alkyl, mit ein bis drei Resten $C_1$-$C_4$-Alkyl substituiertes Phenyl-$C_1$-$C_4$-alkyl, Phenyl-$C_1$-$C_4$-alkoxy, -CN, $C_1$-$C_{18}$-Alkyl-$S(=O)_t$- oder Phenyl-$S(=O)_t$-;

$R_5$ H, $-CH_2-COOR_{13}$, $C_1$-$C_4$-Alkyl oder -CN;

$R_6$ H, $-COOR_{13}$, $C_1$-$C_{17}$-Alkyl oder Phenyl;

$R_7$ $C_1$-$C_{18}$-Alkyl; Cyclohexyl; $C_3$-$C_{18}$-Alkenyl; Phenyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; oder $-C(=O)-R_{12}$;

$R_8$ H oder $C_1$-$C_{18}$-Alkyl;

$R_9$ $C_1$-$C_{18}$-Alkyl, Phenyl oder Phenyl-$C_1$-$C_4$-alkyl;

$R_{10}$ H oder $-CH_3$;

$R_{11}$ und $R_{11}'$, unabhängig voneinander, $C_1$-$C_4$-Alkyl oder Phenyl oder mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl;

$R_{12}$ $C_1$-$C_{18}$-Alkyl, $C_2$-$C_{18}$-Alkenyl oder Phenyl;

$R_{13}$ $C_1$-$C_{18}$-Alkyl, $C_3$-$C_{18}$-Alkenyl oder Phenyl;

l eine Zahl 0-19;

p eine Zahl 0-10;

q eine Zahl 1-8;

r eine Zahl 0-18; und

t eine Zahl 0, 1 oder 2 bedeuten.

Bevorzugt ist der Einsatz von Verbindungen der Formel (I), worin die Reste

$R_1$, unabhängig voneinander, -A, $-CH_2-CH(XA)-CH_2-O-R_7$, $-CR_8R_8'-(CH_2)_l-XA$, $-CH_2-CH(OA)-R_9$, $-CH_2-CH(OH)-CH_2-XA$,

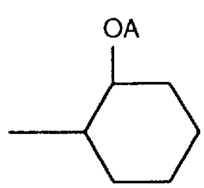
,

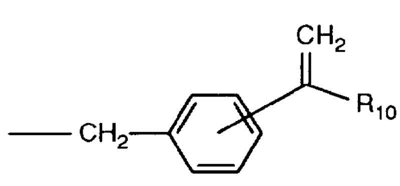

oder $-CHR_8-(CH_2)_r-C(=O)-O-CH_2-CH(OH)-CH_2-OA$;
$R_2$ H, $C_1$-$C_4$-Alkyl, $C_1$-$C_4$-Alkoxy, $C_3$-Alkenyl, F, Cl oder Phenyl;
$R_2$' $C_1$-$C_4$-Alkoxy, $C_3$-Alkenoxy, $-O-COR_{12}$, -OA oder -OH;
$R_3$ und $R_3$', unabhängig voneinander, H, -OH, $-OR_1$, $-OR_{131}$, $C_1$-$C_4$-Alkyl, Cyclohexyl, $C_3$-Alkenyl, F, Cl, Trifluormethyl, Phenyl, Benzyl oder -CN;
$R_4$' und $R_4$", unabhängig voneinander, H, $C_1$-$C_4$-Alkyl, $C_3$-Alkenyl, $C_1$-$C_4$-Alkoxy, $C_3$-Alkenoxy, F, Cl, Trifluormethyl, Phenyl, Phenyl-$C_1$-$C_3$-alkyl oder -CN;
$R_5$ H oder $-CH_3$;
$R_6$ H, $-COOR_{13}$, $-CH_3$ oder Phenyl;
$R_7$ $C_1$-$C_8$-Alkyl, Cyclohexyl, $C_2$-$C_8$-Alkenyl, Phenyl, mit ein bis drei Resten $C_1$-$C_4$-Alkyl oder $C_1$-$C_4$-Alkoxy substituiertes Phenyl, oder Benzyl;
$R_8$ und $R_8$', unabhängig voneinander, H oder $C_1$-$C_{18}$-Alkyl;
$R_9$ $C_1$-$C_{10}$-Alkyl, Phenyl oder Benzyl;
$R_{12}$ H, $C_1$-$C_{18}$-Alkyl, Phenyl, Phenyl-$C_1$-$C_4$-alkyl oder Cyclohexyl;
$R_{13}$ $C_1$-$C_4$-Alkyl, $C_3$-Alkenyl, Cyclohexyl, Phenyl-$C_1$-$C_4$-alkyl oder Phenyl;
$R_4$, $R_{14}$ und $R_{15}$, unabhängig voneinander, H, F, Cl, $C_1$-$C_4$-Alkoxy, $CF_3$, Phenyl, CN oder $C_1$-$C_8$-Alkyl;
$R_{131}$ $C_1$-$C_{18}$-Alkyl; $C_3$-$C_{18}$-Alkyl, welches substituiert ist durch -OH, $C_1$-$C_{18}$-Alkoxy, $C_5$-$C_{12}$-Cycloalkoxy, $-COOR_{13}$, $-CONH_2$, $-COHNR_{132}$, $-CON(R_{132})(R_{133})$, $-NHCOR_{12}$, CN, $-OCOR_{12}$ und/oder Phenoxy; $C_3$-Alkenyl; $C_6$-$C_{12}$-Cycloalkyl; durch ein oder mehrere -O- unterbrochenes $C_3$-$C_{50}$-Alkyl, das unsubstituiert oder durch OH oder $-O-COR_{12}$ substituiert ist; Phenyl; Phenyl-$C_1$-$C_4$-alkyl; $-COR_{12}$; $-SO_2R_{12}$;
X -O- oder $-NR_8$-;
l eine Zahl 1-19; und
r eine Zahl 0-10 bedeuten.

Darunter sind vor allem diejenigen Verbindungen der Formel I von Interesse, worin die Reste
$E_1$ und $E_2$, unabhängig voneinander, jeweils eine Gruppe der Formel Ib oder Ie darstellen

(Ib) 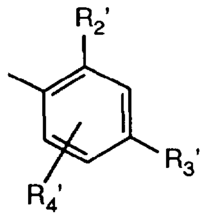 ;

(Ie) 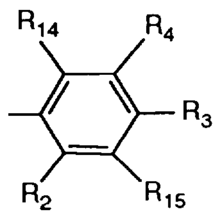

und worin $R_4$' und $R_4$" jeweils in meta-Stellung zum Triazinring stehen.

Besonders bevorzugt sind Verbindungen der Formel (I), worin die Reste
A $-C(=O)-CR_5=CH-R_6$;

$R_1$, unabhängig voneinander, -A, $-CH_2-CH(OA)-CH_2-O-R_7$, $-CH_2-CH(OA)-R_9$,

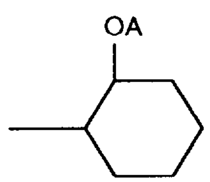 , 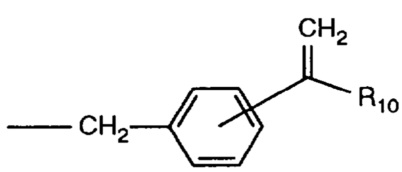

oder $-CHR_8-(CH_2)_l-OA$;
$R_2$ H, $CH_3$, $C_1$-$C_2$-Alkoxy, $C_3$-Alkenoxy oder Cl;
$R_2$' -OH;
$R_3$ H, $-CH_3$, $C_1$-$C_4$-Alkoxy, $C_3$-Alkenoxy, F, Cl, Phenyl, Benzyloxy oder -CN;
$R_3$' $-OR_1$ oder $-OR_{131}$;

$R_4$, $R_{14}$ und $R_{15}$, unabhängig voneinander, H, $OCH_3$, F, Cl, Phenyl, CN oder $CH_3$;
$R_4$' und $R_4$", unabhängig voneinander, H, $-CH_3$, $C_3$-Alkenyl, $-OCH_3$, $C_3$-Alkenoxy, F, Cl, Phenyl-$C_1$-$C_3$-alkyl oder -CN;
$R_5$ H oder $-CH_3$;
$R_6$ H oder $-CH_3$;
$R_7$ $C_1$-$C_8$-Alkyl, Cyclopentyl, Cyclohexyl, $C_3$-Alkenyl, Phenyl oder Benzyl;
$R_8$ H oder $C_1$-$C_{18}$-Alkyl;
$R_9$ $C_1$-$C_{10}$-Alkyl or phenyl;
$R_{12}$ $C_1$-$C_{18}$-Alkyl, Phenyl oder Cyclohexyl;
$R_{131}$ $C_1$-$C_{18}$-Alkyl oder $C_3$-$C_{18}$-Alkyl, welches substituiert ist durch $C_1$-$C_{18}$-Alkoxy, OH, Phenoxy, $-NHCOR_{12}$ und/oder $-OCOR_{12}$; und
l eine Zahl 1-19 bedeuten.

Ganz besonders bevorzugt sind Verbindungen der Formel (I), worin die Reste A $-C(=O)-CR_5=CH-R_6$;

$R_1$, unabhängig voneinander, -A, $-CH_2-CH(OA)-CH_2-O-R_7$, $-CH_2-CH(OA)-R_9$,

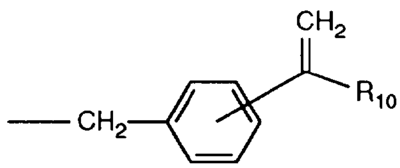

oder $-CH_2-(CH_2)_l-OA$;
$R_2$ H oder $CH_3$;
$R_2$' -OH;
$R_3$ H, $-CH_3$, $OCH_3$, Cl oder Phenyl;
$R_3$' $-OR_1$ oder $-OR_{131}$;
$R_4$ H, Cl, $OCH_3$, F oder $CH_3$;
$R_4$' und $R_4$" H oder $CH_3$;
$R_{14}$ und $R_{15}$ Wasserstoff, $CH_3$ oder $OCH_3$;
$R_5$ H oder $-CH_3$;
$R_6$ H;
$R_7$ $C_1$-$C_8$-Alkyl;
$R_9$ $C_1$-$C_{10}$-Alkyl;
$R_{12}$ $C_1$-$C_8$-Alkyl;
$R_{131}$ $C_1$-$C_{18}$-Alkyl oder $C_3$-$C_{18}$-Alkyl, welches substituiert ist durch $-OCOR_{12}$; und
l eine Zahl 1-10 bedeuten.

Speziell bevorzugt sind Verbindungen der Formel (I), worin mindestens einer der Reste $R_3$ oder $R_3$' die Bedeutung $-OR_1$ hat.

Einen Gegenstand besonderen Interesses bildet die erfindungsgemäße Verwendung von solchen Verbindungen der Formel I, worin $E_1$ der Formel Ib und $E_2$ der Formel Ia, insbesondere der Formel Ie, entspricht. Darunter sind speziell gemischt substituierte Verbindungen bevorzugt, worin weder $R_3$ noch $R_3$' die Bedeutung $-OR_1$ umfassen; besonders solche, worin $R_2$' OH darstellt und $R_3$' $C_1$-$C_{18}$-Alkoxy, oder $C_2$-$C_{18}$-Alkoxy bedeutet, welches durch -O- unterbrochen oder durch OH, $-OCOR_{12}$ und/oder $C_1$-$C_{18}$-Alkoxy oder $C_5$-$C_{12}$-Cycloalkoxy substituiert ist, wobei $R_{12}$ $C_1$-$C_{18}$-Alkyl, Phenyl, Phenyl-$C_1$-$C_4$-alkyl oder $C_5$-$C_{12}$-Cycloalkyl ist; dies sind vor allem solche Verbindungen, worin $R_1$ die Bedeutung $-CR_8R_8'-(CH_2)_l-XA$ hat.

Bevorzugt werden polymere Verbindungen eingesetzt, welche aus mindestens einem Monomeren der Formel (I) aufgebaut sind. Es kommen hierfür sowohl Homopolymere wie auch Copolymere in Frage, wobei die Copolymeren aus mindestens zwei verschiedenen Verbindungen der Formel (I) aufgebaut werden können oder aber aus mindestens einer Verbindung der Formel (I) und einem weiteren Comonomeren.

Bevorzugte Monomere der Formel (I) sind solche, welche eine Acrylat-Gruppe enthalten, wobei dann im Polymeren A

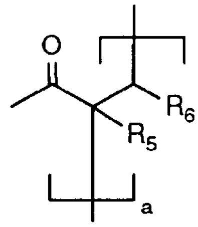

bedeutet und a eine ganze Zahl ist, und solche, welche eine Styrol-Einheit enthalten, wobei dann im Polymeren A

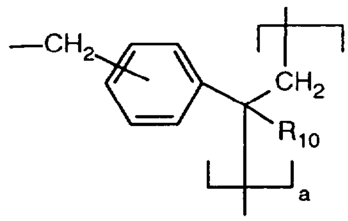

bedeutet und a eine ganze Zahl ist. Vorzugsweise ist a eine Zahl aus dem Bereich 3-100000, besonders 4-1000.

Die Endgruppen der erfindungsgemäß verwendbaren Polymere ergeben sich in bekannter Weise aus den Bedingungen der Polymerisationsreaktion.

Für die die Polymeren aufbauenden Monomeren der Formel (I) gelten die oben näher erläuterten Bevorzugungen. Bevorzugt sind somit Homopolymere, welche aus Monomeren einer als bevorzugt genannten Verbindung der Formel (I) aufgebaut sind. Bevorzugt sind somit Copolymere, welche aus Monomeren mindestens zweier als bevorzugt genannten Verbindungen der Formel (I) aufgebaut sind. Analoges gilt für die besonderen Bevorzugungen.

Als weitere Comonomere (von Verbindungen der Formel (I) verschiedene Comonomere) kommen unter anderem in Alphastellung ungesättigte Carbonsäuren wie beispielsweise Acryl- und Methacrylsäure, Vinylether, Styrol, Vinylpyridin, Acrylnitril, Vinylpyrrolidon oder deren Derivate in Betracht.

Von besonderer Bedeutung ist der Einsatz anderer copolymerisierbarer Stabilisatoren, beispielsweise von ethylenisch ungesättigten Derivaten von sterisch gehinderten Aminen (HALS), 2-(2'-Hydroxyphenyl)benztriazolen, 2-Hydroxy-benzophenonen, Zimtsäurederivaten oder sterisch gehinderten Phenolen.

In Copolymeren, welche neben Einheiten aus mindestens einer Verbindung der Formel I noch weitere Comonomere enthalten, beträgt das Mengenverhältnis an weiteren Comonomeren : Monomeren der Formel I vorzugsweise bis zu 10: 1; es liegt vor allem im Bereich 1 : 1 bis 5 : 1.

Entsprechende, als Comonomer verwendbare HALS sind allgemein durch das Strukturelement

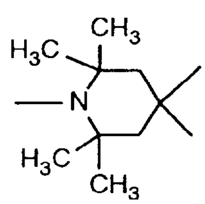

gekennzeichnet, wobei die drei offenen Bindungen durch H oder einen organisch-chemischen Substituenten abgesättigt sind und mindestens eine polymerisierbare, ethylenisch ungesättigte Doppelbindung im Molekül enthalten ist; entsprechende Verbindungen sind unter anderem in US-A-4 942 238, US-A-4 983 737 und EP-A-634 399 und dort zitierter Literatur beschrieben.

Entsprechende, als Comonomer verwendbare 2-(2'-Hydroxyphenyl)benztriazole sind allgemein durch das Strukturelement

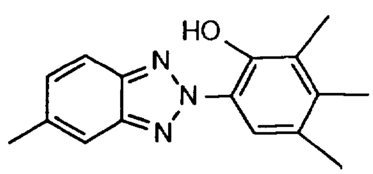

gekennzeichnet, wobei die vier offenen Bindungen durch H oder einen organisch-chemischen Substituenten abgesättigt sind und mindestens eine polymerisierbare, ethylenisch ungesättigte Doppelbindung im Molekül enthalten ist; entsprechende Verbindungen sind unter anderem in US-A-5099027, US-A-4528311, US-A-5147902, Research Disclosure 32592, US-A-4785063, US-A-4892915, US-A-4611061, EP-A-190003, EP-A-508744, US-A-4716234, US-A-3493539, US-A-5234807, US-A-5256359, US-A-5385815, US-A-5372922, JP-A-03-139590, EP-A-431868, JP-A-03-8547, GB-A-2232667, EP-A-282294, EP-A-343996, EP-A-133164, EP-A-131468, J. Macromol. Sci., Pure Appl. Chem. A30

(9-10), 741 (1993) und Polm. Bull. 12 (5), 375 (1984), sowie in dort zitierter Literatur beschrieben.
Entsprechende, als Comonomer verwendbare 2-Hydroxy-benzophenone sind allgemein durch das Strukturelement

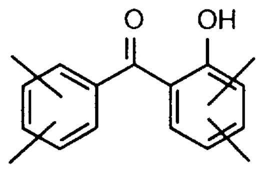

gekennzeichnet, wobei die vier offenen Bindungen durch H oder einen organisch-chemischen Substituenten abgesättigt sind und mindestens eine polymerisierbare, ethylenisch ungesättigte Doppelbindung im Molekül enthalten ist; entsprechende Verbindungen sind unter anderem in CH-B-383001 und CH-B-376899 und dort zitierter Literatur beschrieben.

Entsprechende, als Comonomer verwendbare Zimtsäurederivate sind allgemein durch das Strukturelement

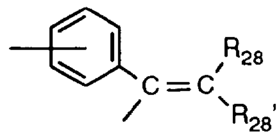

gekennzeichnet, wobei $R_{28}$ und $R_{28}'$ unabhängig voneinander CN oder $COOR_{13}$ sind, $R_{13}$ die weiter oben angegebenen Bedeutungen umfaßt und die beiden offenen Bindungen durch H oder einen organisch-chemischen Substituenten abgesättigt sind und mindestens eine polymerisierbare, ethylenisch ungesättigte Doppelbindung im Molekül enthalten ist.
Entsprechende, als Comonomer verwendbare sterisch gehinderte Phenole sind allgemein durch das Strukturelement

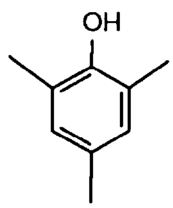

gekennzeichnet, wobei die drei offenen Bindungen durch organisch-chemische Substituenten abgesättigt sind und mindestens eine polymerisierbare, ethylenisch ungesättigte Doppelbindung im Molekül enthalten ist, meist im zur Hydroxylgruppe p-ständigen Substituenten; entsprechende Verbindungen sind unter anderem in US-A-3 708 520 und dort zitierter Literatur beschrieben.

Bevorzugt sind als weitere Comonomere (von Verbindungen der Formel (I) verschiedene Comonomere) Verbindungen der folgenden Formeln (II)-(VII)

(II)

$R_{18}$-CH=C($R_{17}$)-C(=O)-X'-$R_{20}$,
worin X' -O- oder -$NR_{19}$- ist;
$R_{17}$ H, $C_1$-$C_4$-Alkyl, -$CH_2$-$COOR_{21}$, -Cl oder -CN;
$R_{18}$ H, -$COOR_{21}$ oder -$CH_3$;
$R_{19}$ H, $C_1$-$C_8$-Alkyl, $C_4$-$C_{12}$-Cycloalkyl, durch -N$(R_X)_2$ substituiertes $C_1$-$C_4$-Alkyl, -S(=O)-$R_X$, -C$(CH_3)_2$-$CH_2$-C(=O)-$CH_3$, -C$(CH_3)_2$-$CH_2$-$SO_3$M, -$(CH_2)_s$-$SO_3$M oder

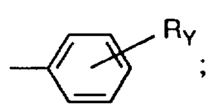

$R_{20}$ H; $C_1$-$C_{18}$-Alkyl; $C_2$-$C_{18}$-Alkenyl; durch ein oder mehrere O-Atome unterbrochenes $C_2$-$C_{18}$-Alkyl, das durch OH substituiert sein kann; oder -$(CH_2)_s$-$SO_3$M;

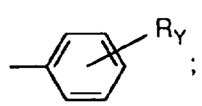

-$CH_2$F; -$CH_2$Cl; -$CH_2$CN; -$CH_2CH_2$Cl;
-$CH_2CH_2$CN; -$CH_2CH_2$-$COOR_X$; $C_7$-$C_{11}$-Phenylalkyl; Naphthyl; durch -N$(R_X)_2$ substituiertes $C_1$-$C_4$-Alkyl; Adamantyl; $C_6$-$C_{12}$-Cycloalkyl;
$R_{21}$ H, $C_1$-$C_{18}$-Alkyl, Phenyl oder $C_2$-$C_{18}$-Alkenyl;
$R_X$ $C_1$-$C_4$-Alkyl oder Phenyl;

$R_Y$ H, $C_1$-$C_{12}$-Alkyl, Phenyl, -CO-$OR_X$, -CN, -F, -Cl;
M H oder ein Alkalimetall; und
s eine Zahl zwischen 1 und 5 bedeuten.

(III)

$R_{22}$-C(=O)-O-CH=$CH_2$,
worin $R_{22}$ $C_1$-$C_{19}$-Alkyl oder Phenyl b

(IV) 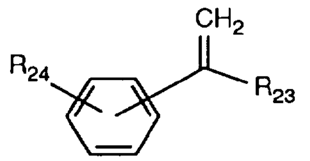 ,

worin $R_{23}$ H oder -$CH_3$;
$R_{24}$ H, -$CR_{23}$=$CH_2$, -C(O)-Phenyl oder
M H oder ein Alkalimetall bedeutet.

(V) 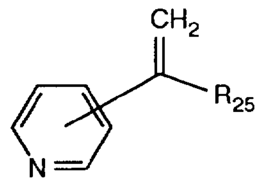 ,

worin $R_{25}$ H oder -$CH_3$ bedeutet.

(VI)

$CH_2$=$CR_{26}$-$R_{27}$,
worin $R_{26}$ H, -F, -Cl oder -$CH_3$ und
$R_{27}$ -Cl, -Br, -F oder -CN bedeutet.

(VII) 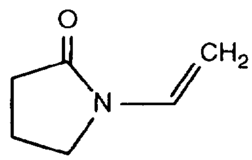 ,

sowie die oben genannten polymerisierbaren ethylenisch ungesättigten Derivate von sterisch gehinderten Aminen (HALS), 2-(2'-Hydroxyphenyl)benztriazolen, 2-Hydroxybenzophenonen und sterisch gehinderten Phenolen.

Die möglichen Bedeutungen für die Substituenten in den Formeln (II)-(VII) entsprechen denen wie sie für die Verbindungen der Formel (I) weiter oben angegeben sind.

Bedeuten Substituenten in obigen Formeln durch ein oder mehrere O-Atome unterbrochenes Alkyl mit bis zu 18 Kohlenstoffatomen, so kommen hierfür Reste wie -($CH_2$-$CH_2$-O)$_{1-8}$-$CH_3$, -($CH_2$-$CH_2$-O)$_{1-8}$-$C_2H_5$ oder auch -($CH_2$-$CH_2$-$CH_2$-O)$_{1-5}$-$CH_3$ in Frage

Bedeuten Substituenten in obigen Formeln Alkalimetall, so kommt hierfür Li, Na oder K in Frage.

Besonders bevorzugte weitere Comonomere (von Verbindungen der Formel (I) verschiedene Comonomere) sind Verbindungen der Formeln (II), (III), (IV) und (VII).

Bevorzugt sind Copolymere, welche durch Polymerisation mindestens einer bevorzugten Verbindung der Formel (I) und mindestens einem der Comonomeren der Formeln (II)-(VII) erhältlich sind.

Besonders bevorzugt sind Copolymere, welche durch Polymerisation mindestens einer besonders bevorzugten Verbindung der Formel (I) und mindestens einem der Comonomeren der Formeln (II), (III), (IV) oder (VII) erhältlich sind.

Ganz besonders bevorzugt sind Copolymere, welche durch Polymerisation mindestens einer ganz besonders bevorzugten Verbindung der Formel (1) und mindestens einem der Comonomeren der Formeln (II), (III), (IV) oder (VII), wobei

$R_{17}$ H oder -$CH_3$;
$R_{18}$ H oder -$CH_3$;
$R_{19}$ H, $C_1$-$C_4$-Alkyl, -C($CH_3$)$_2$-$CH_2$-$SO_3M$ oder -($CH_2$)$_s$-$SO_3M$;
$R_{20}$ H, $C_1$-$C_8$-Alkyl oder durch ein oder mehrere O-Atome unterbrochenes $C_2$-$C_8$-Alkyl;
R22 -$CH_3$;
$R_{23}$ und $R_{24}$ H;
M H, Li, Na oder K
X -O- oder -$NR_{19}$-; und
s die Zahl 2 oder 3 bedeuten;

erhältlich sind.

Ein weiterer Gegenstand vorliegender Erfindung betrifft ein fotografisches Aufzeichnungsmaterial, welches neben dem oben beschriebenen Monomeren der Formel I bzw. einem entsprechenden Homo- oder Copolymeren zusätzlich einen Stabilisator aus der Klasse der sterisch gehinderten Amine (HALS) enthält. Vorzugsweise ist das sterisch gehinderte Amin in derselben Schicht enthalten wie der erfindungsgemäße UV-Absorber. Bei dem sterisch gehinderten Amin handelt es sich allgemein um ein cyclisches sterisch gehindertes Amin, insbesondere eine Verbindung aus der Reihe der Derivate von Polyalkylpiperidinen oder -piperazinen, die mindestens eine Gruppe der Formeln HALS-II oder HALS-III

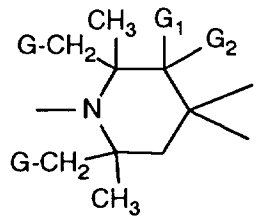

(HALS-II)

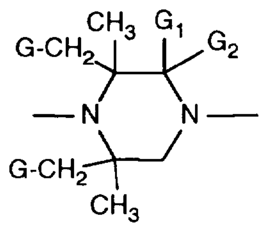

(HALS-III)

enthalten, worin G Wasserstoff oder Methyl ist und $G_1$ und $G_2$ Wasserstoff, Methyl oder gemeinsam =O bedeuten; vorzugsweise sind die Polyalkylpiperidingruppen der Formel II oder III in 4-Stellung mit einem oder zwei polaren Substituenten oder einen polaren Spiro-Ringsystem substituiert.

Ein sterisch gehindertes Amin wird in diesem speziellen fotografischen Aufzeichnungsmaterial zweckmäßig in einer Menge von 1 mg bis 200 mg pro $m^2$ Trägermaterial eingesetzt.

Beispiele für entsprechende sterisch gehinderte Amine sind die folgenden:
Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidy1)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)-ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro [4,5] decan und Epichlorhydrin.

Bevorzugt zur Verwendung in den erfindungsgemäßen fotografischen Aufzeichnungsmaterialien sind insbesondere Verbindungen vom Typ HALS-II, welche am Stickstoffatom durch -CO-R, -O-R, Benzyl oder Allyl substituiert sind, wobei R einen organisch-chemischen Rest, meist einen Kohlenwasserstoffrest von 1 bis 30 Kohlenstoffatomen, darstellt. Beispiele für diese bevorzugten Costabilisatoren finden sich in obiger Liste.

Die genannten Verbindungen der Formel (I) sind zum Teil bekannt oder lassen sich nach im Prinzip bekannten Methoden herstellen. Die folgenden Reaktionsschemata zeigen ein prinzipielles, zweistufiges (a+b) Verfahren zur Herstellung von Verbindungen der Formel (I), welche sich von Monoresorcinoltriazinen ableiten. Analog lassen sich auch von Bis-oder Trisresorcinol abgeleitete Verbindungen herstellen. Weitere Erläuterungen zu möglichen Herstellungsverfahren finden sich in EP-A-0 434 608.

a) Alkylierung der para-OH-Gruppe des Resorcinolrestes (durch beispielsweise einen Glycidylether oder einen ω-Bromalkohol):

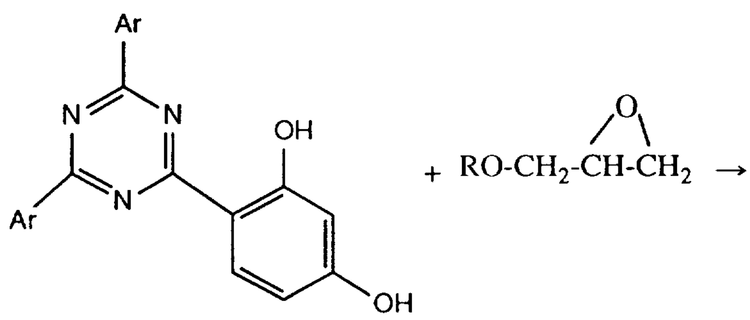

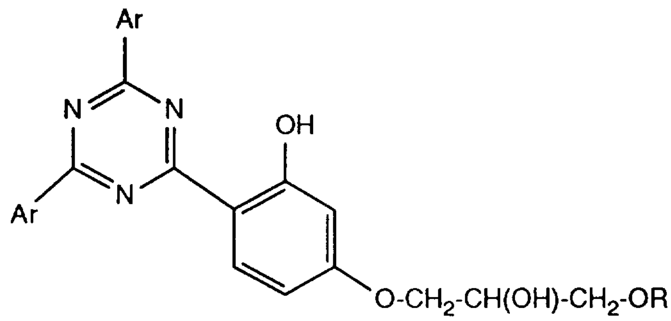

(Ar: substituierter oder unsubstituierter Phenylrest; R: z.B. Alkylrest)

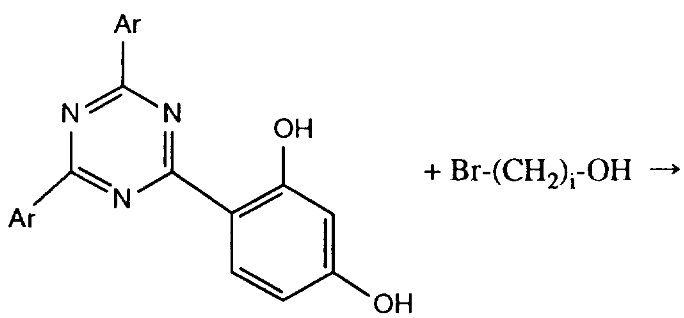

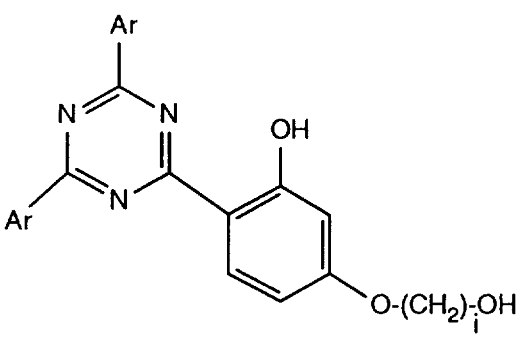

(Ar: substituierter oder unsubstituierter Phenylrest; i: 1-20)

b) Acrylierung bzw. Methacrylierung der aliphatischen OH-Gruppe

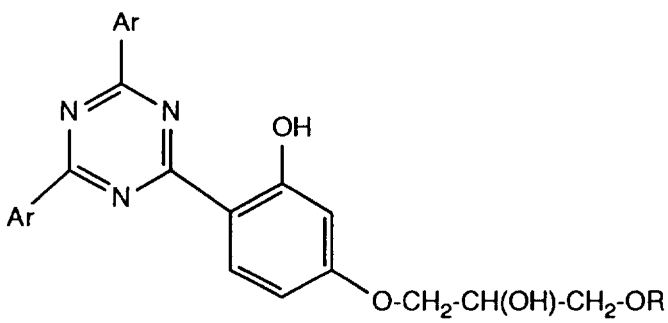

$+ CH_2=CR_1-C(=O)Cl \rightarrow$

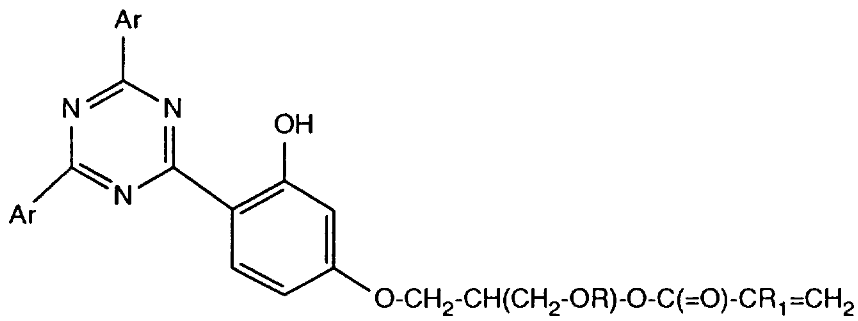

(Ar: substituierter oder unsubstituierter Phenylrest; R: z.B. Alkyl; $R_1$: H oder $-CH_3$)

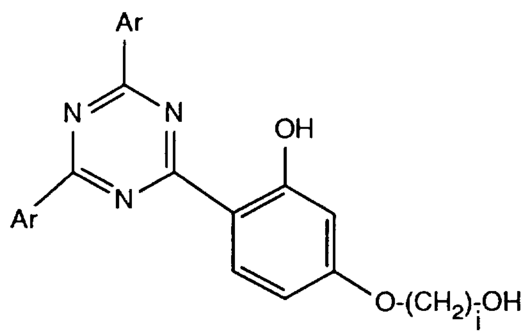

$+ CH_2=CR_1-C(=O)Cl \rightarrow$

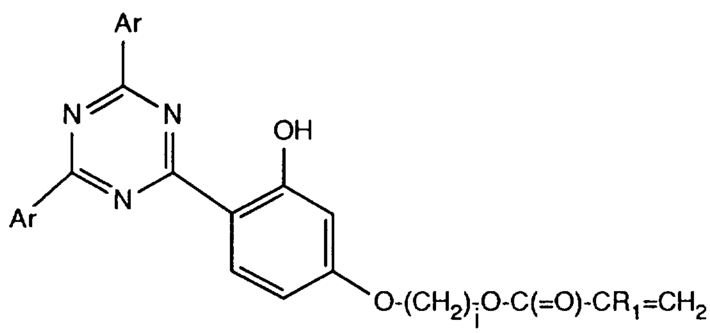

(Ar: substituierter oder unsubstituierter Phenylrest; i: 1-20; $R_1$: H oder $-CH_3$)

Die Polymerisation einer der monomeren Verbindungen der Formel (I) kann durch radikalische, anionische oder kationische Initiatoren initiiert werden. Vorzugsweise verwendet man radikalische Initiatoren, die beim Erwärmen in Radikale zerfallen, wie z.B. organische Peroxide oder Hydroperoxide, Azoverbindungen oder Redox-Katalysatoren. Die Polymerisation kann auch durch energiereiche Strahlung initiiert werden. Die Polymerisation kann in Lösung, Emulsion, Dispersion oder in Masse ausgeführt werden. Dem Fachmann sind diese Verfahren bekannt. Weiterhin werden in der EP-A-0 577 122 Seite 9, Zeile 46 bis Seite 10, Zeile 35 geeignete Polymerisationsverfahren beschrieben.

Beispiele für Verbindungen der Formel (I) sind:

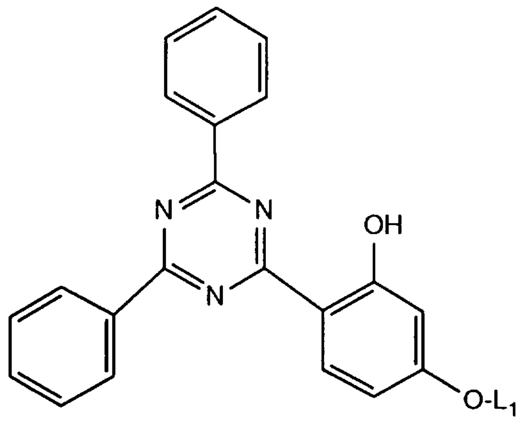

| Nr. | $L_1$ |
|---|---|
| (100) | $-CH_2-CH(CH_2-O-n-C_4H_9)-O-C(O)-CH=CH_2$ ($n-C_4H_9$: n-Butyl) |
| (101) | $-CH_2-CH(CH_2-O-n-C_4H_9)-O-C(O)-C(CH_3)=CH_2$ |
| (102) | $-(CH_2)_{11}-O-C(O)-CH=CH_2$ |
| (103) | $-(CH_2)_{11}-O-C(O)-C(CH_3)=CH_2$ |
| (104) | $-CH_2-CH(n-C_4H_9)-O-C(O)-C(CH_3)=CH_2$ |
| (105) | 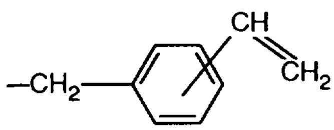 |
| (106) | $-CH_2-CH_2-O-C(O)-C(CH_3)=CH_2$ |
| (107) | $-C(O)-C(CH_3)=CH_2$ |

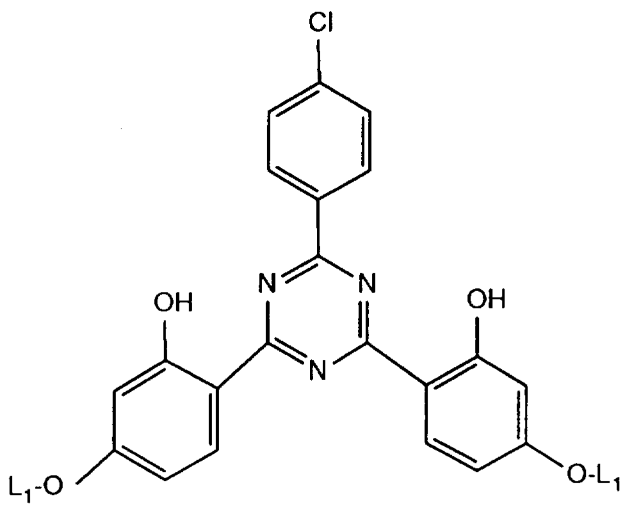

| Nr. | $L_1$ |
|---|---|

(200) $-CH_2-CH_2-O-C(O)-CH=CH_2$

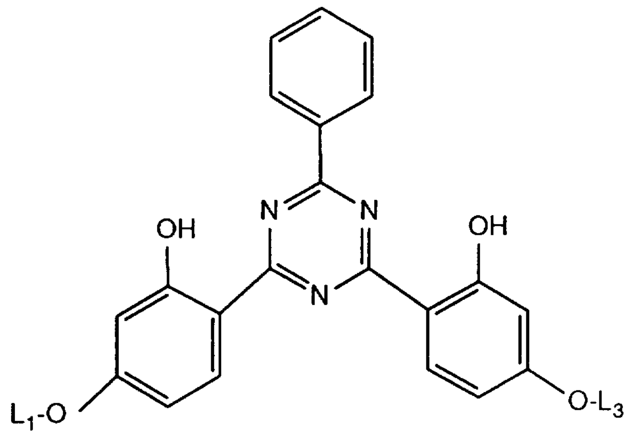

| Nr. | $L_1$, $L_3$ |
|---|---|
| (201) | $-CH_2-CH_2-O-C(O)-C(CH_3)=CH_2$ |
| (202) | $-CH_2-CH(CH_2-O-n-C_4H_9)-O-C(O)-CH=CH_2$ |
| (203) | $-(CH_2)_{11}-O-C(O)-CH=CH_2$ |
| (204) | $-(CH_2)_{11}-O-C(O)-C(CH_3)=CH_2$ |
| (205) | $-CH_2-CH(n-C_4H_9)-O-C(O)-C(CH_3)=CH_2$ |
| (206) | $-CH_2-CH(n-C_4H_9)-O-C(O)-CH=CH_2$ |
| (207) | $L_1$= $n-C_6H_{13}$, $L_3$= $-(CH_2)_{11}-O-C(O)-C(CH_3)=CH_2$ |
| (208) | $L_1$= $-(CH_2)_{11}-O-C(O)-CH_3$, $L_3$= $-(CH_2)_{11}-O-C(O)-C(CH_3)=CH_2$ |

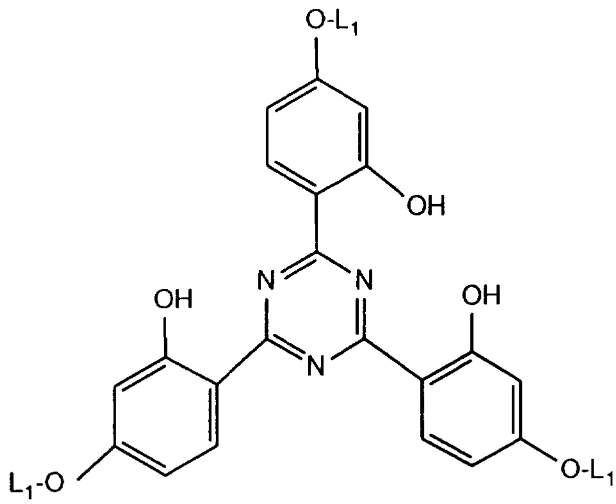

| Nr. | $L_1$ |
|---|---|
| (300) | $-CH_2-CH(CH_2-O-n-C_4H_9)-O-C(O)-CH=CH_2$ ($n-C_4H_9$: n-Butyl) |
| (301) | $-(CH_2)_{11}-O-C(=O)-CH=CH_2$ |
| (302) | $-(CH_2)_{11}-O-C(=O)-C(CH_3)=CH_2$ |

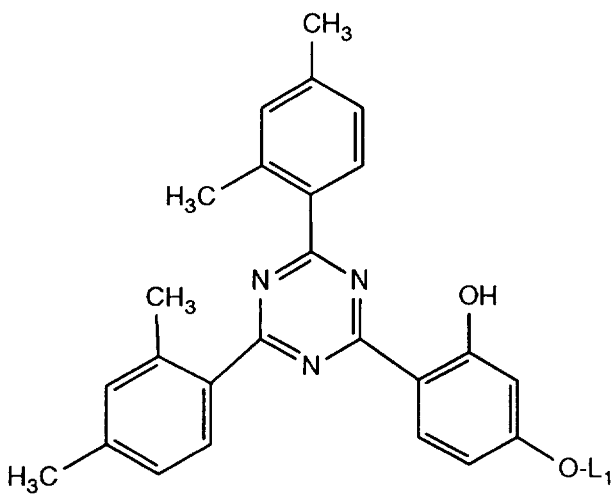

| Nr. | $L_1$ |
|---|---|
| (400) | $-CH_2-CH(CH_2-O-n-C_4H_9)-O-C(O)-C(CH_3)=CH_2$ |
| (401) | $-(CH_2)_{11}-O-C(O)-CH=CH_2$ |
| (402) | $-(CH_2)_{11}-O-C(O)-C(CH_3)=CH_2$ |
| (403) | $-CH_2-CH(n-C_4H_9)-O-C(O)-C(CH_3)=CH_2$ |
| (404) | $-CH_2-CH_2-O-C(O)-C(CH_3)=CH_2$ |

(405) 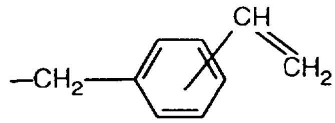

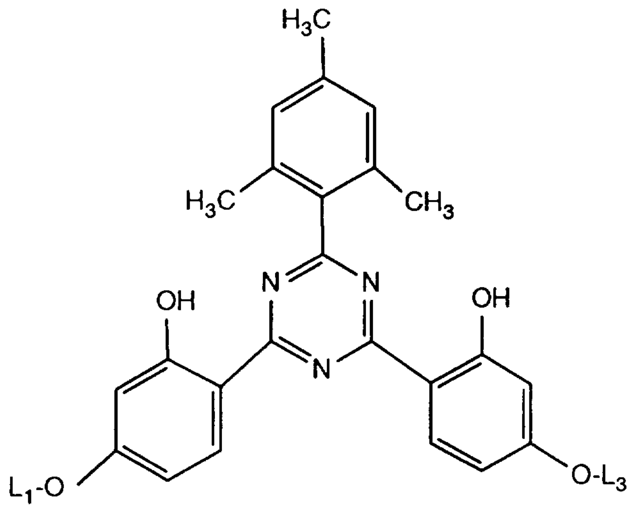

| Nr. | $L_1$, $L_3$ |
|---|---|
| (500) | $-CH_2-CH(CH_2-O-n-C_4H_9)-O-C(O)-CH=CH_2$ |
| (501) | $-CH_2-CH(CH_2-O-n-C_4H_9)-O-C(O)-C(CH_3)=CH_2$ |
| (502) | $-(CH_2)_{11}-O-C(O)-C(CH_3)=CH_2$ |
| (503) | $-CH_2-CH(n-C_4H_9)-O-C(O)-C(CH_3)=CH_2$ |
| (504) | $L_1$= n-$C_6H_{13}$, $L_3$= $-(CH_2)_{11}-O-C(O)-C(CH_3)=CH_2$ |
| (505) | 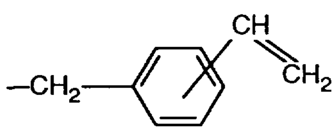 |
| (506) | $L_1$= n-$C_6H_{13}$, $L_3$= $-(CH_2)_{11}-O-C(O)-CH=CH_2$ |

Beispiele für polymere Verbindungen sind:

(600) Homopolymer der Verbindung (204)
(601) Copolymer aus Verbindung (204) und n-Butylacrylat im Molverhältnis 1:4

(602) Homopolymer der Verbindung (207)
(603) Copolymer aus Verbindung (207) und n-Butylacrylat im Molverhältnis 1:4

(604) Homopolymer der Verbindung (504)

(605) Copolymer aus Verbindung (504) und n-Butylacrylat im Molverhältnis 1:4
(606) Copolymer aus Verbindung (504) und n-Dodecyl-methacrylat im Molverhältnis 1:4

(607) Homopolymer der Verbindung (102)
(608) Copolymer aus Verbindung (102) und n-Butylacrylat im Molverhältnis 1:4

(609) Homopolymer der Verbindung (402)
(610) Copolymer aus Verbindung (402) und n-Butylacrylat im Molverhältnis 1:2

(611) Homopolymer der Verbindung (103)
(612) Copolymer aus Verbindung (103) und n-Butylacrylat im Molverhältnis 1:2

(613) Homopolymer der Verbindung (400)
(614) Copolymer aus Verbindung (400) und n-Butylacrylat im Molverhältnis 1:2

(615) Copolymer aus Verbindung (105) und n-Butylacrylat im Molverhältnis 1:2

(616) Copolymer aus Verbindung (405) und n-Butylacrylat im Molverhältnis 1:2.

Das erfindungsgemäße fotografische Aufzeichnungsmaterial kann schwarzweiß- oder farbfotografisches Material sein. Bevorzugt ist farbfotografisches Material.

Beispiele für farbfotografische Materialien sind Farbnegativfilme, Farumkehrfilme, Farbpositivfilme, farbfotografisches Papier, farbumkehrfotografisches Papier, farbempfindliche Materialien für das Farbdiffusionstransfer-Verfahren oder das Silberfarb-Bleichverfahren.

Geeignete Träger zur Herstellung farbfotografischer Materialien sind z.B. Filme und Folien von halbsynthetischen und synthetischen Polymeren, wie Cellulosenitrat, Celluloseacetat, Cellulosebutyrat, Polystyrol, Polyvinylchlorid, Polyethylenterephthalat und Polycarbonat und mit einer Barytschicht oder $\alpha$-Olefinpolymerschicht (z.B. Polyethylen) laminiertes Papier. Diese Träger können mit Farbstoffen und Pigmenten, beispielsweise Titandioxid, gefärbt sein. Sie können auch zum Zwecke der Abschirmung von Licht schwarz gefärbt sein. Die Oberfläche des Trägers wird im allgemeinen einer Behandlung unterzogen, um die Adhäsion der fotografischen Emulsionsschicht zu verbessern, beispielsweise einer Corona-Entladung mit nachfolgendem Antrag einer Substratschicht.

Die farbfotografischen Materialien enthalten üblicherweise mindestens je eine rotempfindliche, grünempfindliche und blauempfindliche Silberhalogenidemulsionsschicht sowie gegebenenfalls Zwischenschichten und Schutzschichten. Bevorzugt enthält das erfindungsgemäße Material die Silberhalogenidemulsionsschichten vom Träger aus in der Reihenfolge blauempfindliche, grünempfindliche und rotempfindliche Schicht. Im erfindungsgemäßen farbfotografischen Material ist der UV-Absorber vorzugsweise in einer Schicht über der grünempfindlichen Schicht, besonders bevorzugt in einer Schicht oberhalb der Silberhalogenidemulsionsschicht(en) enthalten.

Der erfindungsgemäße UV-Absorber ist in dem fotografischen Material vorzugsweise in einer Menge von 0,05 bis 10 g pro $m^2$, besonders 0,1 bis 8, vor allem 0,2 bis 5 g/$m^2$ enthalten.

Ein Beispiel für ein erfindungsgemäßes farbfotografisches Aufzeichnungsmaterial stellt ein Material mit folgender Schichtabfolge dar:

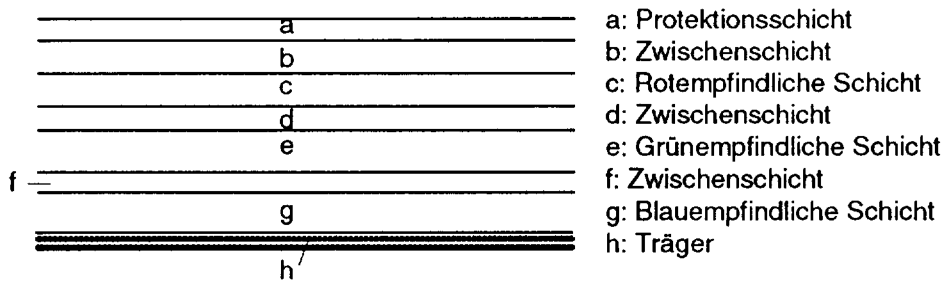


Ein anderes Beispiel ist ein Material mit ähnlichem Schichtaufbau, worin jedoch Schicht a fehlt. Der erfindungsgemäße UV-Absorber der Formel (I) bzw. das entsprechende Homooder Copolymer ist bei der dargestellten Schichtabfolge z.B. zweckmäßig in Schicht b, c und/oder d enthalten, besonders in b und/oder c, vor allem in b.

Bevorzugt ist allgemein ein fotografisches Aufzeichnungsmaterial enthaltend eine Verbindung der Formel (I) bzw. ein entsprechendes Homo- oder Copolymer in einer Schicht oberhalb der Silberhalogenidemulsionsschicht(en). Weiterhin bevorzugt ist fotografisches Aufzeichnungsmaterial enthaltend mindestens je eine rotempfindliche und grünempfindliche Silberhalogenidemulsionsschicht sowie dazwischen eine Zwischenschicht, wobei mindestens eine Verbindung der Formel (I) bzw. ein entsprechendes Homo- oder Copolymer in der Zwischenschicht zwischen der rotempfindlichen und der grünempfindlichen Silberhalogenidemulsionsschicht enthalten ist. Ganz besonders bevorzugt ist fotografisches Aufzeichnungsmaterial enthaltend mindestens je eine rotempfindliche, grünempfindliche und blauempfindliche Silberhalogenidemulsionsschicht sowie mindestens zwei zwischen den genannten Schichten liegende Zwischenschichten und eine Schutzschicht, wobei mindestens eine Verbindung der Formel (I) bzw. ein entsprechendes Homo- oder Copolymer in einer Schicht oberhalb der grünempfindlichen Silberhalogenidemulsionsschicht enthalten ist und die Silberhalogenidemulsionsschichten Dunkellager- und/oder Lichtstabilisatoren enthalten.

Wesentliche Bestandteile der fotografischen Emulsionsschichten sind Bindemittel, Silberhalogenidkörnchen und Farbkuppler.

Als Bindemittel wird vorzugsweise Gelatine verwendet. Diese kann jedoch ganz oder teilweise durch andere synthetische, halbsynthetische oder auch natürlich vorkommende Polymere ersetzt werden. Synthetische Gelatineersatzstoffe sind beispielsweise Polyvinylalkohol, Poly-N-vinylpyrrolidon, Polyacrylamide, Polyacrylsäure oder deren Derivate, insbesondere deren Mischpolymerisate. Natürlich vokommende Gelatineersatzstoffe sind beispielsweise andere Proteine wie Albumin oder Casein, Cellulose, Zucker, Stärke oder Alginate. Halbsynthetische Gelatineersatzstoffe sind in der Regel modifizierte Naturprodukte. Cellulosederivate wie Hydroxyalkylcellulose sowie Gelatinederivate, die durch Umsetzung mit Alkylierungs- oder Acylierungsmitteln oder durch Aufpfropfung von polymerisierbaren Monomeren erhalten worden sind, sind Beispiele hierfür.

Die Bindemittel sollen über eine ausreichende Menge an funktionellen Gruppen verfügen, so daß durch Umsetzung mit geeigneten Härtungsmitteln genügend widerstandsfähige Schichten erzeugt werden können. Solche funktionellen Gruppen sind insbesondere Aminogruppen, aber auch Carboxylgruppen, Hydroxylgruppen und aktive Methylengruppen.

Die vorzugsweise verwendete Gelatine kann durch sauren oder alkalischen Aufschluß erhalten sein. Es kann auch oxidierte Gelatine verwendet werden. Die Herstellung solcher Gelatinen wird beispielsweise in The Science and Technology of Gelatine, herausgegeben von A.G. Ward und A. Courts, Academic Press 1977, Seite 295 ff beschireben. Die jeweils eingesetzte Gelatine soll einen möglichst geringen Gehalt an fotografisch aktiven Verunreinigungen enthalten (Inertgelatine). Gelatinen mit hoher Viskosität und niedriger Quellung sind besonders vorteilhaft.

Das als lichtempfindlicher Bestandteil in dem fotografischen Material befindliche Silberhalogenid kann als Halogenid Chlorid, Bromid oder Iodid bzw. Mischungen davon enthalten. Beispielsweise kann der Halogenidanteil wenigstens einer Schicht zu 0 bis 15 mol-% aus Iodid, zu 0 bis 100 mol-% aus Chlorid und zu 0 bis 100 mol-% aus Bromid bestehen. Im Falle von Farbnetagiv- und Farbumkehrfilmen werden üblicherweise Silberbromidiodidemulsionen, im Falle von Farbnegativ- und Farbumkehrpapier üblicherweise Silberchloridbromidemulsionen mit hohem Chloridanteil, beispielsweise mindestens 90 mol % Silberchlorid, bis zu reinen Silberchloridemulsionen verwendet. Es kann sich um überwiegend kompakte Kristalle handeln, die z.B. regulär kubisch oder oktaedrisch sind oder Übergangsformen aufweisen können, vorzugsweise können aber auch plättchenförmige Kristalle vorliegen, deren durchschnittliches Verhältnis von Durchmesser zu Dicke bevorzugt wenigstens 5:1 ist, wobei der Durchmesser eines Kornes definiert ist als der Durchmesser eines Kreises mit einem Kreisinhalt entsprechend der projizierten Fläche des Kornes. Die Schichten können aber auch tafelförmige Silberhalogenidkristalle aufweisen, bei denen das Verhältnis von Durchmesser zu Dicke wesentlich größer als 5:1 ist, z.B. 12:1 bis 30:1.

Die Silberhalogenidkörner können auch einen mehrfach geschichteten Kornaufbau aufweisen, im einfachsten Fall mit einem inneren und einem äußeren Kornbereich (core/shell), wobei die Halogenidzusammensetzung und/oder sonstige Modifizierungen, z.B. Dotierungen der einzelnen Kornbereiche unterschiedlich sind. Die mittlere Korngröße der Emulsionen liegt vorzugsweise zwischen 0,2 µm und 2,0 µm, die Korngrößenverteilung kann sowohl homo- als auch heterodispers sein. Homodisperse Korngrößenverteilung bedeutet, daß 95 % der Körner nicht mehr als ± 30 % von der mittleren Korngröße abweichen. Die Emulsionen können neben dem Silberhalogenid auch organische Silbersalze enthalten, z.B. Silberbenztriazolat oder Silberbehenat.

Es können zwei oder mehrere Arten von Silberhalogenidemulsionen, die getrennt hergestellt werden, als Mischung verwendet werden.

Die fotografischen Emulsionen können nach verschiedenen Methoden (z.B. P. Glafkides, Chimie et Physique Photographique, Paul Montel, Paris (1967), G.F. Duffin, Phtographic Emulsion Chemistry, The Focal Press, London (1966), V.L. Zelikman et al, Making and Coating Photographic Emulsion, The Focal Press, London (1966) aus löslichen Silbersalzen und löslichen Halogeniden hergestellt werden.

Die Fällung des Silberhalogenids erfolgt bevorzugt in Gegenwart des Bindemittels, z.B. der Gelatine und kann im sauren, neutralen oder alkalischen pH-Bereich durchgeführt werden, wobei vorzugsweise Silberhalogenidkomplexbildner zusätzlich verwendet werden. Zu letzteren gehören z.B. Ammoniak, Thioether, Imidazol, Ammoniumthiocyanat oder überschüssiges Halogenid. Die Zusammenführung der wasserlöslichen Silbersalze und der Halogenide erfolgt wahlweise nacheinander nach dem single-jet- oder gleichzeitig nach dem double-jet-Verfahren oder nach beliebiger Kombination beider Verfahren. Bevorzugt wird die Dosierung mit steigenden Zuflußraten, wobei die "kritische" Zufuhrgeschwindigkeit, bei der gerade noch keine Neukeime entstehen, nicht überschritten werden sollte. Der p-Ag-Bereich kann während der Fällung in weiten Grenzen variieren, vorzugsweise wird das sogenannte pAg-gesteuerte Verfahren benutzt, bei dem ein bestimmter pAg-Wert konstant gehalten oder ein definiertes pAg-Profil während der Fällung durchfahren wird. Neben der bevorzugten Fällung bei Halogenidüberschuß ist aber auch die sogenannte inverse Fällung bei Silberhalogenidüberschuß möglich. Außer durch Fällung können die Silberhalogenidkristalle auch durch physikalische Reifung (Ostwaldreifung), in Gegenwart von überschüssigem Halogenid und/oder Silberhalogenidkomplexierungsmittel

wachsen. Das Wachstum der Emulsionskörper kann sogar überwiegend durch Ostwaldreifung erfolgen, wobei vorzugsweise eine feinkörnige, sogenannte Lippmann-Emulsion, mit einer schwerer löslichen Emulsion gemischt und auf letzterer umgelöst wird.

Während der Fällung und/oder der physikalischen Reifung der Silberhalogenidkörner können auch Salze oder Komplexe von Metallen, wie Cd, Zn, Pb, Tl, Bi, Ir, Rh, Fe vorhanden sein.

Ferner kann die Fällung auch in Gegenwart von Sensibilisierungsfarbstoffen erfolgen. Komplexierungsmittel und/oder Farbstoffe lassen sich zu jedem beliebigen Zeitpunkt unwirksam machen, z.B. durch Aenderung des pH-Wertes oder durch eine oxidative Behandlung.

Nach abgeschlossener Kristallbildung oder auch schon zu einem früheren Zeitpunkt werden die löslichen Salze aus der Emulsion entfernt, z.B durch Nutschen und Waschen, durch Flocken und Waschen, durch Ultrafiltration oder durch Ionenaustauscher.

Die Silberhalogenidemulsion wird im allgemeinen einer chemischen Sensibilisierung unter definierten Bedingungen - pH, pAg, Temperatur, Gelatine-, Silberhalogenid- und Sensibilisatorkonzentration - bis zum Erreichen des Empfindlichkeits- und Schleieroptimums unterworfen. Die Verfahrensweise ist z.B. bei H. Frieser "Die Grundlagen der Photographischen Prozesse mit Silberhalogeniden" Seite 675-734, Akademische Verlagsgesellschaft (1968) beschrieben.

Dabei kann die chemische Sensibilisierung unter Zusatz von Verbindungen von Schwefel, Selen, Tellur und/oder Verbindungen der Metalle der VIII. Nebengruppe des Periodensystems (z.B. Gold, Platin, Palladium, Iridium) erfolgen, weiterhin können Thiocyanatverbindungen, oberflächenaktive Verbindungen, wie Thioether, heterocyclische Stickstoffverbindungen (z.B. Imidazole, Azaindene) oder auch spektrale Sensibilisatoren (beschrieben z.B. bei F. Hamer "The Cyanine Dyes and Related Compounds", 1964, bzw. Ullmanns Encyclopädie der technischen Chemie, 4. Auflage, Bd. 18, S. 431ff, und Research Disclosure 17643 (Dez. 1978), Kapitel III) zugegeben werden. Ersatzweise oder zusätzlich kann eine Reduktionssensibilisierung unter Zugabe von Reduktionsmitteln (Zinn-II-Salze, Amine, Hydrazinderivate, Aminoborane, Silane, Formamidinsulfinsäure) durch Wasserstoff, durch niedrigen pAg (z.B. kleiner 5) und/oder hohen pH (z.B. über 8) durchgeführt werden.

Die fotografischen Emulsionen können Verbindungen zur Verhinderung der Schleierbildung oder zur Stabilisierung der fotografischen Funktion während der Produktion, der Lagerung oder der fotografischen Verarbeitung enthalten.

Besonders geeignet sind Azaindene, vorzugsweise Tetra- und Pentaazaindene, insbesondere solche, die mit Hydroxyl- oder Aminogruppen substituiert sind. Derartige Verbindungen sind z.B. von Birr, Z. Wiss. Phot. 47 (1952), S. 2-58 beschrieben worden. Weiter können als Antischleiermittel Salze von Metallen wie Quecksilber oder Cadmium, aromatische Sulfon- oder Sulfinsäure wie Benzolsulfinsäure, oder stickstoffhaltige Heterocyclen wie Nitrobenzimidazol, Nitroindazol, gegebenenfalls subsitutierte Benztriazole oder Benzthiazoliumsalze eingesetzt werden. Besonders geeignet sind Mercaptogruppen enthaltende Heterocyclen, z.B. Mercaptobenzthiazole, Mercaptobenzimidazole, Mercaptotetrazole, Mercaptothiadiazole, Mercaptopyrimidine, wobei diese Mercaptoazole auch eine wasserlöslichmachende Gruppe, z.B. eine Carboxylgruppe oder Sulfogruppe, enthalten können. Weitere geeignete Verbindungen sind in Research Disclosure 17643 (Dez. 1978), Kapitel VI, veröffentlicht.

Die Stabilisatoren können den Silberhalogenidemulsionen vor, während oder nach deren Reifung zugesetzt werden. Selbstverständlich kann man die Verbindungen auch anderen fotografischen Schichten, die einer Halogensilberschicht zugeordnet sind, zusetzen.

Es können auch Mischungen aus zwei oder mehreren der genannten Verbindungen eingesetzt werden.

Die fotografischen Emulsionsschichten oder andere hydrophile Kolloidschichten des erfindungsgemäß hergestellten lichtempfindlichen Materials können oberflächenaktive Mittel für verschiedene Zwecke enthalten, wie Überzügshilfen, zur Verhinderung der elektrischen Aufladung, zur Verbesserung der Gleiteigenschaften, zum Emulgieren der Dispersion, zur Verhinderung der Adhäsion und zur Verbesserung der fotografischen Charakteristika (z.B. Entwicklungsbeschleunigung, hoher Kontrast, Sensibilisierung usw.). Neben natürlichen oberflächenaktiven Verbindungen, z.B. Saponin, finden hauptsächlich synthetische oberflächenaktive Verbindungen (Tenside) Verwendung; nicht-ionische Tenside, z.B. Alkylenoxidverbindungen, Glycerinverbindungen oder Glycidolverbindungen, kationische Tenside, z.B. höhere Alkylamine, quartäre Ammoniumsalze, Pyridinverbindungen und andere heterocyclische Verbindungen, Sulfoniumverbindungen oder Phosphoniumverbindungen, anionische Tenside, enthaltend eine Säuregruppe, z.B. Carbonsäure-, Sulfonsäure-, eine Phosphorsäure-, Schwefelsäureester- oder Phosphorsäureestergruppe, ampholytische Tenside, z.B. Aminosäure- und Aminosulfonsäureverbindungen sowie Schwefel- oder Phosphorsäureester eines Aminoalkohols.

Die fotografischen Emulsionen können unter Verwendung von Methinfarbstoffen oder anderen Farbstoffen spektral sensibilisiert werden. Besonders geeignete Farbstoffe sind Cyaninfarbstoffe, Merocyaninfarbstoffe und komplexe Merocyaninfarbstoffe.

Eine Übersicht über die als Spektralsensibilisatoren geeigneten Polymethinfarbstoffe, deren geeignete Kombinationen und supersensibilisierend wirkenden Kombinationen enthält Research Disclosure 17643 (Dez. 1978), Kapitel IV.

Insbesondere sind die folgenden Farbstoffe - geordnet nach Spekiralgebieten - geeignet:

1. als Rotsensibilisatoren

9-Ethylcarbocyanine mit Benzthiazol, Benzselenazol oder Naphthothiazol als basische Endgruppen, die in 5- und/oder 6-Stellung durch Halogen, Methyl, Methoxy, Carbalkoxy, Aryl substituiert sein können sowie 9-Ethyl-naphthoxathia- bzw. -selencarbocyanine und 9-Ethyl-naphthothiazoxa- bzw. -benzimidazocarbocyanine, vorausgesetzt, daß die Farbstoffe mindestens eine Sulfoalkylgruppe am heterocyclischen Stickstoff tragen.

2. als Grünsensibilisatoren
9-Ethylcarbocyanine mit Benzoxazol, Naphthoxazol oder einem Benzoxazol und einem Benzthiazol als basische Endgruppen sowie Benzimidazocarbocyanine, die ebenfalls weiter substituiert sein können und ebenfalls mindestens eine Sulfoalkylgruppe am heterocyclischen Stickstoff enthalten müssen.

3. als Blausensibilisatoren
symmetrische oder asymmetrische Benzimidazo-, Oxa-, Thia- oder Selenacyanine mit mindestens einer Sulfoalkylgruppe am heterocyclischen Stickstoff und gegebenenfalls weiteren Substituenten am aromatischen Kern, sowie Apomerocyanine mit einer Rhodaningruppe.

Als Beispiele seinen, insbesondere für Negativ- und Umkehrfilm, die nachfolgend aufgeführten Rotsensibilisatoren RS, Grünsensibilisatoren GS und Blausensibilisatoren BS genannt, die jeweils einzeln oder in Kombination untereinander eingesetzt werden können, z.B. RS-1 und RS-2, sowie GS-1 und GS-2.

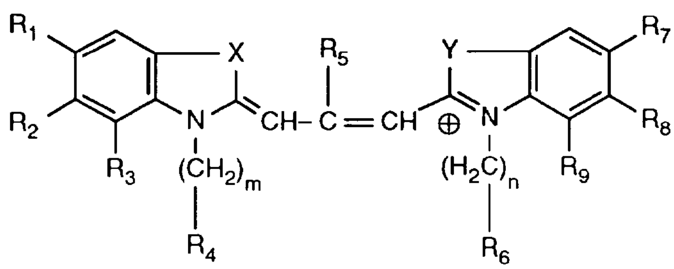

RS-1: $R_1$, $R_3$, $R_7$, $R_9$ = H; $R_2$, $R_8$ = Cl;
$R_4$ = $-SO_3^{\ominus}{}^{\oplus}NH(C_2H_5)_3$; $R_5$ = $-C_2H_5$; $R_6$ = $-SO_3^{\ominus}$;
m, n = 3; X, Y = S;

RS-2: $R_1$, $R_3$, $R_9$ = H; $R_2$ = Phenyl;

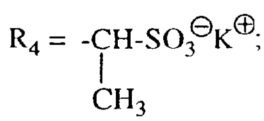

$R_5$=$C_2H_5$; $R_6$=$-SO_3^{\ominus}$; $R_7$, $R_8$=$-OCH_3$; m=2; n=3; X=O; Y=S;

RS-3: $R_1$, $R_9$ = H; $R_2$, $R_3$ zusammen -CH=CH-CH=CH-;
$R_4$=$-SO_3^{\ominus}Na^{\oplus}$; $R_5$=$-C_2H_5$; $R_6$=$-SO_3^{\ominus}$; $R_7$, $R_8$=Cl;
m, n = 3; X = S; Y = N-$C_2H_5$;

RS-4: $R_1$ = $-OCH_3$; $R_2$, $R_8$ = $-CH_3$; $R_3$, $R_4$, $R_7$, $R_9$ = H;
$R_5$ = $-C_2H_5$; $R_6$ = $-SO_3^{\ominus}$; m = 2; n = 4; X = S; Y = Se;

RS-5: $R_1$, $R_7$ = H; $R_2$, $R_3$ sowie $R_8$, $R_9$ zusammen -CH=CH-CH=CH-; $R_4$ =$-SO_3^{\ominus}{}^{\oplus}NH(c_2H_5)_3$; $R_5$ = $C_2H_5$;
$R_6$ = $SO_3^{\ominus}$; m = 2; n = 3; X, Y = S;

GS-1: $R_1$, $R_3$, $R_7$, $R_9$ = H; $R_2$ = Phenyl;

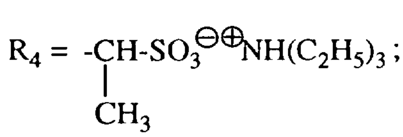

$R_5$ = $-C_2H_5$; $R_6$ = $-SO_3^{\ominus}$;
$R_8$ = Cl; m = 2; m = 3; X, Y = O;

GS-2: $R_1$, $R_2$, $R_7$ ,$R_8$ = Cl; $R_3$, $R_5$, $R_6$, $R_9$ = H;

$R_4$ = 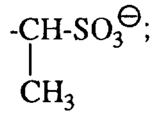

m, n = 2; X, Y = N-$C_2H_5$;

GS-3: $R_1$, $R_7$ = H; $R_2$, $R_3$ sowie $R_8$, $R_9$ zusammen -CH=CH-CH=CH-; $R_4$ = $SO_3^{\ominus}Na^{\oplus}$;
$R_5$ = $C_2H_5$;
$R_6$ = $SO_3^{\ominus}$; m, n=3; X, Y=O;

GS-4: $R_1$, $R_3$, $R_4$, $R_7$, $R_8$, $R_9$ = H; $R_2$ = -$OCH_3$; $R_5$ = -$C_2H_5$;
$R_6$ = $SO_3^{\ominus}$; m=2; n=4; X=O; Y=S;

BS-1:

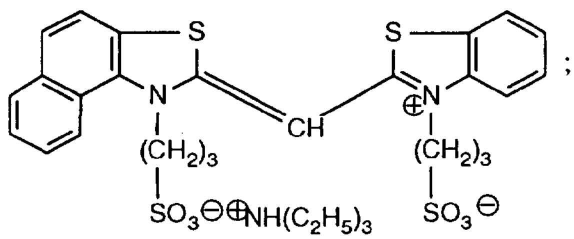

BS-2:

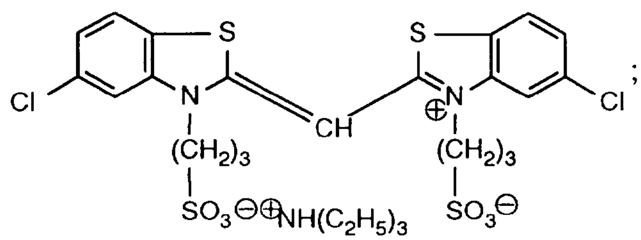

sowie

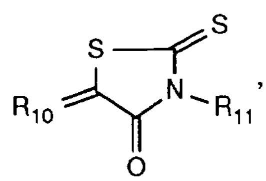

worin in

BS-3:

$R_{10}$ = 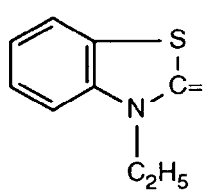 ; $R_{11}$ = -$CH_2$-COOH;

BS-4:

$R_{10}$ = 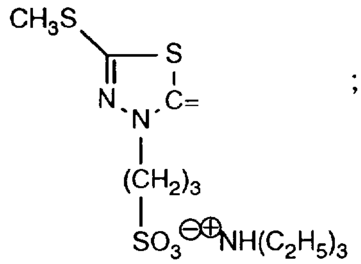 ; $R_{11}$ = $-C_2H_5$;

BS-5:

$R_{10}$ = 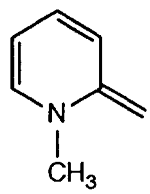 ; $R_{11}$ = $-C_2H_5$.

Auf Sensibilisatoren kann verzichtet werden, wenn für einen bestimmten Spektralbereich die Eigenempfindlichkeit des Silberhalogenids ausreichend ist, beispielsweise die Blauempfindlichkeit von Silberbromiden.

Den unterschiedlich sensibilisierten Emulsionsschichten werden nicht diffundierende monomere oder polymere Farbkuppler zugeordnet, die sich in der gleichen Schicht oder in einer dazu benachbarten Schicht befinden können. Gewöhnlich werden den rotempfindlichen Schichten Cyankuppler, den grünempfindlichen Schichten Magentakuppler und den blauempfindlichen Schichten Gelbkuppler zugeordnet.

Im erfindungsgemäßen Material verwendbare Gelbkuppler sind vorzugsweise Verbindungen der Formel A

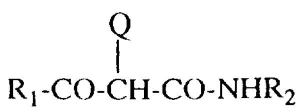 (A),

worin $R_1$ Alkyl oder Aryl ist, $R_2$ Aryl ist und Q Wasserstoff oder eine Gruppe ist, die durch Reaktion mit dem oxidierten Entwickler abgespaltet werden kann.

Eine Gruppe von Gelbkupplern sind solche Verbindungen der Formel A, in denen $R_1$ t-Butyl ist und $R_2$ eine Gruppe der Formel

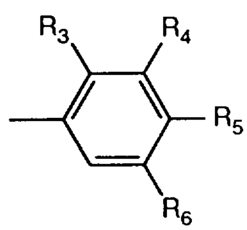

ist, worin $R_3$ Wasserstoff, Halogen, Alkyl oder Alkoxy bedeutet und $R_4$, $R_5$ und $R_6$ Wasserstoff, Halogen, Alkyl, Alkenyl, Alkoxy, Aryl, Carboxy, Alkoxycarbonyl, eine Carbamoylgruppe, eine Sulfon- oder Sulfamoylgruppe, eine Alkylsulfonaminogruppe, Acylaminogruppe, Ureidogruppe oder Aminogruppe bedeuten.

Vorzugsweise sind $R_3$ Chlor oder Methoxy, $R_4$ und $R_5$ Wasserstoff und $R_6$ eine Acylaminogruppe. Hierzu gehören auch die Verbindungen der Formel

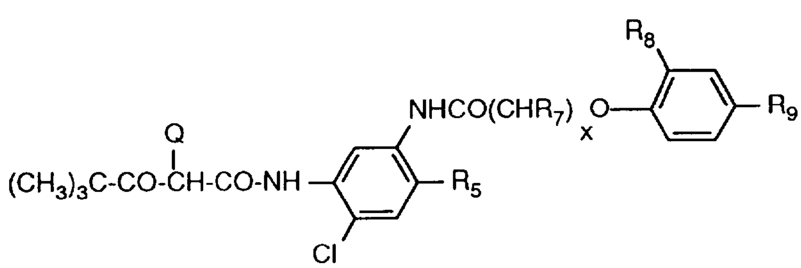 ,

worin x O-4 ist, $R_7$ Wasserstoff oder Alkyl bedeutet und $R_8$ und $R_9$ Alkyl sind.

Eine andere Gruppe von Gelbkupplern entspricht der Formel B

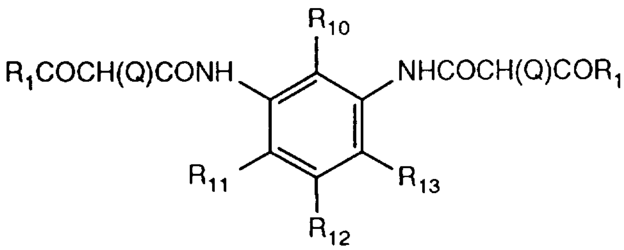

(B),

worin $R_{10}$ Wasserstoff, Halogen oder Alkoxy ist, $R_{11}$, $R_{12}$ und $R_{13}$ Wasserstoff, Halogen, Alkyl, Alkenyl, Alkoxy, Aryl, Carboxyl, Alkoxycarbonyl, eine Carbamoylgruppe, eine Sulfongruppe, Sulfamoylgruppe, Sulfonamidogruppe, Acylaminogruppe, Ureidogruppe oder Aminogruppe bedeuten und $R_1$ und Q die oben angegebene Bedeutung haben.

Dazu gehören Verbindungen der Formel B, in denen $R_1$ t-Butyl ist, $R_{10}$ Chlor ist, $R_{11}$ und $R_{13}$ Wasserstoff sind und $R_{12}$ Alkoxycarbonyl ist.

In den Verbindungen der Formel A und B kann die Abgangsgruppe Q Wasserstoff (4-Äquivalentkuppler) sein oder sie ist eine heterocyclische Gruppe (2-Äquivalentkuppler)

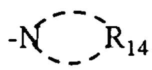 ,

worin $R_{14}$ eine organische zweiwertige Gruppe ist, die den Ring zu einem 4-7-gliedrigen Ring ergänzt, oder Q ist eine Gruppe $-OR_{15}$, worin $R_{15}$ Alkyl, Aryl, Acyl oder ein heterocyclischer Rest ist.

Typische Beispiele für gebräuchliche Gelbkuppler sind die Verbindungen der folgenden Formeln (1 und 2):

1) 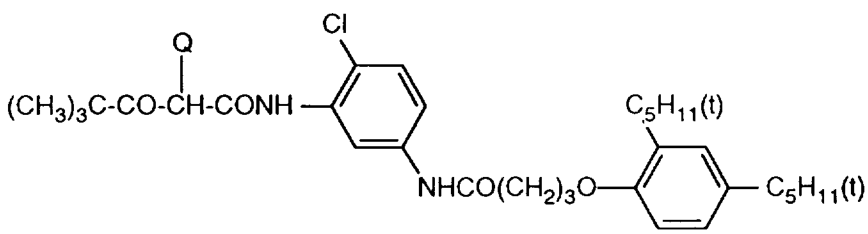 ,

worin

a) Q = 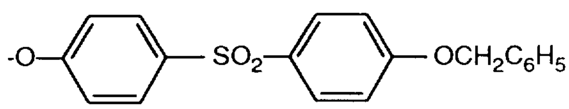

b) Q = 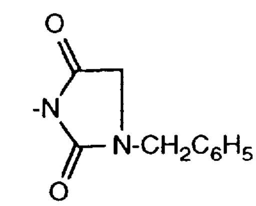

c) Q = 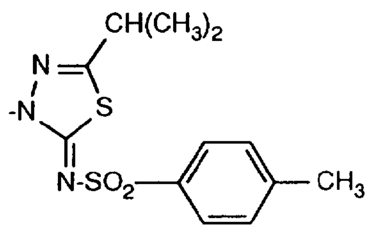

d) Q = 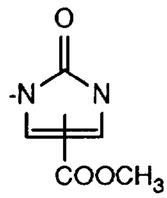

e) Q = 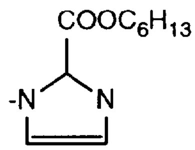

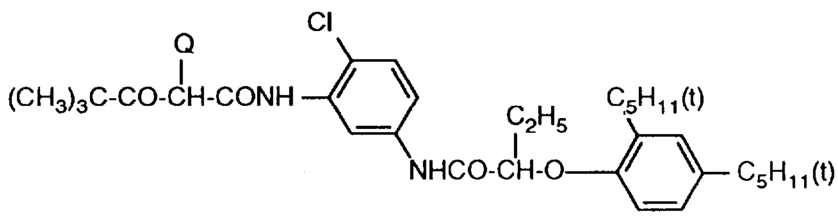

f) Q = 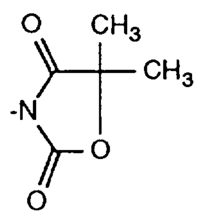

g) Q = 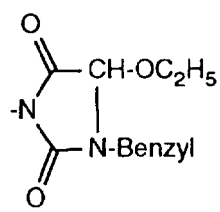

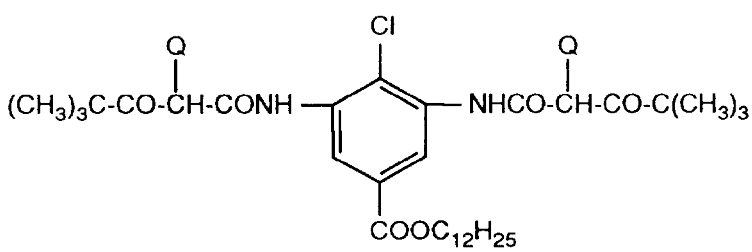

h) Q = 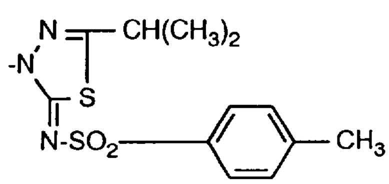 ;

2) 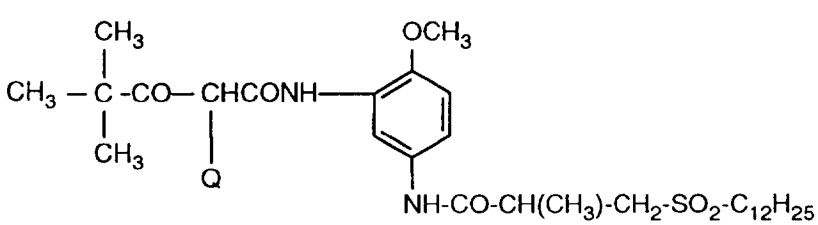 ,

worin

i) Q = 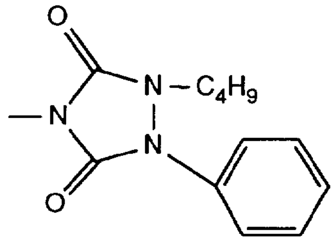 .

Weitere Beispiele für Gelbkuppler sind zu finden in den US-A 2,407,210, 2,778,658, 2,875,057, 2,908,513, 2,908,573, 3,227,155, 3,227,550, 3,253,924, 3,265,506, 3,277,155, 3,408,194, 3,341,331, 3,369,895, 3,384,657, 3,415,652, 3,447,928, 3,551,155, 3,582,322, 3,725,072, 3,891,445, 3,933,501, 4,115,121, 4,401,752 und 4,022,620, 5,118,599, 5,215,878, 5,260,182, 5,294,527, 5,298,383, 5,300,412, 5,306,609, 5,314,797, 5,336,591 in den DE-A 1,547,868, 2,057,941, 2,162,899, 2,163,813, 2,213,461, 2,219,917, 2,261,361, 2,261,362, 2,263,875, 2,329,587, 2,414,006 und 2,422,812, in den GB-A 1,425,020 und 1,077,874 und in JP-A-88/123,047, 4,133,052, 5,080,469, 5,313,323 und in EP-A-447,969, 447,920, 508,398, 510,535, 542,463, 568,198.

Die Gelbkuppler werden üblicherweise in einer Menge von 0,05-2 Mol und vorzugsweise 0,1-1 Mol pro Mol Silberhalogenid verwendet.

Typische und bevorzugte Gelbkuppler entsprechen den Formeln:

(Y-1) 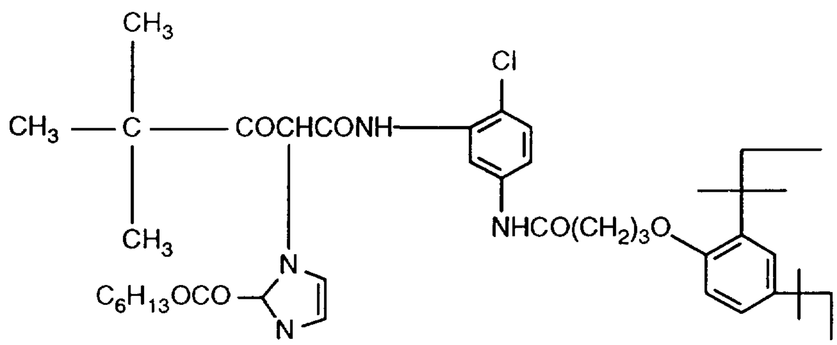

(Y-2) 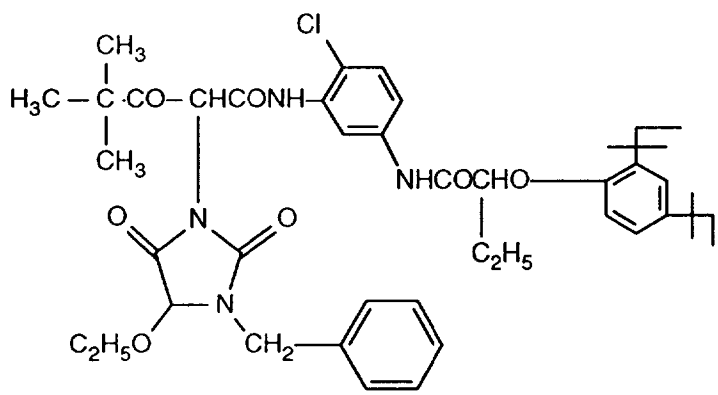

(Y-3)

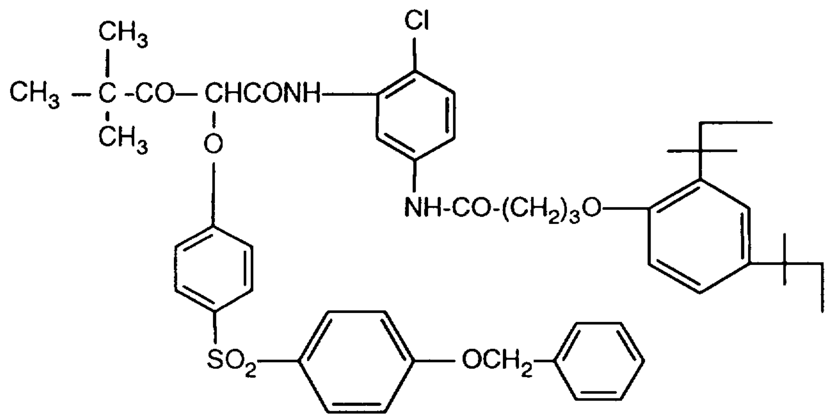

(Y-4)

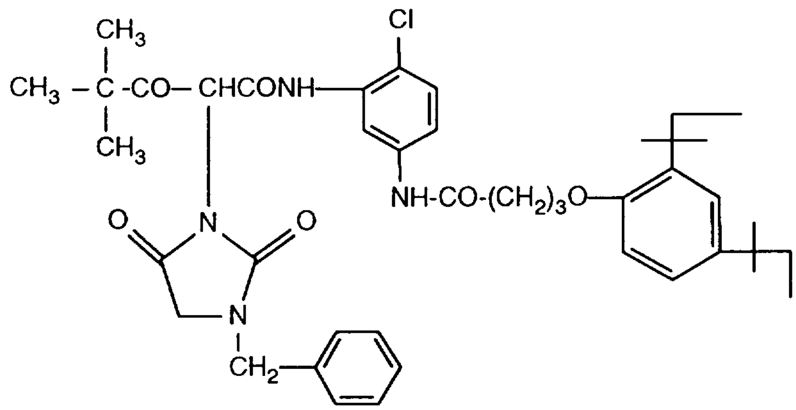

(Y-5)

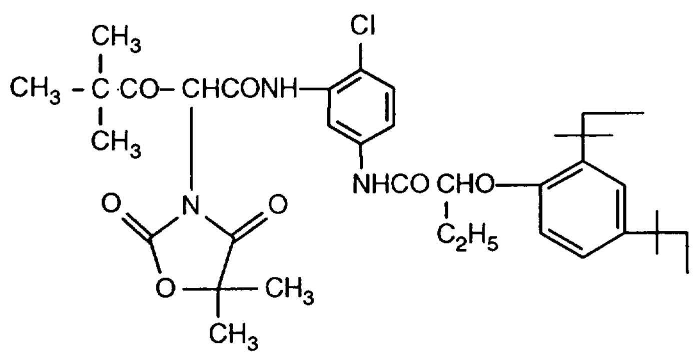

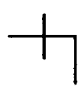 = $-C(CH_3)_2C_2H_5$ = $-C_5H_{11}(t)$

(Y-6)

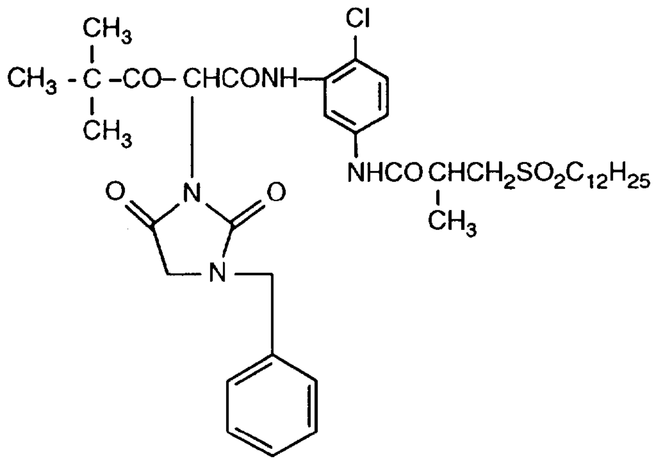

(Y-7)

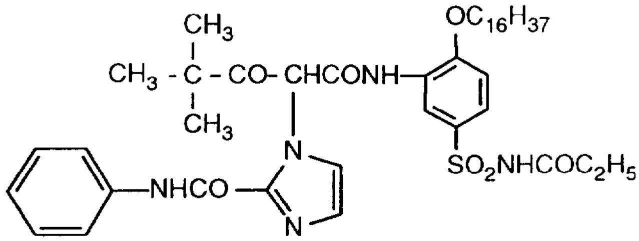

(Y-8)

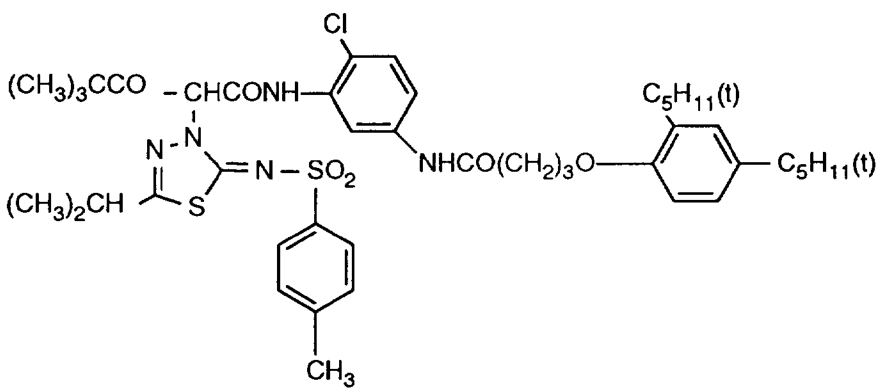

(Y-9)

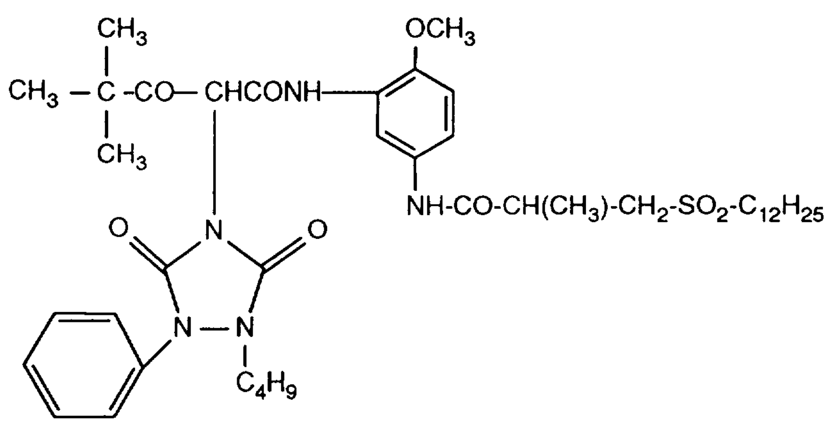

(Y-10)

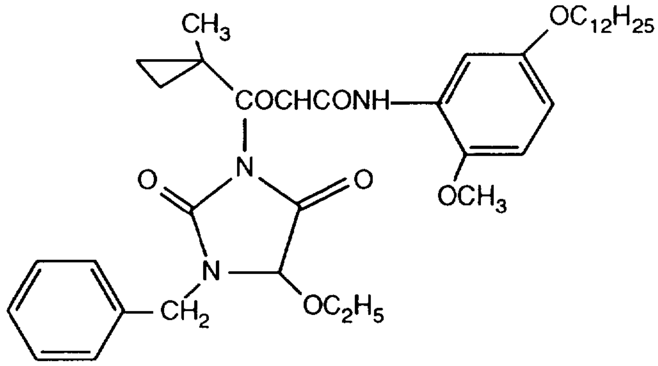

(Y-11)

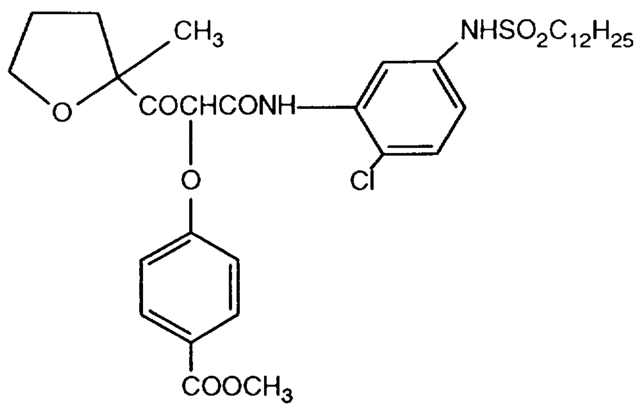

(Y-12)

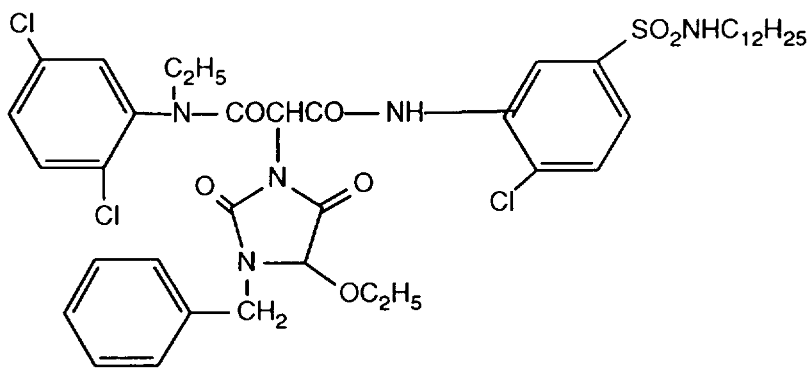

(Y-13)

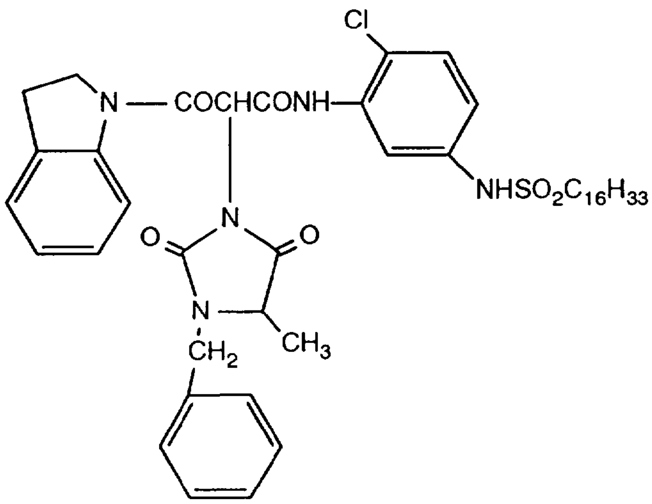

(Y-14)

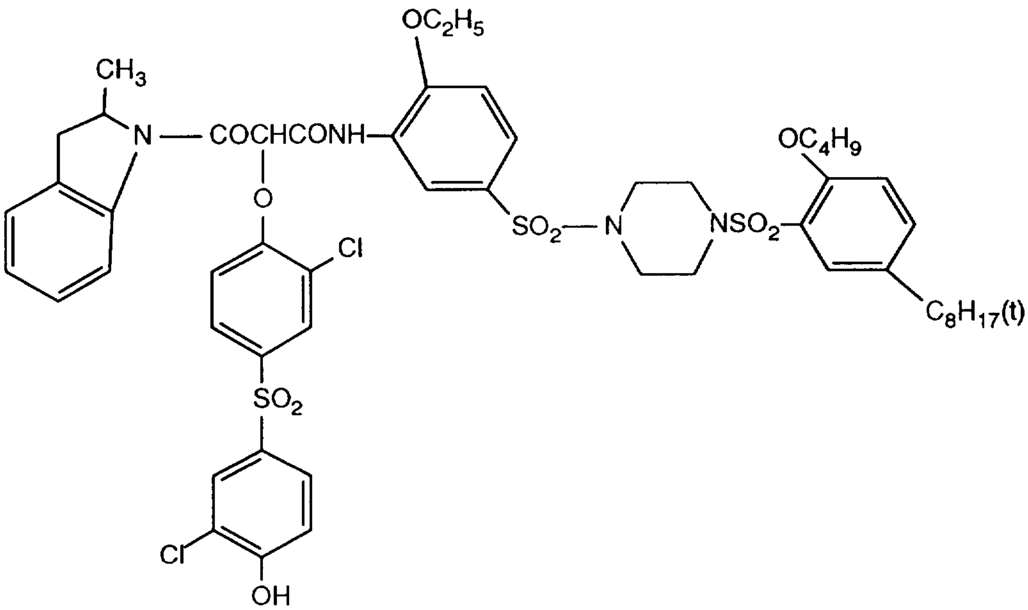

(Y-15)

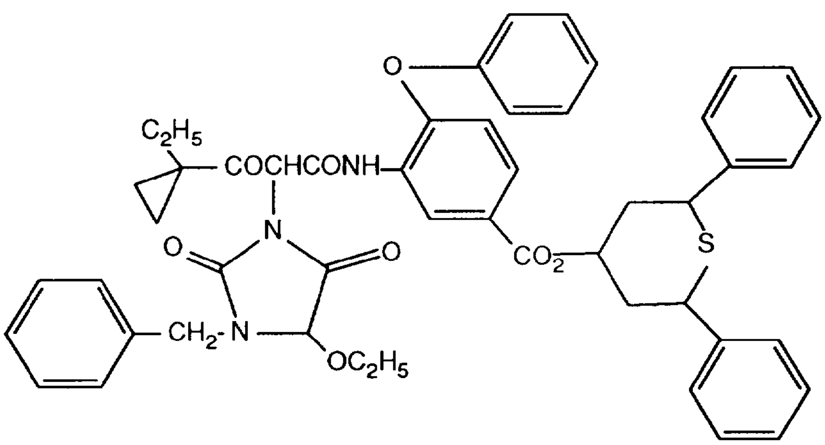

(Y-16)

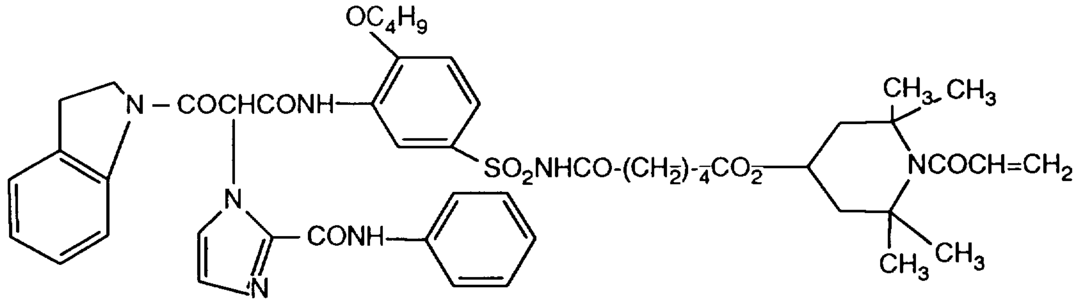

(Y-17)

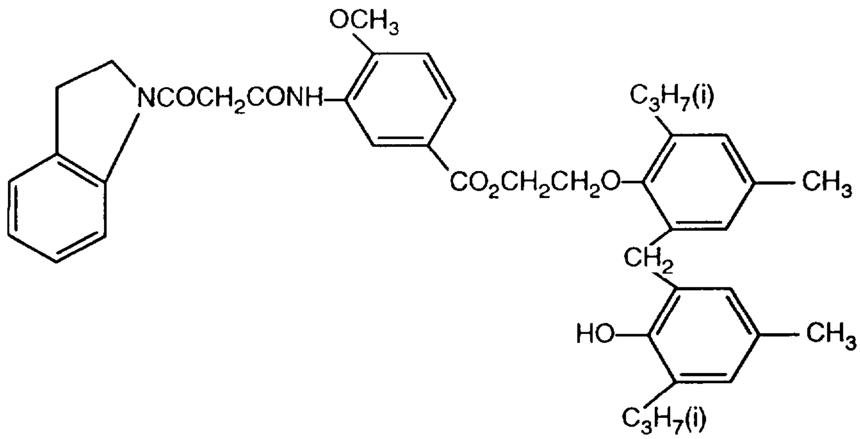

(Y-18)

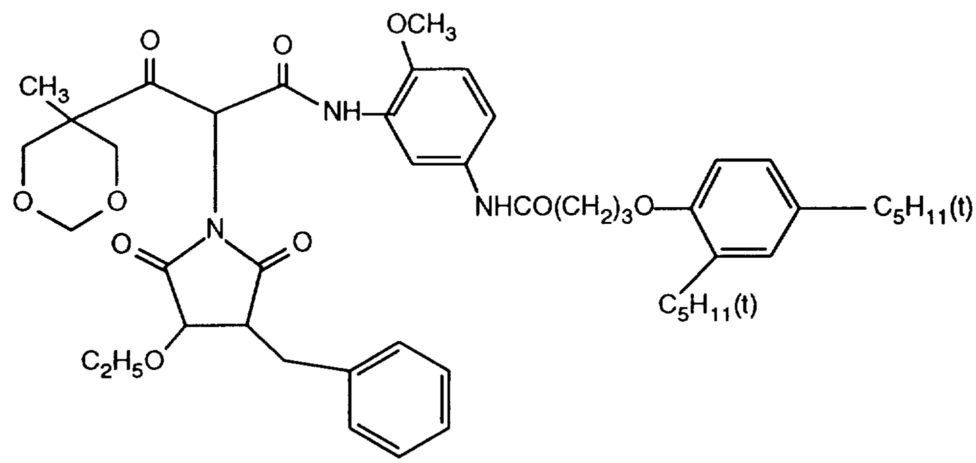

(Y-19)

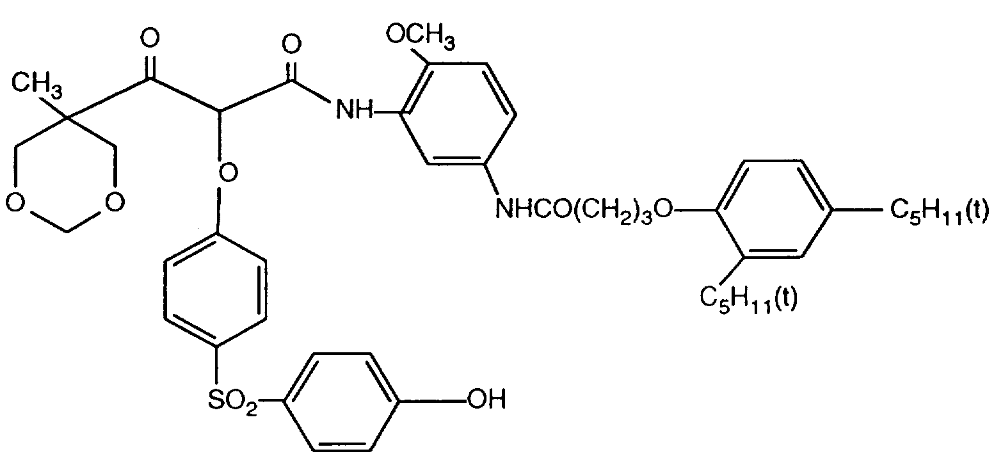

Magentakuppler können z.B. einfache 1-Aryl-5-pyrazolone sein oder mit 5-gliedrigen Heteroringen kondensierte Pyrazolderivate wie z.B. Imidazopyrazole, Pyrazolopyrazole, Pyrazolotriazole oder Pyrazolotetrazole.

Eine Gruppe von Magentakupplern sind 5-Pyrazolone der Formel C,

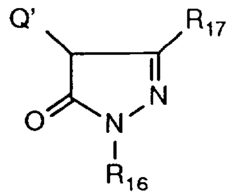

(C),

wie sie in der Britischen Patentschrift 2,003,473 beschrieben sind. Darin ist $R_{16}$ Wasserstoff, Alkyl, Aryl, Alkenyl oder eine heterocyclische Gruppe. $R_{17}$ ist Wasserstoff, Alkyl, Aryl, eine heterocyclische Gruppe, eine Estergruppe, Alkoxygruppe, Alkylthiogruppe, Carboxylgruppe, Arylaminogruppe, Acylaminogruppe, (Thio)-harnstoffgruppe, (Thio)-carbamoylgruppe, Guanidinogruppe oder Sulfonamidogruppe.

Bevorzugt ist $R_{17}$ eine Gruppe

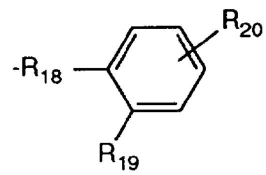

,

worin $R_{18}$ Imino, Acylamino oder Ureido ist, $R_{19}$ Wasserstoff, Halogen, Alkyl oder Alkoxy ist, $R_{20}$ Wasserstoff, Alkyl, Acylamino, Carbamoyl, Sulfamoyl, Sulfonamido, Alkoxycarbonyl, Acyloxy oder eine Urethangruppe ist.

Wenn Q' Wasserstoff ist, so ist der Magentakuppler tetraäquivalent in bezug auf das Silberhalogenid.

Typische Beispiele für diesen Typ von Magentakupplern sind Verbindungen der Formel

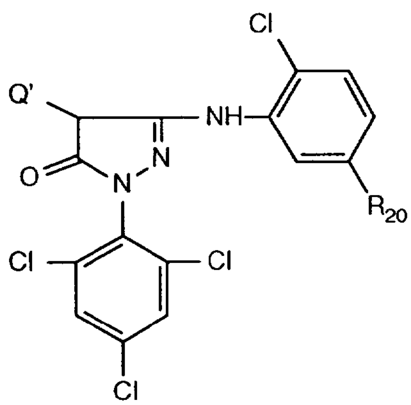

,

worin $R_{20}$ die oben genannten Bedeutungen hat, und Q', wie oben beschrieben, eine Abgangsgruppe ist. Diese Verbindungen liegen bevorzugt im erfindungsgemäßen Material vor.

Weitere Beispiele solcher tetraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866 und 3,933,500 und in JP-A-89/309,058.

Wenn Q' in Formel C nicht Wasserstoff ist sondern eine Gruppe, die bei der Reaktion mit dem oxidierten Entwickler eliminiert wird, so handelt es sich um einen diäquivalenten Magentakuppler. Q kann in diesem Fall z.B. Halogen oder eine über O, S oder N an den Pyrazolring gebundenen Gruppe sein. Solche diäquivalenten Kuppler ergeben eine höhere Farbdichte und sind reaktiver gegenüber dem oxidierten Entwickler als die entsprechenden tetraäquivalenten Magentakuppler.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, 3,262,292. in den EP-A-133,503, 529,784, 530,039, DE-A-2,944,601, JP-A-78/34044, 74/53435, 74/53436, 75/53372 und 75/122935, 3,323,851, 4,018,547, 5,150,429, und WO 93/02392

Typische und bevorzugte Magentakuppler entsprechen der Formeln

(M-1)

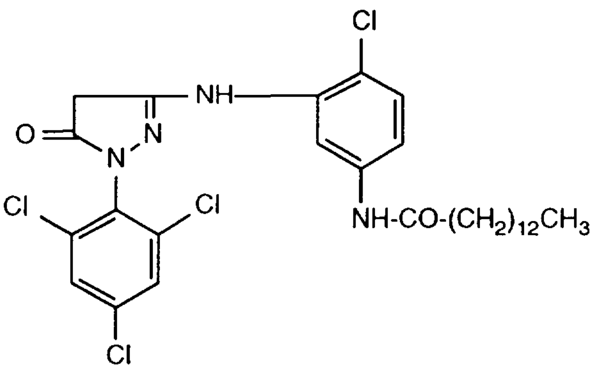

(M-2)

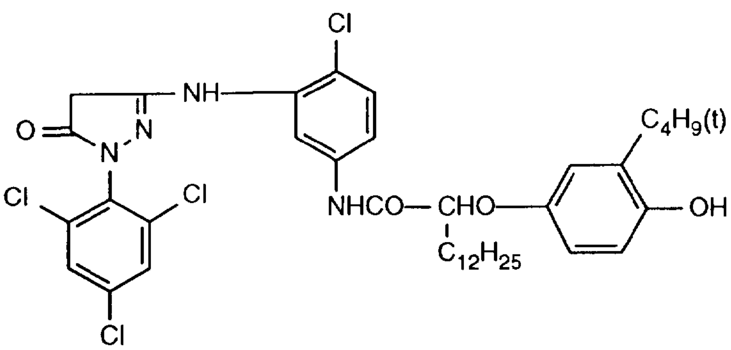

(M-3)

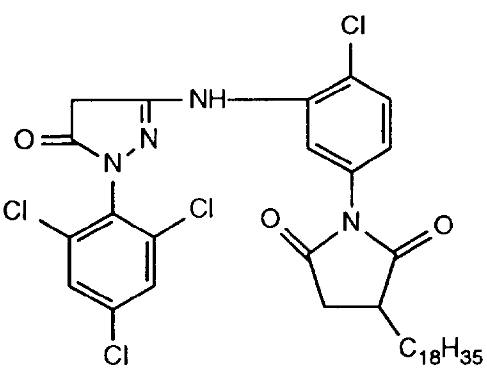

(M-4)

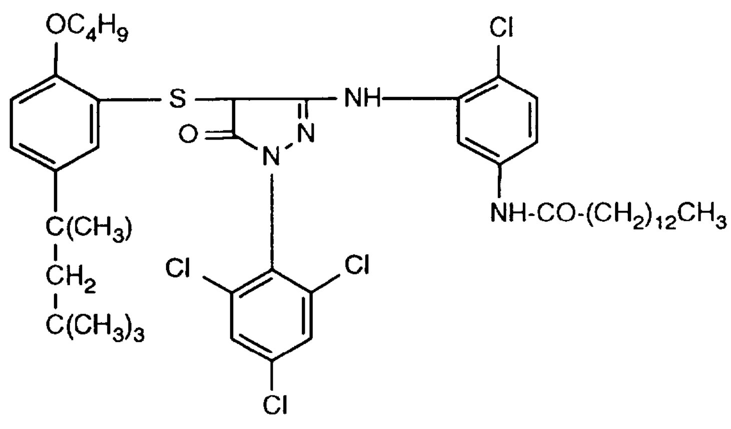

Ueber ein zweiwertiges Q' können 2 Pyrazolonringe verknüpft werden und man erhält dann sogenannte Bis-Kuppler. Solche sind z.B. beschrieben in den US-A-2,632,702, US-A-2,618,864, GB-A-968,461, GB-A-786,859, JP-A-76/37646, 59/4086, 69/16110, 69/26589, 74/37854 und 74/29638. Bevorzugt ist Y eine O-Alkoxyarylthio-Gruppe.

Wie vorstehend erwähnt, können als Magentakuppler auch mit 5-gliedrigen Heterocyclen kondensierte Pyrazole -

sogenannte Pyrazoloazole - verwendet werden. Deren Vorteile gegenüber einfachen Pyrazolen ist, daß sie Farben von größerer Formalin-Beständigkeit und reineren Absorptionsspektren aufweisen.

Magentakuppler vom Pyrazoloazoltyp, welche ebenfalls bevorzugt sind, können durch die Formeln D

(D) 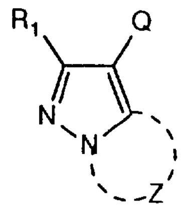

dargestellt werden, worin $R_1$ Wasserstoff oder ein Substituent ist, Z die zur Vervollständigung eines 5-gliedrigen Ringes mit 2 oder 3 Stickstoffatomen notwendigen nichtmetallischen Atome darstellt, wobei dieser Ring substituiert sein kann, und Q Wasserstoff (4-Äquivalentkuppler) oder eine Abgangsgruppe (2-Äquivalentkuppler) ist.

Bevorzugt hiervon sind Magentakuppler der Formeln

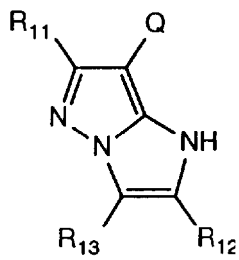

(D-1)

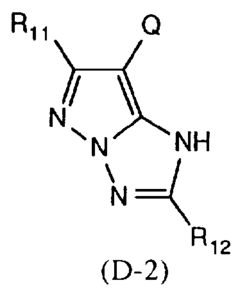

(D-2)

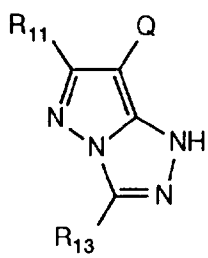

(D-3)

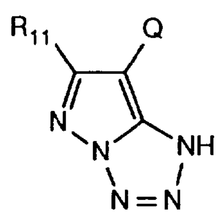

(D-4)

$R_{11}$, $R_{12}$ und $R_{13}$ bedeuten unabhängig voneinander beispielsweise Wasserstoff, Halogen (z.B. Chlor, Brom), eine Grup-

pe der Formel $-CR_3$, worin die Reste R unabhängig voneinander Wasserstoff oder Alkyl sind, Aralkyl, Alkenyl, Alkinyl, Cycloalkyl oder Cycloalkenyl und besonders bevorzugt Methyl, Ethyl, Propyl, Isopropyl, t-Butyl, Tridecyl, 2-Methansulfonylethyl, 3-(3-Pentadecylphenoxy)propyl, 3-(4-(2-(4-(4-Hydroxyphenylsulfonyl)phenoxy)dodecanamido)phenyl)propyl, 2-Ethoxytridecyl, Trifluoromethyl, Cyclopentyl, 3-(2,4-Di-t-Amylphenoxy)propyl); Aryl (z.B. Phenyl, 4-t-Butylphenyl, 2,4-Di-t-amylphenyl, 4-Tetradecaneamidophenyl); Heterocyclyl (z.B. 2-Furyl, 2-Thienyl, 2-Pyrimidinyl, 2-Benzothiazolyl); Cyano; Hydroxy, Nitro; Carboxy; Amino; Alkoxy (z.B. Methoxy, Ethoxy, 2-Methoxyethoxy; 2-Dodecylethoxy, 2-Methansulfonylethoxy); Aryloxy (z.B. Phenoxy, 2-Methylphenoxy, 4-t-Butylphenoxy, 3-Nitrophenoxy, 3-t-Butyloxycarbamoylphenoxy, 3-Methoxycarbamoyl); Acylamino (z.B. Acetoamido, Benzamido, Tetradecanamido, 2-(2,4-Di-t-amylphenoxy)butanamido, 4-(3-t-Butyl-4-hydroxyphenoxy)butanamido, 2-(4-(4-Hydroxyphenylsulfonyl)phenoxy)decanamido); Methylbutylamino); Anilino (z.B. Phenylamino, 2-Chloranilino, 2-Chloro-5-tetradecanaminoanilino, 2-Chloro-5-dodecyloxycarbonylanilino, N-Acetylanilino, 2-Chloro-5-(alpha-(3-t-butyl-4-hydroxyphenoxy)dodecanamidoanilino); Ureido (z.B. Phenylureido, Methylureido, N,N-Dibutylureido); Sulfamoylamino (z.B. N,N-Dipropylsulfamoylamino, N-Methyl-N-decylsulfamoylamino); Alkylthio (z.B. Methylthio, Octylthio, Tetradecylthio, 2-Phenoxyethylthio, 3-Phenoxypropylthio, 3-(4-t-Butylphenoxy)-propylthio); Arylthio (z.B. Phenylthio, 2-Butoxy-5-t-octylphenylthio, 3-Pentadecylphenylthio, 2-Carboxyphenylthio, 4-Tetradecanamidophenylthio); Alkoxycarbonylamino (z.B. Methoxycarbonylamino, Tetradecyloxycarbonylamino); Sulfonamido (z.B. Methansulfonamido, Hexadecansulfonamido, Benzolsulfonamido, p-Toluolsulfonamido, Octadecansulfonamido, 2-Methyloxy-5-t-butylbenzolsulfonamido); Carbamoyl (z.B. N-Ethylcarbamoyl, N,N-Dibutylcarbamoyl, N-(2-Dodecyloxyethyl)-carbamoyl, N-Methyl-N-dodecylcarbamoyl, N-(3-(2,4-Di-t-Amylphenoxy)propyl)-carbamoyl); Sulfamoyl (z.B. N-Ethylsulfamoyl, N,N-Dipropylsulfamoyl, N-2(-Dodecyloxyethyl)-sulfamoyl, N-Ethyl-N-dodecylsulfamoyl, N,N-Diethylsulfamoyl); Sulfonyl (z.B. Methansulfonyl, Octansulfonyl, Benzolsulfonyl, Toluolsulfonyl); Alkoxycarbonyl (z.B. Methoxycarbonyl, Butoxycarbonyl, Dodecyloxycarbonyl, Octadecyloxycarbonyl); heterocyclische Ringoxy (z.B. 1-Phenyltetrazol-5-oxy, 2-Tetrahydropyranyloxy); Azo (z.B. Phenylazo, 4-Methoxyphenylazo, 4-Pivaloylaminophenylazo, 2-Hydroxy-4-propanoylphenylazo); Acyloxy (z.B. Acetoxy); Carbamoyloxy (z.B. N-Methylcarbamoyloxy, N-Phenylcarbamoyloxy); Silyloxy (z.B. Trimethylsilyloxy, Dibutylmethylsilyloxy); Aryloxycarbonylamino (z.B. Phenoxycarbonylamino); Imido (z.B. N-Succinimido, N-Phthalimido, 3-Octadecenylsuccinimido); heterocyclische Ring-thio (z.B. 2-Benzothiazolylthio, 2,4-Diphenyioxy-1,3,5-triazol-6-thio, 2-Pyridylthio); Sulfinyl (z.B. Dodecansulfinyl, 3-Pentadecylphenylsulfinyl, 3-Phenoxyprupylsulfinyl); Phosphonyl (z.B. Phenoxyphosphonyl, Octyloxyphosphonyl, Phenylphosphonyl); Aryloxycarbonyl (z.B. Phenoxycarbonyl); Acyl (z.B. Acetyl, 3-Phenylpropanoyl, Benzoyl, 4-Dodecyloxybenzoyl); Azolyl (z.B. Imidazolyl, Pyrazolyl, 3-Chloro-pyrazol-1-yl).

Diese Substituenten sind gegebenenfalls weiter substituiert, beispielsweise durch Halogen oder durch einen über ein C-, O-, N- oder S-Atom gebundenen organischen Rest.

Die bevorzugten Gruppen $R_{11}$ sind Alkyl, Aryl, Alkoxy, Aryloxy, Alkylthio, Ureido, Urethan und Acylaminogruppen.

$R_{12}$ kann die Bedeutung von $R_{11}$ besitzen und ist vorzugsweise Wasserstoff, Alkyl, Aryl, ein heterocyclischer Ring, Alkoxycarbonyl, Carbamoyl, Sulfamoyl, Sulfinyl, Acyl oder Cyano.

$R_{13}$ kann die Bedeutung von $R_{11}$ haben und ist vorzugsweise Wasserstoff, Alkyl, Aryl, Heterocyclic, Alkoxy, Aryloxy, Alkylthio, Arylthio, Alkoxycarbonyl, Carbamoyl oder Acyl, besonders Alkyl, Aryl, Heterocyclic, Alkylthio oder Arylthio.

Q ist Wasserstoff oder eine Abgangsgruppe wie Halogen, Alkoxy, Aryloxy, Acyloxy, Alkyl- oder Arylsulfonyloxy, Acylamino, Alkyl- oder Aryl-sulfonamido, Alkoxycarbonyloxy, Aryloxycarbonyloxy, Alkyl-, Aryl- oder Heterocyclyl-S-Carbamoylamino, ein 5- oder 6-gliedriger stickstoffhaltiger heterocyclischer Rest, Imido und Arylazo. Diese Gruppen sind gegebenenfalls wie für $R_{11}$ gezeigt weiter substituiert.

Vorzugsweise ist Q Halogen (z.B. Fluor, Chlor, Brom); Alkoxy (z.B. Ethoxy, Dodecyloxy, Methoxyethylcarbamoylmethoxy, Carboxypropyloxy, Methylsulfonylethoxy, Ethoxycarbonylmethoxy); Aryloxy (z.B. 4-Methylphenoxy, 4-Chlorphenoxy, 4-Methoxyphenoxy, 4-Carboxyphenoxy, 3-Ethoxycarboxyphenoxy, 3-Acetylaminophenoxy, 2-Carboxyphenoxy); Acyloxy (z.B. Acetoxy, Tetradecanoyloxy, Benzoyloxy); Alkyl- oder Aryl-sulfonyloxy (z.B. Methansulfonyloxy, Toluolsulfonyloxy); Acylamino (z.B. Dichloracetylamino, Heptafluorobutyrylamino); Alkyl- oder Arylsulfonamido (z.B. Methanesulfonamido, Trifluoromethansulfonamido, p-Toluolsulfonylamido); Alkoxycarbonyloxy (z.B. Ethoxycarbonyloxy, Benzyloxycarbonyloxy); Aryloxycarbonyloxy (z.B. Phenoxycarbonyloxy); Alkyl-, Aryl- oder Heterocyclyl-S- (z.B. Dodecylthio, 1-Carboxydodecylthio, Phenylthio, 2-Butoxy-5-t-octylphenylthio, Tetrazolylthio); Carbamoylamino (z.B. N-Methylcarbamoylamino, N-Phenylcarbamoylamino); 5- oder 6-gliedriger stickstoffhaltiger Ring (z.B. Imidazolyl, Pyrazolyl, Triazolyl, Tetrazolyl, 1,2-Dihydro-2-oxo-1-pyridyl); Imido (z.B. Succinimido, Hydantoinyl); Arylazo (z.B. Phenylazo, 4-Methoxyphenylazo).

Q kann auch entsprechende Bisverbindungen bilden durch Kondensation von 4 äquivalenten Kuppler mit einem Aldehyd oder Keton. Des weiteren kann Q fotografisch wirksame Gruppen enthalten wie Entwicklungsinhibitoren oder Entwicklungsbeschleuniger. Vorzugsweise ist Q Halogen, Alkoxy, Aryloxy, Alkyl-, Aryl-thio, oder eine 5- oder 6-gliedrige stickstoffhaltige heterocyclische Gruppe, die an den Ort der Kupplung über ein Stickstoffatom gebunden ist.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-0 119 860, EP-A-0 173 256, EP-A-0 178

789, EP-A-0 178 788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, JP-A-87/278,552, JP-A-87/279,340, JP-A-88/100,457, JP-A-5,027,391, JP-A-5,053,271, JP-A-5,053,272, JP-A-232,646, JP-A-5,241,286, JP-A-5,241,287, JP-A-5,241,288, JP-A-5,241,289, JP-A-5,241,290, JP-A-5,249,633, JP-A-5,303,181, JP-A-5,323,530, EP-A-0 177 765, EP-A-0 176 804, EP-A-0 170 164, EP-A-0 164 130, EP-A-0 178 794, EP-A-0 487 081, EP-A-0 489 333, EP-A-0 558 145, EP-A-0 568 894, DE-A-35 16 996, DE-A-35 08 766, DE-A-42 40 000, WO 92/10788, WO 92/12464, US-A-5,100,772, US-A-5,254,451, US-A-5,300,407, US-A-5,336,593 und Research Disclosure 81/20919, 84/24531 und 85/25758.

Geeignete Beispiele solcher Kuppler sind:

(M-5)

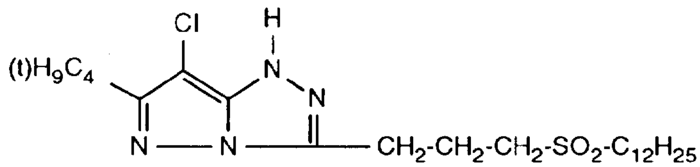

(M-6)

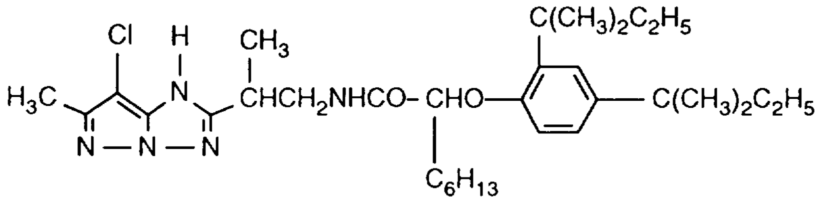

(M-7)

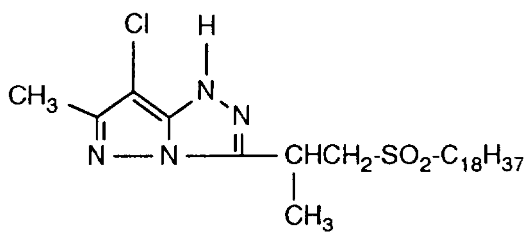

(M-8)

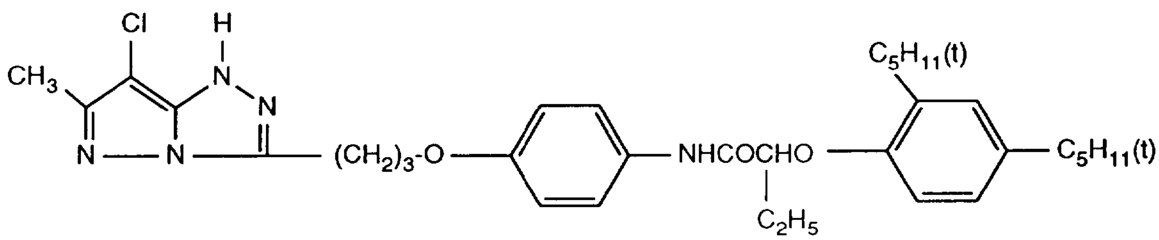

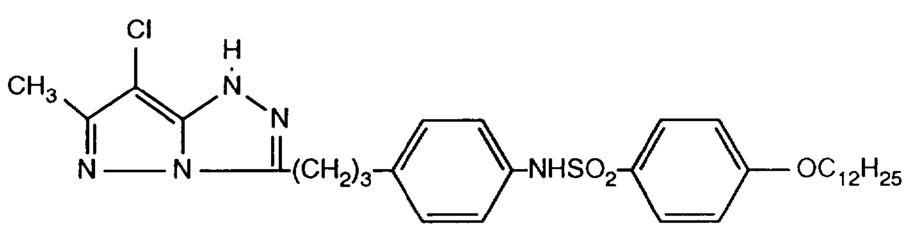

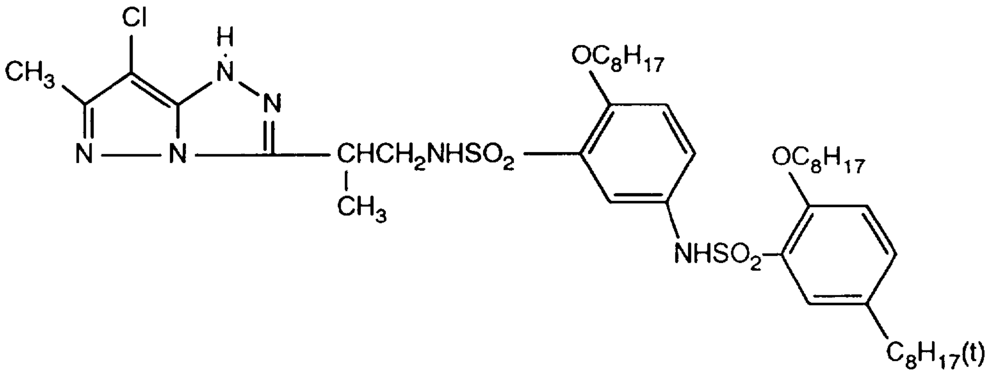

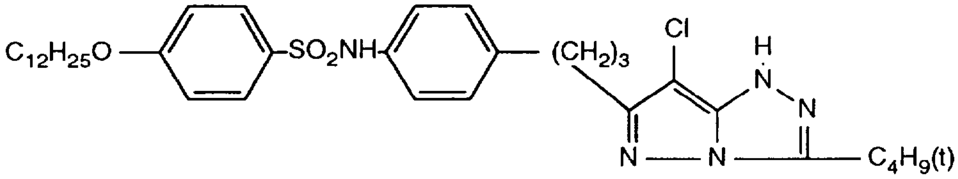

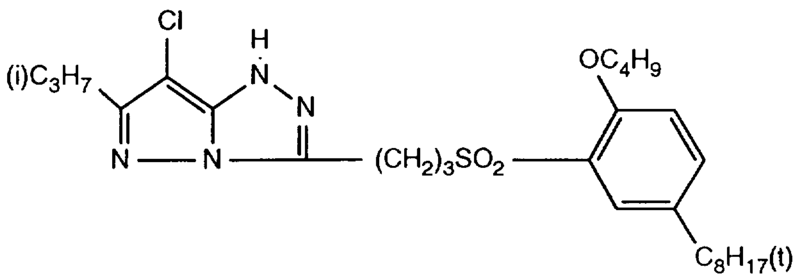

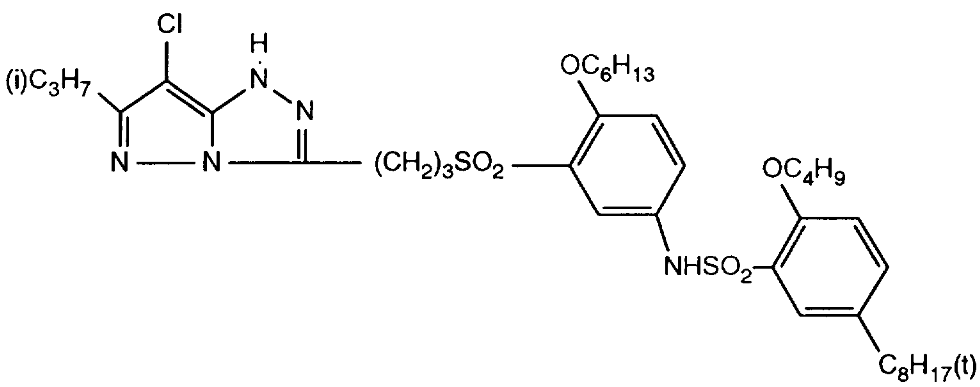

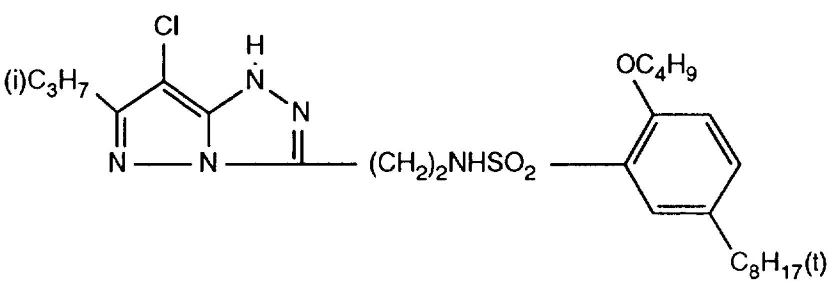

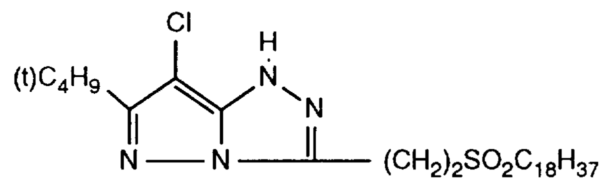

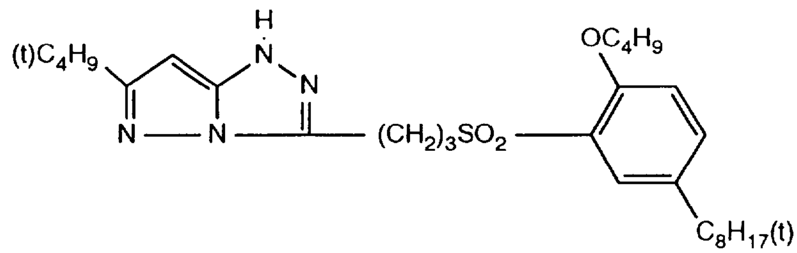

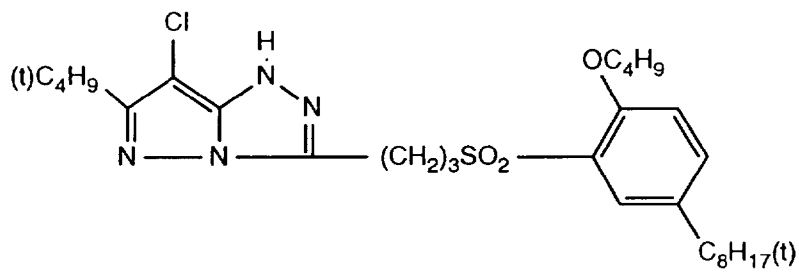

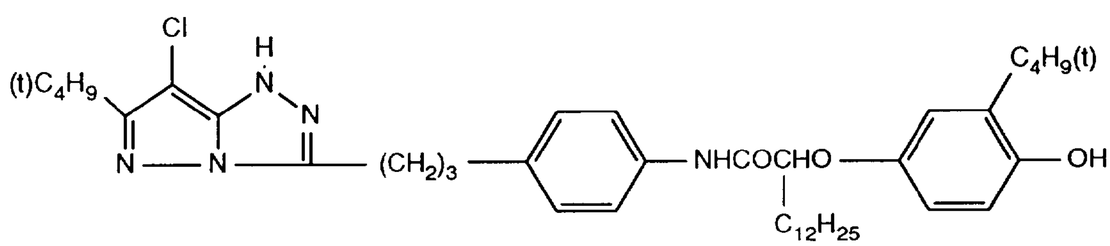

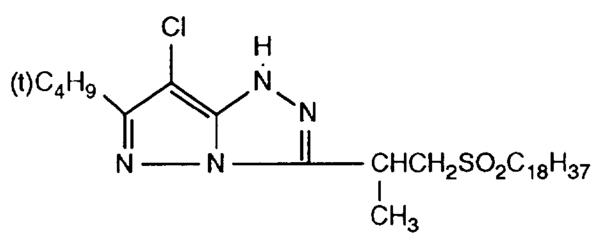

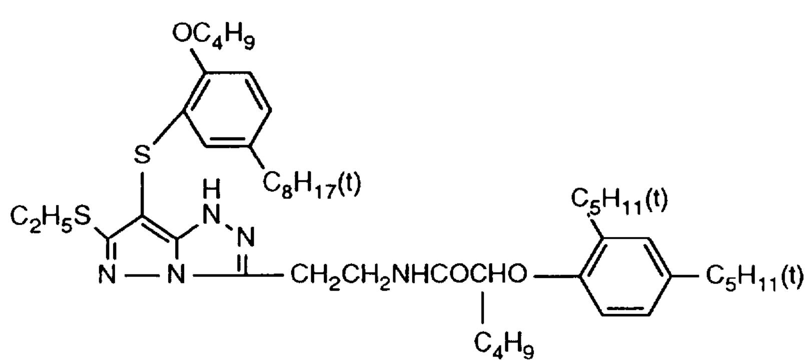

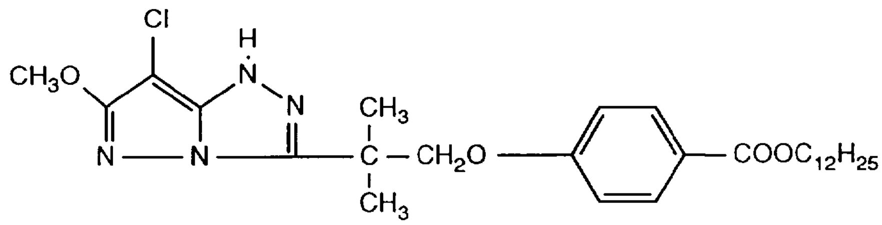

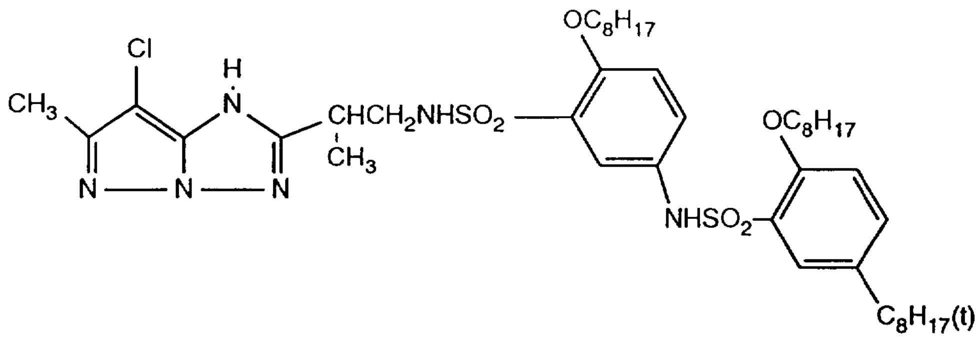

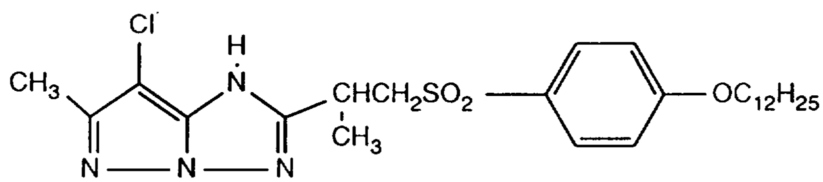

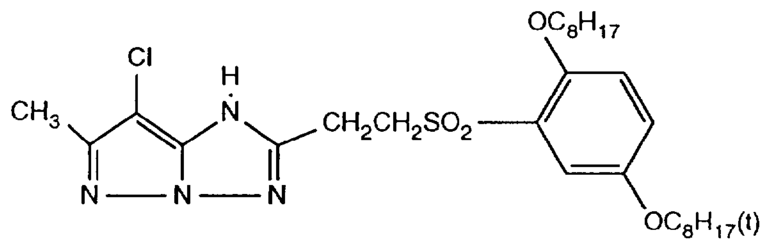

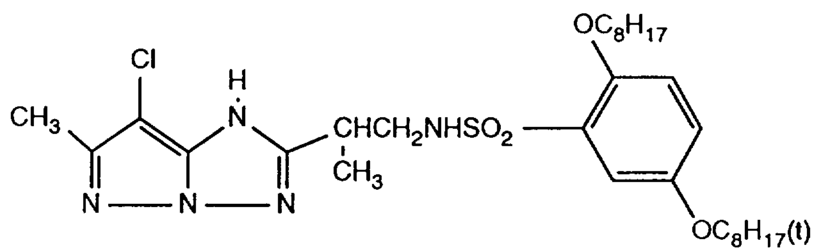

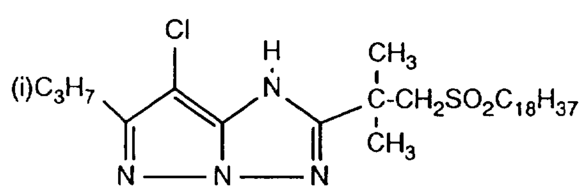

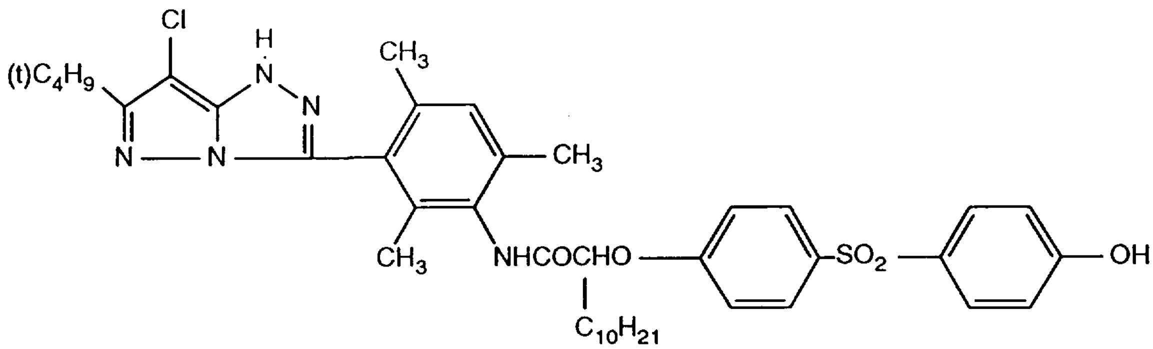

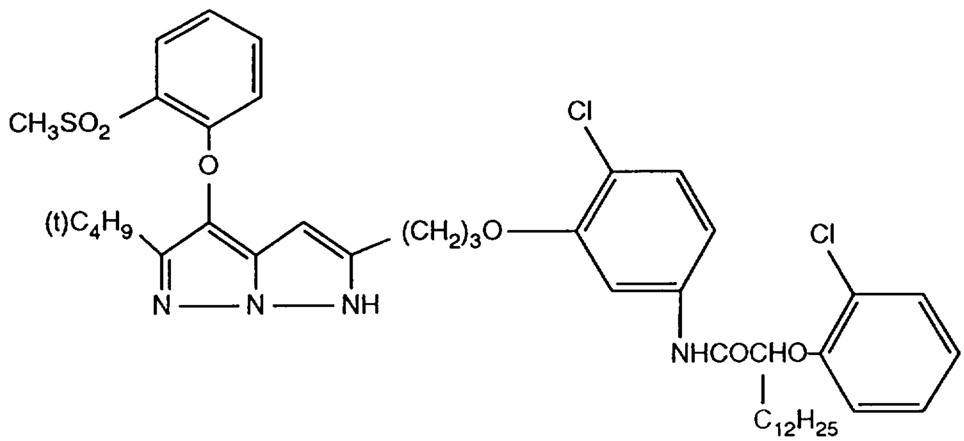

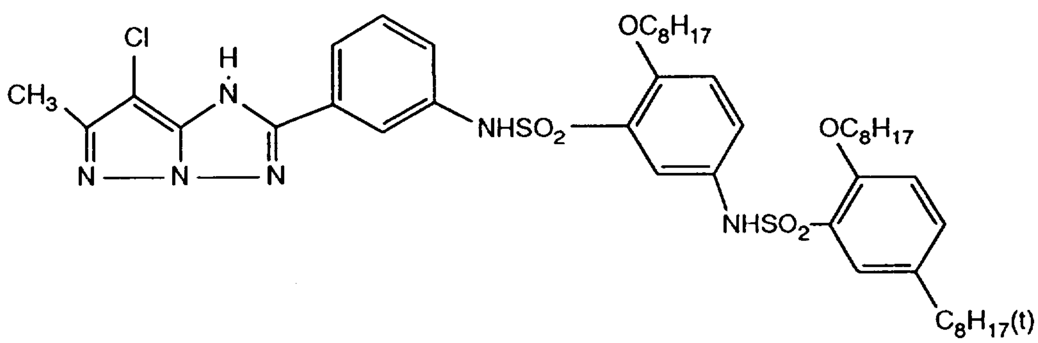

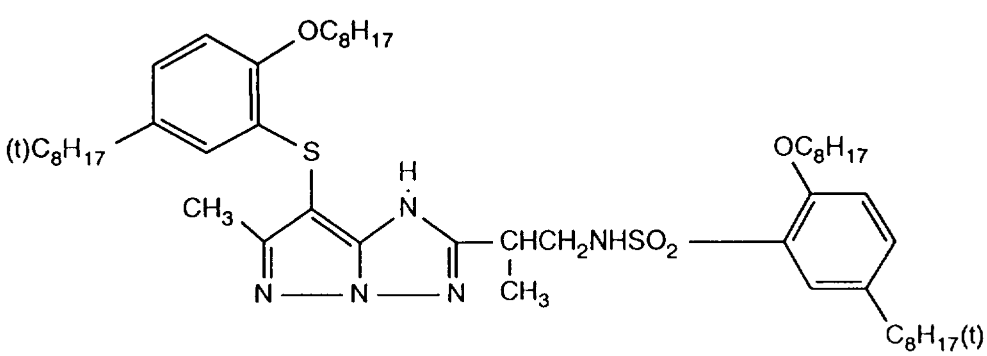

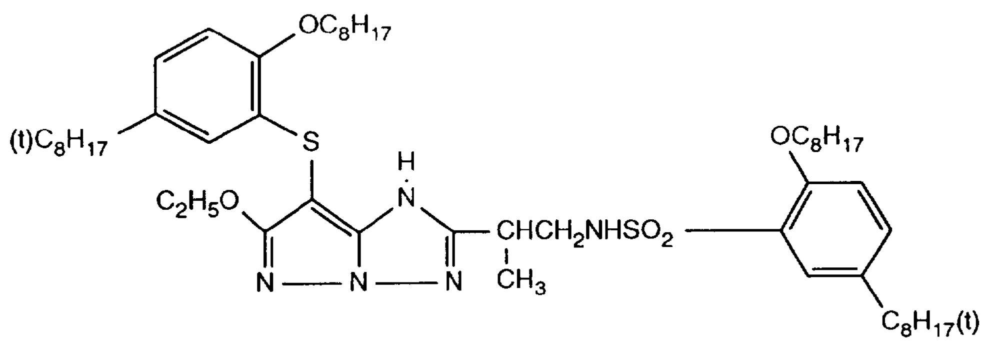

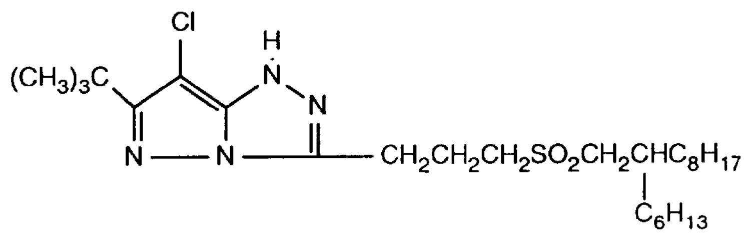

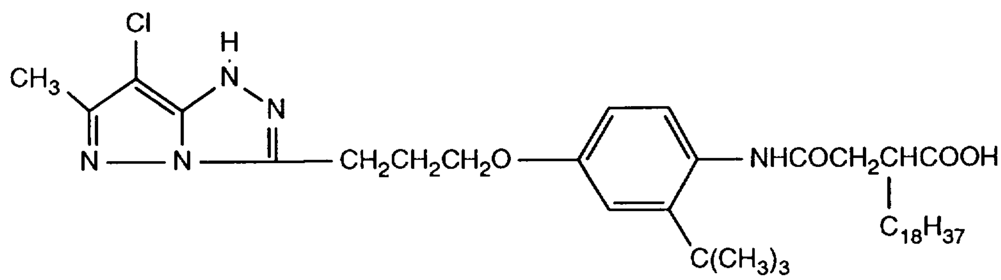

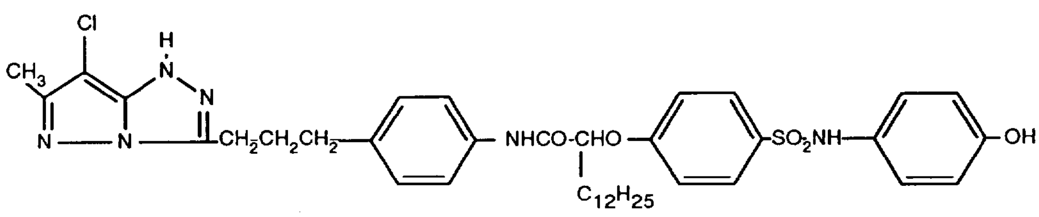

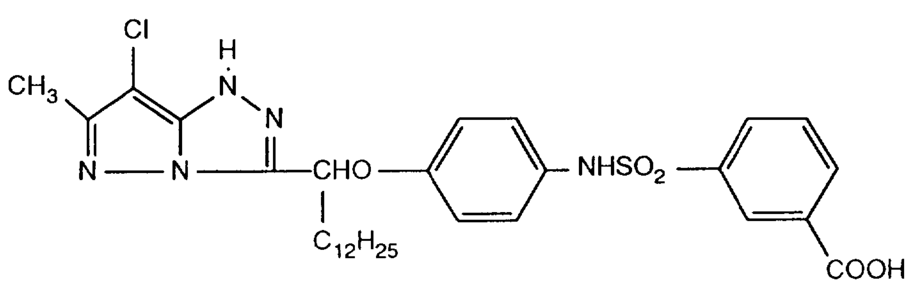

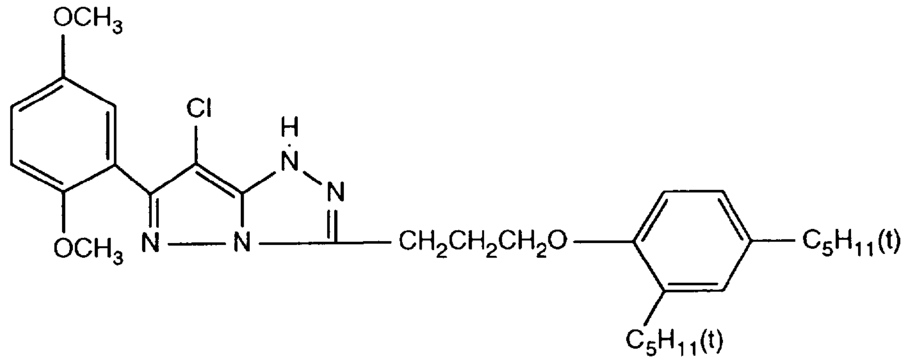

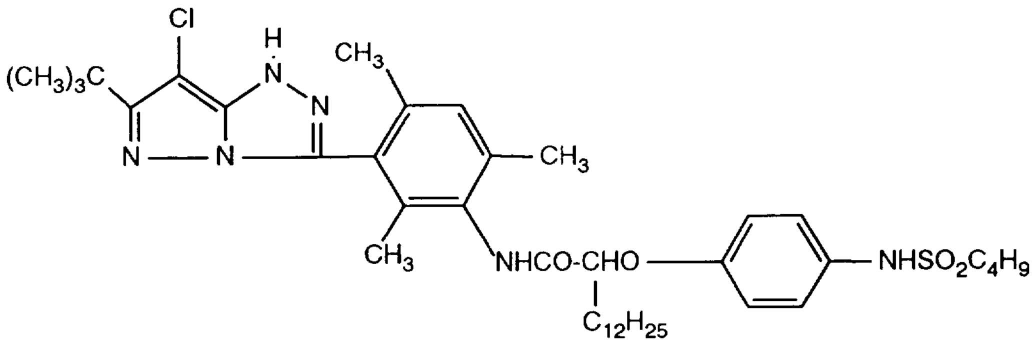

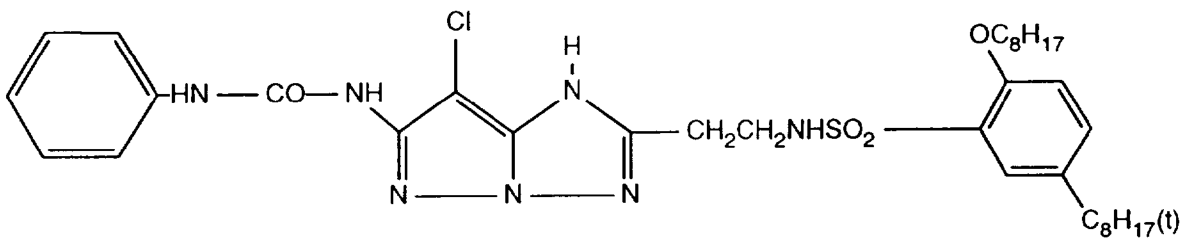

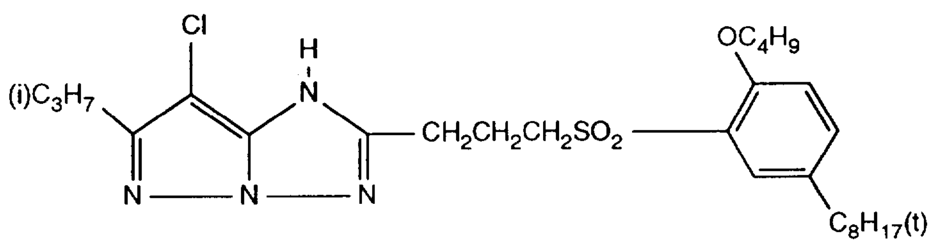

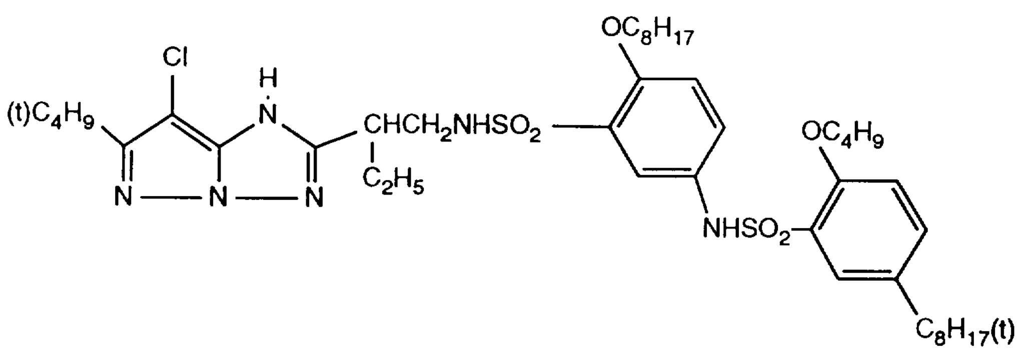

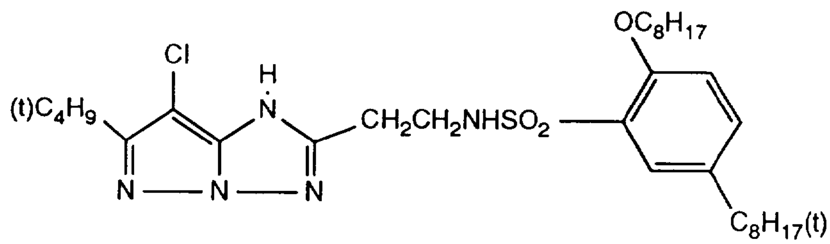

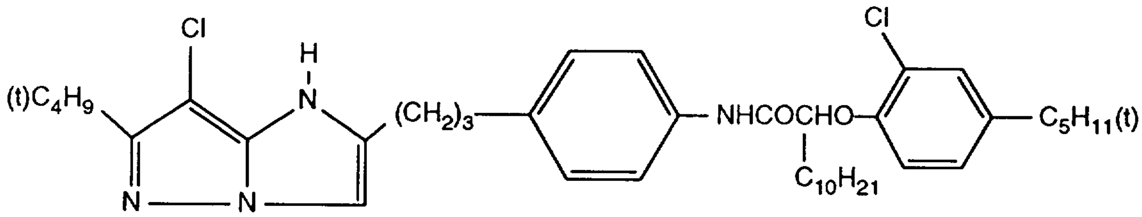

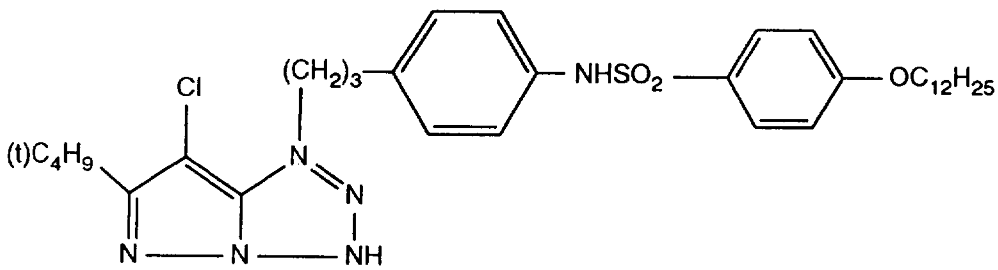

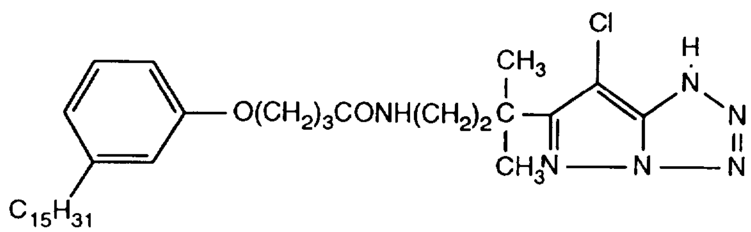

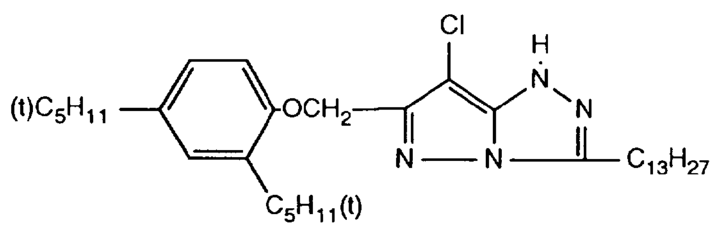

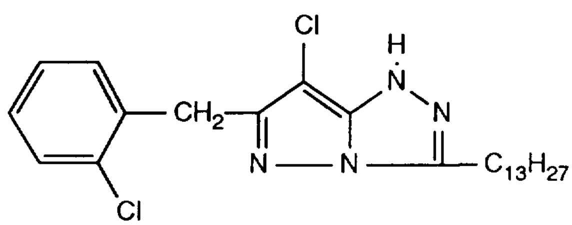

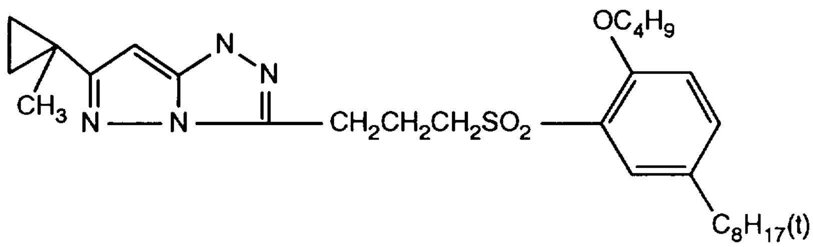

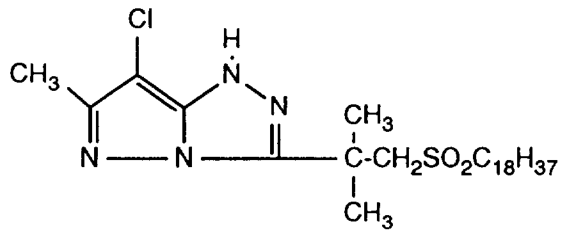

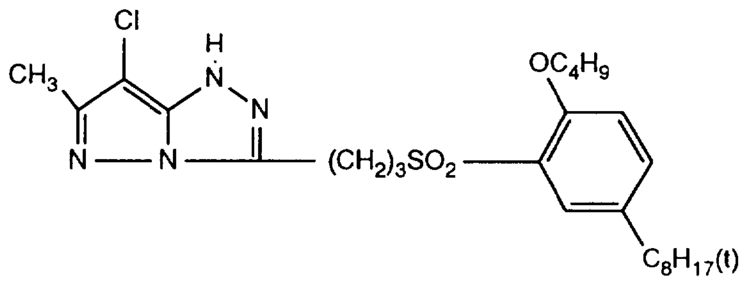

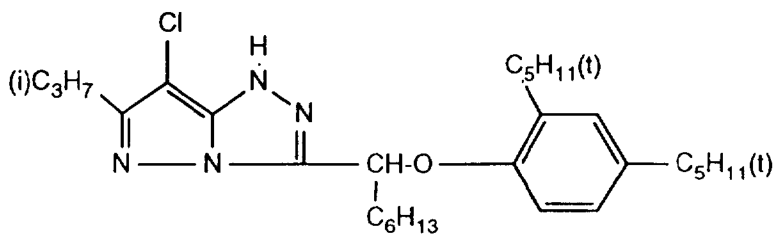

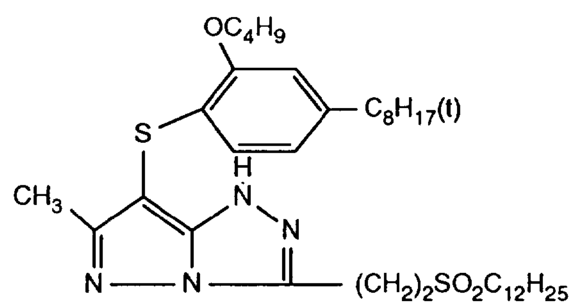

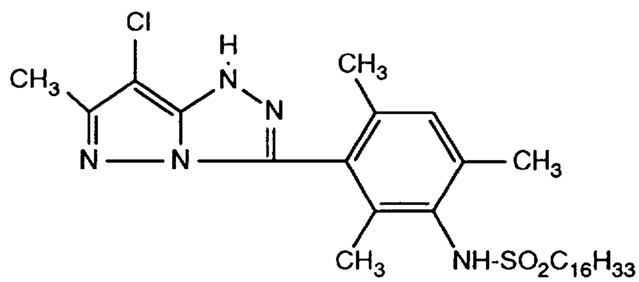

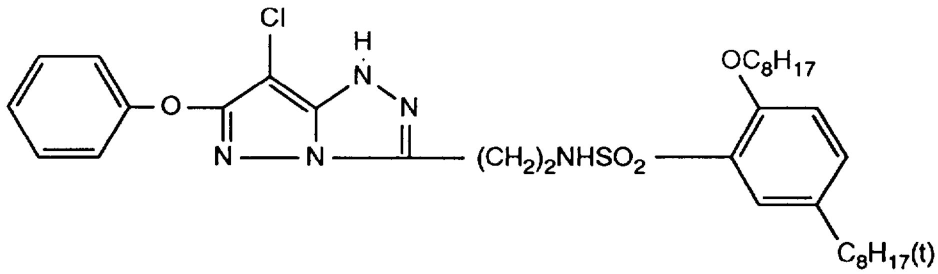

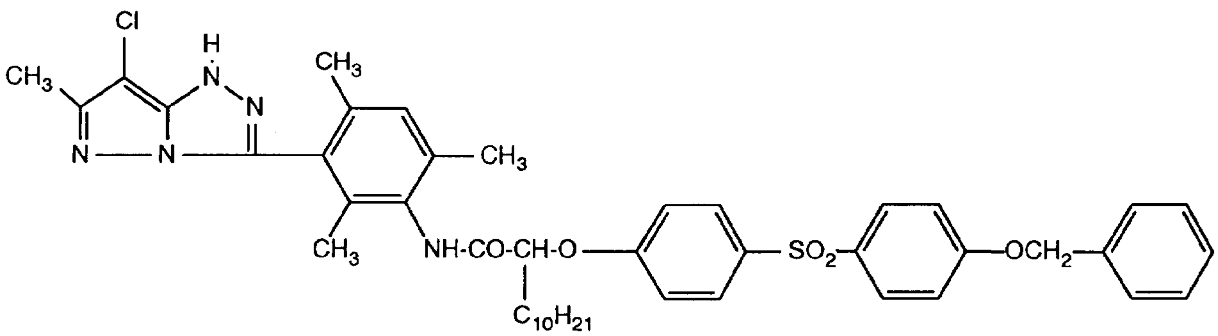

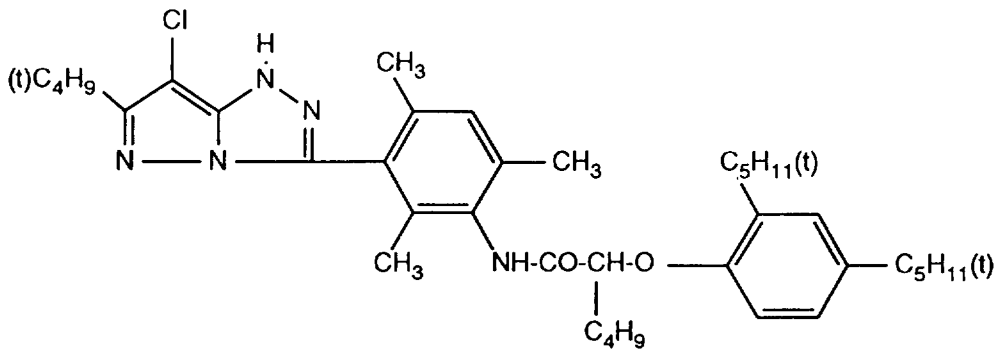

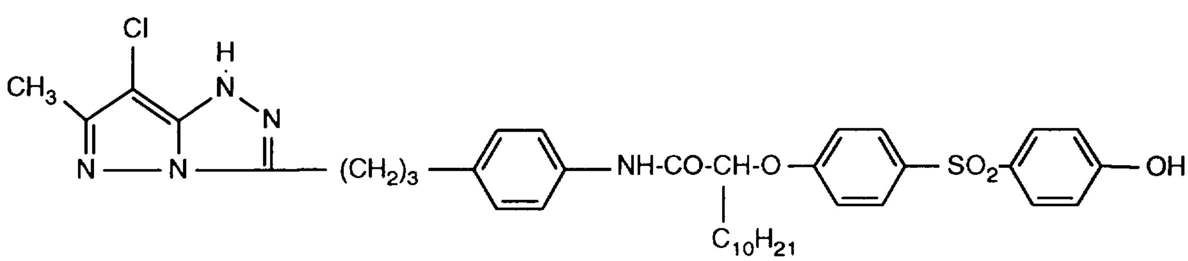

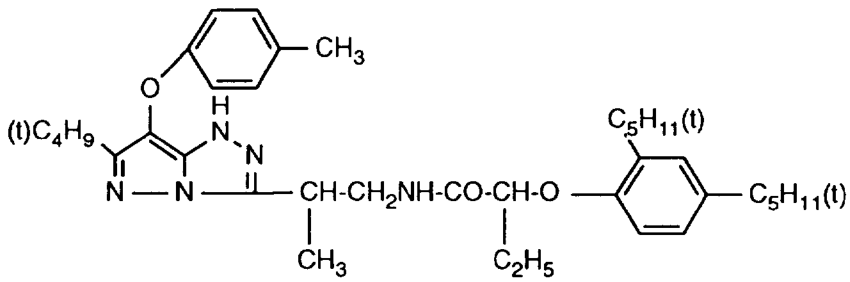

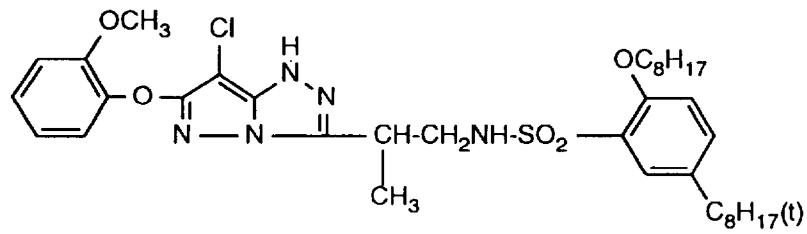

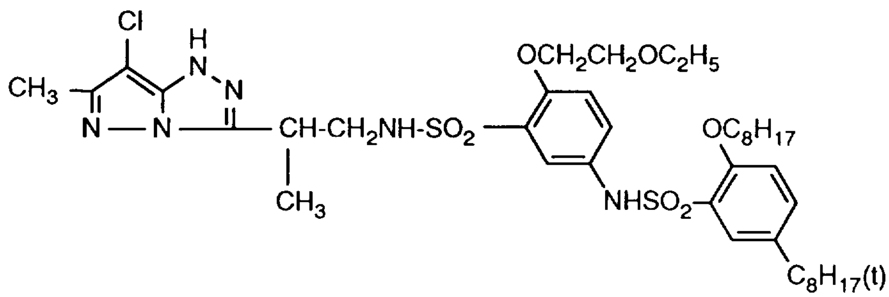

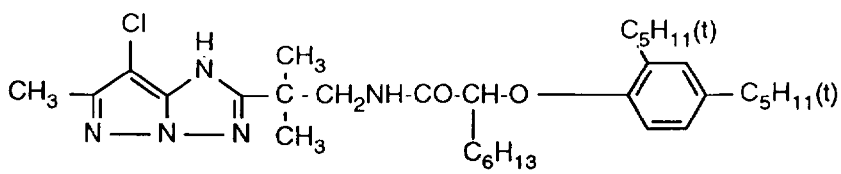

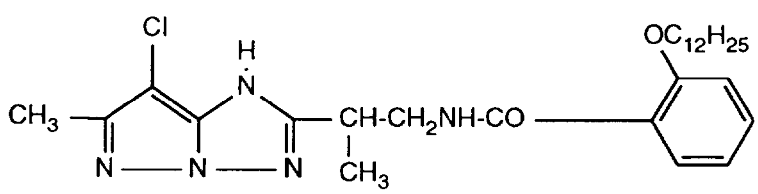

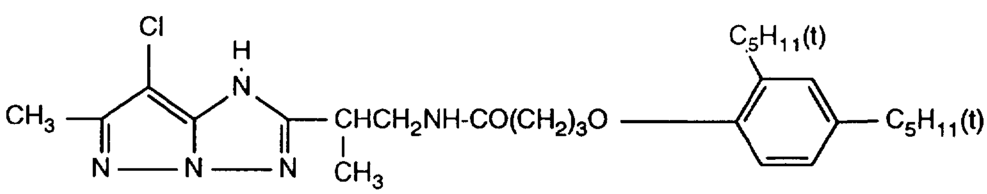

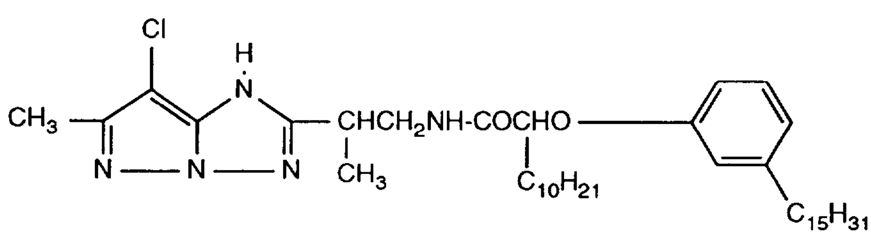

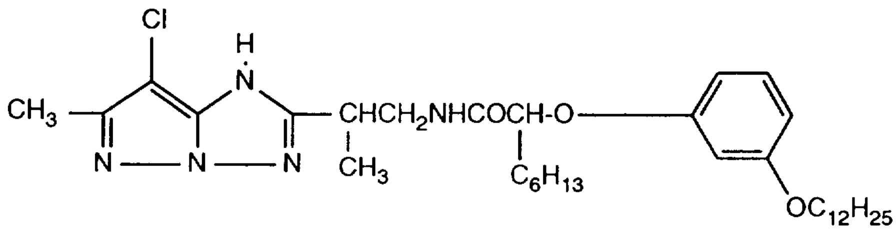

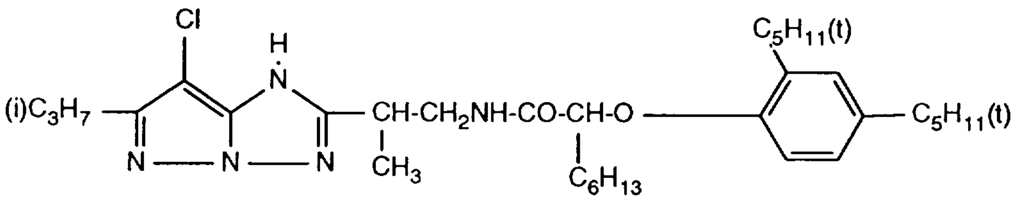

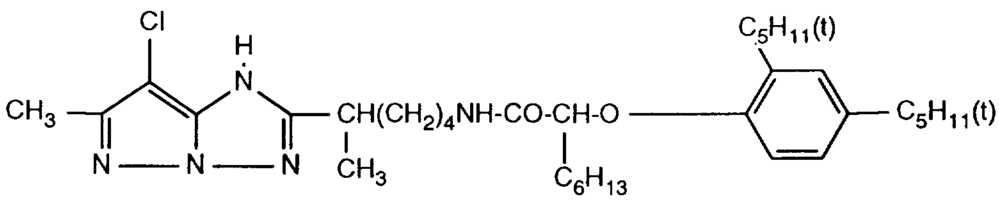

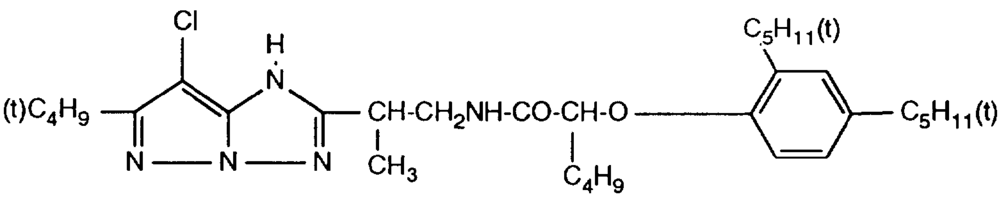

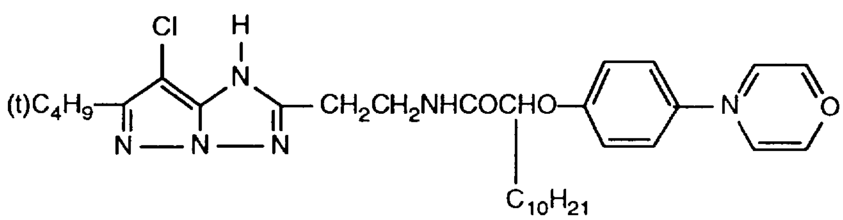

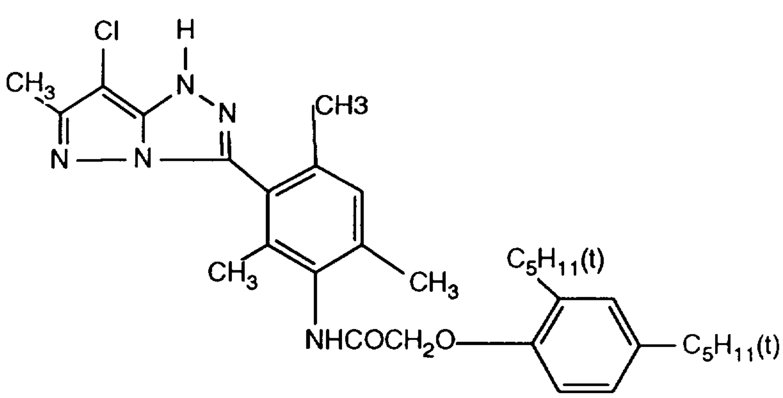

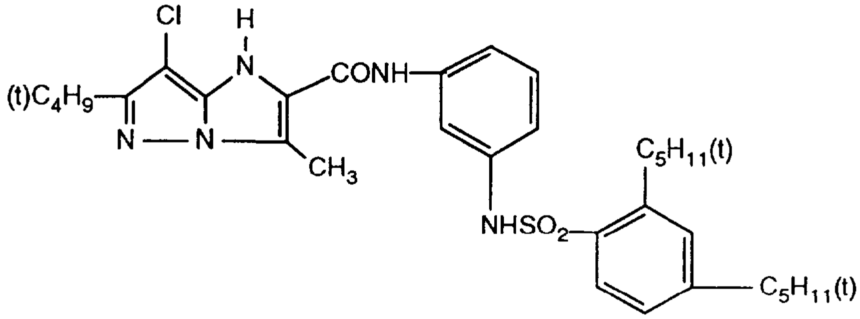

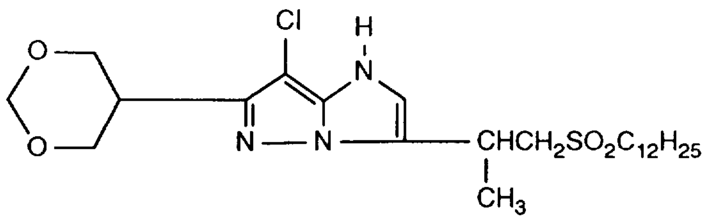

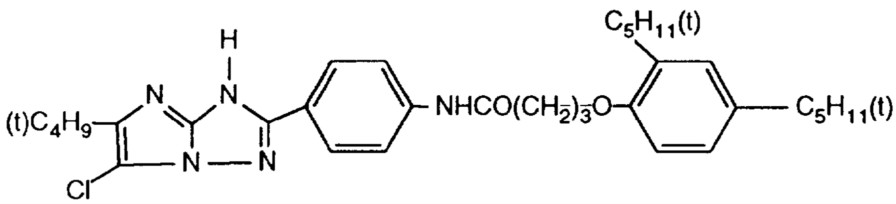

Cyankuppler können z.B. Derivate von Phenol, von 1-Naphthol oder von Pyrazolochinazolon sein. Bevorzugt sind Strukturen der Formel E,

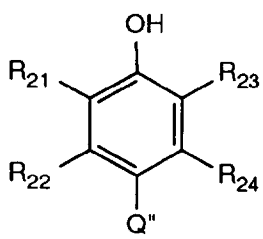

(E),

worin $R_{21}$, $R_{22}$, $R_{23}$ und $R_{24}$ Wasserstoff, Halogen, Alkyl, Carbamoyl, Amino, Sulfonamido, Phosphoramido oder Ureido sind. $R_{21}$ ist vorzugsweise H oder Cl, $R_{22}$ ist vorzugsweise eine Alkyl- oder Aminogruppe. $R_{23}$ ist vorzugsweise eine Aminogruppe und $R_{24}$ ist vorzugsweise Wasserstoff. Q" ist Wasserstoff (4-Äquivalentkuppler) oder eine Abgangsgruppe (2-Äquivalentkuppler), die bei der Reaktion mit den oxidierten Entwickler abgespalten wird. Eine ausführliche Aufzählung von Cyankupplern ist im US-A-4,456,681 zu finden.

In der rotempfindlichen Silberhalogenidemulsionsschicht des erfindungsgemäßen Materials kommen vorzugsweise Cyankuppler der Formel

(E-12)

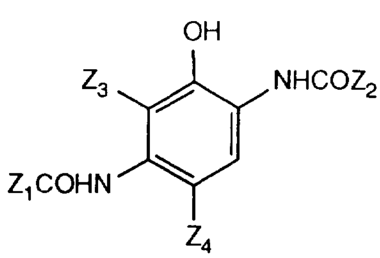

und/oder oder Formel

(E-13) 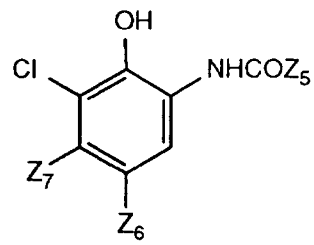

zum Einsatz, worin
$Z_1$ Alkyl, Aryl, $Z_2$ Alkyl, Cycloalkyl, Aryl, eine heterocyclische Gruppe, oder eine Ballastgruppe, $Z_3$ Wasserstoff oder Halogen ist, $Z_1$ und $Z_3$ zusammen einen Ring bilden können, und $Z_4$ Wasserstoff oder eine Abgangsgruppe ist, und $Z_5$ eine Ballastgruppe, $Z_6$ Wasserstoff oder eine Abgangsgruppe und $Z_7$ Alkyl ist.

Beispiele von gebräuchlichen Cyankupplern sind die folgenden:

(E-1) 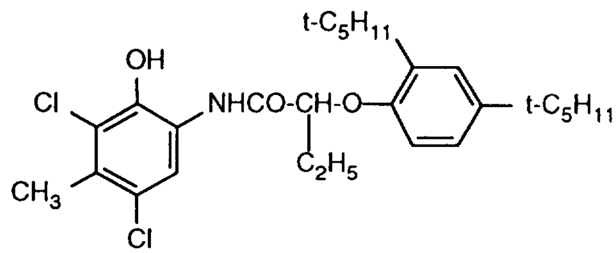

(E-2) 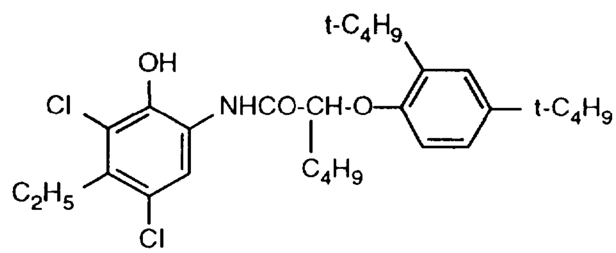

(E-3) 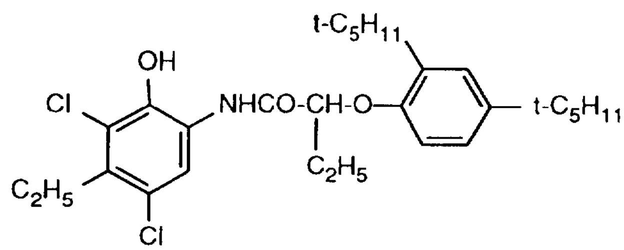

(E-4) 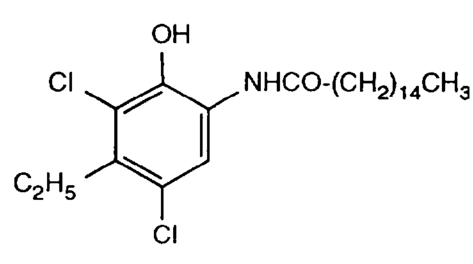

(E-5)

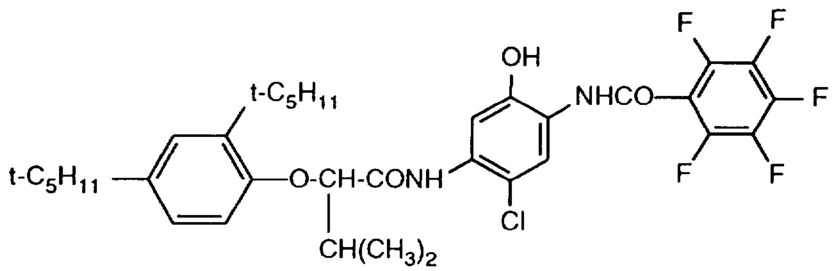

(E-6)

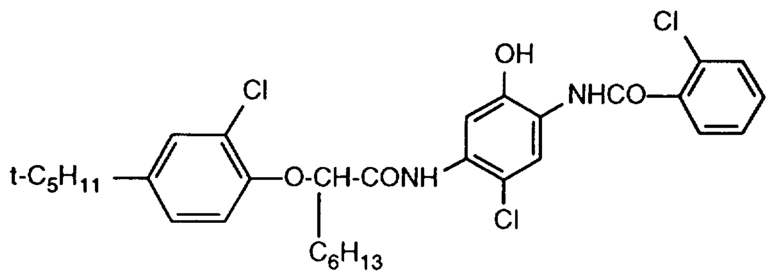

(E-7)

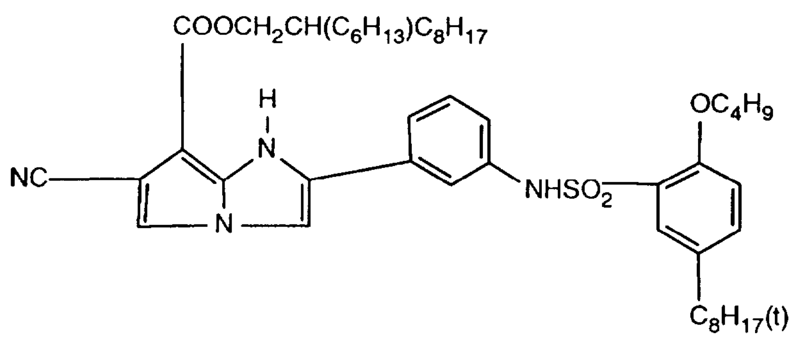

(E-8)

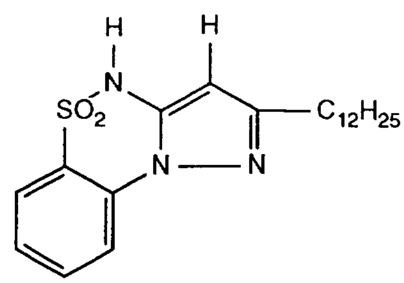

(E-9)

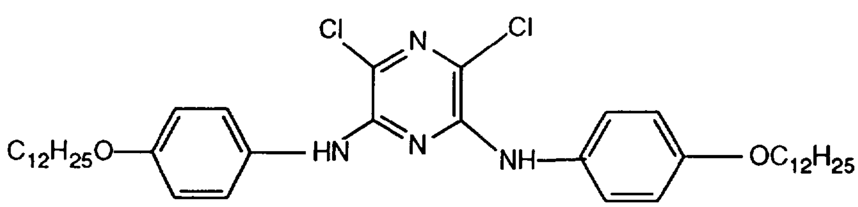

(E-10)

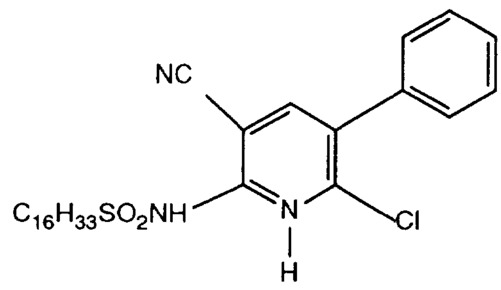

(E-11)

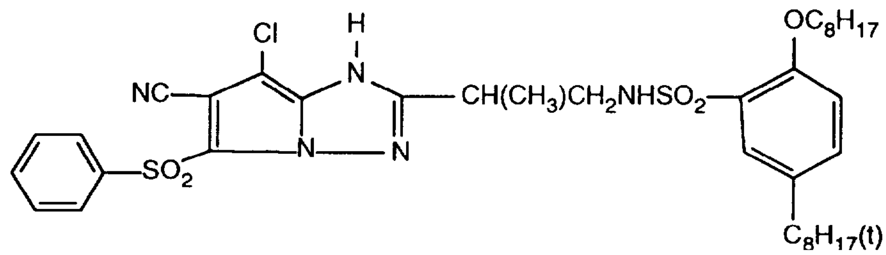

Weitere Beispiele von Cyankupplern sind in folgenden US-A- zu finden: 2,369,929, 2,423,730, 2,434,272, 2,474,293, 2,521,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681, 4,873,183, 4,923,791, 5,143,824, 5,256,526, 5,269,181, 5,262,293, 5,270,153, 5,306,610 und in den EP-A-0 354 549, EP-A-0 398 664, EP-A-0 456 226, EP-A-0 484 909, EP-A-0 487 111, EP-A-0 488 248, EP-A-0 491 197, EP-A-0 544 316, EP-A-0 545 300, EP-A-0 545 305, EP-A-0 556 777, EP-A-0 578 248, EP-A-0 608 133 und JP-A-3,240,053, 3,284,746, 4,009,050, 4,043,346, 4,125,557, 5,262,293, 5,306,610, 6,083,000, 6,083,001.

Zu den 2-Äquivalentkupplern sind solche zu rechnen, die farblos sind, als auch solche, die eine intensive Eigenfarbe aufweisen, die bei der Farbkupplung verschwindet bzw. durch die Farbe des erzeugten Bildfarbstoffes ersetzt wird (Maskenkuppler), und die Weißkuppler, die bei Reaktion mit Farbentwickleroxidationsprodukten im wesentlichen farblose Produkte ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird und dabei entweder direkt oder nachdem aus dem primär abgespaltenen Rest eine oder mehrere weitere Gruppen abgespalten worden sind (z.B. DE-A-27 03 145, DE-A-28 55 697, DE-A-31 05 026, DE-A-33 19 428), eine bestimmte erwünschte fotografische Wirksamkeit entfaltet, z.B. als Entwicklungshinhbitor oder -accelerator. Beispiele für solche 2-Äquivalentkuppler sind die bekannten DIR-Kuppler wie auch DAR- bzw. FAR-Kuppler.

Beispiel für Weißkuppler sind:

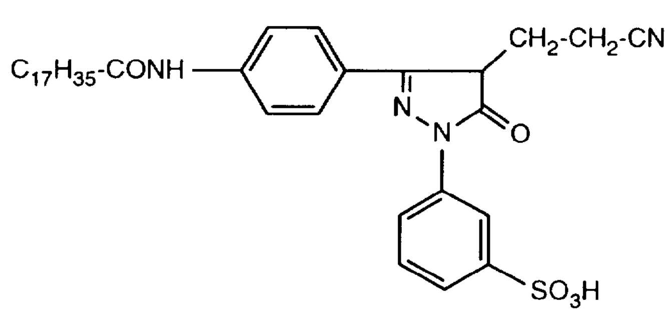

W-1

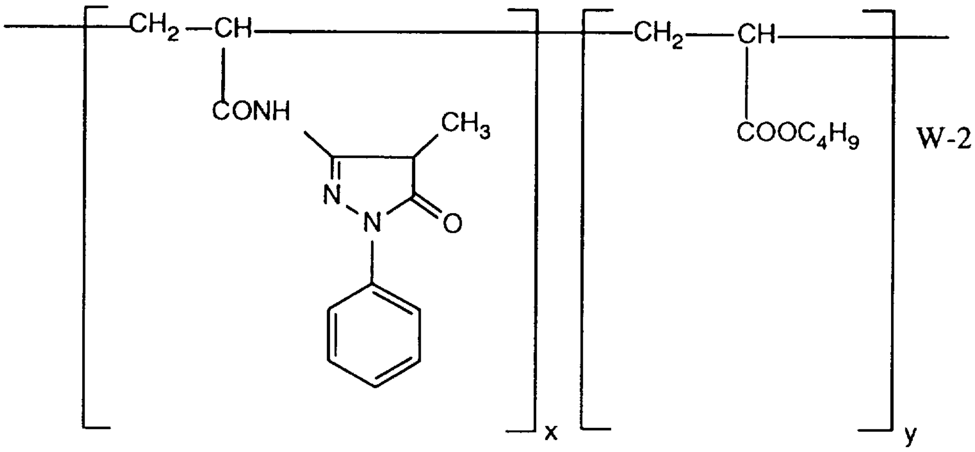

W-2

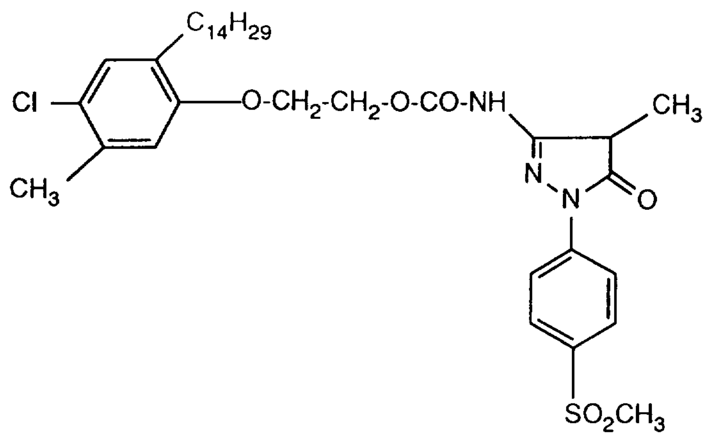

W-3

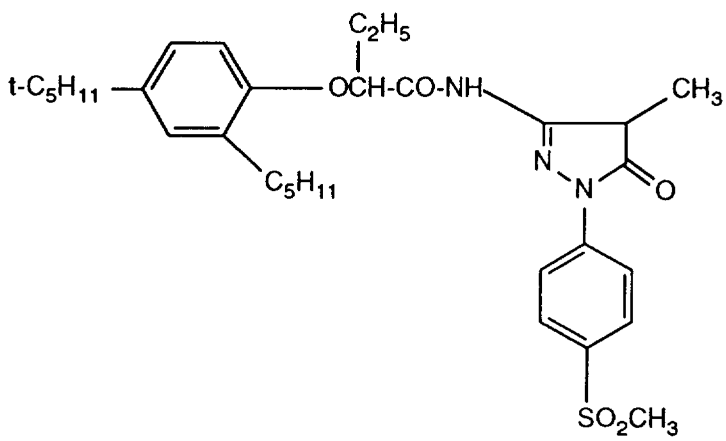

W-4

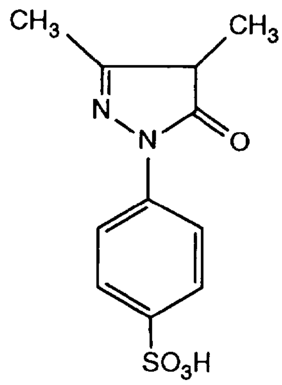

W-5

Beispiele für Maskenkuppler sind

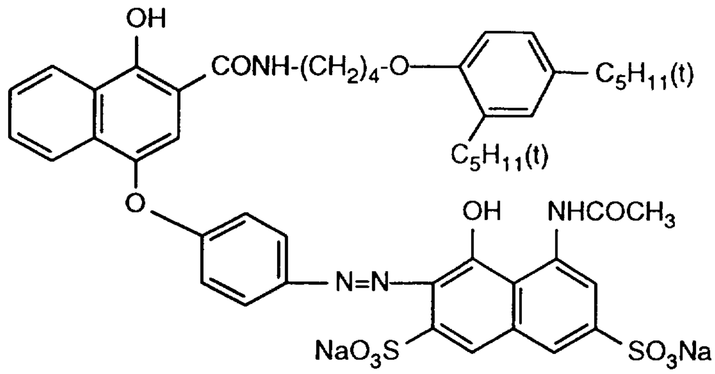

RM-1

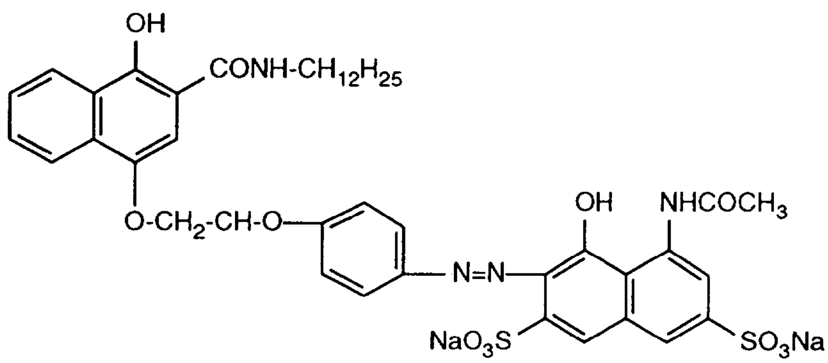

RM-2

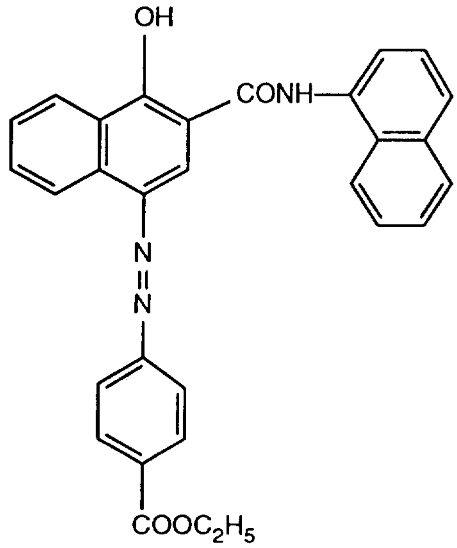

RM-3

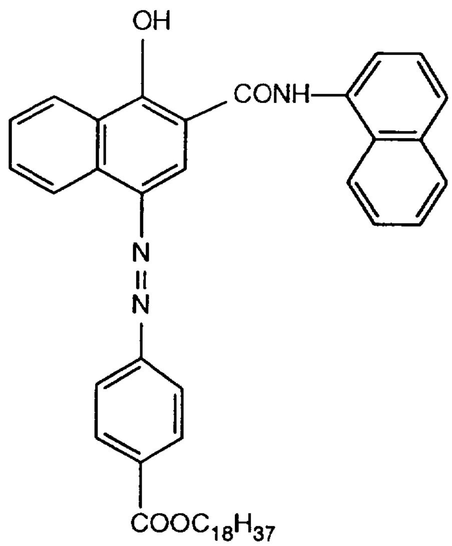

RM-4

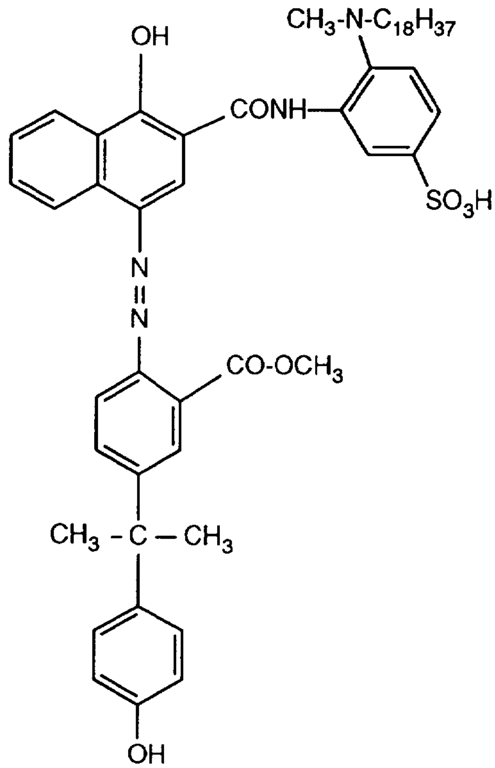

RM-5

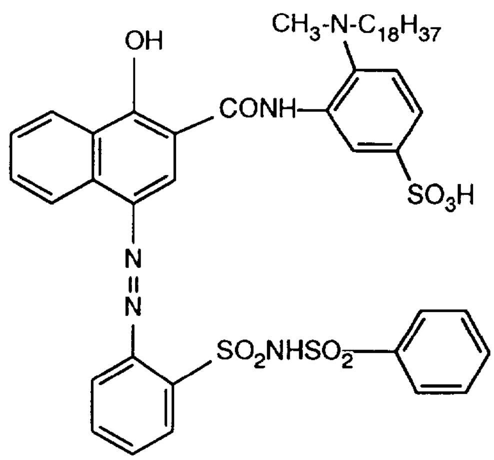

RM-6

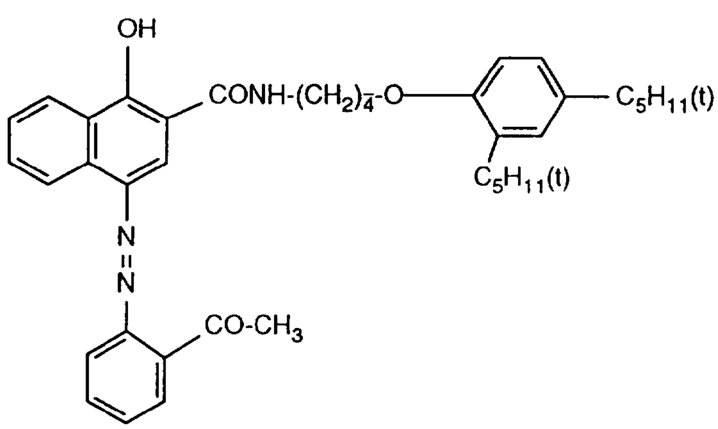

RM-7

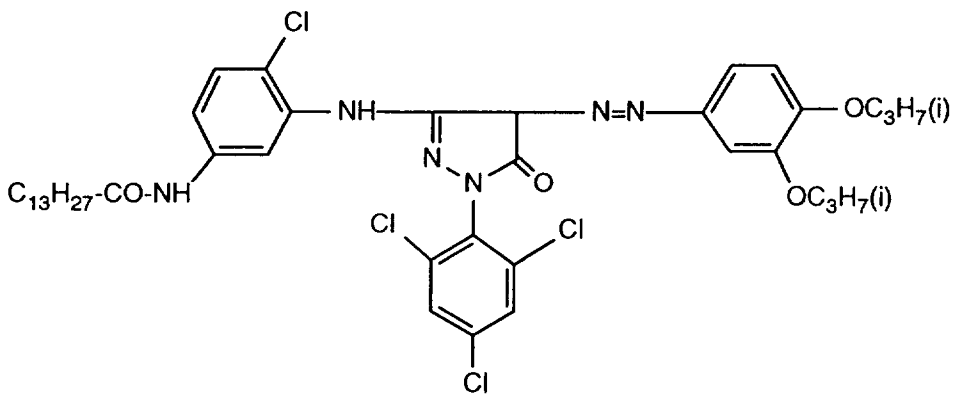

YM-1

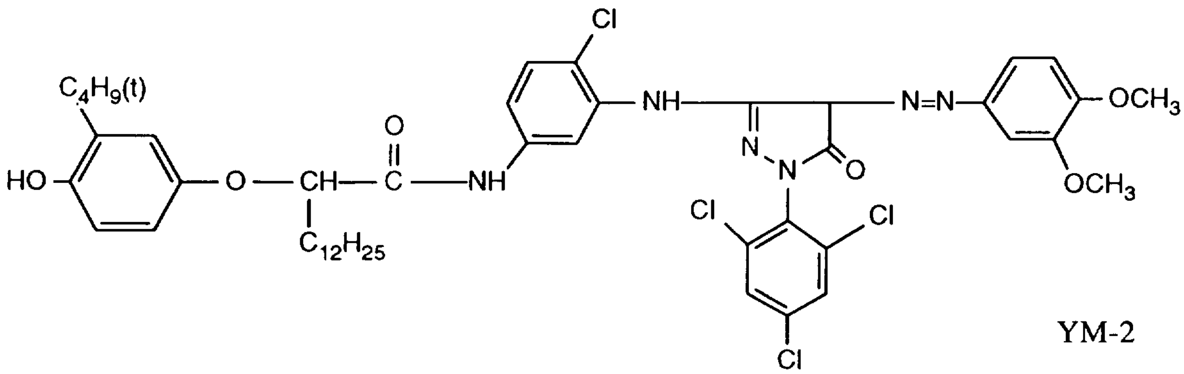

YM-2

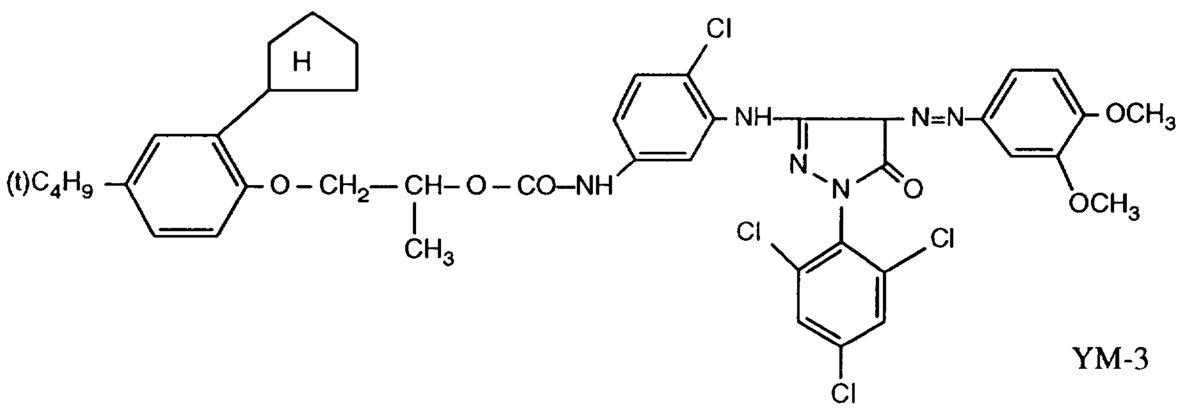

YM-3

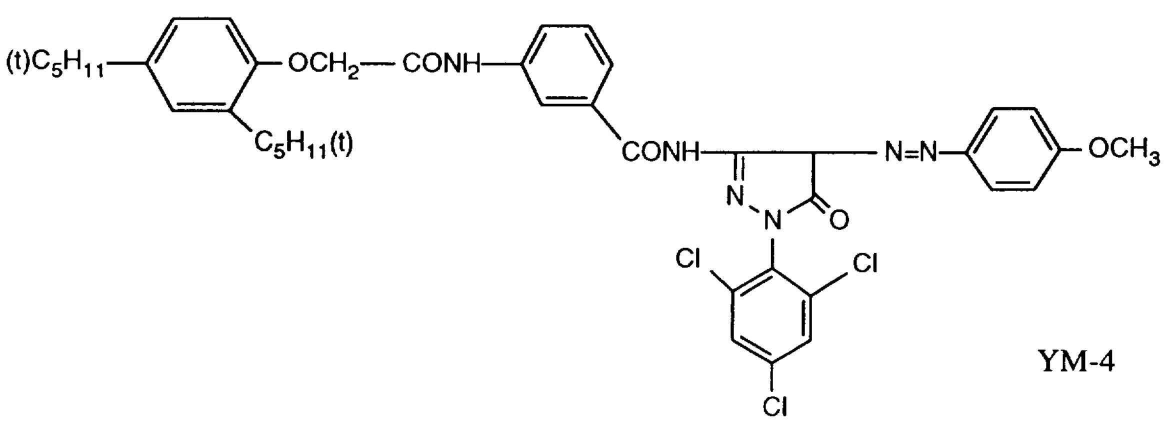

YM-4

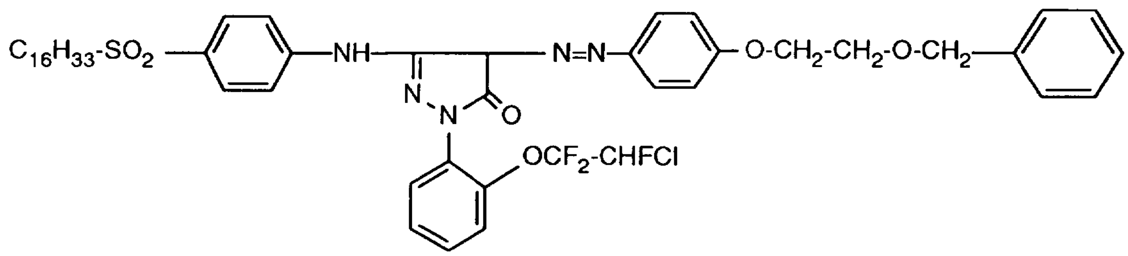

YM-5

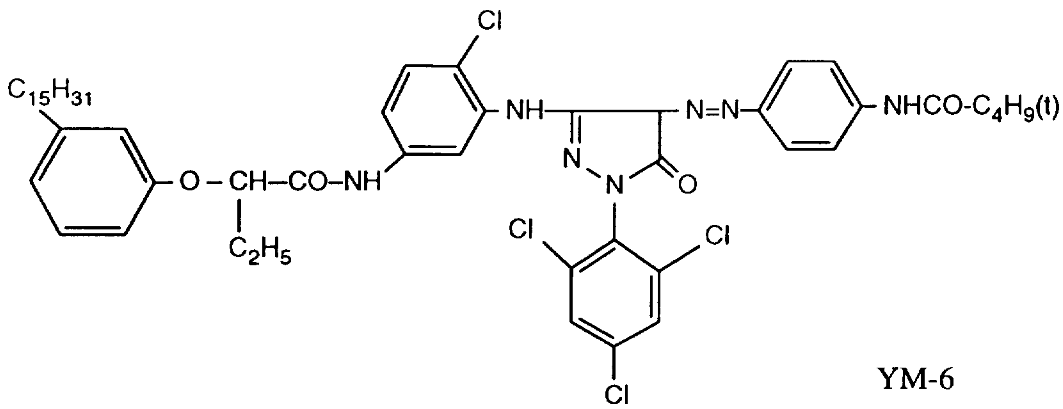

YM-6

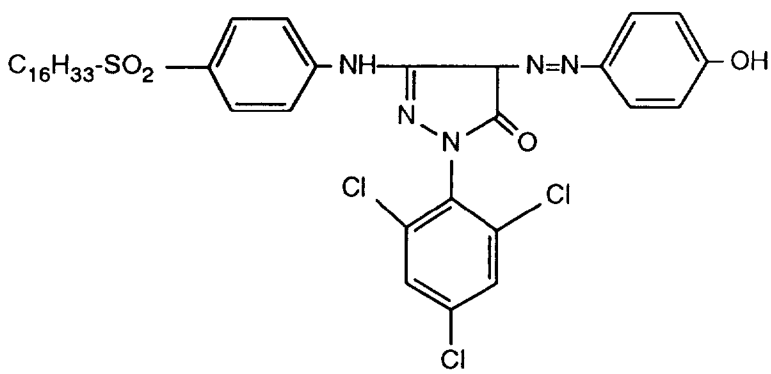

YM-7

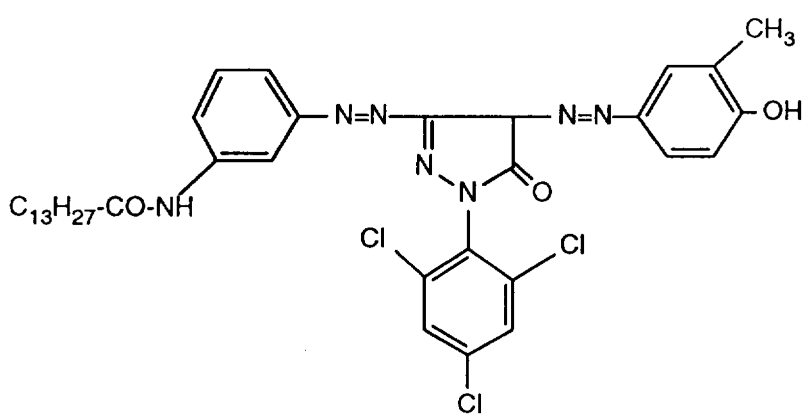

YM-8

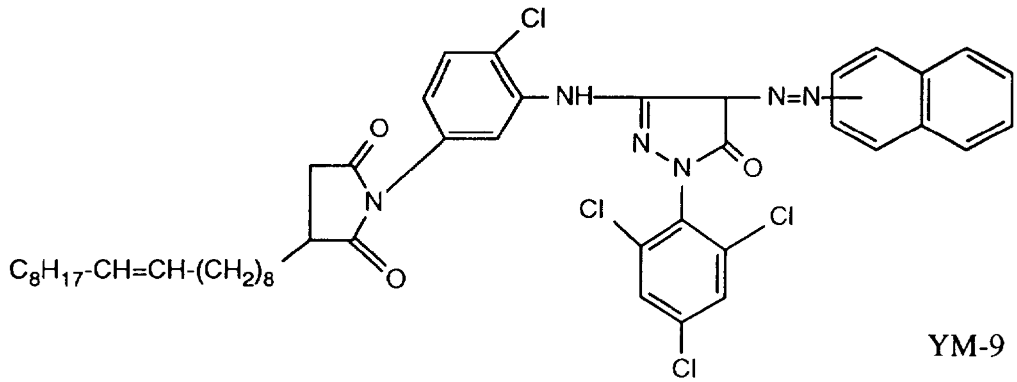

YM-9

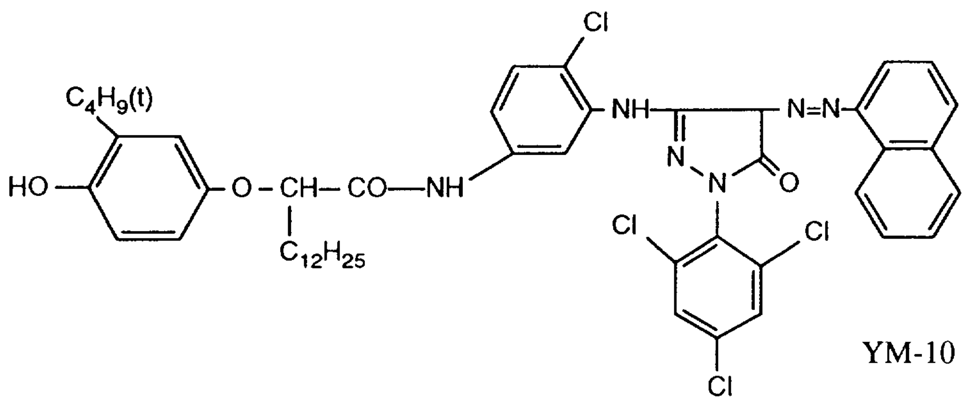

YM-10

DIR-Kuppler, die Entwicklungsinhibitoren vom Azoltyp, z.B. Triazole und Benzotriazole freisetzen, sind in DE-A-24 14 006, 26 10 546, 26 59 417, 27 54 281, 28 42 063, 36 26 219, 36 30 564, 36 36 824, 36 44 416 beschrieben. Weitere Vorteile für die Farbwiedergabe, d.h. Farbtrennung und Farbreinheit, und für die Detailwiedergabe, d.h. Schärfe und Körnigkeit, sind mit solchen DIR-Kupplern zu erzielen, die z.B. den Entwicklungsinhibitor nicht unmittelbar als Folge der Kupplung mit einem oxidierten Farbentwickler abspalten, sondern erst nach einer weiteren Folgereaktion, die beispielsweise mit einer Zeitsteuergruppe erreicht wird. Beispiel dafür sind in DE-A-28 55 697, 32 99 671, 38 18 231, 35 18 797, in EP-A-0 157 146 und 0 204 175, in US-A-4 146 396 und 4 438 393 sowie in GB-A-2 072 363 beschrieben.

DIR-Kuppler, die einen Entwicklungsinhibitor freisetzen, der im Entwicklerbad zu im wesentlichen fotografisch unwirksamen Produkten zersetzt wird, sind beispielsweise in DE-A-32 09 486 und in EP-A-0 167 168 und 0 219 713 beschrieben. Mit dieser Maßnahme wird eine störungsfreie Entwicklung und Verarbeitungskonstanz erreicht.

Bei Einsatz von DIR-Kupplern, insbesondere von solchen, die einen gut diffundierbaren Entwicklungsinhibitor abspalten, lassen sich durch geeignete Maßnahmen bei der optischen Sensibilisierung Verbesserungen der Farbwiedergabe, z.B. eine differenziertere Farbwiedergabe erzielen, wie beispielsweise in EP-A-0 115 304, 0 167 173, GB-A- 2 165 058, DE-A-37 00 419 und US-A-4 707 436 beschrieben.

Die DIR-Kuppler können in einem mehrschichtigen fotografischen Material den unterschiedlichsten Schichten zugesetzt werden, z.B. auch lichtunempfindlichen oder Zwischenschichten. Vorzugsweise werden sie jedoch den lichtempfindlichen Silberhalogenidemulsionsschichten zugesetzt, wobei die charakteristischen Eigenschaften der Silberhalogenidemulsion, z.B. deren Iodidgehalt, die Struktur der Silberhalogenidkörner oder deren Korngrößenverteilung von Einfluss auf die erzielten fotografischen Eigenschaften sind. Der Einfluß der freigesetzten Inhibitoren kann beispielsweise durch den Einbau einer Inhibitorfängerschicht gemäß DE-A-24 31 223 begrenzt werden. Aus Gründen der Reaktivität oder Stabilität kann es vorteilhaft sein, einen DIR-Kuppler einzusetzen, der in der jeweiligen Schicht, in der er eingebracht ist, eine von der in dieser Schicht zu erzeugenden Farbe abweichende Farbe bei der Kupplung bildet.

Zur Steigerung der Empfindlichkeit, des Kontrastes und der maximalen Dichte können vor allem DAR- bzw. FAR-Kuppler eingesetzt werden, die einen Entwicklungsbeschleuniger oder ein Schleiermittel abspalten. Verbindungen dieser Art sind beispielsweise in DE-A-25 34 466, 32 09 110, 33 33 355, 34 10 616, 34 29 545, 34 41 823, in EP-A-0 089 834, 0 110 511, 0 118 087, 0 147 765 und in US-A- 4 618 572 und 4 656 123 beschrieben.

Als Beispiel für den Einsatz in BAR-Kuppler (Bleach Accelerator Releasing Coupler) wird auf EP-A-193 389 verwiesen.

Es kann vorteilhaft sein, die Wirkung einer aus einem Kuppler abgespaltenen fotografisch wirksamen Gruppe dadurch zu modifizieren, daß eine intermolekulare Reaktion dieser Gruppe nach ihrer Freisetzung mit einer anderen Gruppe gemäß DE-A-35 06 805 eintritt.

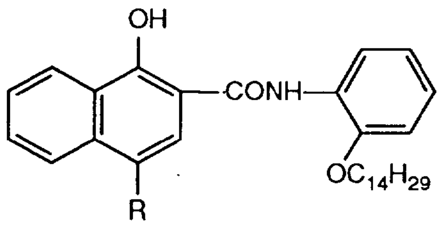

R = 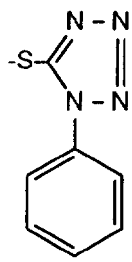 DIR-1

R = 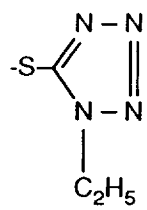 DIR-2

R = 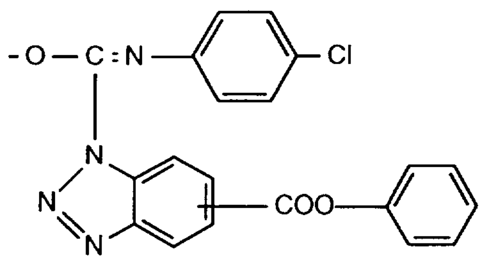 DIR-3

R = 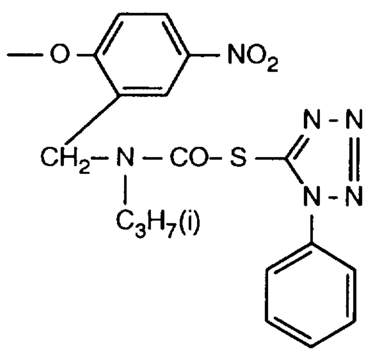 DIR-4

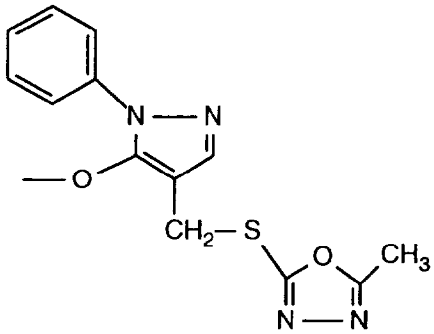

DIR-5

R =

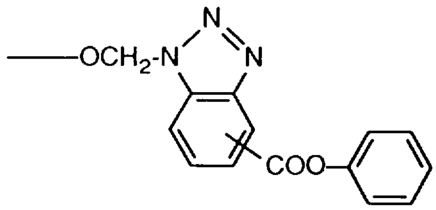

DIR-6

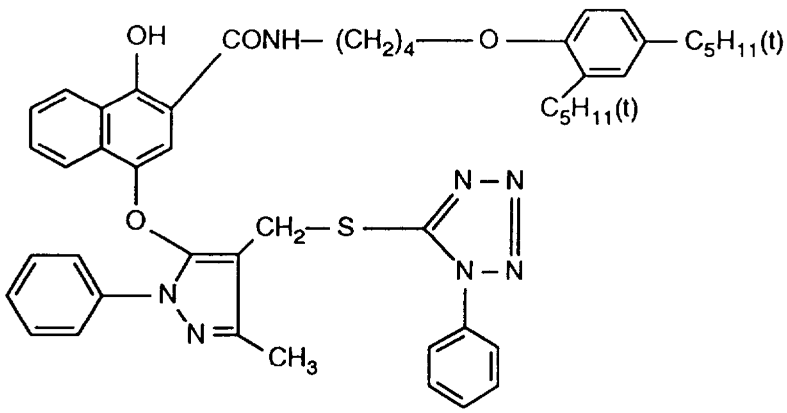

DIR-7

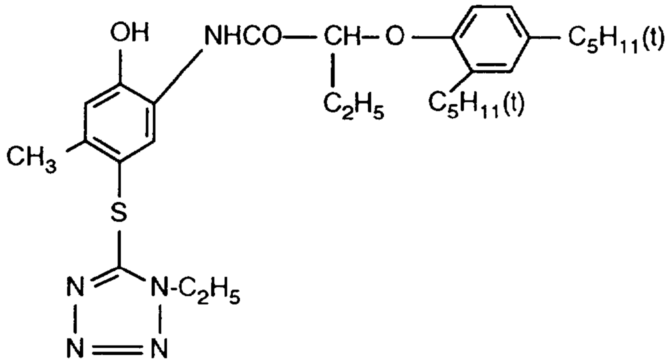

DIR-8

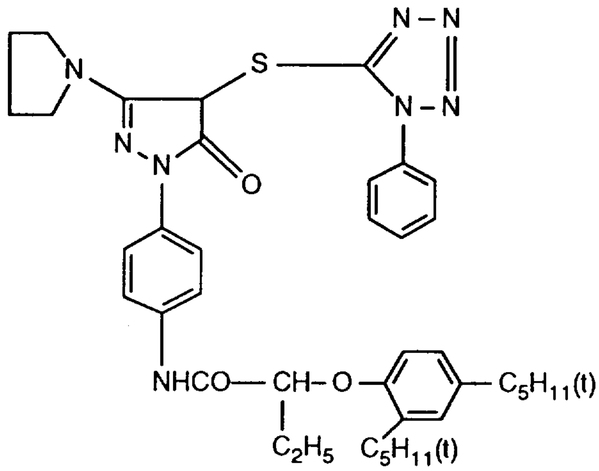

DIR-9

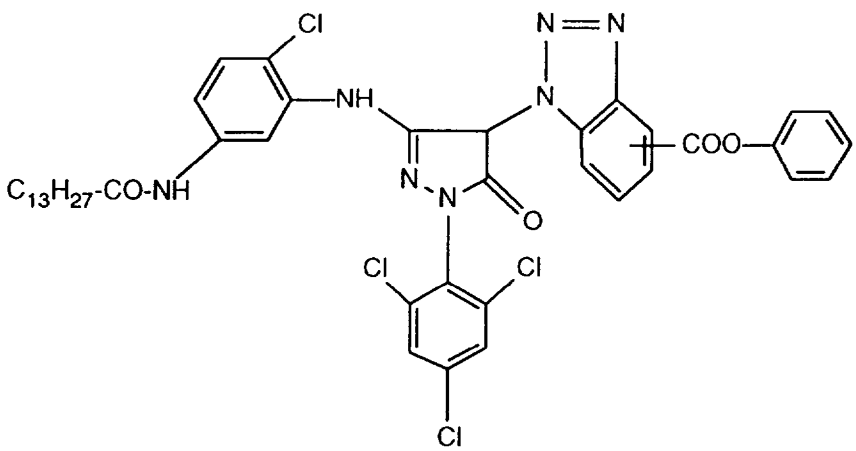

DIR-10

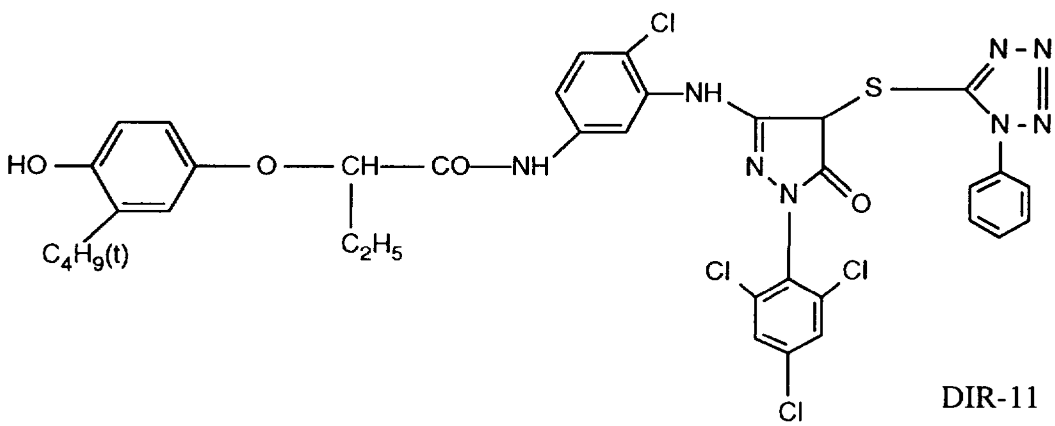

DIR-11

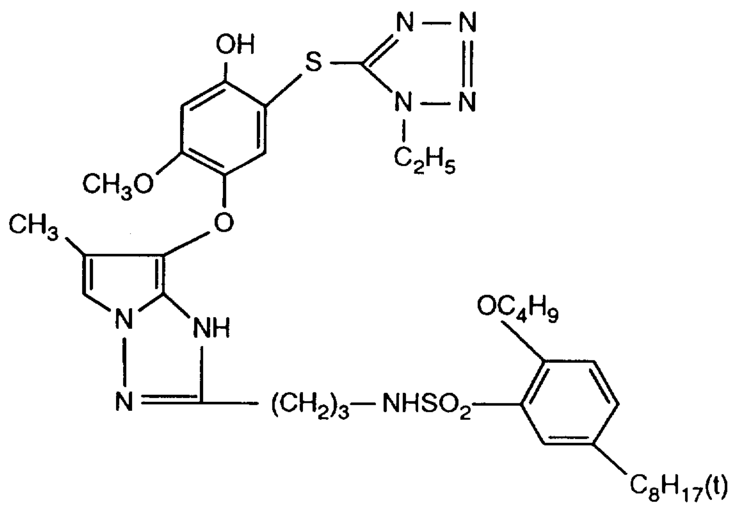

DIR-12

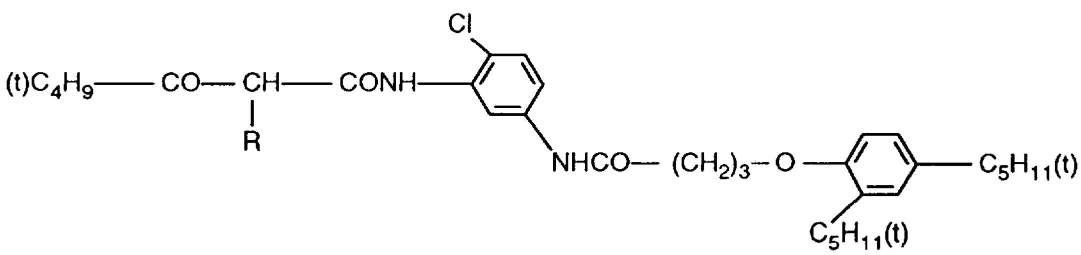

R = 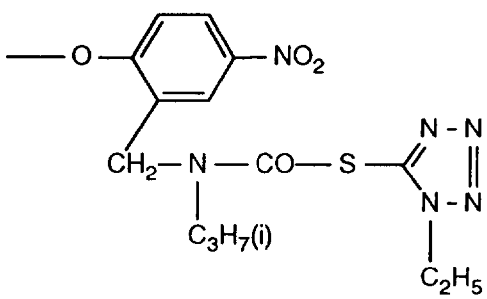

DIR-13

R = 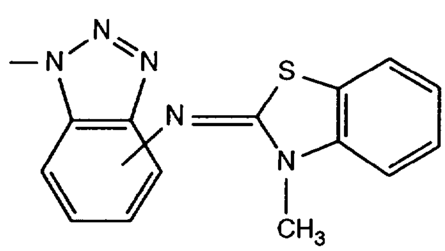

DIR-14

R = 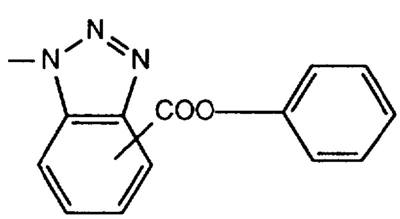

DIR-15

R = 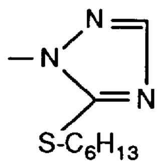 DIR-16

R = 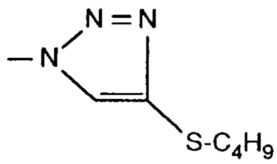 DIR-17

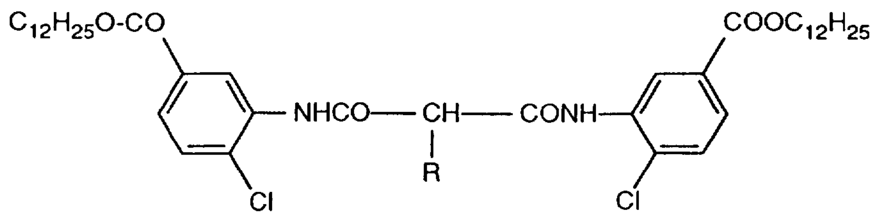

R = 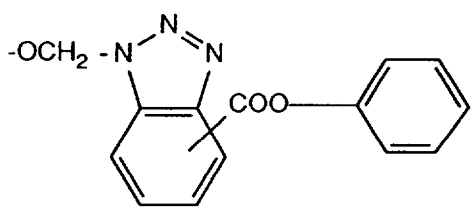 DIR-18

R = 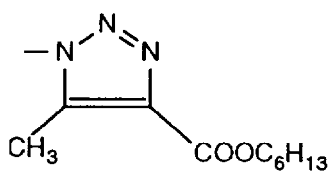 DIR-19

R = 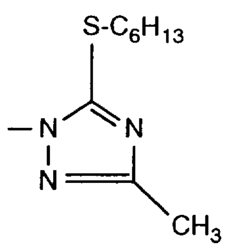 DIR-20

R = 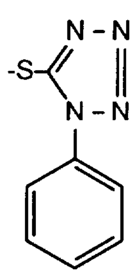 DIR-21

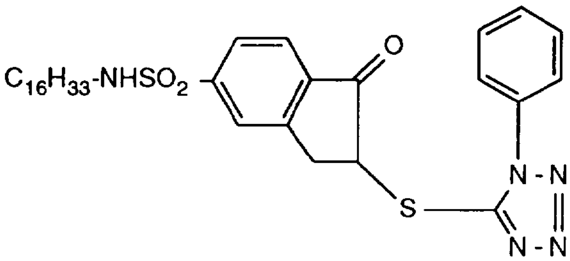

DIR-22

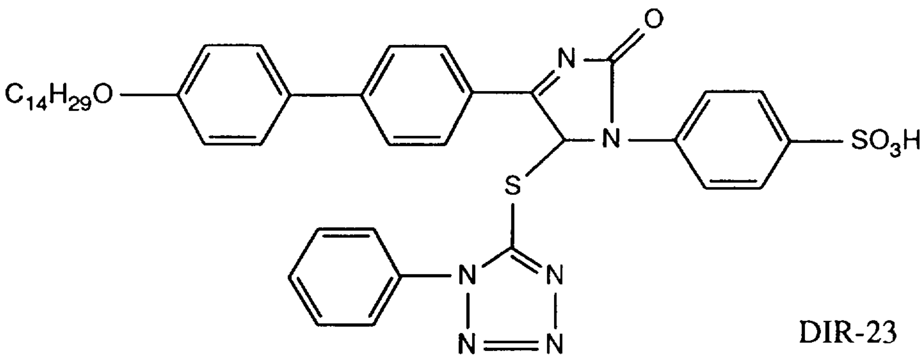

DIR-23

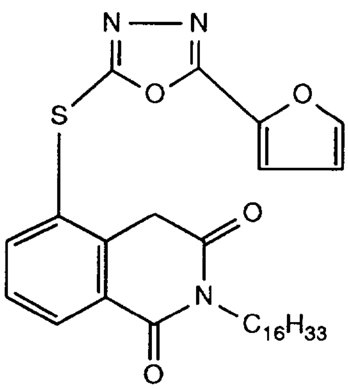

DIR-24

Beispiele für DAR-Kuppler

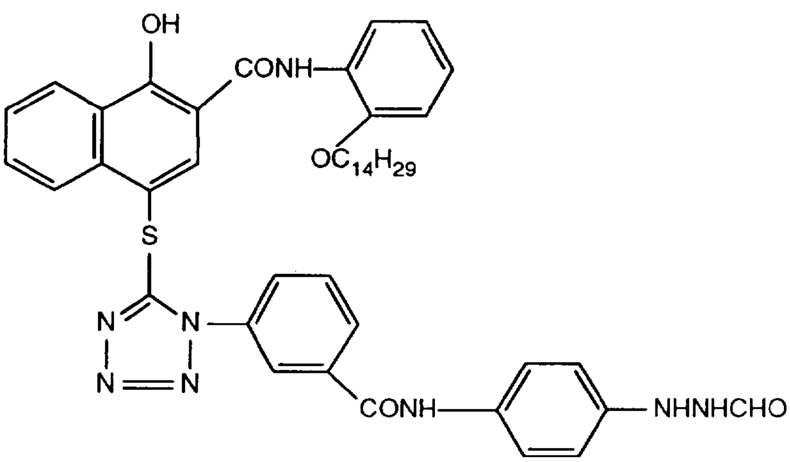

DAR-1

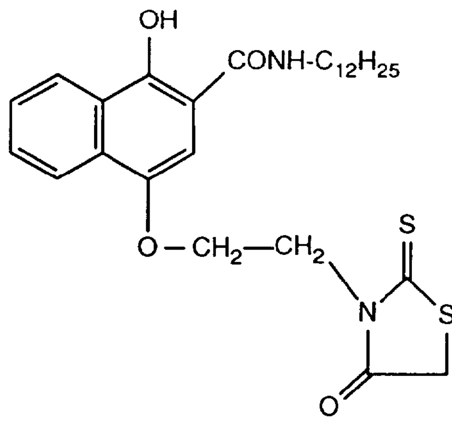

DAR-2

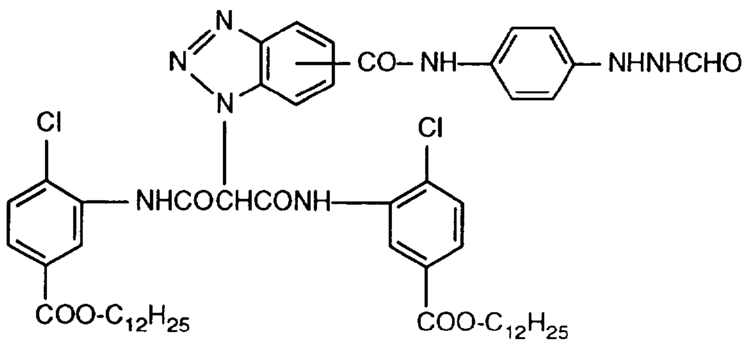

DAR-3

Da bei den DIR-, DAR- bzw. FAR-Kupplern hauptsächlich die Wirksamkeit des bei der Kupplung freigesetzten Restes erwünscht ist und es weniger auf die farbbildenden Eigenschaften dieser Kuppler ankommt, sind auch solche DIR-, DAR- bzw. FAR-Kuppler geeignet, die bei der Kupplung im wesentlichen farblose Produkte ergeben (DE-A-15 47 640).

Der abspaltbare Rest kann auch ein Ballastrest sein, so daß bei der Reaktion mit Farbentwickleroxidationsprodukten Kupplungsprodukte erhalten werden, die diffusionsfähig sind oder zumindest eine schwache bzw. eingeschränkte Beweglichkeit aufweisen (US-A-4 420 556).

Das Material kann weiterhin von Kupplern verschiedene Verbindungen enthalten, die beispielsweise einen Entwicklungsinhibitor, einen Entwicklungsbeschleuniger einen Bleichbeschleuniger, einen Entwickler, ein Silberhalogenidlösungsmittel, ein Schleiermittel oder ein Antischleiermittel in Freiheit setzen könne, beispielsweise sogenannte DIR-Hydrochinone und andere Verbindungen, wie sie beispielsweise in US-A-4 636 546, 4 345 024, 4 684 604 und in DE-A-31 45 640, 25 15 213, 24 47 079 und in EP-A-198 438 beschrieben sind. Diese Verbindungen erfüllen die gleiche Funktion wie die DIR-, DAR- oder FAR-Kuppler, außer daß sie keine Kupplungsprodukte bilden.

Hochmolekulare Farbkuppler sind beispielsweise in DE-A-1 297 417, DE-A-24 07 569, DE-A-31 48 125, DE-A-32 17 200, DE-A-33 20 079, DE-A- 33 24 932, DE-A-33 31 743, DE-A-33 40 376, EP-A-27 284, US-A-4 080 211 beschrieben. Die hochmolekularen Farbkuppler werden in der Regel durch Polymerisation von ethylenisch ungesättigten monomeren Farbkupplern hergestellt. Sie können aber auch durch Polyaddition oder Polykondensation erhalten werden.

Die Einarbeitung der Kuppler oder anderer Verbindungen in Silberhalogenidemulsionsschichten kann in der Weise erfolgen, daß zunächst von der betreffenden Verbindung eine Lösung, eine Dispersion oder eine Emulsion hergestellt und dann der Gießlösung für die betreffende Schicht zugefügt wird. Die Auswahl des geeigneten Lösungs- oder Dispersionsmittels hängt von der jeweiligen Löslichkeit der Verbindung ab.

Methoden zum Einbringen von in Wasser im wesentlichen unlöslichen Verbindungen durch Mahlverfahren sind beispielsweise in DE-A-26 09 741 und DE-A-26 09 742 beschrieben.

Hydrophobe Verbindungen können auch unter Verwendung von hochsiedenden Lösungsmitteln, sogenannten Ölbildnern, in die Gießlösung eingebracht werden. Entsprechende Methoden sind beispielsweise in US-A-2 322 o27, US-A-2 801 170, US-A-2 801 171 und EP-A-0 043 037 beschrieben.

Anstelle der hochsiedenden Lösungsmitteln können Oligomere oder Polymere, sogenannte polymere Ölbildner Verwendung finden.

Die Verbindungen können auch in Form beladener Latices in die Gießlösung eingebracht werden. Verwiesen wird beispielsweise auf DE-A-25 41 230, DE-A-25 41 274, DE-A-28 35 856, EP-A-0 014 921, EP-A-0 069 671, EP-A-0 130 115, US-A-4 291 113.

Die diffusionsfeste Einlagerung anionischer wasserlöslicher Verbindungen (z.B. von Farbstoffen) kann auch mit Hilfe von kationischen Polymeren, sogenannten Beizenpolymeren erfolgen.

Die erfindungsgemäß verwendeten UV-Absorber der Formel I bzw. entsprechenden Homo- oder Copolymere können allein oder zusammen mit dem Farbkuppler und gegebenenfalls weiteren Zusätzen in das farbfotografische Material eingearbeitet werden, indem man sie in hochsiedenden organischen Lösungsmitteln vorlöst.

Geeignete hochsiedende Lösungsmittel sind z.B. Phthalsäurealkylester, Phosphonsäureester, Phosphorsäureester, Citronensäureester, Benzoesäureester, Amide, Fettsäureester, Trimesinsäureester,. Alkohole, Phenole, Anilinderivate und Kohlenwasserstoffe.

Beispiele für geeignete hochsiedende Lösungsmittel sind Dibutylphthalat, Dicyclohexylphthalat, Di-2- ethylhexylphthalat, Decylphthalat, Triphenylphosphat, Tricresylphosphat, 2-Ethylhexyldiphenylphosphat, Tridecylphosphat, Tributoxyethylphosphat, Trichlorpropylphosphat, Di-2-ethylhexylphenylphosphat, 2-Ethylhexylbenzoat, Dodecylbenzoat, 2-Ethylhexyl-p-hydroxybenzoat, Diethyldodecanamid, N-Tetradecylpyrrolidon, Isostearylalkohol, 2,4-Di-t-amylphenol, Dioctylacelat, Glycerintributyrat, Isostearyllactat, Trioctylcitrat, N,N-Dibutyl-2-butoxy-5-t-octylanilin, Paraffin, Didecylbenzol und Diisopropylnaphthalin.

Weitere Details über verwendbare hochsiedende Lösungsmittel sind in den folgenden Veröffentlichungen zu finden:

Phosphate: GB-A-791,219, BE-A-755,248, JP-A-76/76739, 78/27449, 78/218,252, 78/97573, 79/148,133, 82/216,177, 82/93323 und 83/216,177 und EP-A265,296.

Phthalate: GB-A-791,219, JP-A-77/98050, 82/93322, 82/216,176, 82/218,251, 83/24321, 83/45699, 84/79888.

Amide: GB-A-791,129, JP-A-76/105,043, 77/13600, 77/61089, 84/189,556, 87/239,149, US-A-928,741, EP-A-270,341, WO 88/00723.

Phenole: GB-A-820,329, FR-A-1,220,657, JP-A-69/69946, 70/3818, 75/123,026, 75/82078, 78/17914, 78/21166, 82/212,114 und 83/45699.

Andere sauerstoffhaltige Verbindungen: US-A-3,748,141, 3,779,765, JP-A-73/75126, 74/101,114, 74/10115, 75/101,625, 76/76740, 77/61089, EP-A-304,810 und BE-A-826,039.

Sonstige Verbindungen: JP-A-72/115,369, 72/130,258, 73/127,521, 73/76592, 77/13193, 77/36294, 79/95233, 91/2,748, 83/105,147 und Research Disclosure 82/21918.

Die Menge an hochsiedendem Lösungsmittel liegt z.B. im Bereich von 50 mg bis 2 g pro $m^2$ Träger, vorzugsweise von 200 mg bis 1 g pro $m^2$.

Gegebenenfalls können die UV-Absorber auch ohne Öl in der Gelatineschicht dispergiert werden; Research Disclosure 88/296017 und 89/303070.

Weiter kann der UV-Absorber oder ein Gemisch von UV-Absorbern so in mindestens eine der fotografischen Schichten eingebracht werden, daß ein Latex mit kleinen lipophilen Partikeln (typischer Durchmesser 0,02 bis 2 $\mu$m) hergestellt wird, der sowohl den UV-Absorber als auch ein hydrophobes Polymer enthält. Eine entsprechende Technik ist beispielsweise in Spalte 17 der US-A-5 200 307 für Benztriazole beschrieben. Erfindungsgemäß können nun UV-Absorber der Formel I bzw. entsprechende Homo- oder Copolymere, allein oder in Kombination mit einem anderen UV-Absorber derselben oder einer anderen Klasse, z.B. der 2-Hydroxyphenylbenztriazole, zusammen mit einem hydrophoben Polymer in einem geeigneten organischen Lösungsmittel wie beispielsweise Ethylacetat gelöst werden; diese Lösung kann anschließend emulgiert und zu einem Latex in Wasser oder wässriger Gelatine dispergiert werden. Nach Abtrennen des organischen Lösungsmittels kann der Latex in das fotografische System eingebracht werden. Als hydrophobes Polymer eignet sich dabei z.B. ein Homo- oder Copolymer, wie es durch Polymerisation von weiter oben beschriebenen ethylenisch ungesättigten Monomeren der Formeln II bis VII erhalten werden kann. In bestimmten Fällen kann es sich bei dem hydrophoben Polymer um ein Kondensationspolymer handeln, z.B. um einen Polyester wie 1,4-Butandiol-adipinsäure-polyester oder Polycaprolakton. Zusätzlich kann auch ein hochsiedendes organisches Lösungsmittel zur Anwendung gelangen, wenn beispielsweise der eingesetzte UV-Absorber nicht flüssig ist. Auch Mischungen von geeigneten organischen Lösungsmitteln können zweckmäßig eingesetzt werden.

Gegenstand der Erfindung ist daher auch ein fotografisches Aufzeichnungsmaterial, dadurch gekennzeichnet, daß es in mindestens einer der Schichten zusätzlich zu dem copolymeren UV-Absorber ein hydrophobes Polymer enthält. Dieses kann beispielsweise ein hydrophobes Homo- oder Copolymer aus Monomeren der weiter oben beschriebenen Formeln II bis VII sein. Vorzugsweise enthält dieses Polymer kein Polyoxyalkylen, keine Hydroxylgruppen und keine Sulfongruppen.

Eine andere Technik, beispielsweise in GB-A-2 016 017 oder US-A-5 372 922 analog beschrieben, besteht darin, einen gemäß obiger Beschreibung durch Emulsionspolymerisation hergestellten Latex umfassend kleine wasserunlösliche, ein Lösungsmittel enthaltende Partikel mit dem erfindungsgemäßen UV-Absorber zu versetzen; der UV-Absorber wird daraufhin in die Partikel aufgenommen. Anschließend kann der beladene Latex in das fotografische System eingebracht werden.

Die Erfindung betrifft daher weiterhin ein fotografisches Aufzeichnungsmaterial enthaltend in mindestens einer der

Schichten den copolymeren UV-Absorber und das hydrophobe Polymer, welches dadurch erhältlich ist, daß der UV-Absorber und das hydrophobe Polymer in einem organischen Lösungsmittel gelöst und hierauf in wässrigem Milieu emulgiert, dispergiert und als Latex ins fotografische System eingebracht werden, sowie ein entsprechendes Herstellungsverfahren für fotografisches Aufzeichnungsmaterial.

Jede der unterschliedlich sensibilisierten, lichtempfindlichen Schichten kann aus einer einzigen Schicht bestehen oder auch zwei oder mehr Silberhalogenidemulsionsteilschichten umfassen (DE-C-1 121 470). Dabei sind rotempfindliche Silberhalogenidemulsionsschichten dem Schichtträger häufig näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtempfindliche gelbe Filterschicht befindet.

Bei geeignet geringer Eigenempfindlichkeit der grün- bzw. rotempfindlichen Schichten kann man unter Verzicht auf die Gelbfilterschicht andere Schichtanordnungen wählen, bei denen auf den Träger z.B. die blauempfindlichen, dann die rotempfindlichen und schließlich die grünempfindlichen Schichten folgen.

Die in der Regel zwischen Schichten unterschiedlicher Spektralempfindlichkeit angeordneten nicht lichtempfindlichen Zwischenschichten können Mittel enthalten, die eine unerwünschte Diffusion von Entwickleroxidationsprodukten aus einer lichtempfindlichen in eine andere lichtempfindliche Schicht mit unterschiedlicher spektraler Sensibilisierung verhindern.

Geeignete Mittel, die auch Scavenger oder EOP-Fänger genannt werden, werden in Research Disclosure 17 643 (Dez. 1978), Kapitel VII, 17 842 (Feb. 1979) und 18 716 (Nov. 1979), Seite 650 sowie in EP-A-0 069 070, 0 098 072, 0 124 877, 0 125 522 beschrieben.

Beispiele für besonders geeignete Verbindungen sind:

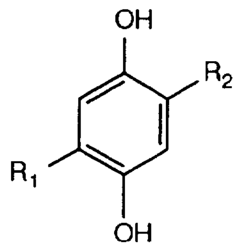

$R_1$, $R_2$ = $C_8H_{17}(t)$
$C_{12}H_{25}(S)$
$C_6H_{13}(t)$

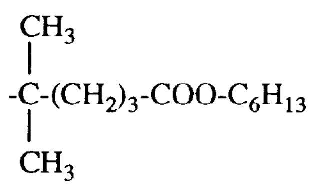

$C_8H_{17}(s)$
$C_{15}H_{31}(t)$

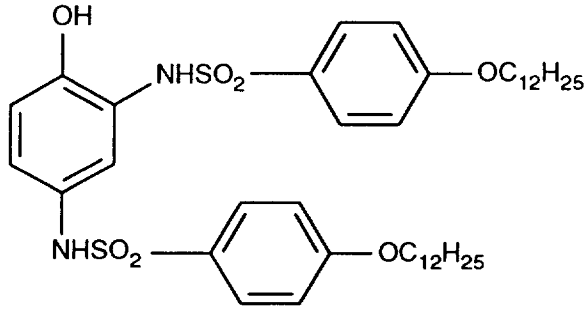

Liegen mehrere Teilschichten gleicher spektraler Sensibilisierung vor, so können sich diese hinsichtlich ihrer Zusammensetzung, insbesondere was Art und Menge der Silberhalogenidkörnchen betrifft unterscheiden. Im allgemeinen wird die Teilschicht mit höherer Empfindlichkeit von Träger entfernter angeordnet sein als die Teilschicht mit geringerer Empfindlichkeit. Teilschichten gleicher spektraler Sensibilisierung können zueinander benachbart oder durch andere Schichten, z.B. durch Schichten anderer spektraler Sensibilisierung getrennt sein. So können z.B. alle hochempfindlichen und alle niedrigempfindlichen Schichten jeweils zu einem Schichtpaket zusammengefaßt sein (DE-A-19 58 709, DE-A-25 30 645, DE-A-26 22 922).

Das fotografische Material kann weiterhin UV-Licht absorbierende Verbindungen, Weißtöner, Abstandshalter, Filterfarbstoffe, Formalinfänger, Lichtschutzmittel, Antioxidantien, $D_{Min}$-Farbstoffe, Zusätze zur Verbesserung der Farbstoff-, Kuppler- und Weißentstabilisierung sowie zur Verringerung des Farbschleiers, Weichmacher (Latices), Biocide und anderes enthalten.

Die fotografischen Schichten im erfindungsgemäßen Material, insbesondere die Schichten b, c und/oder d im oben beispielhaft beschriebenen farbfotografischen Material, können auch weitere UV-Absorber enthalten. Beispiele für solche UV-Absorber sind Benztriazole, 2-Hydroxybenzophenone, Oxanilide, Cyanoacrylate, Salicylsäureester, Acrylnitrilderivate oder Thiazoline.

Solche UV-Absorber sind z.B. in folgenden Veröffenlichungen näher erläutert: US-A-3,314,794, 3,352,681, 3,705,805, 3,707,375, 4,045,229, 3,700,455, 3,700,458, 3,533,794, 3,698,907, 3,705,805, 3,738,837, 3,762,272, 4,163,671, 4,195,999, 4,309,500, 4,431,726, 4,443,543, 4,576,908, 4,749,643, GB-A-1,564,089, EP-A-190,003 und JP-A-71/2784, 81/111,826, 81/27,146, 88/53,543 und 88/55,542. Bevorzugte UV-Absorber sind Benzotriazole, insbesondere 2-(2-Hydroxyphenyl)-benztriazole.

Bevorzugt ist auch fotografisches Aufzeichnungsmaterial enthaltend zusätzlich einen nicht der Formel (I) entsprechenden UV-Absorber aus der Reihe der Hydroxyphenyltriazine, wie sie beispielsweise in US 5,300,414 und US 5,364,749 beschrieben sind.

Beispiele besonders geeigneter Verbindungen sind:

Benzotriazolverbindungen der Formel AII

(AII)

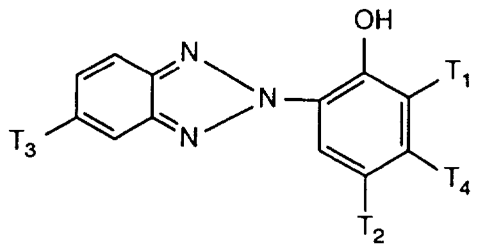

worin $T_1$, $T_2$ und $T_3$ unabhängig voneinander Wasserstoff, Halogen, Alkyl, Alkyl substituiert mit einer Carbonsäureestergruppe, Alkoxy, Aryloxy, Hydroxyl oder Acyloxy sind, und $T_4$ Wasserstoff, Alkoxy, Aryloxy oder Acyloxy ist.

Beispiele für HBT-Verbindungen der Formel AII sind:

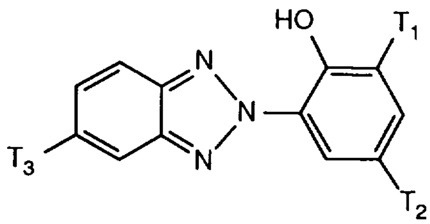

| HBT-Nr. | $T_1$ | $T_2$ | $T_3$ |
|---|---|---|---|
| HBT-1 | H | $CH_3$ | H |
| HBT-2 | H | $C(CH_3)_3$ | H |
| HBT-3 | $C(CH_3)_3$ | $CH_3$ | Cl |
| HBT-4 | $C(CH_3)_3$ | $C(CH_3)_3$ | Cl |
| HBT-5 | $C(CH_3)_2C_2H_5$ | $C(CH_3)_2C_2H_5$ | H |
| HBT-6 | $CH(CH_3)C_2H_5$ | $C(CH_3)_3$ | H |
| HBT-7 | $C(CH_3)_2$- 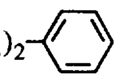 | $C(CH_3)_2$- 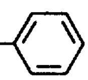 | H |
| HBT-8 | $C(CH_3)_3$ | $CH_2CH_2COOC_8H_{17}$ (Isomere)* | Cl |
| HBT-9 | $C(CH_3)_3$ | $CH_2CH_2COOC_8H_{17}$ (Isomere)* | H |
| HBT-10 | $C_{12}H_{25}$ (Isomere)* | $CH_3$ | H |

*Hauptprodukt

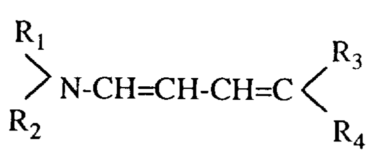

$R_1$, $R_2$ = $-C_6H_{13}(n)$; $R_3$, $R_4$ = -CN

$R_1, R_2 = -C_2H_5; R_3 =$ 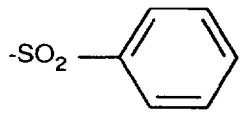 $; R_4 = -CO-OC_8H_{17}$

$R_1, R_2 = -C_2H_5; R_3 =$ 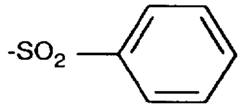 $; R_4 = -COO-C_{12}H_{25}$

$R_1, R_2 = -CH_2=CH-CH_2; R_3, R_4 = -CN$

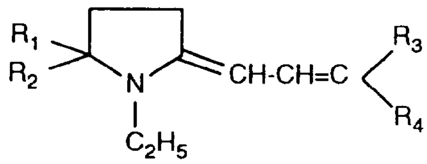

$R_1, R_2 = H; R_3 = -CN; R_4 = -CO-NHC_{12}H_{25}$

$R_1, R_2 = -CH_3; R_3 = -CN; R_4 = -CO-NHC_{12}H_{25}$

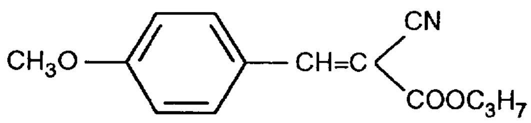

Es können auch ultraviolettabsorbierende Kuppler (wie Cyankuppler des α-Naphtholtyps) und ultraviolettabsorbierende Polymere verwendet werden. Diese Ultraviolettabsorbentien können durch Beizen in einer speziellen Schicht fixiert sein.

Für sichtbares Licht geeignete Filterfarbstoffe umfassen Oxonolfarbstoffe, Hemioxonolfarbstoffe, Styrylfarbstoffe, Merocyaninfarbstoffe, Cyaninfarbstoffe und Azofarbstoffe. Von diesen Farbstoffen werden Oxonolfarbstoffe, Hemioxonolfarbstoffe und Merocyaninfarbstoffe besonders vorteilhaft verwendet.

Geeignete Weißtöner sind z.B. in Research Disclosure 17 643 (Dez. 1978), Kapitel V, in US-A-2 632 701, 3 269 840 und in GB-A-852 075 und 1 319 763 beschrieben.

Bestimmte Bindemittelschichten, insbesondere die vom Träger am weitesten entfernte Schicht, aber auch gelegentlich Zwischenschichten, insbesondere, wenn sie während der Herstellung die vom Träger am weitesten entferte Schicht darstellen, können fotografisch inerte Teilchen anorganischer oder organischer Natur enthalten, z.B. als Mattierungsmittel oder als Abstandshalter (DE-A-33 31 542; DE-A-34 24 893, Research Disclosure 17 643 (Dez. 1978), Kapitel XVI).

Der mittlere Teilchendurchmesser der Abstandshalter liegt insbesondere im Bereich von 0,2 bis 10 µm. Die Abstandshalter sind wasserunlöslich und können alkaliunlöslich oder alkalilöslich sein, wobei die alkalilöslichen im allgemeinen im alkalischen Entwicklungsbad aus dem fotografischen Material entfernt werden. Beispiele für geeignete Polymere sind Polymethylacyrlat, Copolymere aus Acrylsäure und Methylmethacrylat sowie Hydroxypropylmethylcellulosehexahydrophthalat.

Geeignete Formalinfänger sind z.B.

$H_2N-CONH-(CH_2)_2-NH-CONH_2$,

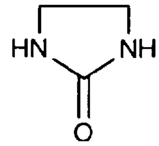 ,

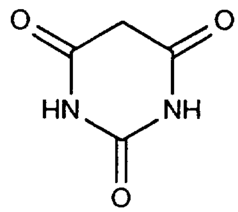 , 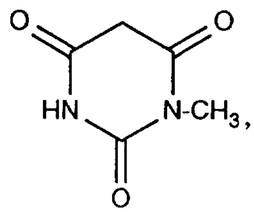 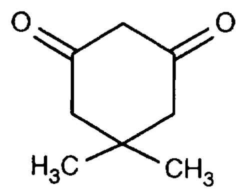 ,

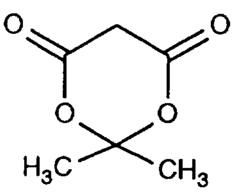 , 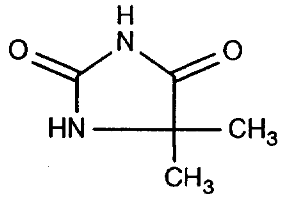 , 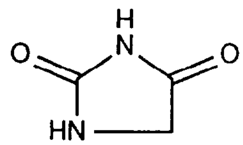 ,

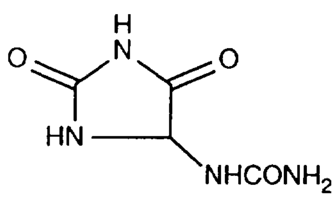 , 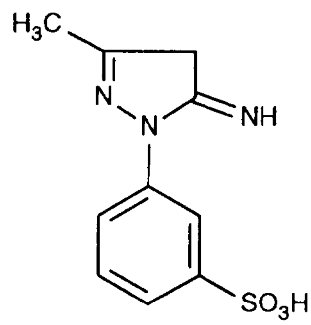 , 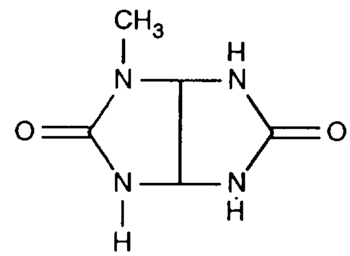

Die fotografischen Schichten können auch phenolische Verbindungen enthalten, die als Lichtschutzmittel für das Farbbild sowie als Mittel gegen Farbschleier wirken. Sie können in einer lichtempfindlichen Schicht (Farbschicht) oder in einer Zwischenschicht enthalten sein, allein oder zusammen mit anderen Additiven. Solche Verbindungen sind z.B. in den folgenden Veröffentlichungen näher beschrieben: US-A-3,700,455, 3,591,381, 3,573,052, 4,030,931, 4,174,220, 4,178,184, 4,228,235, 4,268,593, 4,279,990, 4,346,165, 4,366,226, 4,447,523, 4,528,264, 4,581,326, 4,562,146, 4,559,297, GB-A-1,309,277, 1,547,302, 2,023,862, 2,135,788, 2,139,370, 2,156,091; DE-A-2,301,060, 2,347,708, 2,526,468, 2,621,203, 3,323,448; DD-A-200,691, 214,468; EP-A-106,799, 113,124, 125,522, 159,912, 161,577, 164,030, 167,762, 176,845, 246,766, 320,776; JP-A-74/134,326, 76/127,730, 76/30462, 77/3822, 77/154,632, 78/10842, 79/48535, 79/70830, 79/73032, 79/147,038, 79/154,325, 79/155,836, 82/142,638, 83/224,353, 84/5246, 84/72443, 84/87456, 84/192,246, 84/192,247, 84/204,039, 84/204,040, 84/212,837, 84/220,733, 84/222,836, 84/228,249, 86/2540, 86/8843, 86/18835, 86/18836, 87/11456, 87/42245, 87/62157, 86/6652, 89/137,258 sowie in Research Disclosure 79/17804.

Die fotografischen Schichten können auch gewisse Phosphor-III-Verbindungen, insbesondere Phosphite und Phosponite, enthalten. Diese fungieren als Lichtschutzmittel für die Farbbilder sowie als Dunkellager-Stabilisator für Magentakuppler. Man setzt sie vorzugsweise den hochsiedenden Lösungsmitteln zu, zusammen mit dem Kuppler. Solche Phosphor-III-Verbindungen sind z.B. in den folgenden Veröffentlichungen näher beschrieben: US-A-4,407,935, US-A-4,436,811, US-A-4,956,406, EP-A-181,289, JP-A-73/32728, JP-A-76/1420 und JP-A-55/66741.

Die fotografischen Schichten können auch metallorganische Komplexe enthalten, die Lichtschutzmittel für die Farbbilder sind, insbesondere für die Magenta-Farbstoffe. Solche Verbindungen und deren Kombination mit anderen Additiven sind z.B. in folgenden Veröffentlichungen näher beschrieben: US-A-4,050,938, 4,239,843, 4,241,154, 4,242,429, 4,241,155, 4,242,430, 4,273,854, 4,246,329, 4,271,253, 4,242,431, 4,248,949, 4,245,195, 4,268,605, 4,246,330, 4,269,926, 4,245,018, 4,301,223, 4,343,886, 4,346,165, 4,590,153; JP-A-81/167,138, 81/168,652, 82/30834, 82/161,744; EP-A-137,271, 161,577, 185,506; DE-A-2,853,865.

Die fotografischen Schichten können auch Hydrochinonverbindungen enthalten. Diese wirken als Lichtschutzmittel für die Farbkuppler und für die Farbbilder sowie als Abfänger von oxidiertem Entwickler in Zwischenschichten. Sie werden vor allem in der Magentaschicht verwendet. Solche Hydrochinon-Verbindungen und deren Kombinationen mit anderen Additiven sind z.B. in folgenden Veröffentlichungen näher beschrieben:
US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866, 4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978, 4,430,425, 4,277,558, 4,489,155, 4,504,572, 4,559,297, FR-A-885,982; GB-A-891,158, 1,156,167, 1,363,921, 2,022,274, 2,066,975, 2,071,348, 2,081,463, 2,117,526, 2,156,091;

DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577;JP-A-75/33733, 75/21249, 77/128,130, 77/146,234, 79/70036, 79/133,131, 81/83742, 81/87040, 81/109,345, 83/134,628, 82/22237, 82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Die fotografischen Schichten können auch Derivate von Hydrochinonethern enthalten. Diese Verbindungen wirken als Lichtschutzmittel und sind besonders geeignet zur Stabilisierung von Magenta-Farbstoffen. Solche Verbindungen und deren Kombination mit anderen Additiven sind z.B. in folgenden Veröffentlichungen näher beschrieben: US-A 3,285,937, 3,432,300, 3,519,429, 3,476,772, 3,591,381, 3,573,052, 3,574,627, 3,573,050, 3,698,909, 3,764,337, 3,930,866, 4,113,488, 4,015,990, 4,113,495, 4,120,723, 4,155,765, 4,159,910, 4,178,184, 4,138,259, 4,174,220, 4,148,656, 4,207,111, 4,254,216, 4,134,011, 4,273,864, 4,264,720, 4,279,990, 4,332,886, 4,436,165, 4,360,589, 4,416,978, 4,385,111, 4,459,015, 4,559,297; GB-A 1,347,556, 1,366,441, 1,547,392, 1,557,237, 2,135,788; DE-A 3,214,567; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/70830, 79/73032, 79/95233, 79/145,530, 80/21004, 80/50244, 80/52057, 80/70840, 80/139,383, 81/30125, 81/151,936, 82/34552, 82/68833, 82/204,306 82/204,037, 83/134,634, 83/207,039, 84/60434, 84/101,650, 84/87450, 84/149,348, 84/182,785, 86/72040, 87/11455, 87/62157, 87/63149, 86/2151, 86/6652, 86/48855, 89/309,058 sowie in Research Disclosure 78/17051.

Die fotografischen Schichten, insbesondere die den erfindungsgemäßen UV-Absorber enthaltende(n) Schicht(en), können auch Lichtschutzmittel vom Typ der sterisch gehinderten Amine enthalten. Vorzugsweise handelt es sich dabei um ein 2,2,6,6-Tetraalkylpiperidinderivat, das mindestens eine Gruppe der Formel

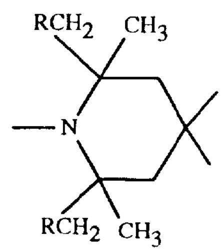

enthält, worin R Wasserstoff oder Methyl, insbesondere Wasserstoff, ist. Beispiele für einsetzbare gehinderte Amine sind u.a. die folgenden Verbindungen:

Bis(2,2,6,6-tetramethyl-piperidin-4-yl)-sebacat, Bis(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidy1)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis(1-octyloxy-2,2,6,6-tetramethylpiperidyl)-sebacat, Bis(1-octyloxy-2,2,6,6-tetramethylpiperidyl)-succinat, Kondensationsprodukt aus N,N'-Bis(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)-ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondenstionsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyi-4-piperidyl)-n-dodecylsuccinimid, 2-Undecyl-7,7,9,9-tetramethyl-1-oxa-3,8-diaza-4-oxo-spiro[4,5] decan, Umsetzungsprodukt von 7,7,9,9-Tetramethyl-2-cycloundecyl-1-oxa-3,8-diaza-4-oxospiro[4,5]decan und Epichlorhydrin.

Beispiele für besonders geeignete Verbindungen in den fotografischen Schichten sind:

(ST-1)

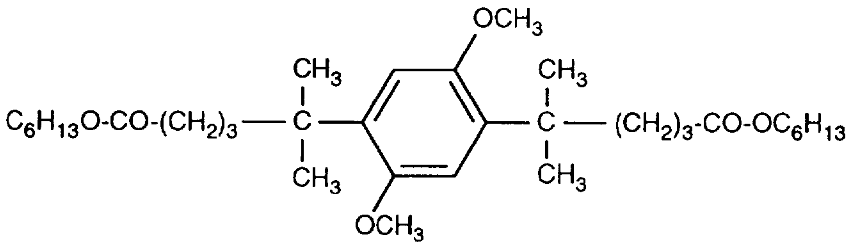

(ST-2)

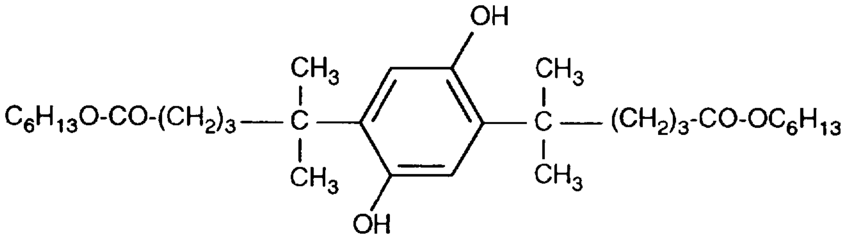

(ST-3)

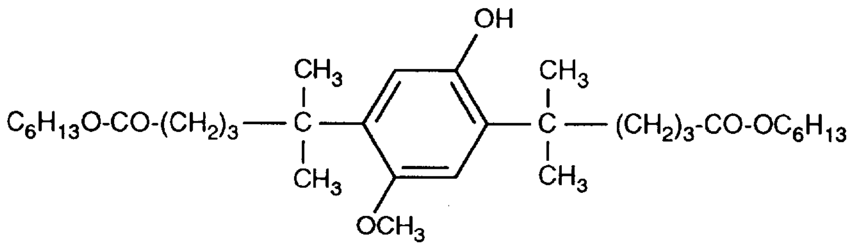

(ST-4)

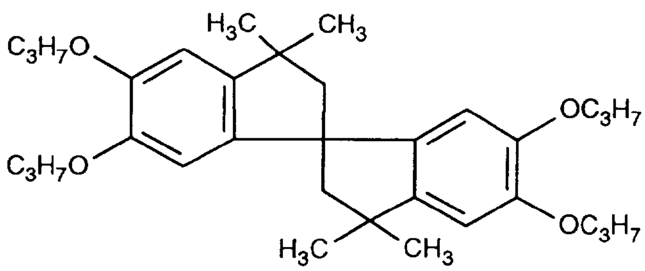

(ST-5)

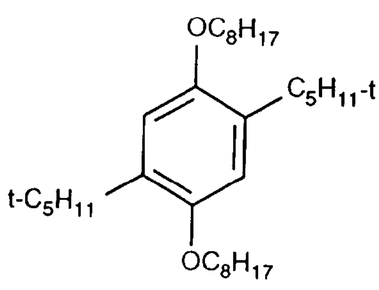

(ST-6)

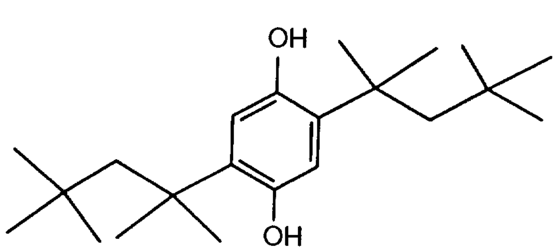

(ST-7)

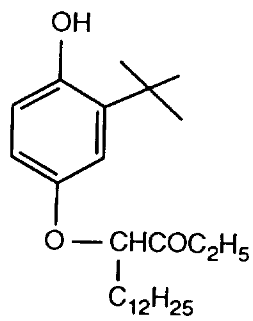

(ST-8)

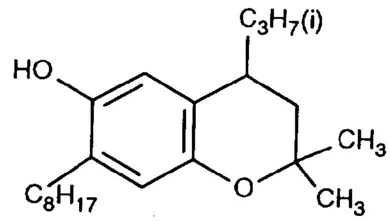

(ST-9)

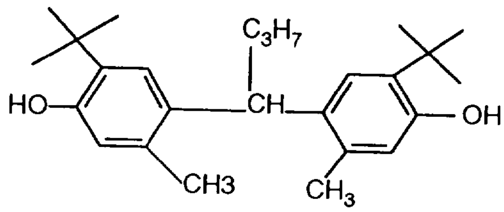

(ST-10)

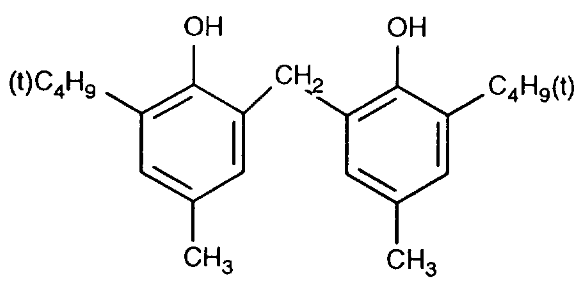

(ST-11)

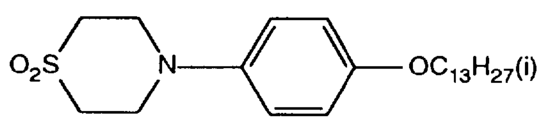

(ST-12)

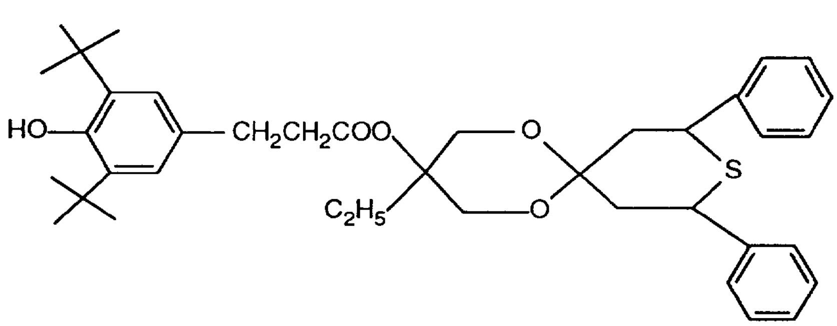

(ST-13)

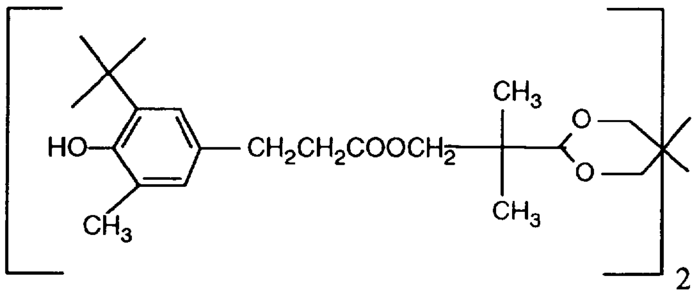

(ST-14)

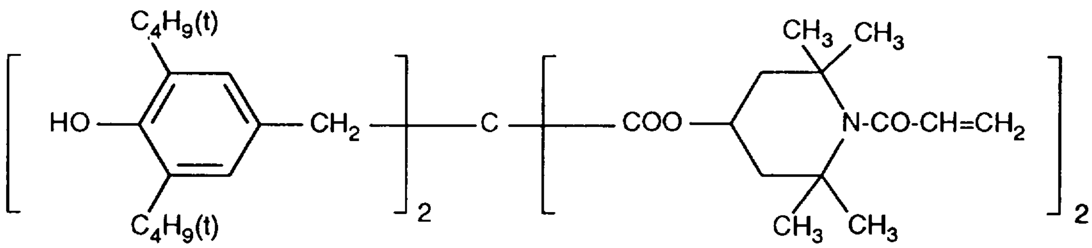

(ST-15)

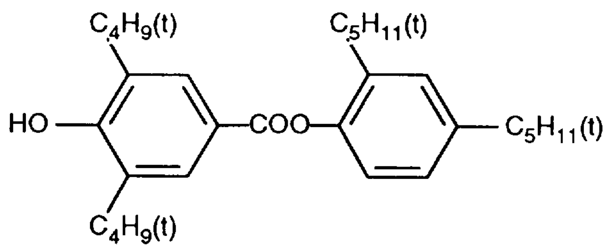

(ST-16)

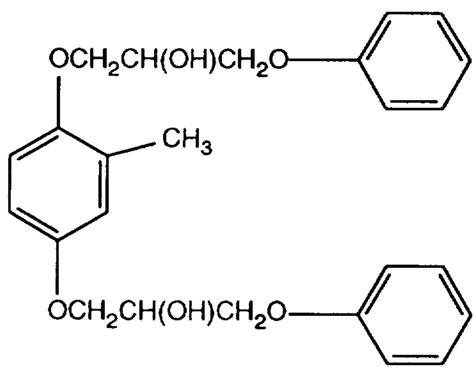

(ST-17)

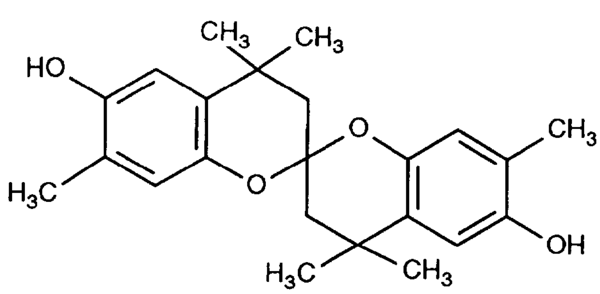

(ST-18)

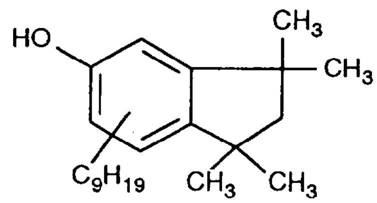

(ST-19)

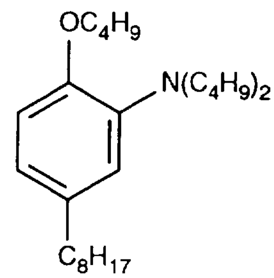

sowie die als EOP-Fänger aufgeführten Verbindungen.

Die Schichten des fotografischen Materials können mit den üblichen Härtungsmitteln gehärtet werden. Geeignete Härtungsmittel sind z.B. Formaldehyd, Glutaraldehyd und ähnliche Aldehydverbindungen, Diacetyl, Cyclopentadion und ähnliche Ketonverbindungen, Bis-(2-chlorethylharnstoff), 2-Hydroxy-4,6-dichlor-1,3,5-triazin und andere Verbindungen, die reaktives Halogen enthalten (US-A-3 288 775, US-A-2 732 303, GB-A-974 723 und GB-A-1 167 207), Divinylsulfonverbindungen, 5-Acetyl-1,3-di-acryloylhexahydro-1,3,5-triazin und andere Verbindungen, die eine reaktive Olefinbindung enthalten (US-A-3 635 718, US-A-3 232 763 und GB-A-994 869); N-Hydroxymethylphthalimid und andere N-Methylolverbindungen (US-A-2 732 316 und US-A-2 586 168); Isocyanate (US-A-3 103 437); Aziridinverbindungen (US-A-3 017 280 und US-A-2 983 611); Säurederivate (US-A-2 725 294 und US-A-2 725 295); Verbindungen vom Carbodiimidtyp (US-A-3 100 704); Carbamoylpyridiniumsalze (DE-A-22 25 230 und US-A-24 39 511); Carbamoylpyridiniumverbindungen (DE-A-24 08 814); Verbindungen mit einer Phosphor-Halogen-Bindung (JP-A-113 929/83); N-Carbonyloximid-Verbindungen (JP-A-43353/81); N-Sulfonyloximido-Verbindungen (US-A-4 111 926), Dihydrochinolinverbindungen (US-A-4 013 468), 2-Sulfonyloxypyridiniumsalze (JP-A-110 762/81), Formamidiniumsalze (EP-A-0 162 308), Verbindungen mit zwei oder mehr N-Acyloximino-Gruppen (US-A-4 052 373), Epoxyverbindungen (US-A-3 091 537), Verbindungen vom Isoxazoltyp (US-A-3 321 313 und US-A-3 543 292); Halogencarboxyaldehyde, wie Mucochlorsäure; Dioxanderivate, wie Dihydroxydioxan und Di-chlordioxan; und anorganische Härter, wie Chromalaun und Zirkonsulfat.

Die Härtung kann in bekannter Weise dadurch bewirkt werden, daß das Härtungsmittel der Gießlösung für die zu härtende Schicht zugesetzt wird, oder dadurch, daß die zu härtende Schicht mit einer Schicht überschichtet wird, die ein diffusionsfähiges Härtungsmittel enthält.

Unter den aufgeführten Klassen gibt es langsam wirkende und schnell wirkende Härtungsmittel sowie sogenannte Soforthärter, die besonders vorteilhaft sind. Unter Soforthärtern werden Verbindungen verstanden, die geeignete Bindemittel so vernetzen, daß unmittelbar nach Beguß, spätestens nach 24 Stunden, vorzugsweise spätestens nach 8 Stunden die Härtung so weit abgeschlossen ist, daß keine weitere durch die Vernetzungsreaktion bedingte Aenderung der Sensitometrie und der Quellung des Schichtverbandes auftritt. Unter Quellung wird die Differenz von Naßschichtdicke und Trockenschichtdicke bei der wäßrigen Verarbeitung des Films verstanden (Photogr. Sci., Eng. 8 (1964), 275; Photogr. Sci., Eng. (1972), 449).

Bei diesen mit Gelatine sehr schnell reagierenden Härtungsmitteln handelt es sich z.B. um Carbamoylpyridiniumsalze, die mit freien Carboxylgruppen der Gelatine zu reagieren vermögen, so daß letztere mit freien Aminogruppen der Gelatine unter Ausbildung von Peptidbindungen und Vernetzung der Gelatine reagieren.

Geeignete Beispiele für Soforthärter sind z.B. Verbindungen der allgemeinen Formel

(a)

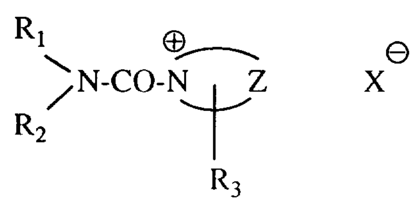

worin

$R_1$ Alkyl, Aryl oder Aralkyl bedeutet,

$R_2$ die gleiche Bedeutung wie $R_1$ hat oder Alkylen, Arylen, Aralkylen oder Alkaralkylen bedeutet, wobei die zweite Bindung mit einer Gruppe der Formel

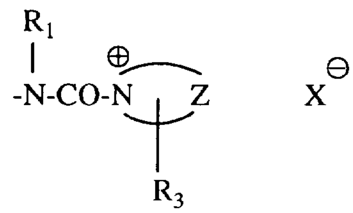

verknüpft ist, oder

$R_1$ und $R_2$ zusammen die zur Vervollständigung eines gegebenenfalls substituierten heterocyclischen Ringes, beispielsweise eines Piperidin-, Piperazin- oder Morpholinringes erforderlichen Atome bedeuten, wobei der Ring z.B. durch $C_1$-$C_3$-Alkyl oder Halogen substituiert sein kann,

$R_3$ für Wasserstoff, Alkyl, Aryl, Alkoxy, $-NR_4-COR_5$, $-(CH_2)_m-NR_8R_9$,

$-(CH_2)_n-CONR_{13}R_{14}$ oder 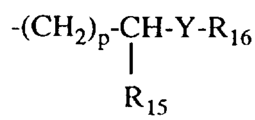

oder ein Brückenglied oder eine direkte Bindung an eine Polymerkette steht, wobei

$R_4$, $R_6$, $R_7$, $R_9$, $R_{14}$, $R_{15}$, $R_{17}$, $R_{18}$, und $R_{19}$ Wasserstoff oder $C_1$-$C_4$-Alkyl,

$R_5$ Wasserstoff, $C_1$-$C_4$-Alkyl oder $NR_6R_7$,

$R_8$ $-COR_{10}$

$R_{10}$ $NR_{11}R_{12}$

$R_{11}$ $C_1$-$C_4$-Alkyl oder Aryl, insbesondere Phenyl,

$R_{12}$ Wasserstoff, $C_1$-$C_4$-Alkyl oder Aryl, insbesondere Phenyl,

$R_{13}$ Wasserstoff, $C_1$-$C_4$-Alkyl oder Aryl, insbesondere Phenyl,

$R_{16}$ Wasserstoff, $C_1$-$C_4$-Alkyl, $-COR_{18}$ oder $CONHR_{19}$,

m eine Zahl 1 bis 3

n eine Zahl 0 bis 3

p eine Zahl 2 bis 3 und

Y O oder $NR_{17}$ bedeuten oder

$R_{13}$ und $R_{14}$ gemeinsam die zur Vervollständigung eines gegebenenfalls substituierten heterocyclischen Ringes, beispielsweise eines Piperidin-, Piperazin- oder Morpholinringes erforderlichen Atome darstellen, wobei der Ring z.B. durch $C_1$-$C_3$-Alkyl oder Halogen substituiert sein kann,

Z — die zur Vervollständigung eines 5- oder 6-gliedriegn aromatischen heterocyclischen Ringes, gegebenenfalls mit anelliertem Benzolring, erforderlichen C-Atome und

$X^{\ominus}$ — ein Anion bedeuten, das entfällt, wenn bereits eine anionische Gruppe mit dem übrigen Molekül verknüpft ist;

(b) 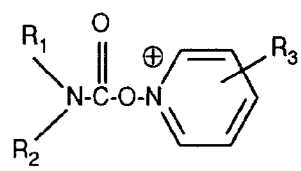 $X^{\ominus}$

worin

$R_1$, $R_2$, $R_3$ und $X^{\ominus}$ — die für Formel (a) amgegebene Bedeutung besitzen.

Es gibt diffusionsfähige Härtungsmittel, die auf alle Schichten innerhalb eines Schichtverbandes in gleicher Weise härtend wirken. Es gibt aber auch schichtbegrenzt wirkende, nicht diffundierende, niedermolekulare und hochmolekulare Härter. Mit ihnen kann man einzelnen Schichten, z.B. die Schutzschicht besonders stark vernetzen. Dies ist wichtig, wenn man die Silberhalogenid-Schicht wegen der Silberdeckkrafterhöhung wenig härtet und mit der Schutzschicht die mechanischen Eigenschaften verbessern muß (EP-A-0 114 699).

Farbfotografische Negativmaterialien werden üblicherweise durch Entwickeln, Bleichen, Fixieren und Wässern oder durch Entwickeln, Bleichen, Fixieren und Stabilisieren ohne nachfolgende Wässerung verarbeitet, wobei Bleichen und Fixieren zu einem Verarbeitungsschritt zusammengefaßt sein können. Als Farbentwicklerverbindung lassen sich sämtliche Entwicklerverbindungen verwenden, die die Fähigkeit besitzen, in Form ihres Oxidationsproduktes mit Farbkupplern zu Azomethin- bzw. Indophenolfarbstoffen zu reagieren. Geeignete Farbentwicklerverbindungen sind aromatische, mindestens eine primäre Aminogruppe enthaltende Verbindungen vom p-Phenylendiamintyp, beispielsweise N,N-Dialkyl-p-phenylendiamine wie N,N-Diethyl-p-phenylendiamin, 1-(N-Ethyl-N-methansulfonamidoethyl)-3-methyl-p-phenylendiamin und I-(N-Ethyl-N-methoxyethyl)-3-methyl-p-phenylendiamin. Weitere brauchbare Farbentwickler sind beispielsweise in J. Amer. Chem. Soc. 73, 3106 (1951) und G. Haist, Modern Photographic Processing, 1979, John Wiley and Sons, New York 545 ff. beschrieben.

Nach der Farbentwicklung kann ein saures Stoppbad oder eine Wässerung folgen.

Ueblicherweise wird das Material unmittelbar nach der Farbentwicklung gebleicht und fixiert. Als Bleichmittel können z.B. Fe(III)-Salze unf Fe(III)-Komplexsalze wie Ferricyanide, Dichromate, wasserlösliche Kobalt-Komplexe verwendet werden. Besonders bevorzugt sind Eisen-(III)-Komplexe von Aminopolycarbonsäuren, insbesondere z.B. von Ethylendiamintetraessigsäure, Propylendiamintetraessigsäure, Diethylentriaminpentaessigsäure, Nitrilotriessigsäure, Iminodiessigsäure, N-Hydroxyethyl-ethylendiamintriessigsäure, Alkyliminodicarbonsäuren und von entsprechenden Phosphonsäuren. Geeignete als Bleichmittel sind weiterhin Persulfate und Peroxide, z.B. Wasserstoffperoxid.

Auf das Bleichfixierbad oder Fixierbad folgt meist eine Wässerung, die als Gegenstromwässerung ausgeführt ist oder aus mehreren Tanks mit eigener Wasserzufuhr besteht.

Günstige Ergebnisse können bei Verwendung eines darauf folgenden Schlußbades, das keinen oder nur wenig Formaldehyd enthält, erhalten werden.

Die Wässerung kann aber durch ein Stabilisierbad vollständig ersetzt werden, das üblicherweise im Gegenstrom geführt wird. Dieses Stabilisierbad übernimmt bei Formaldehydzusatz auch die Funktion eines Schlußbades.

Bei Farbumkehrmaterialien erfolgt zunächst eine Entwicklung mit einem Schwarz-Weiß-Entwickler, dessen Oxidationsprodukt nicht zur Reaktion mit den Farbkupplern befähigt ist. Es schließt sich eine diffuse Zweitbelichtung und dann Entwicklung mit einem Farbentwickler, Bleichen und Fixieren an.

Ein weiterer Gegenstand der vorliegenden Erfindung ist ein Verfahren zum Stabilisieren von fotografischem Aufzeichnungsmaterial enthaltend auf einem Träger mindestens eine Silberhalogenidemulsionsschicht sowie gegebenenfalls mindestens eine Zwischenschicht und/oder eine Protektionsschicht, dadurch gekennzeichnet, daß mindestens einer der genannten Schichten ein UV-Absorber der Formel (I) bzw. ein entsprechendes Homo- oder Copolymer, wie sie weiter vorne näher beschrieben sind bzw. gemäß Anspruch 1, zugegeben wird.

Ein weiterer Gegenstand der vorliegenden Erfindung ist die Verwendung von Verbindungen der Formel (I) bzw. ein entsprechendes Homo- oder Copolymer, wie sie weiter vorne näher beschrieben sind bzw. gemäß Anspruch 1, zum Stabilisieren von fotografischem Aufzeichnungsmaterial enthaltend auf einem Träger mindestens eine Silberhalogenidemulsionsschicht sowie gegebenenfalls mindestens eine Zwischenschicht und/oder eine Protektionsschicht.

Für das erfindungsgemäße Verfahren, die erfindungsgemäße Verwendung und die erfindungsgemäßen Verbindungen der Formel (I) gelten sinngemäß die weiter vorne bei dem erfindungsgemäßen fotografischen Aufzeichnungsmaterial näher beschriebenen Bevorzugungen.

Die folgenden Beispiele beschreiben den Erfindungsgegenstand weiter, ohne ihn auf die Beispiele zu beschränken. Darin bedeuten Teile Gewichtsteile und % Gewichts-%; ist in einem Beispiel Raumtemperatur erwähnt, so ist darunter eine Temperatur im Bereich 20-25°C zu verstehen. Diese Vorgaben gelten jeweils sofern nichts anderes angegeben ist. Direkt hinter chemischen Symbolen stehende Zahlen bedeuten Indices der chemischen Formel, auch wenn sie nicht tiefgestellt sind.

Es gelten folgende Abkürzungen:

| | |
|---|---|
| THF | Tetrahydrofuran |
| AIBN | α,α'-Azoisobutyronitril |
| abs. | wasserfrei |
| Smp. | Schmelzpunkt bzw. Schmelzbereich |
| mmHg | Torr (1 mmHg = 133,3224 Pa) |
| MALDI | Matrix Assisted Laser Desorption Ionization |
| MS | Massenspektromtrie |
| NMR | Kernmagnetische Resonanz |
| GC | Gaschromatographie |
| GPC | Gelpermeationschromatographie |
| DSC | Differential-Scan Calorimetrie |
| Mn | Zahlenmittel der Molmasse (Einheit g/Mol) |
| Mw | Massenmittel der Molmasse (Einheit g/Mol) |
| Tg | Glastemperatur. |

Beispiel 1: Unter Stickstoff wird eine Mischung von 14,2 g (30 mmol) 2,4-Diphenyl-6-[2-hydroxy-4-(3-n-butoxy-2-hydroxy-propoxy-)phenyl-]1,3,5-triazin, 3,5 g (38 mmol)

Acryloylchlorid, 0,4 g (5 mmol) Pyridin in 100 ml Toluol bei 70°C während 24 Stunden erhitzt. Nach Zugabe von 4,5 g (44 mmol) Triethylamin wird für weitere 6 Stunden bei 70°C erhitzt. Man läßt abkühlen und filtriert den festen Rückstand ($(CH_3CH_2)_3N{\cdot}HCl$) ab. Das Filtrat wird eingedampft und man erhält 17,5 g des harzähnlichen Rohproduktes. Durch Säulenchromatografie (Kieselgel 60; 230-400 mesh; Eluiermittel $CH_2Cl_2/CH_3OH$ 95/5) erhält man 12,5 g (79 % Ausbeute) 2,4-Diphenyl-6-[2-hydroxy-4-(3-n-butoxy-2-acryloyloxy-propoxy-)phenyl-]1,3,5-triazin (Verbindung 100) als gelbliches Harz.

Das $^1$H-NMR-Spektrum ($CDCl_3$, 300 MHz) ist in Übereinstimmung mit dem gewünschten Produkt.

| Elementaranalyse für $C_{31}H_{31}N_3O_5$(525,60): | | | |
|---|---|---|---|
| Theorie: | C: 70,84 | H: 5,94 | N: 7,99 % |
| Gefunden: | C: 70,80 | H: 5,85 | N: 8,02 % |

Beispiel 2: Unter Stickstoff wird eine Mischung von 14,2 g (30 mmol)

2,4-Diphenyl-6-[2-hydroxy-4-(3-n-butoxy-2-hydroxy-propoxy-)phenyl-]1,3,5-triazin, 8,0 g (76 mmol) Methacryloylchlorid, 0,4 g (5 mmol) Pyridin in 100 ml Toluol bei 80°C während 48 Stunden erhitzt. Der Überschuß Methacryloylchlorid und das Toluol werden am Rotavapor entfernt. Nach Zugabe von 100 ml Toluol und 4,5 g (44 mmol) Triethylamin wird für 5 Stunden bei 75°C erhitzt. Man läßt abkühlen und filtriert den festen Rückstand ($(CH_3CH_2)_3N{\cdot}HCl$) ab. Das Filtrat wird eingedampft und man erhält 18,4 g des Rohproduktes. Durch Säulenchromatografie (Kieselgel 60; 230-400 mesh; Durchmesser 8 cm, h = 30 cm; Eluiermittel $CH_2Cl_2$) erhält man 8,5 g 2,4-Diphenyl-6-[2-hydroxy-4-(3-n-butoxy-2-methacryloyloxy-propoxy-)phenyl-]1,3,5-triazin (Verbindung 101) als gelbliches Harz.

Das $^1$H-NMR-Spektrum ($CDCl_3$, 300 MHz) ist in Übereinstimmung mit dem gewünschten Produkt.

| Elementaranalyse für $C_{32}H_{33}N_3O_5$(539,63): | | | |
|---|---|---|---|
| Theorie:<br>Gefunden: | C: 71,22<br>C: 70,42 | H: 6,16<br>H: 6,28 | N: 7,79 %<br>N: 7,57 % |

Zwischenprodukt für Beispiel 3: 2-[2-Hydroxy-4-(11-hydroxy-undecyloxy)-phenyl]-4,6-diphenyl-1,3,5-triazin

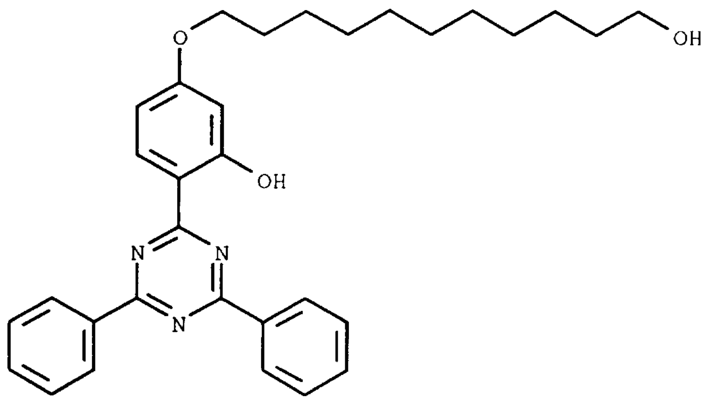

Eine Mischung aus 170,7 g (0,5 mol) of 2-(2,4-dihydroxyphenyl)-4,6-diphenyl-1,3,5-triazine, 28,1g (0,5 mol) pulverisiertem Kaliumhydroxid (Fluka, >85%) in 1000 ml Diglyme (Fluka, 99%) wird unter Stickstoff auf 80 °C erhitzt. Zu der gelben Lösung werden 155,4 g (0,6 mol) 11-Brom-1-undecanol (Fluka, 97%) gegeben. Die Mischung wird für 40 Stunden auf 100 °C gehalten. Anschließend wird der Feststoff heiß abfiltriert. Das beim Abkühlen auf 0°C kristallisierte Filtrat wird mit Hexan gewaschen und bei 50 °C/70 mmHg getrocknet. Man erhält 219,5 g (85,84 %) 2-[2-Hydroxy-4-(11-hydroxy-undecyl-oxy-)phenyl-]-4,6-diphenyl-1,3,5-triazin als Feststoff vom Smp. 131 - 132 °C.

| | | | | |
|---|---|---|---|---|
| Analyse: $C_{32}H_{37}N_3O_3$<br>(511,67) | berechnet<br>gefunden | C 76,12<br>C 75,06 | H 7,29<br>H 7,46 | N 8,21<br>N 8,13 |

Beispiel 3: 2-[2-Hydroxy-4-(11-acryloyl-oxy-undecyl-oxy)-phenyl-]-4,6-diphenyl-1,3,5-triazin

Einer Mischung aus 51,2 g (0,1 mol) 2-[2-Hydroxy-4-(1 1-hydroxy-undecyloxy-)-phenyl]-4,6-diphenyl)-1,3,5-triazin, 22,2 g (0,22 mol) Triethylamin, 500 ml Toluol (Merck, 99,5 %) unter Stickstoff wird tropfenweise binnen 20 min. bei 15 bis 20 °C 13,3 g (0,105 mol) 3-Chlorpropionsäurechlorid zugesetzt. Die weiße Suspension wird filtriert, das Filtrat mit Wasser gewaschen, mit $Na_2SO_4$ getrocknet und eingedampft. Der Rückstand wird in Toluol aufgenommen, durch eine Schüttung aus Kieselgel 60 filtriert und mit Toluol eluiert.

Man erhält 44,8 g (79,2 %) 2-[2-Hydroxy-4-(11-acryloyloxy-undecyloxy)-phenyl]-4,6-diphenyl-1,3,5-triazin (Verbindung 102)

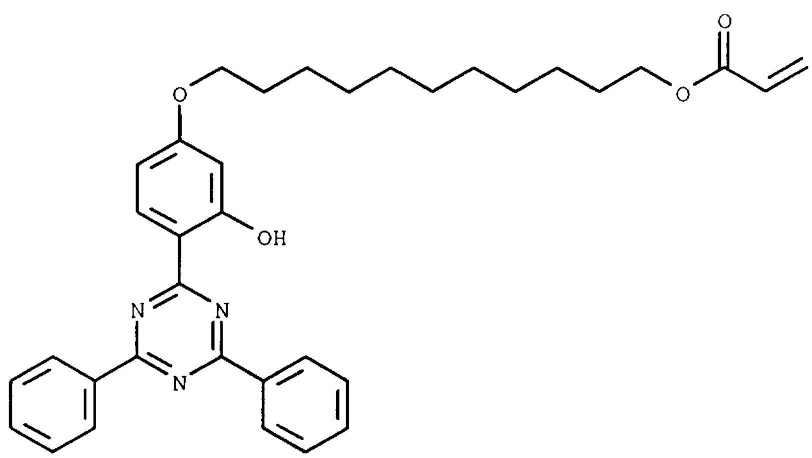

als weißen Feststoff vom Smp. 123 - 125 °C.

| | | | | |
|---|---|---|---|---|
| Analyse: C35H39N3O4 (565,71) | berechnet<br>gefunden | C 74,31<br>C 74,22 | H 6,95<br>H 7,08 | N 7,43 %<br>N 7,45 % |

Beispiel 4: 2-[2-Hydroxy-4-(11-methacryloyloxy-undecyloxy)phenyl]-4,6-diphenyl-1,3,5-triazin

Die Titelverbindung (Verbindung 103) wird nach der in Beispiel 3 angegebenen Methode hergestellt; Schmelzpunkt 94-96 °C.

Beispiel 4b: Mischung von 2-[2-Hydroxy-4-(3-vinylbenzyl-oxy)-phenyl-]-4,6-bis-(2,4-dimethylphenyl)-1,3,5-triazin.und 2-[2-Hydroxy-4-(4-vinylbenzyl-oxy)-phenyl-]-4,6-bis-(2,4-dimethylphenyl)-1,3,5-triazin

Die Titelverbindung (Verbindung 105) wird nach der in Beispiel 8b angegebenen Methode hergestellt; Schmp.: 158-160°C

Beispiel 4c: 2-[2-Hydroxy-4-(2-methacryloyloxy-ethoxy)phenyl]-4,6-diphenyl-1,3,5-triazin

Die Titelverbindung (Verbindung 106) wird als weißer Feststoff nach der in Beispiel 3 angegebenen Methode erhalten..

| | | | | |
|---|---|---|---|---|
| Analyse: C27H23N3O4 (453.50) | berechnet.<br>gefunden | C 71.57<br>C 70.70 | H 5.11<br>H 5.38 | N 9.27<br>N 9.00 |

Beispiel 4d:

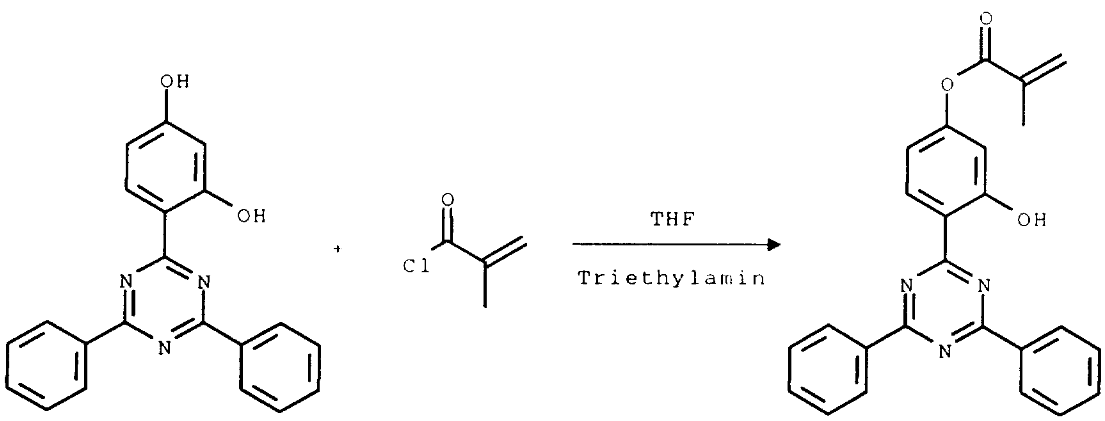


68.3g (0.2 Mol) 2-(2,4-Dihydroxyphenyl)-4,6-diphenyl-1,3,5-triazin werden in 500ml THF unter Stickstoff suspendiert. Dann wird 22.3g (0.22 Mol) Triethylamin zugegeben. Bei einer Temperatur welche 20°C nicht übersteigt wird 22.9g (0.22 Mol) Methacrylsäurechlorid zugetropft. Nach 2.5h Reaktionszeit wird die Reaktionsmischung filtriert, und das Filtrat eingeengt. Das Produkt wird in Dichlormethan aufgenommen, und die organische Phase mit Wasser gewaschen und über $MgSO_4$ getrocknet. Man filtriert über Silicagel und engt die organische Phase zur Trockne ein. Das Rohprodukt wird aus Ethylcellosolve umkristallisiert. Man erhält 43.6g (53.2%) der Verbindung (107) als hellgelbes Pulver mit dem Schmelzpunkt 158-160°C.

| | | | | |
|---|---|---|---|---|
| Analyse: | berechnet %:<br>gefunden %: | C: 73.34<br>C: 73.13 | H: 4.68<br>H: 4.79 | N: 10.26<br>N: 10.23 |

Beispiel 5: 2,4-Bis-(2,4-dimethylphenyl)-6-[2-hydroxy-4-(3-n-butyloxy-2-methacryloyloxypropoxy-)-phenyl-] -1,3,5-triazin

Die Titelverbindung (Verbindung 400) vom Schmelzpunkt 3°C wird nach der in Beispiel 2 angegebenen Methode erhalten.

Zwischenprodukt für Beispiel 6: 2-[2-Hydroxy-4-(11-hydroxy-undecyloxy)-phenyl-]-4,6-bis-(2,4-dimethylphenyl)-1,3,5-triazin.

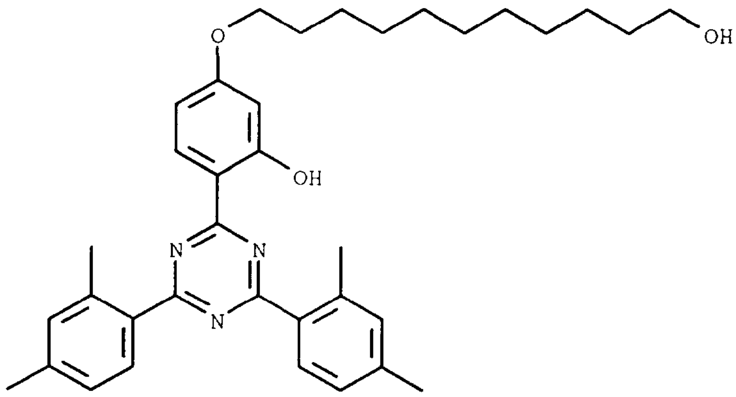

Unter Stickstoff wird eine Mischung aus 79,5 g (0,2 mol) 2-(2,4-Dihydroxyphenyl)-4,6-(2,4-dimethylphenyl)-1,3,5-triazin, 11,2 g (0,2 mol) pulverisiertem KOH (Fluka, >85%) in 500 ml Diglyme (Fluka, 99%) auf 80 °C erhitzt. Zur gelben Lösung werden 59,6 g (0,23 mol) 11-Brom-1-undecanol (Fluka, 97%) gegeben. Die Mischung wird für 46 Stunden auf 100 °C gehalten. Anschließend wird heiß filtriert und das Filtrat auf 0°C abgekühlt. Das kristalline Produkt wird filtriert, mit Hexan gewaschen und bei 50°C/ 70 mmHg getrocknet.

Man erhält 81,0 g (71,4 %) 2-[2-Hydroxy-4-(11-hydroxy-undecyloxy)phenyl]-4,6-bis-(2,4-dimethyl-phenyl)-1,3,5-triazine, als hellgelben Feststoff; F. 95 - 96°C.

| | | | | |
|---|---|---|---|---|
| Analyse: $C_{36}H_{45}N_3O_3$ (567,78) | berechnet: | C 76,16 | H 7,99 | N 7,40 |
| | gefunden: | C 75,42 | H 7,92 | N 7,39 |

Beispiel 6: 2-[2-Hydroxy-4-(11-acryloyloxy-undecyloxy)phenyl]-4,6-bis-(2,4-dimethylphenyl)-1,3,5-triazin (Verbindung Nr. 401)

Unter Stickstoff wird eine Mischung aus 56,8 g (0,1 mol) 2-[2-Hydroxy-4-(11-hydroxyundecyloxy)phenyl]-4,6-bis-(2,4-dimethylphenyl)-1,3,5-triazin, 12,4 g (0,133mol) Acryloylchlorid (Fluka, 97 %), 0,4 g Hydrochinon (Fluka, 98 %) und 2,0 ml Pyridin in 500 ml Toluol (Merck, 99,5 %) für 30 Stunden auf 80 °C erhitzt. Anschließend wird die Lösung auf 50°C abgekühlt und mit 21,3 ml (0,154 mol) Triethylamin versetzt, gefolgt von weiteren 6 Stunden bei 70°C. Feststoff und Lösungsmittel werden abgetrennt, und das feste gelbe Produkt wird mit Toluol durch eine Schicht aus Kieselgel 60 filtriert. Das Produkt wird aus Ispropanol umkristallisiert. Man erhält 47,0 g (75,5%) 2-[2-Hydroxy-4-(11-acryloyloxy-undecyloxy)phenyl]-2,4-bis-(2,4-dimethylphenyl)-1,3,5-triazin vom Smp. 81 - 83°C.

| | | | | |
|---|---|---|---|---|
| Analyse: C39H47N3O4 (621,82) | Berechnet: | C 75,33 | H 7,62 | N 6,76 |
| | Gefunden: | C 74,18 | H 7,75 | N 6,54 |

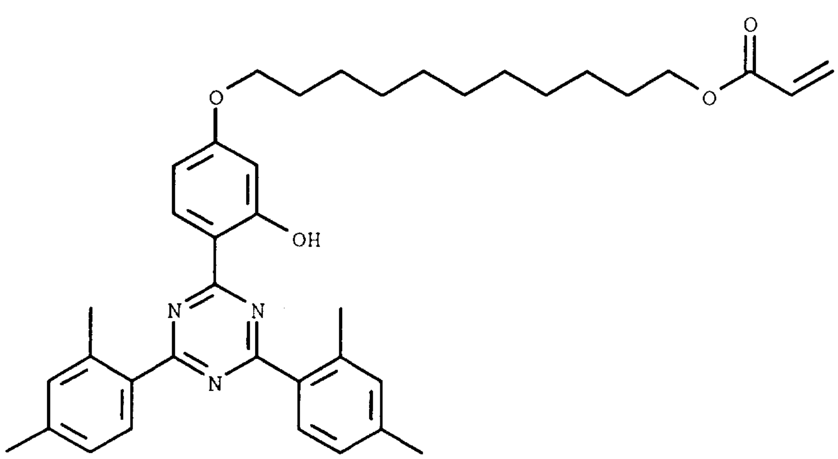

Beispiel 7: 2,4-Bis-(2,4-dimethylphenyl)-6-[2-hydroxy-4-(11-methacryloyloxy-undecyloxy)-phenyl-] -1,3,5-triazin

Die Titelverbindung (Verbindung 402) vom Schmelzpunkt 71-73°C wird nach der in Beispiel 6 angegebenen Methode erhalten..

Beispiel 8a: 2,4-Bis-(2,4-dimethylphenyl)-6-[2-hydroxy-4-(2-methacryloyloxy-ethoxy)-phenyl]-1,3,5-triazin

Die Titelverbindung (Verbindung 404) vom Schmelzpunkt 132-133°C wird nach der in Beispiel 3 angegebenen Methode erhalten..

Beispiel 8b: Mischung aus 2-[2-Hydroxy-4-(3-vinylbenzyl-oxy)-phenyl-]-4,6-bis-(2,4-dimethylphenyl)-1,3,5-triazin und 2-[2-Hydroxy-4-(4-vinylbenzyl-oxy)-phenyl-]-4,6-bis-(2,4-dimethylphenyl)-1,3,5-triazin (Verbindung Nr. 405).

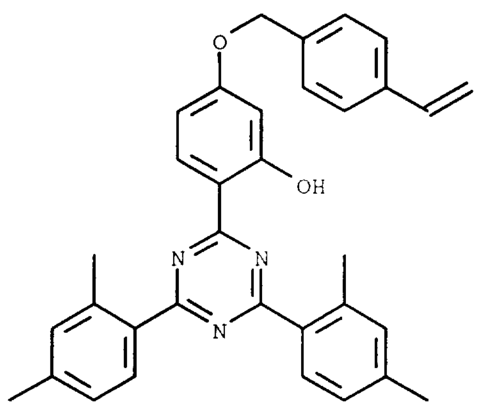

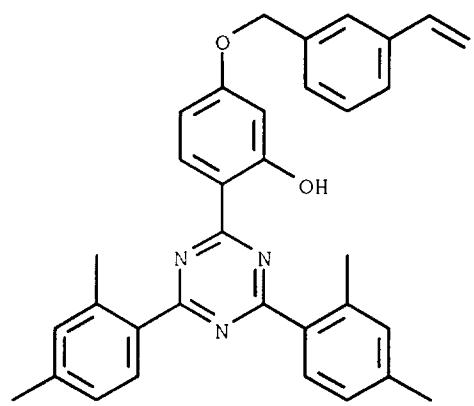

Unter Stickstoff wird eine Mischung aus 19,9 g (0,05 mol) 2-[2,4-Dihydroxy-phenyl]-4,6-bis-(2,4-dimethylphenyl) -1,3,5-triazin, 8,4 g (0,055 mol) Vinylbenzylchorid (Fluka, 98 %; Isomerenmischung aus 70% meta- und 30 % para-Vinylbenzylchorid), 3,1 g (0,055 mol) KOH und 100 ml Diglyme für 3 Stunden auf 110 °C erhitzt. Die Suspension wird abgekühlt, 1 lt Wasser wird zugesetzt, und der Feststoff wird abfiltriert und aus Isopropanol umkristallisiert. Man erhält 19,6 g (76,3%) 2-[2-Hydroxy-4-(3-/or 4-vinylbenzyl-oxy)-phenyl-]-2,4-bis-(2,4-dimethylphenyl)-1,3,5-triazin (Verbindung Nr. 405) vom Smp. 110 - 114°C.

| | | | | |
|---|---|---|---|---|
| Analyse: C34H31N3O2 (513,64) | Berechnet: | C 79,51 | H 6,08 | N 8,18 |
| | Gefunden: | C 79,53 | H 5,98 | N 7,98 |

Beispiel 9: Unter Stickstoff wird eine Mischung von 5,0 g (11 mmol)

2-Phenyl-4,6-bis-[2-hydroxy-4-(2-hydroxy-ethoxy-)phenyl-]1,3,5-triazin, 3,4 g (33 mmol) Methacryloylchlorid, 1,1 g (14 mmol) Pyridin in 35 ml Toluol bei 80°C während 16 Stunden erhitzt. Der Überschuß Methacryloylchlorid und das Toluol werden bei 60°C/60 mmHg entfernt. Nach Zugabe von 40 ml Toluol und 3,8 g (38 mmol) Triethylamin wird für 4 Stunden bei 80°C erhitzt. Man läßt abkühlen und filtriert den festen Rückstand ab. Durch Säulenchromatografie (Kieselgel 60; 230-400 mesh; Durchmesser 8 cm; h = 30 cm; Eluiermittel Toluol/Ethylacetat 1/1) erhält man 3,84 g des Rohproduktes, welches man aus 180 ml Ethylacetat umkristallisiert. Man erhält 1,64 g 2-Phenyl-4,6-bis-[2-hydroxy-4-(2-methacryloyloxy-ethoxy-)phenyl-]1,3,5-triazin (Verbindung 201) als gelblichen Feststoff (Schmelzpunkt: 154-159°C).

Das $^1$H-NMR-Spektrum ($CDCl_3$, 300 MHz) ist in Übereinstimmung mit dem gewünschten Produkt.

| Elementaranalyse für $C_{33}H_{31}N_3O_8$(597,62): | | | |
|---|---|---|---|
| Theorie: | C: 66,32 | H: 5,23 | N: 7,03 % |
| Gefunden: | C: 66,04 | H: 5,37 | N: 7,03 % |

Beispiel 10: Unter Stickstoff wird eine Mischung von 12,7 g (20 mmol)

2-Phenyl-4,6-bis-[2-hydroxy-4-(3-n-butoxy-2-hydroxy-propoxy-)phenyl-]1,3,5-triazin, 4,3 g (46 mmol) Acryloylchlorid, 0,4 g (5 mmol) Pyridin in 80 ml Toluol bei 70°C während 16 Stunden erhitzt. Nach Abkühlung auf 50°C und Zugabe von 8,0 g (80 mmol) Triethylamin wird für 6 Stunden bei 80°C erhitzt. Man läßt abkühlen und filtriert den festen Rückstand ($(CH_3CH_2)_3N{\cdot}HCl$) ab. Das Filtrat wird eingedampft und danach in 200 ml Methylenchlorid aufgenommen. Man filtriert die Lösung durch eine Kieselgelschicht (Kieselgel 60; 230-400 mesh), wäscht mit 450 ml Methylenchlorid. Nach Entfernen des Lösungsmittels und Trocknen bei 80°C erhält man 8,8 g (59 % Ausbeute) 2-Phenyl-4,6-bis-[2-hydroxy-4-(3-n-butoxy-2-acryloyloxy-propoxy-)phenyl-]1,3,5-triazin (Verbindung 202) als orangefarbenes Harz.

Das $^1$H-NMR-Spektrum ($CDCl_3$, 300 MHz) ist in Übereinstimmung mit dem gewünschten Produkt.

Elementaranalyse für $C_{41}H_{47}N_3O_{10}$ (741,84):

| | | | |
|---|---|---|---|
| Theorie: | C: 66,38 | H: 6,39 | N: 5,66 % |
| Gefunden: | C: 66,09 | H: 6,50 | N: 5,40 % |

Herstellung der Ausgangsverbindung für die Beispiele 11 und 12: Unter Stickstoff wird zu einer Lösung aus 20,0 g (54 mmol)

2-Phenyl-4,6-bis-(2,4-dihydroxy-phenyl)-1,3,5-triazin, 6,6 g (109 mmol) Kaliumhydroxid und 150 ml Diglyme bei 80°C 32,4 g (128 mmol) 11-Brom-1-undecanol gegeben. Die Mischung wird 14 Stunden bei 100°C erhitzt. Es wird heiß abfiltriert und das Filtrat auf 0°C abgekühlt. Der daraus kristallisierte Feststoff wird abfiltien, gepreßt und 24 Stunden unter reduziertem Druck (60 mmHg; 60°C) getrocknet, worauf man 27,6 g (72 % Ausbeute) 2-Phenyl-4,6-bis-[2-hydroxy-4-(11-hydroxy-undecyloxy-)-phenyl]-1,3,5-triazin als hellgelben Feststoff mit dem Schmelzpunkt 126-135°C erhält.

Das $^1$H-NMR-Spektrum ($CDCl_3$, 300 MHz) ist in Übereinstimmung mit dem gewünschten Produkt.

Beispiel 11: Unter Stickstoff wird eine Mischung von 10,8 g (15 mmol) 2-Phenyl-4,6-bis-[2-hydroxy-4-(11-hydroxy-undecyloxy-)-phenyl]-1,3,5-triazin, 3,6 g (40 mmol)

Acryloylchlorid, 0,2 g Hydrochinon, 0,3 g (3,8 mmol) Pyridin in 80 ml Toluol bei 78°C während 16 Stunden erhitzt. Der Überschuß Acryloylchlorid und Toluol werden am Rotavapor entfernt. Der Rückstand wird in 100 ml Toluol gelöst und nach Zugabe von 16,2 g (160 mmol) Triethylamin wird für 5 Stunden bei 80°C erhitzt. Man läßt abkühlen und filtriert den festen Rückstand ($(CH_3CH_2)_3N{\cdot}HCl$) ab. Das Filtrat wird eingedampft und danach in 50 ml Methylenchlorid aufgenommen. Man filtriert die Lösung durch Kieselgel (Kieselgel 60; 230-400 mesh; Durchmesser 6 cm; h = 4 cm), wäscht mit 400 ml Methylenchlorid nach und erhält nach Abzug des Lösungsmittels und zweistündigem Trocknen (80°C/0,1 mmHg) 10,7 g (86 % Ausbeute) 2-Phenyl-4,6-bis-[2-hydroxy-4-(11-acryloyloxy-undecyloxy-)-phenyl]-1,3,5-triazin (Verbindung 203) als hellgelben Feststoff (Schmelzpunkt: 93,3°C; mit DSC bestimmt).

Das $^1$H-NMR-Spektrum ($CDCl_3$, 300 MHz) ist in Übereinstimmung mit dem gewünschten Produkt.

Beispiel 12: Unter Stickstoff wird eine Mischung von 10,8 g (15 mmol) 2-Phenyl-4,6-bis-[2-hydroxy-4-(11-hydroxy-undecyloxy-)-phenyl]- 1,3,5-triazin, 4,0 g (38 mmol)

Methacryloylchlorid, 0,2 g Hydrochinon, 0,3 g (3,8 mmol) Pyridin in 80 ml Toluol bei 80°C während 16 Stunden erhitzt. Der Überschuß Methacryloylchlorid und Toluol werden am Rotavapor entfernt. Der Rückstand wird in 100 ml Toluol gelöst und nach Zugabe von 8,1 g (80 mmol) Triethylamin und 0,1 g Hydrochinon wird für 4 Stunden bei 70°C erhitzt. Man läßt abkühlen und filtriert den festen Rückstand ($(CH_3CH_2)_3N{\cdot}HCl$) ab. Das Filtrat wird eingedampft und danach in 50 ml Methylenchlorid aufgenommen. Man filtriert die Lösung durch Kieselgel (Kieselgel 60; 230-400 mesh; Durchmesser 6 cm; h = 4 cm), wäscht mit 400 ml Methylenchlorid nach und erhält nach Abzug des Lösungsmittels und zweistündigem Trocknen (80°C/0,1 mmHg) 10,9 g (85 % Ausbeute) 2-Phenyl-4,6-bis-[2-hydroxy-4-(11-methacryloyloxy-undecyloxy-)-phenyl]-1,3,5-triazin (Verbindung 204) als hellgelben Feststoff (Schmelzpunkt: 68,3°C; mit DSC bestimmt).

Das $^1$H-NMR-Spektrum ($CDCl_3$, 300 MHz) ist in Übereinstimmung mit dem gewünschten Produkt.

Beispiel 13: Verbindung (205) wird nach der in Beispiel 11 beschriebenen Methode erhalten. Tg = 17 °C.

| | | | | |
|---|---|---|---|---|
| Analyse: C41H47N3O8 (709,84) | Berechnet:<br>Gefunden: | C 69,37<br>C 68,53 | H 6,67<br>H 6,67 | N 5,92 %<br>N 6,03 % |

Beispiel 14: Verbindung (206) wird nach der in Beispiel 11 beschriebenen Methode erhalten. Tg = 15 °C.

| | | | | |
|---|---|---|---|---|
| Analyse: C39H43N3O8 (681,79) | Berechnet: | C 68,71 | H 6,36 | N 6,16 % |
| | Gefunden: | C 68,65 | H 6,44 | N 6,49 % |

Zwischenprodukt für Beispiel 15: 2-Phenyl-4-(2-hydroxy-4-n-hexyloxy-phenyl)-6-[2-hydroxy-4-(11-hydroxy-undecyloxy)phenyl]-1,3,5-triazin

Unter Stickstoff wird eine Mischung aus 37,3 g 2-Phenyl-4,6-bis-(2,4-dihydroxyphenyl)-1,3,5-triazine (0,100 mol), 6,6 g pulverisiertem KOH ( Fluka, >85 %, 0,100 mol), 25,9 g 11-Brom-1-undecanol ( Fluka, 97 %, 0,103 mol), und 0,6 g (3,6 mmol) Kaliumiodid (Merck, 99,5 % ) in 160 mL Diethylenglycol-dimethylether (Diglyme, Fluka, 99% ) unter Rühren für 4,5 Stunden auf 110°C erhitzt. Nach Abkühlen auf 50°C werden weitere 6,6 g (0,100 mol) pulverisiertes KOH ( Fluka, >85 % ) und 17,0 g (0,103 mol) 1-Bromhexan (Fluka, 98 %) zugesetzt. Die Mischung wird unter Rühren für 14 Stunden auf 105°C gehalten; anschließend wird heiß filtriert und das Filtrat am Rotovapor eingedampft. Säulenchromatografie [Kieselgel 60, 230-400 mesh; 10 cm Durchmesser; h = 30 cm; Eluiermittel $CH_2Cl_2$] liefert als erstes eluiertes Produkt das Dihexyl-Derivat, das zweite eluierte Produkt ist das erwünschte Titelprodukt; als letztes erhält man einen Anteil an einer mit 2 Gruppen 11-Hydroxy-undecyl dialkylierten Verbindung.

Nach Trocknen bei 60°C/60 mmHg für 24 h erhält man 22,5 g ( 35,8 %) 2-Phenyl-4-(2-hydroxy-4-n-hexyloxy-phenyl) -6-[2-hydroxy-4-(1 1-hydroxy-undecyloxy)-phenyl]-1,3,5-triazin vom Smp. 96-99°C.

1H NMR (CDCl3, 300 MHz)-Spektrum ist konsistent mit dem erwünschten Produkt.

| | | | | |
|---|---|---|---|---|
| Analyse C38H49N3O5 (627,83) | Berechnet: | C 72,70 | H 7,87 | N 6,69 % |
| | Gefunden: | C 72,19 | H 8,01 | N 6,88 % |

Beispiel 15: 2-Phenyl-4-(2-hydroxy-4-n-hexyloxy-phenyl)-6-[2-hydroxy-4-(11-methacryloyloxy-undecyloxy)phenyl] -1,3,5-triazin (Verbindung 207)

Unter Stickstoff wird eine Mischung aus 22,0 g (35,0 mmol) 2-Phenyl-4-(2-hydroxy-4-n-hexyloxy-phenyl)-6-[2-hydroxy-4-(1-hydroxy-undecyloxy)phenyl]-1,3,5-triazin, 4,4g (42,0 mmol) Methacryloylchlorid (Fluka, 97 %) und 0,5 g (6,3 mmol) Pyridin in 100 mL Toluol (Merck, 99,5 %) für 21 Stunden auf 80-85°C erhitzt. Nach Abkühlen auf 55°C werden 8,9 g (87,5 mmol) Triethylamin (Fluka, 99,5 %) zugesetzt. Die Mischung wird für weitere 7 Stunden auf 80°C gehalten. Anschließend wird heiß filtriert und das Filtrat eingedampft. Das Rohprodukt (25,7 g ) wird in 120 mL $CH_2Cl_2$ gelöst, durch eine Schicht aus Kieselgel 60 (230-400 mesh; 6,5 cm Durchmesser; h = 5 cm) filtriert und mit 380mL CH2Cl2 eluiert. Abtrennen des Lösungsmittels und Trocknen bei 80°C/0,1 mmHg für 2,5 h liefern 22,3 g (91,4 %) 2-Phenyl-4-(2-hydroxy-4-hexyloxyphenyl)-6-[2-hydroxy-4-(11-methacryloyloxy-undecyloxy)-phenyl]-1,3,5-triazin als orangefarbenes Harz; Tg = -13°C (DSC).

1H NMR (CDCl3, 300 MHz )-Spektrum ist konsistent mit dem gewünschten Produkt.

| | | | | |
|---|---|---|---|---|
| Analyse C42H53N3O6 (695,90) | Berechnet: | C 72,46 | H 7,68 | N 6,04 % |
| | Gefunden: | C 72,14 | H 7,48 | N 5,98 % |

Beispiel 16: 2-Phenyl-4-[2-hydroxy-4-(11-acetyloxy-undecyloxy)phenyl]-6-[2-hydroxy-4-(11-methacryloyloxy-undecyloxy)phenyl]-1,3,5-triazin (Verbindung 208).

Eine Mischung von 20,0 g (28,0 mmol) 2-Phenyl-4,6-bis-[2-hydroxy-4-(11-hydroxyundecyloxy)phenyl]-1,3,5-triazin (Zwischenprodukt für Beispiele 11 und 12), 3,1 g (29,4 mmol) Methacryloylchlorid (Fluka, 97%), 0,2 g Hydrochinon (Fluka, 98%), 0,3 g (3,8 mmol) Pyridin in 115 ml Toluol zuerst für 5 Stunden unter Rühren auf 80°C erhitzt, anschließend wird auf 60°C abgekühlt, 4,62 g (58,8 mmol) Essigsäurechlorid zugegeben und die Mischung für weitere 18 h unter Rühren auf 60°C gehalten. Nach Abtrennen des Lösungsmittels und des Überschusses an Essigsäurechlorid (Rotavap) wird in 100 ml Toluol gelöst und nach Zusatz von 7,1 g (70 mmol) Triethylamin für 6 h unter Rühren auf 70°C erhitzt. Nach Abkühlen wird der Feststoff ($[H_5C_2]_3N \cdot HCl$) abfiltriert und das Filtrat eingedampft. Das Rohprodukt wird in 100 mL $CH_2Cl_2$ gelöst, durch eine Schicht aus Kieselgel 60 (230-400 mesh) filtriert und mit 1000 mL $CH_2Cl_2$ /Methanol (95:5) eluiert. Abtrennen des Lösungsmittels und Trocknen bei 40°C/60 mmHg für 48 h liefern 20,8 g (90,1 %) der Verbindung (208) vom Smp. 70-76°C.

Beispiel 17: Unter Stickstoff wird eine Mischung von 7,0 g (14,1 mmol)

2-(4-Chlorphenyl)-4,6-bis-[2-hydroxy-4-(2-hydroxy-ethoxy-)phenyl-]1,3,5-triazin, 8,52 g (94,1 mmol) Acryloylchlorid, 0,3 g (3,8 mmol) Pyridin in 180 ml Toluol bei 75°C während 14 Stunden erhitzt. Der Überschuß Acryloylchlorid und das Toluol werden am Rotavapor entfernt. Nach Zugabe von 100 ml Toluol und 5,0 g (48 mmol) Triethylamin wird für 14 Stunden bei 90°C erhitzt. Man läßt abkühlen und filtriert den festen Rückstand ($(CH_3CH_2)_3N{\cdot}HCl$) ab. Das Filtrat wird eingedampft und aus Ethylacetat umkristallisiert. Man erhält 4,4 g (52 % Ausbeute) 2-(4-Chlorphenyl)-4,6-bis-[2-hydroxy-4-(2-acryloyloxy-ethoxy-)phenyl-]1,3,5-triazin (Verbindung 200) als gelblichen Feststoff (Schmelzpunkt: 115-120°C).

Das $^1H$-NMR-Spektrum ($CDCl_3$, 300 MHz) ist in Übereinstimmung mit dem gewünschten Produkt.

| Elementaranalyse für $C_{31}H_{26}ClN_3O_8$(604,02): | | | | |
|---|---|---|---|---|
| Theorie: | C: 61,64 | H: 4,34 | N: 6,96 | Cl: 5,87 % |
| Gefunden: | C: 61,36 | H: 4,49 | N: 6,98 | Cl: 6,02 % |

Zwischenprodukt für Beispiele 18 bis 22b

(i) 2-Mesityl-4,6-dichlor-1,3,5-triazin

Eine Lösung von 109,5 g (0,55 Mol) 2-Brom-mesitylen (Reinheit 98%) in 150 ml abs. THF (Reinheit 99,5%) wird innerhalb von 1 ½ Stunden unter Stickstoff zu einer gerührten, auf 60°C temperierten Suspension von 14,6 g (0,60 Mol) Magnesiumspänen (Reinheit 99,8%) in 100 ml abs. THF gegeben, der ein Iodkristall beigegeben ist. Anschließend wird die Mischung für 30 Minuten auf Rückflußtemperatur gehalten (68°C).

Nach Abkühlen wird das erhaltene Grignard-Reagens in einen Tropftrichter überführt und tropfenweise zu einer Lösung von 96,0 g (0,52 Mol) Cyanurchlorid (98%) in 270 ml THF gegeben. Während der Zugabe, die 1 ½ Stunden dauert, wird durch Kühlen eine Temperatur zwischen 15 und 30°C gehalten. Anschließend wird die Mischung bei 25°C für 2 Stunden gerührt, dann auf 2 1 einer Eis-Wasser-Mischung enthaltend 80 ml 32 % HCl (0,81 Mol) gegossen. Nach einstündigem Rühren wird filtriert. Der Filterkuchen wird in 1000 ml Wasser suspendiert, 30 Min. gerührt und wieder filtriert. Diese Operation wird noch zweimal wiederholt. Der Filterkuchen wird für 24 h bei 25°C und einem Druck von 60 mmHg (8000 Pa) über $P_2O_5$ getrocknet. 171,0 g Rohprodukt werden anschließend in Toluol gelöst, heiß filtriert, und durch Zusatz von Hexan und Kühlen auf 0°C kristallisiert.

Nach Abfiltrieren und Trocknen werden 82,8 g des Titelproduktes (Verbindung i) erhalten

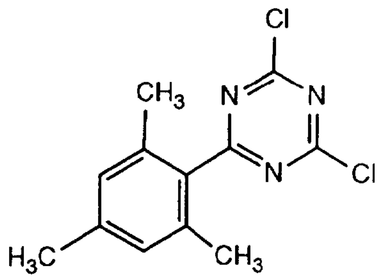

Verbindung i

mit dem Smp. 85-91°C.

$^1H$-NMR ($CDCl_3$, 300 MHz): δ 2,22 (s, 6H); 2,32 (s, 3H); 6,95 (s, 2H).

(ii) 2-Mesityl-4,6-bis(2,4-dihydroxyphenyl)-1,3,5-triazin

Zu einer Suspension von 130,0 g (0,485 Mol) 2-Mesityl-4,6-dichlor-1,3,5-triazin (Verbindung 1) in 300 ml Benzin vom Siedebereich 110-140°C und 385 ml Sulfolan werden unter Rühren 148,7 g (1,21 Mol) wasserfreies Aluminiumtrichlorid (Reinheit 98%) gegeben. Die Mischung erwärmt sich dabei auf 45°C. Dazu wird während 45 Min. eine Lösung von 133,5 g (1,21 Mol) Resorcin (Reinheit 98%) in 155 ml Sulfolan gegeben. Die Mischung wird unter HCl-Entwicklung für 5 h 30 Min. auf 80-85°C erwärmt. Die obere Phase (Benzin) wird entfernt, die untere, dickflüssige Phase wird heiß in eine gerührte Mischung aus 2,1l Methanol und 2,1l Wasser gegeben. Nach Rühren für die Dauer von 14 h wird der Feststoff abfiltriert, in 2,2 l 1-molarer HCl für 1 h gerührt und wieder abfiltriert. Der Filterkuchen wird in 1000 ml Wasser

suspendiert, 30 Min. gerührt und wieder filtriert. Diese Operation wird noch zweimal wiederholt. Der Filterkuchen wird für 24 h bei 80°C und einem Druck von 60 mmHg (8000 Pa) getrocknet. Es werden 170,5 g des Titelproduktes (Verbindung ii) erhalten der Formel

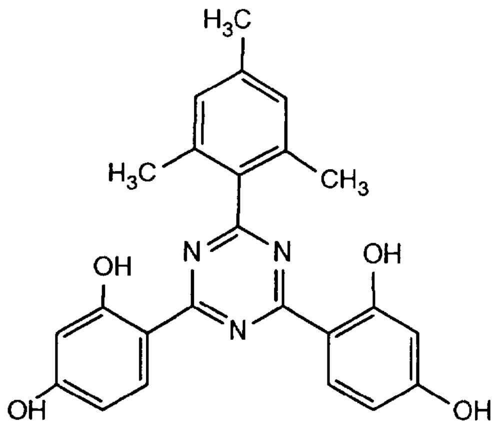

mit dem Smp. 230-234°C.

Beispiel 18: Verbindung (500) wird nach der in Beispiel 10 beschriebenen Methode als gelbes Harz erhalten; Tg.: 9 °C.

| | | | | |
|---|---|---|---|---|
| Analyse (C44H53N3O10): (783,92) | Berechnet: Gefunden: | C 67,42 C 67,27 | H 6,81 H 6,91 | N 5,36 % N 5,66 % |

Beispiel 19: Verbindung (501) wird nach der in Beispiel 10 beschriebenen Methode als gelbes Harz erhalten; Tg.: -2 °C.

| | | | | |
|---|---|---|---|---|
| Analyse (C46H57N3O10): (811,98) | Berechnet: Gefunden: | C 68,04 C 68,01 | H 7,08 H 7,10 | N 5,18 % N 4,92 % |

Beispiel 20: Verbindung (502) wird nach der in Beispiel 12 beschriebenen Methode erhalten.

| | | | | |
|---|---|---|---|---|
| Analyse (C54H73N3O8): (892,19) | Berechnet: Gefunden: | C 72,70 C 71,69 | H 8,25 H 8,09 | N 4,71 % N 4,75 % |

Beispiel 21: Verbindung (503) wird nach der in den Beispielen 9 und 11 beschriebenen Methode als gelbes Harz erhalten; Tg.: 22 °C.

| | | | | |
|---|---|---|---|---|
| Analyse (C44H53N3O8): (751,92) | Berechnet: Gefunden: | C 70,28 C 69,81 | H 7,11 H 6,87 | N 5,59 % N 5,67 % |

Zwischenprodukt für Beispiele 22 und 22b: 2-Mesityl-4-(2-hydroxy-4-n-hexyloxy-phenyl)-6-[2-hydroxy-4-(11-hydroxy-undecyloxy)phenyl]-1,3,5-triazin

Unter Stickstoff wird eine Mischung von 83,0 g (0,200 mol) 2-Mesityl-4,6-bis-(2,4-dihydroxy-phenyl)-1,3,5-triazin, 13,2 g (0,200 mol) pulverisiertem KOH (Fluka, >85%), 51,8g (0,206 mol) 11-Brom-1-undecanol ( Fluka, 97 %) und 1,2 g (7,2 mmol) Kaliumiodid (Merck, 99,5 %) in 300 mL Diethylenglycol-dimethylether (Diglyme, Fluka, 99%) unter Rühren für 3 Stunden auf 120°C erhitzt. Nach Abkühlen auf 60°C werden weitere 13,2 g pulverisiertes KOH und 34,0 g 1-Bromhexan (0,206 mol; Fluka, 98 %) zugesetzt. Die Mischung wird für 16 Stunden bei 110°C gerührt. Anschließend wird heiß filtriert und das Filtrat am Rotovapor eingedampft. Das Rohprodukt (152,1 g) wird chromatografiert [Kieselgel 60; 230-400 mesh; Säule 10 cm Durchmesser, h = 30 cm; Eluiermittel: Toluol/Methanol 98:2], wobei die zweite Fraktion das gewünschte Produkt enthält. Nach Abtrennen des Lösungsmittels und Trocknen bei 110°C/0,1 mmHg für 2 Stunden erhält

man 55,2 g ( 41,2 %) 2-Mesitylyl-4-(2-hydroxy-4-n-hexyloxy-phenyl)-6-[2-hydroxy-4-(11-hydroxy-undecyloxy)-phenyl] -1,3,5-triazin als gelbes Harz, welches bei 25°C langsam kristallisiert.

1H NMR (CDCl3, 300 MHz)-Spektrum ist konsistent mit dem Titelprodukt.

| | | | | |
|---|---|---|---|---|
| Analyse (C41H55N3O5): (669,91) | Berechnet: Gefunden: | C 73,51 C 73,55 | H 8,27 H 8,47 | N 6,27 % N 6,29 % |

Beispiel 22: 2-Mesityl-4-(2-hydroxy-4-n-hexyloxyphenyl)-6-[2-hydroxy-4-(11-methacryloyloxy-undecyloxy)-phenyl] -1,3,5-triazin (Verbindung 504).

Unter Stickstoff wird eine Mischung von 10,9 g (16,0 mmol) 2-mesityl-4-(2-hydroxy-4-n-hexyloxyphenyl)-6-[2-hydroxy-4-(11-hydroxy-undecyloxy)-phenyl]-1,3,5-triazin, 2,0g (19,2 mmol) Methacryloylchlorid (Fluka, 97 %) und 0,4 g (5,0 mmol) Pyridine in 70 mL Toluol ( Merck, 99,5 % ) für 16 Stunden auf 80-85°C erhitzt. Nach Abkühlen auf 55°C werden 4,1 g (40,0 mmol) Triethylamin ( Fluka, 99,5 % ) zugesetzt. Anschließend wird für weitere 6 Stunden auf 80°C erhitzt und dann heiß filtriert und das Filtrat eingedampft. Das Rohprodukt (13,6 g ) wird in 100 mL CH2Cl2 gelöst, durch eine Kieselgel 60 Schicht (230-400 mesh; 6,5 cm Durchmesser; h = 4 cm) filtriert und mit 380mL CH2Cl2 eluiert. Entfernen des Lösungsmittels und Trocknen bei 80°C/0,1 mmHg für 2 Stunden 30 min. liefert 22,3 g (91,4 %) 2-Mesityl-4-(2-hydroxy-4-hexyloxyphenyl)-6-[2-hydroxy-4-(11-methacryloyloxy-undecyloxy)-phenyl]-1,3,5-triazin (Verbindung 504) als gelbes Harz; Tg.: -10 °C (DSC). 1H NMR (CDCl3, 300 MHz )-Spektrum ist konsistent mit dem Titelprodukt.

| | | | | |
|---|---|---|---|---|
| Analyse (C45H59N3O6): (737,98) | Berechnet: Gefunden: | C 73,24 C 73,01 | H 8,06 H 7,76 | N 5,69 % N 5,61 % |

Beispiel 22a: Verbindung (505) wird nach der in Beispiel 8b beschriebenen Methode als orangefarbenes Harz erhalten; Tg. 13,3°C (DSC).

| | | | | |
|---|---|---|---|---|
| Analyse (C42H37N3O4): (647,78) | Berechnet: Gefunden: | C 77,88 C 77,64 | H 5,76 H 5,76 | N 6,49 % N 5,66 % |

Beispiel 22b: 2-Mesityl-4-(2-hydroxy-4-n-hexyloxyphenyl)-6-[2-hydroxy-4-(11-acryloyloxyundecyloxy)-phenyl] -1,3,5-triazin (Verbindung 506).

Unter Stickstoff wird eine Mischung von 27,3 g (40,8 mmol) 2-Mesityl-4-(2-hydroxy-4-n-hexyloxyphenyl)-6-[2-hydroxy-4-(11-hydroxy-undecyloxy)-phenyl]- 1,3,5-triazin, 4,4g (49,0 mmol) Acryloylchlorid ( Fluka, 97 % ), 0,1 g (0,9 mmol) Hydrochinon (Fluka, 99%) und 0,75 g (9,5 mmol) Pyridin in 170 mL Toluol (Merck, 99,5 %) unter Rühren für 17 Stunden auf 70-75°C erhitzt. Nach Abkühlen auf 50°C werden 20,0 g (197,6 mmol) Triethylamin ( Fluka, 99,5 % ) zugesetzt. Anschließend wird für weitere 6 Stunden auf 85°C erhitzt und dann heiß filtriert und das Filtrat eingedampft. Das Rohprodukt wird in 100 mL einer Mischung aus 98 **Volumen**teilen Toluol und 2 **Volumen**teilen Methanol gelöst, durch eine Kieselgel 60 Schicht (230-400 mesh; 6,5 cm Durchmesser; h = 5 cm) filtriert und mit 400mL der Toluol/Methanol-Mischung eluiert. Entfernen des Lösungsmittels und Trocknen bei 80°C/0,1 mmHg für 3 Stunden liefert 24,3 g (82,3 %) 2-Mesityl-4-(2-hydroxy-4-hexyloxyphenyl)-6-[2-hydroxy-4-(11-acryloyloxy-undecyloxy)phenyl]-1,3,5-triazin (Verbindung 506) als gelbes Harz; Tg. -14,2°C (DSC).

1H NMR (CDCl3, 300 MHz )-Spektrum ist konsistent mit dem Titelprodukt.

| | | | | |
|---|---|---|---|---|
| Analyse C44H57N3O6 (723,96) | Berechnet: Gefunden: | C 73,00 C 72,81 | H 7,94 H 7,70 | N 5,80 % N 5,53 % |

Beispiel 23: Unter Stickstoff wird eine Mischung von 15,9 g (20 mmol)

2,4,6-Tris-[2-hydroxy-4-(3-n-butoxy-2-hydroxy-propoxy-)phenyl-]1,3,5-triazin, 7,3 g (80 mmol) Acryloylchlorid, 0,4 g (5 mmol) Pyridin in 120 ml Toluol bei 75°C während 24 Stunden unter Rühren erhitzt. Der Überschuß Acryloylchlorid und Toluol werden am Rotavapor entfernt. Der Rückstand wird in 100 ml Toluol gelöst und nach Zugabe von 10,1 g (100 mmol) Triethylamin wird für 6 Stunden bei 80°C gerührt. Man läßt abkühlen und filtriert den festen Rückstand ( $(CH_3CH_2)_3N{\cdot}HCl$) ab. Das Filtrat wird eingedampft und danach in 100 ml Methylenchlorid aufgenommen. Man filtriert die Lösung durch eine Kieselgelschicht (Kieselgel 60; 230-400 mesh; Durchmesser 10 cm; h = 5 cm), wäscht mit 2000

ml Methylenchlorid nach und erhält nach Abzug des Lösungsmittels und Trocknen (90°C/0,5 mmHg) 12,0 g (63 % Ausbeute) 2,4,6-Tris-[2-hydroxy-4-(3-n-butoxy-2-acryloyloxy-propoxy)phenyl-]1,3,5-triazin (Verbindung 300) als hellgelbes Harz.

Das $^1H$-NMR-Spektrum ($CDCl_3$, 300 MHz) ist in Übereinstimmung mit dem gewünschten Produkt.

| Elementaranalyse für $C_{51}H_{63}N_3O_{15}$(958,07): | | | |
|---|---|---|---|
| Theorie: | C: 63,94 | H: 6,63 | N: 4,39 % |
| Gefunden: | C: 63,24 | H: 6,57 | N: 4,02 % |

Herstellung der Ausgangsverbindung für die Beispiele 24 und 25:

Unter Stickstoff wird zu einer Lösung aus 20,3 g (50 mmol)

2,4,6-Tris-(2,4-dihydroxy-phenyl)-1,3,5-triazin, 9,3 g (141 mmol) Kaliumhydroxid und 150 ml Diglyme bei 80°C 42,9 g (170 mmol)11-Brom-1-undecanol gegeben. Die Mischung wird 16 Stunden bei 100°C erhitzt. Es wird heiß abfiltriert und das Filtrat auf 0°C abgekühlt. Der kristallisierte Feststoff wird abfiltiert, gepreßt und 48 Stunden unter reduziertem Druck (60 mmHg; 70°C) getrocknet, worauf man 24,3 g (53 % Ausbeute) 2,4,6-Tris-[2-hydroxy-4-(11-hydroxy-undecyloxy)-phenyl]-1,3,5-triazin als hellgelben, harzähnlichen Feststoff erhält.

Das $^1H$-NMR-Spektrum ($CDCl_3$, 300 MHz) ist in Übereinstimmung mit dem gewünschten Produkt.

Beispiel 24:

Unter Stickstoff wird eine Mischung von 10,1 g (11 mmol) 2,4,6-Tris-[2-hydroxy-4-(11-hydroxy-undecyloxy-)-phenyl] - 1,3,5-triazin, 3,6 g (40 mmol) Acryloylchlorid, 0,2 g Hydrochinon, 0,3 g (3,8 mmol) Pyridin in 80 ml Toluol bei 80°C während 18 Stunden erhitzt. Der Überschuß Acryloylchlorid und Toluol werden am Rotavapor entfernt. Der Rückstand wird in 100 ml Toluol gelöst und nach Zugabe von 16,2 g (160 mmol) Triethylamin und 0,1 g Hydrochinon wird für 5 Stunden bei 78°C gerührt. Man läßt abkühlen und filtriert den festen Rückstand ($(CH_3CH_2)_3N{\cdot}HCl$) ab. Das Filtrat wird eingedampft und danach in 100 ml Methylenchlorid aufgenommen. Man filtriert die Lösung durch eine Kieselgelschicht (Kieselgel 60; 230-400 mesh; Durchmesser 6 cm; h = 4 cm), wäscht mit 400 ml Methylenchlorid nach und erhält nach Abzug des Lösungsmittels und zweistündigem Trocknen (70°C/0,1 mmHg) 7,7 g (65 % Ausbeute 2,4,6-Tris-[2-hydroxy-4-(11-acryloyloxy-undecyloxy-)-phenyl]-1,3,5-triazin (Verbindung 301) als hellgelben Feststoff (Schmelzpunkt: 72,8°C; mit DSC bestimmt).

Das $^1H$-NMR-Spektrum ($CDCl_3$, 300 MHz) ist in Übereinstimmung mit dem gewünschten Produkt.

Beispiel 25:

Unter Stickstoff wird eine Mischung von 10,1 g (11 mmol) 2,4,6-Tris-[2-hydroxy-4-(11-hydroxy-undecyloxy-)-phenyl] -1,3,5-triazin, 4,2 g (40 mmol) Metacryloylchlorid, 0,2 g Hydrochinon, 0,3 g (3,8 mmol) Pyridin in 80 ml Toluol bei 80°C während 18 Stunden erhitzt. Der Überschuß Acryloylchlorid und Toluol werden am Rotavapor entfernt. Der Rückstand wird in 100 ml Toluol gelöst und nach Zugabe von 8,1 g (80 mmol) Triethylamin und 0,1 g Hydrochinon wird für 5 Stunden bei 70°C gerührt. Man läßt abkühlen und filtriert den festen Rückstand ab. Das Filtrat wird eingedampft und danach in 100 ml Methylenchlorid aufgenommen. Man filtriert die Lösung durch eine Kieselgelschicht (Kieselgel 60; 230-400 mesh; Durchmesser 6 cm; h = 4 cm), wäscht mit 400 ml Methylenchlorid nach und erhält nach Abzug des Lösungsmittels und zweistündigem Trocknen (70°C/0,1 mmHg) 7,7 g (65 % Ausbeute) 2,4,6-Tris-[2-hydroxy-4-(11-methacryloyloxy-undecyloxy-)-phenyl]-1,3,5-triazin (Verbindung 302) als hellgelben Feststoff (Schmelzpunkt: 60,2°C; mit DSC bestimmt).

Das $^1H$-NMR-Spektrum ($CDCl_3$, 300 MHz) ist in Übereinstimmung mit dem gewünschten Produkt.

Beispiel 28: Homopolymer von 2-Phenyl-4-(2-hydroxy-4-hexyloxyphenyl)-6-[2-hydroxy-4-(11-methacryloyl-oxy-undecyl-oxy)phenyl]-1,3,5-triazin (Verbindung 602).

In einem 100ml Dreihalskolben wird unter Argon eine Lösung von 5,2 g ( 7,5 mmol) 2-(Phenyl)-4-(2-hydroxy-4-hexyloxyphenyl)-6-(2-hydroxy-11-methacryloyloxyundecyl-oxyphenyl)-1,3,5-triazin (Verbindung 207) in 40 mL Toluol ( Fluka, 99,5 % ) mit 40mg (0,22 mmol) $\alpha,\alpha'$-Azo-isobutyronitril (AIBN, Fluka, 98 % ) und 70mg (0,75 mmol) n-Butyl-mercaptan ( Fluka, 97 %) versetzt.

Die Mischung wird unter Rühren für 16 Stunden auf 85°C gehalten. Nach Abkühlen wird die klare gelbe Lösung tropfenweise unter Rühren zu einer Lösung von 400 mL Acetonitril gegeben ( Fluka, 99,5 %).

Der Niederschlag wird dekantiert und in 30 mL Toluol aufgenommen. Nach Abzug des Lösungsmittels und zweistündigem Trocknen bei 80°C/0,1 mmHg erhält man 3,35 g (64 %) des Titelproduktes (Verbindung 602); Tg.: 29,9°C.

1H NMR (CDCl3, 300 MHz) Spectrum ist konsistent mit dem gewünschten Produkt. (kein Vinyl-H-Signal)

| | |
|---|---|
| MALDI-MS: | Mn =1698<br>Mw = 3251 |

Beispiel 29: Copolymer von 2-Phenyl-4-(2-hydroxy-4-hexyl-oxy-phenyl)-6-[2-hydroxy-4-(11-methacryloyloxy-undecyloxy)-phenyl]-1,3,5-triazin und n-Butylacrylat im Molverhältnis 1: 4

In einem 100ml Dreihalskolben wird unter Argon eine Lösung von 5,2 g ( 7,5 mmol) 2-(Phenyl)-4-(2-hydroxy-4-hexyloxyphenyl)-6-(2-hydroxy-1 1-methacryloyloxy-undecyloxyphenyl)-1,3,5-triazin (Verbindung 207) und 3,8 g (30 mmol) n-Butylacrylat (Fluka, 99%) in 40 mL Toluol ( Fluka, 99,5 % ) mit 200mg (1,12mmol) α,α'-Azoisobutyronitril (AIBN, Fluka, 98 % ) und 300 mg (3,75 mmol) n-Butyl-mercaptan ( Fluka, 97 % ) versetzt.

Die Mischung wird unter Rühren für 17 Stunden auf 85°C gehalten. Nach Abkühlen wird die klare gelbe Lösung tropfenweise unter Rühren zu einer Lösung von 400 mL Acetonitril gegeben ( Fluka, 99,5 %).

Der Niederschlag wird dekantiert und in 30 mL Toluol aufgenommen; feste Verunreinigungen werden durch Filtrieren entfernt. Nach Abzug des Lösungsmittels und zweistündigem Trocknen bei 80°C/0,1 mmHg erhält man 6,60 g (73 %) des Titelproduktes (Verbindung 603); Tg.: -3,5°C.

| | | |
|---|---|---|
| MALDI-MS: | Mn = 2905; | Mw = 4199 |

Beispiel 30: Verbindung (604) wird nach der in Beispiel 28 angegebenen Methode hergestellt; man erhält ein orangegelbes Harz, Tg. = 49.8 °C.

| | | | | |
|---|---|---|---|---|
| [C45H59N3O6]<br>( 737.98 ) | berechnet<br>gefunden | C 73.24<br>C 72.57 | H 8.06<br>H 8.43 | N 5.69 %<br>N 5.49 % |

| | | |
|---|---|---|
| MALDI-MS: | Mn = 1920; | Mw = 4198 |

Beispiel 31: Verbindung (605) wird nach der in Beispiel 29 angegebenen Methode hergestellt; man erhält ein orangefarbenes Harz, Tg. = -4.2 °C.

| [C45H59N3O6]1 [C7H12O2]4 | | | |
|---|---|---|---|
| berechnet<br>gefunden | C 70.11<br>C 70.71 | H 8.62<br>H 8.74 | N 3.36 %<br>N 3.67 % |

| | | |
|---|---|---|
| MALDI-MS: | Mn = 3238; | Mw = 4923 |

Beispiel 31 a: Verbindung (606) wird nach der in Beispiel 29 angegebenen Methode hergestellt, wobei an Stelle von n-Butylacrylat 30 mmol n-Dodecylmethacrylat eingesetzt werden; Tg. -33,4°C.

| | |
|---|---|
| MALDI-MS: | Mn = 2023 g/Mol<br>Mw = 3661 g/Mol |

Beispiel 32: Verbindung (607) wird nach der in Beispiel 28 angegebenen Methode hergestellt; man erhält einen weißen Feststoff.

| | | | | |
|---|---|---|---|---|
| [C35H39N3O4]<br>( 565.71 ) | berechnet<br>gefunden | C 74.31<br>C 74.13 | H 6.95<br>H 7.16 | N 7.43 %<br>N 7.27 % |

| | | |
|---|---|---|
| Mn = 1938; | Mw = 3054 | (MALDI-MS) |

Beispiel 33: Verbindung (608) wird nach der in Beispiel 29 angegebenen Methode hergestellt.

| [C35H39N3O4]1[C7H12O2]4 | | | |
|---|---|---|---|
| berechnet<br>gefunden | C 70.17<br>C 70.70 | H 8.13<br>H 8.33 | N 3.90 %<br>N 4.60 % |

| | | |
|---|---|---|
| MALDI-MS: | Mn = 2310; | Mw = 3341 |

Beispiel 34: Verbindung (609) wird nach der in Beispiel 28 angegebenen Methode hergestellt; man erhält ein gelbes Harz.

| | | | | |
|---|---|---|---|---|
| [C40H49N3O4]<br>(635.85) | berechnet<br>gefunden | C 75.56<br>C 74.02 | H 7.77<br>H 8.06 | N 6.61 %<br>N 6.06 % |

| | | |
|---|---|---|
| Mn = 1781; | Mw = 3669 | (MALDI-MS) |

Beispiel 35: Verbindung (610) wird nach der in Beispiel 29 angegebenen Methode hergestellt, wobei jedoch nur die halbe Menge an n-Butylacrylat eingesetzt wird.

| [C40H49N3O4]1[C7H12O2]2 | | | |
|---|---|---|---|
| berechnet<br>gefunden | C 72.70<br>C 71.81 | H 8.25<br>H 8.41 | N4.71 %<br>N 4.68 % |

| | | |
|---|---|---|
| GPC: | Mn = 1908; | Mw = 3111 |

Beispiel 36: Verbindung (611) wird nach der in Beispiel 28 angegebenen Methode hergestellt; man erhält einen gelben Feststoff vom Schmelzpunkt 85,7 °C (DSC).

| | | | | |
|---|---|---|---|---|
| [C36H41N3O4]<br>( 579.74 ) | berechnet<br>gefunden | C 74.58<br>C 73.90 | H 7.13<br>H 7.15 | N 7.25 %<br>N 7.03 % |

| | | |
|---|---|---|
| Mn = 2405; | Mw = 3701 | (MALDI-MS) |

Beispiel 37: Verbindung (612) wird nach der in Beispiel 35 angegebenen Methode hergestellt; man erhält ein gelbes Harz, Tg. = 15,8 °C.

| [C36H41N3O4]1[C7H12O2]2 | | | |
|---|---|---|---|
| berechnet<br>gefunden | C 71.83<br>C 71.71 | H 7.84<br>H 7.61 | N 5.03 %<br>N 5.19 % |

| | | |
|---|---|---|
| Mn = 3241; | Mw = 4920 | (MALDI-MS) |

Beispiel 38: Verbindung (613) wird nach der in Beispiel 28 angegebenen Methode hergestellt; man erhält einen gelben Feststoff, Tg. = 59,1 °C.

| | | | | |
|---|---|---|---|---|
| [C36H41N3O5] ( 595.74 ) | berechnet<br>gefunden | C 72.58<br>C 72.25 | H 6.94<br>H 6.95 | N 7.05 %<br>N 6.63 % |

| | | |
|---|---|---|
| Mn = 2405; | Mw = 5533 | (MALDI-MS) |

Beispiel 39: Verbindung (614) wird nach der in Beispiel 35 angegebenen Methode hergestellt; man erhält ein orangefarbenes Harz, Tg. = 35,7 °C.

| [C36H41N3O5]1[C7H12O2]2 | | | |
|---|---|---|---|
| berechnet<br>gefunden | C 70.48<br>C 70.61 | H 7.69<br>H 7.76 | N 4.93 %<br>N 5.35 % |

| | | |
|---|---|---|
| Mn = 3612; | Mw = 5264 | (MALDI-MS) |

Beispiel 40: Verbindung (615) wird nach der in Beispiel 35 angegebenen Methode hergestellt; Tg. 66,5°C.

| | | |
|---|---|---|
| MALDI-MS: | Mn = 2111 g/Mol; | Mw = 3174 g/Mol |

Beispiel 41: Verbindung (616) wird nach der in Beispiel 35 angegebenen Methode hergestellt; Tg. 59,8°C.

| [C34H31N3O2]1[C7H12O2]2 | | |
|---|---|---|
| MALDI-MS: | Mn = 2223 g/Mol; | Mw = 3634 g/Mol |

Anwendungsbeispiele

Beispiel 50: Auf ein mit Polyethylen beschichtetes Trägermaterial wird eine Gelatineschicht aufgetragen, die Silberbromid und den Gelbkuppler (Y-9) enthält. Eine weitere Gelatineschicht enthält den erfindungsgemäßen UV-Absorber (UVA).

Die Zusammensetzung der Gelatineschichten ergibt sich aus folgender Tabelle (Komponenten je $m^2$ Trägermaterial):

| Komponente | AgBr-Schicht | UVA-Filterschicht |
|---|---|---|
| Gelatine | 5.15 g | 1.2 g |
| Härtungsmittel | 300 mg | 40 mg |
| Netzmittel | 85 mg | 200 mg |
| Silberbromid | 260 mg | - |
| Gelbkuppler Y-9 | 854 mg | - |
| Trikresylphosphat | 285 mg | - |
| UV-Absorber (605) | - | 350 mg |

Als Härter wird das Kaliumsalz von 2,4-Dichlor-6-hydroxytriazin verwendet, als Netzmittel Triton B (Benzyltrimethylammoniumhydroxid).

Als Vergleich dient ein gleichartiger Schichtaufbau ohne erfindungsgemäßen UV-Absorber.

Auf die so erhaltenen Proben wird jeweils ein Stufenkeil mit einem Dichteunterschied von 0,30 logE pro Stufe aufbelichtet und anschließend gemäß den Vorschriften des Herstellers im Verarbeitungsprozeß RA-4 der Firma Kodak für Negativ-Farbpapiere verarbeitet.

Nach Belichtung und Verarbeitung wird die Remissionsüichte im Blaubereich für die Gelbstufe mit einer Dichte zwischen 0,9 und 1,1 des Keils geniessen. Dann wird der Keil in einem Atlas-Belichtungsgerät mit total 60 kJ/cm$^2$ belichtet und erneut die Remissionsdichte gemessen. Der Gelbfarbstoffverlust ($-\Delta D_B$) in Prozent ist in der folgenden Tabelle angegeben.

Tabelle

| Gelbfarbstoffverlust ($-\Delta D_B$, nach Belichtung | | |
|---|---|---|
| Gelbkuppler | UVA (Verbindung Nr.) | $-\Delta D_B$ |
| Y-9 | - | 80 % |
| Y-9 | (605) | 40 % |

Der Einsatz eines erfindungsgemäßen UVA führt zu einem effektiven Schutz des Gelbfarbstoffes.

Beispiel 51: Auf einen Polyesterträger wird eine Gelatineschicht der folgenden Zusammensetzung (je m$^2$) in der üblichen Art und Weise aufgebracht.

| Komponente | Menge |
|---|---|
| Gelatine | 1200 mg |
| Trikresylphosphat | 510 mg |
| Härtungsmittel | 40 mg |
| Netzmittel | 100 mg |
| Verb. d. Formel (I) | 400 mg |

Das Härtungsmittel ist: Kaliumsalz von 2-Hydroxy-4,6-dichlor-1,3,5-triazin. Das Netzmittel ist Natrium-4,8-diisobutyl-naphthalin-2-sulfonat.

Die Gelatineschichten werden bei 20°C während 7 Tagen getrocknet.

Bei Verwendung der Verbindungen (202), (300), (600) oder (601) erhält man klare transparente Schichten, welche für ein fotografisches Aufzeichnungsmaterial geeignet sind.

Beispiel 52: Auf ein mit Polyethylen beschichtetes Trägermaterial wird eine Gelatineschicht aufgetragen, die Silberbromid, Magentakuppler (M-6) und als Stabilisator eine Verbindung der Formel (I) enthält.

Die Gelatineschicht enthält folgende Komponenten (je m$^2$ Trägermaterial):

| Komponente | AgBr-Schicht |
|---|---|
| Gelatine | 5.15 g |
| Härtungsmittel | 300 mg |
| Netzmittel | 85 mg |
| Silberbromid | 260 mg |
| Magentakuppler | 325 mg |
| Trikresylphosphat | 162 mg |
| Stabilisator | 114 mg |

Als Härter wird das Kaliumsalz von 2,4-Dichlor-6-hydroxytriazin verwendet, als Netzmittel das Natriumsalz der Diisobutylnaphthalinsulfonsäure.

Auf die so erhaltenen Proben wird jeweils ein Stufenkeil mit einem Dichteunterschied von 0,15 logE pro Stufe aufbelichtet und anschließend gemäß den Vorschriften des Herstellers im Verarbeitungsprozeß RA-4 der Firma Kodak für

Negativ-Farbpapiere verarbeitet.

Nach Belichtung und Verarbeitung wird die Remissionsdichte im Grünbereich für die Magentastufe mit einer Dichte zwischen 0,9 und 1,1 des Keils gemessen. Dann wird der Keil hinter einem UV-Absorber-Filter in einem Atlas-Belichtungsgerät mit 15 kJ/cm$^2$ belichtet und erneut die Remissionsdichte gemessen. Der Magentafarbstoffdichteverlust (-ΔD) wird durch die Verbindungen (202), (300), (600) oder (601) als Stabilisator stark verringert im Vergleich zu einer Probe, die keinen Stabilisator enthält.

**Patentansprüche**

1. Fotografisches Aufzeichnungsmaterial enthaltend auf einem Träger mindestens eine Silberhalogenidemulsionsschicht sowie gegebenenfalls mindestens eine Zwischenschicht und/oder eine Protektionsschicht, wobei mindestens eine der genannten Schichten einen UV-Absorber enthält, dadurch gekennzeichnet, daß der genannte UV-Absorber ein durch Additionspolymerisation eines Monomeren der Formel I erhaltenes Homopolymer, ein Copolymer mindestens zweier verschiedener Verbindungen der Formel 1, oder ein Copolymer mindestens einer Verbindung der Formel 1 und mindestens einer weiteren ethylenisch ungesättigten Verbindung ist

(I) 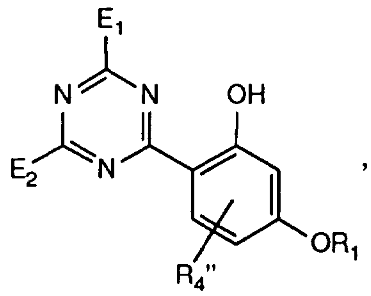 ,

worin die Reste

$E_1$ und $E_2$, unabhängig voneinander, jeweils eine Gruppe der Formel Ia oder Ib darstellen

(Ia) 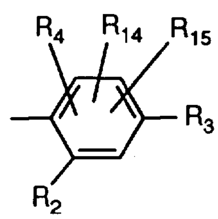

(Ib) 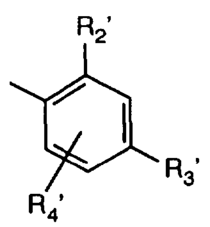 ;

$R_1$, unabhängig voneinander, -A, $-CH_2-CH(XA)-CH_2-O-R_7$, $-CR_8R'_8-(CH_2)_l-XA$, $-CH_2-CH(OA)-R_9$, $-CH_2-CH(OH)-CH_2-XA$,

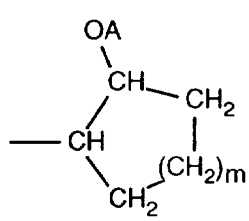 ,

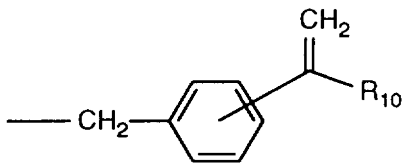 ,

$-CH_2-C(=CH_2)-R_{10}$, $-(CH_2)_p-SiR_{11}R_{11}'-CH=CH_2$, $-C(=O)-(CH_2)_q-CH=CH_2$, $-CHR_8-(CH_2)_r-C(=O)-O-CH_2-CH(OH)-CH_2-OA$, $-CR_8R'_8-(CH_2)_l-C(=O)-XA$ oder $-C(=O)-O-CH_2-C(=CH_2)-R_{10}$; wobei A $-C(=O)-CR_5=CH-R_6$ ist;

$R_2$, unabhängig voneinander, H, $C_1$-$C_{12}$-Alkyl, $C_5$-$C_{12}$-Cycloalkyl, $C_3$-$C_6$-Alkenyl, Halogen, Phenyl oder Trifluormethyl;

$R_2'$, unabhängig voneinander, $C_1$-$C_{18}$-Alkoxy, $C_3$-$C_{18}$-Alkenoxy, $-O-CO-R_{12}$, -OH oder -OA;

$R_3$ und $R_3'$, unabhängig voneinander, H, -OH, $-OR_1$, $-OR_{131}$, $C_1$-$C_{18}$-Alkyl, $C_3$-$C_{18}$-Alkenyl, $C_6$-$C_{12}$-Cycloalkyl, Halogen, Trifluormethyl, Phenyl, Phenyl-$C_1$-$C_4$-Alkyl, -CN, $C_1$-$C_{18}$-Alkyl-S(=O)$_t$- oder Phenyl-S(=O)$_t$-;

$R_4$, $R_4'$ und $R_4''$, unabhängig voneinander, H, $C_1$-$C_{18}$-Alkyl $C_3$-$C_6$-Alkenyl, $-OR_{131}$, Halogen, Trifluormethyl, Phenyl, Phenyl-$C_1$-$C_4$-alkyl, mit ein bis drei Resten $C_1$-$C_4$-Alkyl substituiertes Phenyl-$C_1$-$C_4$-alkyl, -CN, $C_1$-$C_{18}$-Alkyl-S(=O)$_t$- oder Phenyl-S(=O)$_t$-;

$R_5$ H, $-CH_2-COOR_{13}$, $C_1$-$C_4$-Alkyl oder -CN;

$R_6$ H, $-COOR_{13}$, $C_1$-$C_{17}$-Alkyl oder Phenyl;

$R_7$ $C_1$-$C_{18}$-Alkyl; $C_5$-$C_{12}$-Cycloalkyl; $C_3$-$C_{18}$-Alkenyl; Phenyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; durch ein oder mehrere -O- unterbrochenes $C_3$-$C_{50}$-Alkyl; 1-Adamantyl; 2-Adamantyl; Norbornyl; Norbornan-2-methyl; $-C(=O)-R_{12}$; -A;

$R_8$ und $R_8'$, unabhängig voneinander, H, $C_1$-$C_{18}$-Alkyl; Phenyl; Phenyl-$C_1$-$C_4$-alkyl; oder mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl;

$R_9$ $C_1$-$C_{18}$-Alkyl, Phenyl oder Phenyl-$C_1$-$C_4$-Alkyl;

$R_{10}$ H oder $-CH_3$;

$R_1$ und $R_{11}'$, unabhängig voneinander, $C_1$-$C_4$-Alkyl oder Phenyl oder mit ein bis drei Resten $C_1$-$C_8$-Alky-A $C_1$-$C_8$-Alkoxyl $C_3$-$C_8$-Alkenoxy, Halogen oder Trifluormethyl substituiertes Phenyl;

$R_{12}$ H, $C_1$-$C_{18}$-Alkyl, Phenyl, Phenyl-$C_1$-$C_4$-alkyl, $C_5$-$C_{12}$-Cycloalkyl, $C_1$-$C_{12}$-Alkoxy, Phenoxy, Norbornan-2-yl, 5-Norbornen-2-yl, 1-Adamantyl;

$R_{13}$ $C_1$-$C_{18}$-Alkyl, $C_3$-$C_{18}$-Alkenyl, Phenyl, $C_5$-$C_{12}$-Cycloalkyl, durch ein oder mehrere -O- unterbrochenes $C_3$-$C_{50}$-Alkyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; 2-Adamantyl; Norbornyl, Norbornan-2-methyl;

$R_{14}$ und $R_{15}$, unabhängig voneinander, H, $C_1$-$C_{18}$-Alkyl, $C_3$-$C_{18}$-Alkenyl, $C_6$-$C_{12}$-Cycloalkyl, Halogen, Trifluormethyl, Phenyl, Phenyl-$C_1$-$C_4$-alkyl- -CN, $C_1$-$C_{18}$-Alkyl-(S=O)$_t$-, Phenyl-(S=O)$_t$- oder $-OR_{131}$;

$R_{131}$ $C_1$-$C_{18}$-Alkyl; $C_1$-$C_{18}$-Alkyl, welches substituiert ist durch -OH, $C_1$-$C_{18}$-Alkoxy, $C_5$-$C_{12}$-Cycloalkoxy, $C_3$-$C_6$-Alkenyloxy, Halogen, $-COOR_{13}$, $-CONH_2$, $-COHNR_{132}$, $-CON(R_{132})(R_{133})$, $-NHCOR_{12}$, CN, $-OCOR_{12}$, Phenoxy, und/oder durch $C_1$-$C_{18}$-Alkyl, $C_1$-$C_{18}$-Alkoxy oder Halogen substituiertes Phenoxy; $C_3$-$C_{18}$-Alkenyl; $C_6$-$C_{12}$-Cycloalkyl; durch $C_1$-$C_4$-Alkyl und/oder $-OCOR_{12}$ substituiertes $C_6$-$C_{12}$-Cycloalkyl; durch ein oder mehrere -O- unterbrochenes $C_3$-$C_{50}$-Alkyl; durch ein oder mehrere -O- unterbrochenes $C_3$-$C_{50}$-Alkyl, welches durch -OA oder $-O-CO-R_{12}$ substituiert ist; Phenyl; Phenyl-$C_1$-$C_4$-alkyl; $-COR_{12}$; $-SO_2R_{12}$;

$R_{132}$ und $R_{133}$, unabhängig voneinander, $C_1$-$C_{12}$-Alkyl, $C_3$-$C_{12}$-Alkoxyalkyl, $C_4$-$C_{16}$-Dialkylaminoalkyl, $C_5$-$C_{12}$-Cycloalkyl; oder

$R_{132}$ und $R_{133}$ gemeinsam $C_3$-$C_9$-Alkylen, $C_3$-$C_9$-Oxaalkylen oder -Azaalkylen;

X $-NR_8-$, -O-, $-NH-(C_nH_{2n})-NH-$ oder $-O-(C_kH_{2k})-NH-$;

k eine Zahl 2-4;

l eine Zahl 0-19;

m eine Zahl 2-8;

n eine Zahl 0-4;

p eine Zahl 0-10;

q eine Zahl 1-8;

r eine Zahl 0-18; und

t eine Zahl 0, 1 oder 2 bedeuten.

**2.** Fotografisches Aufzeichnungsmaterial gemäß Anspruch 1 enthaltend auf einem Träger mindestens eine Silberhalogenidemulsionsschicht sowie gegebenenfalls mindestens eine Zwischenschicht und/oder eine Protektionsschicht, wobei mindestens eine der genannten Schichten einen UV-Absorber enthält, dadurch gekennzeichnet, daß der UV-Absorber ein durch Additionspolymerisation eines Monomeren der Formel Ic erhaltenes Homopolymer, ein Copolymer mindestens zweier verschiedener Verbindungen der Formel Ic, oder ein Copolymer mindestens einer Verbindung der Formel Ic und mindestens einer weiteren ethylenisch ungesättigten Verbindung ist

(Ic) 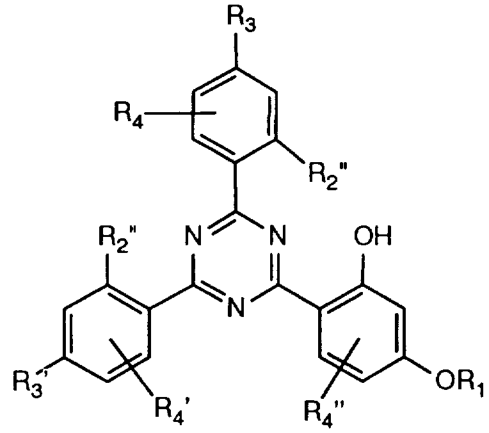 ,

entspricht, worin die Reste

A $-C(=O)-CR_5=CH-R_6$;

$R_1$, unabhängig voneinander, -A, $-CH_2-CH(OA)-CH_2-O-R_7$, $-CHR_8-(CH_2)_l-OA$, $-CH_2-CH(OA)-R_9$, $-CH_2-CH(OH)-CH_2-OA$,

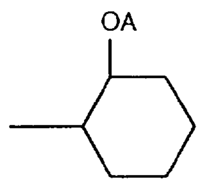 ,

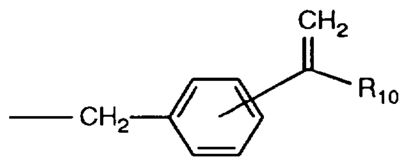 ,

$-CH_2-C(=CH_2)-R_{10}$, $-(CH_2)_p-SiR_{11}R_{11}'-CH=CH_2$, $-C(=O)-(CH_2)_q-CH=CH_2$, $-CHR_8-(CH_2)_r-C(=O)-O-CH_2-CH(OH)-CH_2-OA$ oder $-C(=O)-O-CH_2-C(=CH_2)-R_{10}$;

$R_2$", unabhängig voneinander, H, -OH, -OA, $C_1$-$C_{12}$-Alkyl, Cyclohexyl, $C_3$-$C_6$-Alkenyl, $C_1$-$C_{18}$-Alkoxyl $C_3$-$C_{18}$-Alkenoxyl Halogen, Phenyl oder Trifluormethyl;

$R_3$ und $R_3$', unabhängig voneinander, H, -OH, $-OR_1$, $C_1$-$C_{12}$-Alkyl, Cyclohexyl, $C_3$-$C_6$-Alkenyl, $C_1$-$C_{18}$-Alkoxyl, $C_3$-$C_{18}$-Alkenoxy, Halogen, Trifluormethyl, Phenyl, Phenyloxy, Phenyl-$C_1$-$C_4$-alkyl, Phenyl-$C_1$-$C_4$-alkoxy, -CN, $C_1$-$C_{18}$-Alkyl-$S(=O)_t$- oder Phenyl-$S(=O)_t$-;

$R_4$, $R_4$' und $R_4$", unabhängig voneinander, H, $C_1$-$C_{12}$Alkyl $C_3$-$C_6$-Alkenyl, $C_1$-$C_{18}$-Alkoxy, $C_3$-$C_{18}$-Alkenoxy, Halogen, Trifluormethyl, Phenyl, Phenyloxy, Phenyl-$C_1$-$C_4$-alkyl, mit ein bis drei Resten $C_1$-$C_4$-Alkyl substituiertes Phenyl-$C_1$-$C_4$-alkyl, Phenyl-$C_1$-$C_4$-alkoxy, -CN, $C_1$-$C_{18}$-Alkyl-$S(=O)_t$- oder Phenyl-$S(=O)_t$-;

$R_5$ H, $-CH_2-COOR_{13}$, $C_1$-$C_4$-Alkyl oder -CN;

$R_6$ H, $-COOR_{13}$, $C_1$-$C_{17}$-Alkyl oder Phenyl;

$R_7$ $C_1$-$C_{18}$-Alkyl; Cyclohexyl; $C_3$-$C_{18}$-Alkenyl; Phenyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; oder $-C(=O)-R_{12}$;

$R_8$ H oder $C_1$-$C_{18}$-Akyl;

$R_9$ $C_1$-$C_{18}$-Alkyl, Phenyl oder Phenyl-$C_1$-$C_4$-alkyl;

$R_{10}$ H oder $-CH_3$;

$R_1$ und $R_{11}$', unabhängig voneinander, $C_1$-$C_4$-Alkyl oder Phenyl oder mit ein bis drei Resten $C_1$-$C_8$-Aykyl, $C_1$-$C_8$-Alkoxy, $C_3$-$C_8$-Alkenoxy, Halogen oder Trifluormethyl substituiertes Phenyl;

$R_{12}$ $C_1$-$C_{18}$-Alkyl, $C_2$-$C_{18}$-Alkenyl oder Phenyl;

$R_{13}$ $C_1$-$C_{18}$-Alkyl, $C_3$-$C_{18}$-Alkenyl oder Phenyl;

l eine Zahl 0-19;

p eine Zahl 0-10;

q eine Zahl 1-8;

r eine Zahl 0-18; und
t eine Zahl 0, 1 oder 2 bedeuten.

**3.** Fotografisches Aufzeichnungsmaterial gemäß Anspruch 1, worin in Formel (I)

$R_1$, unabhängig voneinander, -A, $-CH_2-CH(XA)-CH_2-O-R_7$, $-CR_8R_8'-(CH_2)_l-XA$, $-CH_2-CH(OA)-R_9$, $-CH_2-CH(OH)-CH_2-XA$,

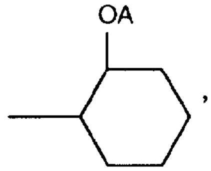

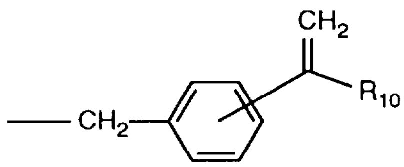

oder $-CHR_8-(CH_2)_r-C(=O)-O-CH_2-CH(OH)-CH_2-OA$;
$R_2$ H, $C_1$-$C_4$-Alkyl, $C_1$-$C_4$-Alkoxy, $C_3$-Alkenyl, F, Cl oder Phenyl;
$R_2'$ $C_1$-$C_4$-Alkoxy $C_3$-Alkenoxy, $-O-COR_{12}$, -OA oder -OH;
$R_3$ und $R_3'$, unabhängig voneinander, H, -OH, $-OR_1$, $-OR_{131}$, $C_1$-$C_4$-Alkyl, Cyclohexyl, $C_3$-Alkenyl, F, Cl, Trifluormethyl, Phenyl, Benzyl oder -CN;
$R_4'$ und $R_4''$, unabhängig voneinander, H, $C_1$-$C_4$-Alkyl, $C_3$-Alkenyl, $C_1$-$C_4$-Alkoxy, $C_3$-Alkenoxy, F, Cl, Trifluormethyl, Phenyl, Phenyl-$C_1$-$C_3$-alkyl oder -CN;
$R_5$ H oder $-CH_3$;
$R_6$ H, $-COOR_{13}$, $-CH_3$ oder Phenyl;
$R_7$ $C_1$-$C_8$-Alkyl, Cyclohexyl, $C_2$-$C_8$-Alkenyl, Phenyl, mit ein bis drei Resten $C_1$-$C_4$-Alkyl oder $C_1$-$C_4$-Alkoxy substituiertes Phenyl, oder Benzyl;
$R_8$ und $R_8'$, unabhängig voneinander, H oder $C_1$-$C_{18}$-Alkyl;
$R_9$ $C_1$-$C_{10}$-Alkyl, Phenyl oder Benzyl;
$R_{12}$ H, $C_1$-$C_{18}$-Alkyl, Phenyl, Phenyl-$C_1$-$C_4$-alkyl oder Cyclohexyl;
$R_{13}$ $C_1$-$C_4$-Alkyl, $C_3$-Alkenyl, Cyclohexyl, Phenyl-$C_1$-$C_4$-alkyl oder Phenyl;
$R_4$, $R_{14}$ und $R_{15}$, unabhängig voneinander, H, F, Cl, $C_1$-$C_4$-Alkoxy, $CF_3$, Phenyl, CN oder $C_1$-$C_8$-Alkyl;
$R_{131}$ $C_1$-$C_{18}$-Alkyl; $C_3$-$C_{18}$-Alkyl, welches substituiert ist durch -OH, $C_1$-$C_{18}$-Alkxyl, $C_5$-$C_{12}$-Cycloalkoxy, $-COOR_{13}$, $-CONH_2$, $-COHNR_{132}$, $-CON(R_{132})(R_{133})$, $-NHCOR_{12}$, CN, $-OCOR_{12}$ und/oder Phenoxy; $C_3$-Alkenyl; $C_6$-$C_{12}$-Cycloalkyl; durch ein oder mehrere -O- unterbrochenes $C_3$-$C_{50}$-Alkyl, das unsubstituiert oder durch OH oder $-O-COR_{12}$ substituiert ist; Phenyl; Phenyl-$C_1$-$C_4$-alkyl; $-COR_{12}$; $-SO_2R_{12}$;
X -O- oder $-NR_8-$;
l eine Zahl 1-19; und
r eine Zahl 0-10 bedeuten.

**4.** Fotografisches Aufzeichnungsmaterial gemäß Anspruch 1, wobei der UV-Absorber von einer Verbindung der Formel I abgeleitet ist, worin die Reste

$E_1$ und $E_2$, unabhängig voneinander, jeweils eine Gruppe der Formel Ib oder Ie darstellen

(Ib) 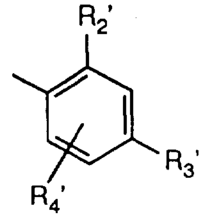 ;

(Ie) 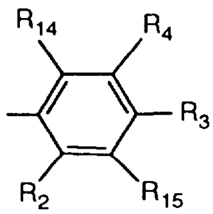

und worin $R_4$' und $R_4$" jeweils in meta-Stellung zum Triazinring stehen.

**5.** Fotografisches Aufzeichnungsmaterial gemäß Anspruch 1, worin die Reste

A $-C(=O)-CR_5=CH-R_6$;
$R_1$, unabhängig voneinander, -A, $-CH_2-CH(OA)-CH_2-O-R_7$, $-CH_2-CH(OA)-R_9$,

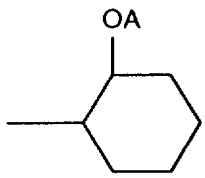 , 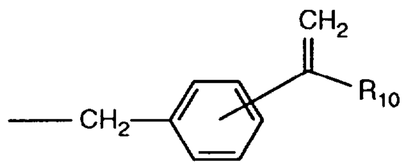

oder $-CHR_8-(CH_2)_l-OA$;
$R_2$ H, $CH_3$, $C_1$-$C_2$-Alkoxy, $C_3$-Alkenoxy oder Cl;
$R_2$' -OH;
$R_3$ H, $-CH_3$, $C_1$-$C_4$-Alkoxy, $C_3$-Alkenoxy, F, Cl, Phenyl, Benzyloxy oder -CN;
$R_3$' $-OR_1$ oder $-OR_{131}$;
$R_4$, $R_{14}$ und $R_{15}$, unabhängig voneinander, H, $OCH_3$, F, Cl, Phenyl, CN oder $CH_3$;
$R_4$' und $R_4$", unabhängig voneinander, H, $-CH_3$, $C_3$-Alkenyl, $-OCH_3$, $C_3$-Alkenoxy, F, Cl, Phenyl-$C_1$-$C_3$-alkyl oder -CN;
$R_5$ H oder $-CH_3$;
$R_6$ H oder $-CH_3$;
$R_7$ $C_1$-$C_8$-Alkyl, Cyclopentyl, Cyclohexyl, $C_3$-Alkenyl, Phenyl oder Benzyl;
$R_8$ H oder $C_1$-$C_{18}$-Alkyl;
$R_9$ $C_1$-$C_{10}$-Alkyl or phenyl;
$R_{12}$ $C_1$-$C_{18}$-Alkyl, Phenyl oder Cyclohexyl;
$R_{131}$ $C_1$-$C_{18}$-Alkyl oder $C_3$-$C_{18}$-Alkyl, welches substituiert ist durch $C_1$-$C_{18}$-Alkoxy, OH, Phenoxy, $-NHCOR_{12}$ und/oder $-OCR_{12}$; und
l eine Zahl 1-19 bedeuten.

**6.** Fotografisches Aufzeichnungsmaterial gemäß Anspruch 1, worin in Formel (I)

A $-C(=O)-CR_5=CH-R_6$;
$R_1$, unabhängig voneinander, -A, $-CH_2-CH(OA)-CH_2-O-R_7$, $-CH_2-CH(OA)-R_9$,

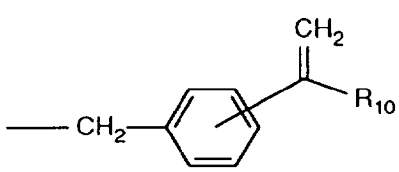

oder $-CH_2-(CH_2)_l-OA$;
$R_2$ H oder $CH_3$;
$R_2$' -OH;
$R_3$ H, $-CH_3$, $OCH_3$, Cl oder Phenyl;
$R_3$' $-OR_1$ oder $-OR_{131}$;
$R_4$ H, Cl, $OCH_3$, F oder $CH_3$;
$R_4$' und $R_4$" H oder $CH_3$;
$R_{14}$ und $R_{15}$ Wasserstoff, $CH_3$ oder $OCH_3$;
$R_5$ H oder $-CH_3$;
$R_6$ H;

$R_7$ $C_1$-$C_8$-Alkyl;
$R_9$ $C_1$-$C_{10}$-Alkyl;
$R_{12}$ $C_1$-$C_8$-Alkyl;
$R_{131}$ $C_1$-$C_{18}$-Alkyl oder $C_3$-$C_{18}$-Alkyl, welches substituiert ist durch $-OCOR_{12}$; und
l eine Zahl 1-10 bedeuten.

**7.** Fotografisches Aufzeichnungsmaterial gemäß Anspruch 1, dadurch gekennzeichnet, daß der UV-Absorber ein durch Additionspolymerisation eines Monomeren der Formel I erhaltenes Homopolymer, ein Copolymer mindestens zweier verschiedener Verbindungen der Formel I, oder ein Copolymer mindestens einer Verbindung der Formel I und mindestens einer weiteren ethylenisch ungesättigten Verbindung ist, wobei das Comonomer ausgewählt ist aus Acrylsäure, Methacrylsäure, Acrylaten, Methacrylaten, Acrylamiden, Methacrylamiden, Vinylethern, Styrol, Styrolderivaten, Vinylpyridinen, Acrylnitril, Methacrylnitril, Vinylpyrrolidon, Derivaten von Vinylpyrrolidon, sowie ethylenisch ungesättigten Derivaten von sterisch gehinderten Aminen, 2-(2'-Hydroxyphenyl)benztriazolen, 2-Hydroxy-benzophenonen, Zimtsäurederivaten oder sterisch gehinderten Phenolen.

**8.** Fotografisches Aufzeichnungsmaterial gemäß Anspruch 7, wobei das weitere Comonomer einer der Formeln (II)-(VII) entspricht

(II)

$R_{18}$-CH=C($R_{17}$)-C(=O)-X'-$R_{20}$,
worin X' -O- oder $-NR_{19}$- ist;
$R_{17}$ H, $C_1$-$C_4$-Alkyl, $-CH_2-COOR_{21}$, -Cl oder -CN;
$R_{18}$ H, $-COOR_{21}$ oder $-CH_3$;
$R_{19}$ H, $C_1$-$C_8$-Alkyl, $C_4$-$C_{12}$-Cycloalkyl, durch $-N(R_X)_2$ substituiertes $C_1$-$C_4$-Alkyl, $-S(=O)-R_X$, $-C(CH_3)_2-CH_2-C(=O)-CH_3$, $-C(CH_3)_2-CH_2-SO_3M$, $-(CH_2)_s-SO_3M$ oder

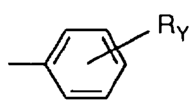
;

$R_{20}$ H; $C_1$-$C_{18}$-Alkyl; $C_3$-$C_{18}$-Alkenyl; durch ein oder mehrere O-Atome unterbrochenes $C_2$-$C_{30}$-Alkyl, das durch OH substituiert sein kann; oder $-(CH_2)_s-SO_3M$;

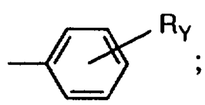
;

$-CH_2F$; $-CH_2Cl$; $-CH_2CN$; $-CH_2CH_2Cl$; $-CH_2CH_2CN$; $-CH_2CH_2-COOR_X$; $C_7$-$C_{11}$-Phenylalkyl; Naphthyl; durch $-N(R_X)_2$ substituiertes $C_1$-$C_4$-Alkyl; Adamantyl; $C_6$-$C_{12}$-Cycloalkyl;
$R_{21}$ H, $C_1$-$C_{18}$-Alkyl, Phenyl oder $C_3$-$C_{18}$-Alkenyl;
$R_X$ $C_1$-$C_4$-Alkyl oder Phenyl;
$R_Y$ H, $C_1$-$C_{12}$-Alkyl, Phenyl, $-CO-OR_X$, -CN, -F, -Cl;
M H oder ein Alkalimetall; und
s eine Zahl zwischen 1 und 5 bedeuten;

(III)

$R_{22}-C(=O)-O-CH=CH_2$ ,
worin $R_{22}$ $C_1$-$C_{19}$-Alkyl oder Phenyl

(IV)

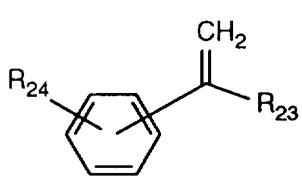
,

worin $R_{23}$ H oder $-CH_3$;
$R_{24}$ H, $-CR_{23}=CH_2$, -C(O)-Phenyl oder $-SO_3M$; und
M H oder ein Alkalimetall bedeutet;

(V)

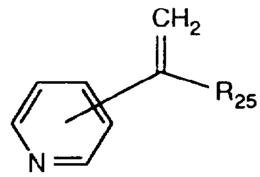,

worin $R_{25}$ H oder $-CH_3$ bedeutet;

(VI)

$CH_2=CR_{26}-R_{27}$,
worin $R_{26}$ H, -F, -Cl oder $-CH_3$ und
$R_{27}$ -Cl, -Br, -F oder -CN bedeutet;

(VII)

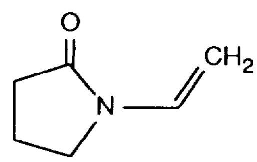,

+

und/oder ein copolymerisierbares ethylenisch ungesättigtes Derivat eines weiteren Stabilisators ist, der dadurch gekennzeichnet ist, daß er das Strukturelement

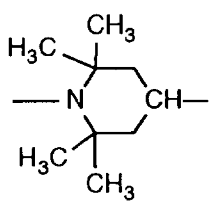,

das Strukturelement

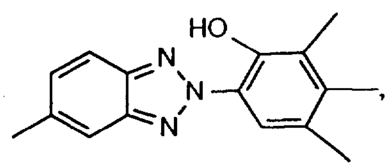,

das Strukturelement

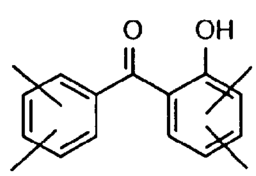,

das Strukturelement

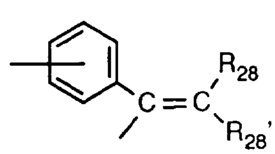,

wobei $R_{28}$ und $R_{28}'$ unabhängig voneinander CN oder $COOR_{13}$ sind und $R_{13}$ weiter oben angegebenen Bedeutun-

gen umfaßt, oder das Strukturelement

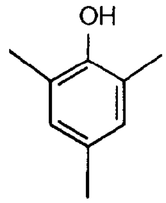

enthält.

**9.** Fotografisches Aufzeichnungsmaterial gemäß Anspruch 8, dadurch gekennzeichnet, daß der UV-Absorber ein Copolymer mindestens einer Verbindung der Formel (I) und mindestens eines weiteren Comonomeren einer der Formeln (II), (III), (IV) oder (VII) ist.

**10.** Fotografisches Aufzeichnungsmaterial gemäß Anspruch 1, enthaltend den UV-Absorber in einer Menge von 0,05 bis 10 g pro $m^2$.

**11.** Fotografisches Aufzeichnungsmaterial gemäß Anspruch 1, enthaltend den UV-Absorber in einer Schicht über der grünempfindlichen Schicht.

**12.** Fotografisches Aufzeichnungsmaterial gemäß Anspruch 1, dadurch gekennzeichnet, daß es in mindestens einer der Schichten zusätzlich zu dem copolymeren UV-Absorber ein hydrophobes Homo- oder Copolymer aus Monomeren der Formel II, III, IV, V, VI und/oder VII gemäß Anspruch 8 enthält.

**13.** Fotografisches Aufzeichnungsmaterial gemäß Anspruch 12 enthaltend in mindestens einer der Schichten den copolymeren UV-Absorber und das hydrophobe Polymer, welches dadurch erhältlich ist, daß der UV-Absorber und das hydrophobe Polymer in einem organischen Lösungsmittel gelöst und hierauf in wässrigem Milieu emulgiert und als Dispersion ins fotografische System eingebracht werden.

**14.** Verwendung einer Verbindung der Formel I gemäß Anspruch 18 oder eines Homopolymers einer Verbindung der Formel (I), eines Copolymers mindestens zweier verschiedener Verbindungen der Formel (I) oder eines Copolymers mindestens einer Verbindung der Formel (I) und mindestens einer weiteren ethylenisch ungesättigten Verbindung gemäß Anspruch 1 als Stabilisator gegen schädigende Effekte durch UV-Strahlung in fotografischem Aufzeichnungsmaterial enthaltend auf einem Träger mindestens eine Silberhalogenidemulsionsschicht sowie gegebenenfalls mindestens eine Zwischenschicht und/oder eine Protektionsschicht.

**15.** Verfahren zum Schützen von fotografischem Aufzeichnungsmaterial, enthaltend auf einem Träger mindestens eine Silberhalogenidemulsionsschicht sowie gegebenenfalls mindestens eine Zwischenschicht und/oder eine Protektionsschicht, vor UV-Strahlung, dadurch gekennzeichnet, daß man in mindestens eine der genannten Schichten einen UV-Absorber der Formel I gemäß Anspruch 18 oder ein Homopolymer einer Verbindung der Formel (I), ein Copolymer mindestens zweier verschiedener Verbindungen der Formel (I) oder ein Copolymer mindestens einer Verbindung der Formel (I) und mindestens einer weiteren ethylenisch ungesättigten Verbindung gemäß Anspruch 1 einarbeitet.

**16.** Verfahren gemäß Anspruch 15, dadurch gekennzeichnet, daß man ein Homopolymer einer Verbindung der Formel (I), ein Copolymer mindestens zweier verschiedener Verbindungen der Formel (I) oder ein Copolymer mindestens einer Verbindung der Formel (I) und mindestens einer weiteren ethylenisch ungesättigten Verbindung einarbeitet.

**17.** Fotografisches Aufzeichnungsmaterial gemäß Anspruch 1 enthaltend zusätzlich einen Stabilisator aus der Klasse der sterisch gehinderten Amine.

**18.** Fotografisches Aufzeichnungsmaterial enthaltend auf einem Träger mindestens eine Silberhalogenidemulsionsschicht sowie gegebenenfalls mindestens eine Zwischenschicht und/oder eine Protektionsschicht, wobei mindestens eine der genannten Schichten einen UV-Absorber enthält, dadurch gekennzeichnet, daß der genannte UV-Absorber eine Verbindung der Formel I ist

(I) 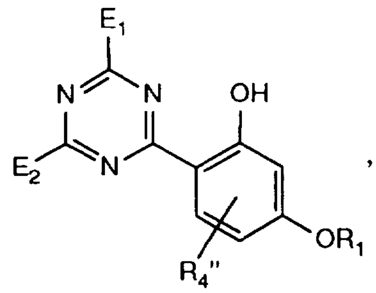 ,

worin die Reste

$E_1$ und $E_2$, unabhängig voneinander, jeweils eine Gruppe der Formel Ia oder Ib darstellen

(Ia) 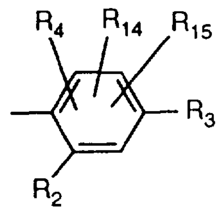

(Ib) 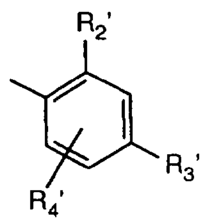 ;

$R_1$, unabhängig voneinander, -A,

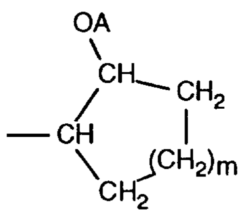 , 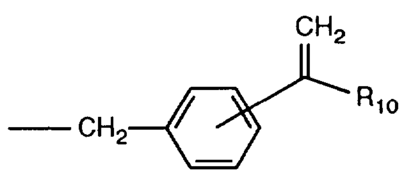 ,

$-CH_2-C(=CH_2)-R_{10}$, $-(CH_2)_p-SiR_{11}R_{11}'-CH=CH_2$, $-C(=O)-(CH_2)_q-CH=CH_2$, $-CHR_8-(CH_2)_r-C(=O)-O-CH_2-CH(OH)-CH_2-OA$, $-CR_8R'_8-(CH_2)_l-C(=O)-XA$ oder $-C(=O)-O-CH_2-C(=CH_2)-R_{10}$, und, im Fall daß $E_1$ oder $E_1$ und $E_2$ eine Gruppe der Formel Ib sind, $R_1$ zusätzlich die Bedeutungen $-CH_2-CH(XA)-CH_2-O-R_7$, $-CR_8R'_8-(CH_2)_l-XA$, $-CH_2-CH(OA)-R_9$, $-CH_2-CH(OH)-CH_2-XA$ umfaßt; wobei

A $-C(=O)-CR_5=CH-R_6$ ist;

$R_2$, unabhängig voneinander, H, $C_1$-$C_{12}$-Alkyl, $C_5$-$C_{12}$-Cycloalkyl, $C_3$-$C_6$-Alkenyl, Halogen, Phenyl oder Trifluormethyl;

$R_2'$, unabhängig voneinander, $C_1$-$C_{18}$-Alkoxy, $C_3$-$C_{18}$-Alkenoxy, $-O-CO-R_{12}$, -OH oder -OA;

$R_3$ und $R_3'$, unabhängig voneinander, H, -OH, $-OR_1$, $-OR_{131}$, $C_1$-$C_{18}$-Alkyl, $C_3$-$C_{18}$-Alkenyl, $C_6$-$C_{12}$-Cycloalkyl, Halogen, Trifluormethyl, Phenyl, Phenyl-$C_1$-$C_4$-Alkyl, -CN, $C_1$-$C_{18}$-Alkyl-$S()O)_t$- oder Phenyl-$S(=O)_t$-;

$R_4$, $R_4'$ und $R_4''$, unabhängig voneinander, H, $C_1$-$C_{18}$-Aklyl, $C_3$-$C_6$-Alkenyl, $-OR_{131}$, Halogen, Trifluormethyl, Phenyl, Phenyl-$C_1$-$C_4$-alkyl, mit ein bis drei Resten $C_1$-$C_4$-Alkyl substituiertes Phenyl-$C_1$-$C_4$-alkyl, -CN, $C_1$-$C_{18}$-Alkyl-$S(=O)_t$- oder Phenyl-$S(=O)_t$-;

$R_5$ H, $-CH_2-COOR_{13}$, $C_1$-$C_4$-Alkyl oder -CN;

$R_6$ H, $-COOR_{13}$, $C_1$-$C_{17}$-Alkyl oder Phenyl;

$R_7$ $C_1$-$C_{18}$-Alkyl; $C_5$-$C_{12}$-Cycloalkyl; $C_3$-$C_{18}$-Alkenyl; Phenyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; durch ein oder mehrere -O- unterbrochenes $C_3$-$C_{50}$-Alkyl; 1-Adamantyl; 2-Adamantyl; Norbornyl; Norbornan-2-methyl; $-C(=O)-R_{12}$; -A;

$R_8$ und $R_8'$, unabhängig voneinander, H, $C_1$-$C_{18}$-Alkyl; Phenyl; Phenyl-$C_1$-$C_4$-alkyl; oder mit ein bis drei Resten

$C_1$-$C_8$-Alkyl, $C_1$-$C_8$-Alkoxy, $C_3$-$C_8$-Alkenoxy, Halogen oder Trifluormethyl substituiertes Phenyl;
$R_9$ $C_1$-$C_{18}$-Alkyl, Phenyl oder Phenyl-$C_1$-$C_4$-alkyl;
$R_{10}$ H oder $-CH_3$;
$R_{11}$ und $R_{11}$', unabhängig voneinander, $C_1$-$C_4$-Alkyl oder Phenyl oder mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl;
$R_{12}$ H, $C_1$-$C_{18}$-Alkyl, Phenyl, Phenyl-$C_1$-$C_4$-alkyl, $C_5$-$C_{12}$-Cycloalkyl, $C_1$-$C_{12}$-Alkoxy, Phenoxy, Norboman-2-yl, 5-Norbornen-2-yl, 1-Adamantyl;
$R_{13}$ $C_1$-$C_{18}$-Alkyl, $C_3$-$C_{18}$-Alkenyl, Phenyl, $C_5$-$C_{12}$-Cycloalkyl, durch ein oder mehrere -O- unterbrochenes $C_3$-$C_{50}$-Alkyl; mit ein bis drei Resten $C_1$-$C_8$-Alkyl, $C_1$-$C_8$-Alkoxy, $C_3$-$C_8$-Alkenoxy, Halogen oder Trifluormethyl substituiertes Phenyl; Phenyl-$C_1$-$C_4$-alkyl; 2-Adamantyl; Norbornyl, Norbornan-2-methyl;
$R_{14}$ und $R_{15}$, unabhängig voneinander, H, $C_1$-$C_{18}$-Alkyl, $C_3$-$C_{18}$-Alkenyl, $C_6$-$C_{12}$-Cycloalkyl, Halogen, Trifluormethyl, Phenyl, Phenyl-$C_1$-$C_4$-alkyl -CN, $C_1$-$C_{18}$-Alkyl-$(S{=}O)_t$-, Phenyl-$(S{=}O)_t$- oder $-OR_{131}$;
$R_{131}$ $C_1$-$C_{18}$-Alkyl; $C_1$-$C_{18}$-Alkyl, welches substituiert ist durch -OH, $C_1$-$C_{18}$-Alkoxy, $C_5$-$C_{12}$-Cycloalkoxy, $C_3$-$C_6$-Alkenyloxy, Halogen, $-COOR_{13}$, $-CONH_2$, $-COHNR_{132}$, $-CON(R_{132})(R_{133})$, $-NHCOR_{12}$, CN, $-OCOR_{12}$, Phenoxy, und/oder durch $C_1$-$C_{18}$-Alkyl, $C_1$-$C_{18}$-Alkoxy oder Halogen substituiertes Phenoxy; $C_3$-$C_{18}$-Alkenyl; $C_6$-$C_{12}$-Cycloalkyl; durch $C_1$-$C_4$-Alkyl und/oder $-OCOR_{12}$ substituiertes $C_6$-$C_{12}$-Cycloalkyl; durch ein oder mehrere -O- unterbrochenes $C_3$-$C_{50}$-Alkyl; durch ein oder mehrere -O- unterbrochenes $C_3$-$C_{50}$-Alkyl, welches durch -OA oder $-O-CO-R_{12}$ substituiert ist; Phenyl;
Phenyl-$C_1$-$C_4$-alkyl; $-COR_{12}$; $-SO_2R_{12}$;
$R_{132}$ und $R_{133}$, unabhängig voneinander, $C_1$-$C_{12}$-Alkyl, $C_3$-$C_{12}$-Alkoxyalkyl, $C_4$-$C_{16}$-Dialkylaminoalkyl, $C_5$-$C_{12}$-Cycloalkyl; oder
$R_{132}$ und $R_{133}$ gemeinsam $C_3$-$C_9$-Alkylen, $C_3$-$C_9$-Oxaalkylen oder -Azaalkylen;
X $-NR_8-$, -O-, $-NH-(C_nH_{2n})-NH-$ oder $-O-(C_kH_{2k})-NH-$;
k eine Zahl 2-4;
l eine Zahl 0-19;
m eine Zahl 2-8;
n eine Zahl 0-4;
p eine Zahl 0-10;
q eine Zahl 1-8;
r eine Zahl 0-18; und
t eine Zahl 0, 1 oder 2 bedeuten.

Europäisches Patentamt

# EUROPÄISCHER RECHERCHENBERICHT

Nummer der Anmeldung
EP 95 81 0602

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X<br>Y | EP-A-0 530 135 (CIBA-GEIGY)<br>* Seite 10; Beispiel 26 *<br>* Seite 41; Tabelle Y *<br>* Seite 43, Zeile 1 - Zeile 22; Ansprüche 1,6 *<br>--- | 18<br>1-17 | G03C1/815 |
| Y | DE-A-43 40 725 (CIBA-GEIGY)<br>* Seite 35, Zeile 27 - Zeile 44 *<br>* Seite 40, Zeile 36 - Zeile 63; Ansprüche 1,5 *<br>--- | 1-17 | |
| Y | CH-A-481 954 (CIBA)<br>* Spalte 12; Beispiel 11 *<br>* Ansprüche *<br>----- | 1-17 | |

RECHERCHIERTE SACHGEBIETE (Int.Cl.6)
G03C
C08K

Der vorliegende Recherchenbericht wurde für alle Patentansprüche erstellt

| Recherchenort | Abschlußdatum der Recherche | Prüfer |
|---|---|---|
| DEN HAAG | 21. Dezember 1995 | Magrizos, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)